# EUROPEAN PATENT APPLICATION

(11) **EP 1 498 125 A1**
(43) Date of publication of application: **19.01.2005**
(21) Application number: 03719177.2
(22) Date of filing: 23.04.2003
(51) Int. Cl.: A61K 31/445, A61K 31/4545, A61K 31/351, A61K 45/00, A61P 1/00, A61P 1/04, A61P 1/14, A61P 1/16, A61P 1/18, A61P 3/04, A61P 3/10, A61P 5/00, A61P 7/00, A61P 7/02, A61P 7/10, A61P 9/00, A61P 9/04, A61P 9/06

(54) **USE OF COMPOUNDS HAVING CCR ANTAGONISM**

(30) Priority: 24.04.2002 JP 2002122832
(71) Applicant: Takeda Pharmaceutical Company Limited, Osaka (JP)
(72) Inventor: TSUCHIMORI, Noboru, Amagasaki-shi, Hyogo 661-0033 (JP); IIZAWA, Yuji, Muko-shi, Kyoto 617-0002 (JP); SHIRAISHI, Mitsuru, Amagasaki-shi, Hyogo 661-0002 (JP); SUGIHARA, Yoshihiro, Ikoma-shi, Nara 630-0111 (JP)
(74) Representative: Lewin, John Harvey
(86) International application number: PCT/JP2003/005172
(87) International publication number: WO 2003/090748

(57) **Abstract**

It is intended to provide preventives/remedies for graft-versus-host disease and/or rejection in organ or bone marrow transplantation, rheumatoid arthritis, autoimmune diseases, allergic diseases, ischemic cerebral cell injury, myocardial infarction, chronic nephritis and arteriosclerosis. The above object can be achieved by preventives/remedies for graft-versus-host disease and/or rejection in organ or bone marrow transplantation, rheumatoid arthritis, autoimmune diseases, allergic diseases, ischemic cerebral cell injury, myocardial infarction, chronic nephritis and arteriosclerosis characterized by containing a specific compound having a CCR (CC chemokine receptor) antagonism.

## Description

### Technical Field

The present invention relates to prophylactic and therapeutic agents for graft-versus-host disease during organ transplantation and/or rejection reactions, and prophylactic and therapeutic agents for rheumatoid arthritis, autoimmune disease, allergy disorders, ischemic brain cell damage, myocardial infarction, chronic nephritis, and arteriosclerosis, which contain a compound having a CC chemokine receptor (hereinafter abbreviated as CCR) antagonistic effect, and also relates to a method for preventing or treating these.

### Background Art

In many cases of renal disease that accompanies glomerulosclerosis, such as IgA nephritis, diabetic nephropathy, and the like, there is a high degree of danger that this will degenerate into renal failure, and thus not only will the QOL of the patient decline, but their prognosis will be poor. Hardening of the glomeruli is caused by excessive extracellular matrix accumulation and enlargement of the mesangium region. However, prior to this point, various types of mediators, cytokines, and the like will be discharged, and tissue remodeling will progress, due to the infiltration of inflammatory cells such as macrophages and the like. When this occurs, groups of molecules referred to as chemokines will activate cells involved in inflammation such as macrophages and the like via chemokine receptors (see for example Non-Patent Document 1). Thus, the present inventors surmised that if the effects of chemokines are inhibited by administering a chemokine antagonist compound, the impact of inflammatory cells such as macrophages and the like on glomerulosclerosis can be controlled. Conventionally, ACE inhibitors and AT1 inhibitors are employed in treatments aimed at controlling renal fibrosis, but are not prescribed to patients whose disease has progressed due to the possibility that changes in hemodynamics will have an adverse impact on renal function. The development of a fibrosis inhibitor that will not have an impact on hemodynamics is desired. Recently, a relation has been reported between a gene polymorph of CCR5 (one type of chemokine receptor) and the rate at which renal disorders occur in Type 2 diabetics (see for example Non-Patent Document 2). In addition, AOP-RANTES (a CCR5 antagonist) will control fibrosis in experimental glomerulonephritis models (see for example Non-Patent Document 3). From these findings, the present inventors surmise that renal disorders can be controlled if chemokine receptors are blocked. However, there have been no reports of a low molecular weight chemokine antagonist compound that will control renal disorders such as glomerulonephritis and the like.

With ischemic cerebrovascular disease represented by cerebral infarction, it is thought that cerebral tissue damage and cerebral function damage caused by a decline in cerebral blood flow due to constriction, blockage, or a decline in perfusion pressure of cerebral blood vessels, metabolic disturbances in brain energy due to a hyperacute decline in cerebral blood flow, abnormal isolation of glutamic acid due to cell membrane depolarization, activation of various types of enzymes (protease, lipase, and others) due to an increase in intracellular calcium, an increase in various types of active oxygen, and the like, play a role therein.

Drugs that are used to treat this include thrombolytic agents (tPA) that are targeted at dissolving blood clots, and antiplatelet and anticoagulant drugs that are targeted at preventing the enlargement and reoccurrence of blood clots. However, the anticipated effects have not been observed because, among other things, some effects have not been clear, and there have been extremely few patients to whom these have been administered in a limited treatment period.

In addition, toward the goal of protecting cerebral tissue by stopping the damage cascade, drugs having various mechanisms of action such as glutamic acid antagonists, calcium antagonists, and antioxidants, have been developed as therapeutic agents for acute stage cerebral infarction. Because of that, effective cerebral protection drugs are earnestly desired. From the viewpoint of the temporal treatment stage, the possibility of cerebral protection drugs targeted at inflammatory reactions that are elicited by primary cerebral tissue damage, and thought to be related to increases in tissue damage is suggested (see for example Non-Patent Document 4).

An increase in various types of inflammatory cytokines at the point of damage due to cerebral ischemia has been confirmed (see for example Non-Patent Documents 5 and 6). These are thought to have an effect on the enlargement and progression of cerebral tissue damage, and thus antagonists of the receptors (CCR, CXCR, CR, CX3CR) that these cytokines act upon are expected to control the enlargement of ischemic cerebral damage that is represented by cerebral infarction (see for example Non-Patent Document 7). In addition, the same enlargement of cerebral tissue damage is seen in other cerebrovascular accidents (cerebral hemorrhage, subarachnoid hemorrhage, and the like), head trauma, and in Alzheimer's disease, multiple sclerosis (MS), AIDS encephalopathy, and the like that are thought to be related to the progression of intracerebral inflammation reactions, and thus these antagonists are also thought to be effective in the progression and enlargement of these central nervous system degenerative diseases. From these findings, the present inventors surmise that these cerebrovascular accidents and head trauma can be controlled if chemokine receptors are blocked. However, it has not been confirmed that compounds having a CCR antagonistic effect are effective in the treatment of these cerebrovascular accidents and head trauma.

Osteoarthritis is a chronic progressive disease that is based upon the deterioration of joint cartilage. In osteoarthritis, the cartilage matrix is destroyed by such things as ageing, excessive dynamic load, and inflammation, and the flexibility and elasticity of the cartilage will be lost to thus produce impairment to joint function. In the treatment of osteoarthritis, it is important to stop or slow cartilage damage. However at present, the drugs that are clinically used against osteoarthritis only treat the symptoms thereof, such as pain and anti-inflammatory agents (steroids, non-steroidal anti-inflammatory drugs), and joint cartilage protective drugs (hyaluronic acid preparations). Thus, drugs that will improve the structural degeneration of joint cartilage are desired as therapeutic agents.

Cartilage damage is caused by the breakdown of the proteoglycan and Type II collagen in the cartilage matrix. The cells that are associated with cartilage damage include the chondrocytes and synoviocytes that form the joints. These cells will increase production of enzymes such as aggrecanase and matrix metalloproteinase due to the stimulation of inflammatory cytokines such as interleukin-I (IL-1), and these enzymes will immediately break down the cartilage matrix. It is thought that this is the target of a cartilage damage inhibitor in this series of processes. With regard to the connection between osteoarthritis and chemokines, it has been reported that the production of various chemokines by chondrocytes and synoviocytes has been observed in osteoarthritis patients, and that this production is powerfully promoted by the stimulation of the inflammatory cytokines (see for example Non-Patent Documents 8 and 9). In fact, high levels of chemokines have been detected in the synovial fluid of osteoarthritis patients (see for example Non-Patent Document 10). More recently, it has been made clear that chemokine receptors are present in both chondrocytes and synoviocytes, specific chemokines will stimulate the production of cytokines and matrix metalloproteinase via these receptors, and that this will induce cartilage damage (see for example Non-Patent Documents 11 and 12), and this suggests that chemokines play an intimate roll in the progress of osteoarthritis. Thus, the present inventors surmised that if the bonding of chemokines to chondrocytes and synoviocytes can be prevented by administering a chemokine antagonist compound, the cartilage damage of osteoarthritis can be controlled. However, there have still been no reports of a low molecular weight chemokine antagonist compound that will control the cartilage damage of osteoarthritis.

With rheumatoid arthritis, the infiltration of inflammatory cells from the microvasculature of the synovial membrane will first occur, and then will progress to chronic inflammation and synoviocyte tylosis. Various types of chemokines will play a role in the infiltration of cells into the joint. With rheumatoid arthritis, the manifestation of CXC chemokines such as IL-8 and GRO, and CC chemokines such as RANTES, MIP-1α, MIP-1β, and MCP-1, is accelerated. These are produced by infiltrating cells and the abnormal growth of synoviocytes. In the same way, the inflammatory cytokines such as TNF-α whose production is accelerated in rheumatoid arthritis will strongly induce the production of chemokines from these cells. The chemokines will affect the cells having the respective receptors that have infiltrated in large numbers inside. the joint, and will further accelerate the infiltration of inflammatory cells. Conventionally, compounds such as methotrexate and the like are used to control the growth of these types of infiltrating cells and the abnormal growth of synoviocytes, and anti-TNF-α treatment methods with biological preparations are performed in order to control the accelerated expression of chemokines. However, the former have strong side effects, and the latter has the problem of high cost, and thus a drug having weak side effects and which can be supplied more inexpensively is desired.

Recently, it has been reported that joint inflammation can be controlled in animal models of rheumatoid arthritis by the administration of an-anti- chemokine antibody (see for example Non-Patent. Document 13) or a chemokine analog (see for example Non-Patent Document 14). In addition, it has been reported that the chronic rheumatoid arthritis incident rate of Caucasians that do not express CCR5 (CCR5Δ32) (one type of chemokine receptor) in immunocytes that are missing 32 base pairs in the CCR5 gene is significantly lower than those having the wild type CCR5 gene (see for example Non-Patent Document 15), and thus suggests that chemokines play an intimate role in chronic rheumatoid arthritis. From these findings, the present inventors surmise that chronic rheumatoid arthritis can be controlled if chemokine receptors are blocked. However, there are no reports of chemokine antagonist compounds that will control chronic rheumatoid arthritis.

Because atherosclerosis is an important risk factor for the occurrence of cardiovascular events, controlling the occurrence of atherosclerosis is thought to be important in the control of cardiovascular events. Up until now, it has been reported that therapeutic agents for hyperlipemia such as statins and the like will improve atherosclerosis, and thus the rate of occurrence of cardiovascular events will improve. However, the rate of effectiveness of these is approximately 30% (see for example Non-Patent Document 16), and thus drugs having another mechanism of action are desired.

The infiltration and activation of monocytes in the vascular wall is the initial step in atherosclerosis, and the monocytes are the primary constituent of unstable plaque that will easily give rise to further ruptures (see for example Non-Patent Documents 17 and 18). MCP-1 is one type of the CC chemokine family, and exhibits strong chemotaxis via CCR2 receptors (see for example Non-Patent Document 19). It has been reported that,the occurrence of atherosclerosis in ApoE and CCR2 double knock-out mice will be controlled in comparison with ApoE knock-out mice (see for example Non-Patent Document 20). From these findings, the present inventors surmise that atherosclerosis can be controlled if chemokine receptors such as CCR2 are blocked. However, there are no reported examples in which the prevention and treatment of atherosclerosis by means of the administration of a compound having a chemokine receptor antagonist effect has been confirmed.

Transplant damage caused by humoral antibodies and macrophages plays a role in graft reactions, but generally speaking, graft rejection are primarily caused by histocompatibility antigens expressed by the transplanted cells being recognized as non-self, and activated T cells grown by the host infiltrating, accumulating in, and attacking the transplant. The infiltration of T cells into the transplant location is induced by a plurality of chemokines that are produced at graft location binding to chemokine receptors on the T cell surface. Thus, the present inventors surmise that if the binding of the chemokines to the T cells is prevented by administering a chemokine antagonist compound, the rejection can be controlled by inhibiting the infiltration of T cells to the transplant. Conventionally, drugs such as cyclosporin and tacrolimus are widely used in clinical settings to control rejection. However, there are problems with this such as a low organ take ratio and strong side effects, and thus a drug is desired in which a higher take ratio can be expected and which has weak side effects.

Recently, the long term graft survival of transplanted organs in a transplant model that employed a mouse in which a specific chemokine receptor was knocked out has been reported (see for example Non-Patent Document 21), as well as the control of rejection in an animal model to which a chemokine antagonist (see for example Non-Patent Document 21) or a chemokine analog (see for example Non-Patent Document 22) was administered. In addition, it has been reported that the graft survival ratio of Caucasian kidney transplant patients that do not express CCR5 (CCR5Δ32) (one type of chemokine receptor) in immunocytes that are missing 32 base pairs in the CCR5 gene is significantly higher than those having the wild type CCR5 gene (see for example Non-Patent Document 23), and thus suggests that chemokines play an intimate role in rejection. However, there are no reports of chemokine antagonist compounds that will control graft rejection.

It is known that the concentration of MCP-1, MIP-1, RANTES, and others belonging to the chemokine family will increase in the blood of acute myocardial infarction patients, and that this correlates with the degree of seriousness of the disease, and thus it is thought that these chemokines play an intimate role in the occurrence and progress of acute myocardial infarction. In addition, because an increase in the expression of CCR1, CCR2 and other chemokine receptors has been identified in end-stage cardiac insufficiency, and because the expression of CCR5 is confirmed in coronary artery atherosclerosis, it is thought that there is a connection between these chemokine receptors and things such as cardiac insufficiency and coronary artery disease. From these findings, the present inventors surmise that the occurrence and progression of acute myocardial infarction can be controlled if chemokine receptors are blocked. However, there are no reports of the effects of chemokine antagonist agents in the prevention and treatment of that cardiovascular disease.

Various types of benzazepine compounds (see for example Patent Documents 1, 2, 3, 4 and 5) and various types of piperidine compounds (see for example Patent Documents 7 and 8) are known as compounds having a CCR antagonist effect and particularly a CCR5 antagonist effect. However, these compounds have been primarily disclosed as therapeutic agents for HIV infection, and have not at all been disclosed with regard to things such as the prevention and treatment of graft-versus-host disease and/or rejection during organ transplantation, and the prevention and treatment of chronic rheumatoid arthritis, autoimmune diseases, allergic disorders, ischemic brain cell damage, myocardial infarction, chronic nephritis, and arteriosclerosis.

In addition, compounds having a specific chemical structure and a chemokine receptor antagonist effect are known to be effective with respect to inflammation, immunodeficiency, asthma, allergic disorders (see for example Patent Document 9), immunodeficiency (see for example Patent Document 10), cardiac insufficiency, inflammation, allergic disorders, dermatitis, conjunctivitis, atherosclerosis, and rheumatoid arthritis (see for example Patent Documents 11 and 12), graft rejection, inflammatory bowel disease, rheumatoid arthritis, and multiple sclerosis (see for example Patent Documents 13 and 14), AIDS, inflammation, immunoregulatory insufficiency, asthma, allergic rheumatism, dermatitis, conjunctivitis, arteriosclerosis, and rheumatoid arthritis (see for example Patent Documents 15, 16, 17, and 18), AIDS, chronic rheumatoid arthritis, nephritis, post-transplant rejection , graft-versus-host disease, diabetes, chronic obstructive pulmonary disease, bronchial asthma, atopic dermatitis, sarcoidosis, fibrosis, pulmonary artery atherosclerosis, and psoriatic inflammatory bowel disease (see for example Patent Document 19), asthma, atopic dermatitis, rheumatoid arthritis, sarcoidosis, parapulmonary fibrosis, arteriosclerosis, psoriasis, multiple sclerosis, post-transplant rejection reactions, and inflammatory bowel disease (see for example Patent Document 20). However, these are no more than the compounds having these specific chemical structures that are known.

Prior art documents relating to the invention of this application are as follows.
Non-patent document 1:
   J Am Soc Nephrol, 2000, Vol. 11, 152
Non-patent document 2:
   Diabetes, 2002, Vol. 51, p. 238
Non-patent document 3:
   Kidney Int, 1999, Vol. 56, p. 2107
Non-patent document 4:
   J Cereb Blood Flow Metab. 21, 683-689 (2001)
Non-patent document 5:
   J Neuroimmunol. 56, 127-134 (1995)
Non-patent document 6:
   Neurosci Lett. 227, 173-176 (1997)
Non-patent document 7:
   J Cereb Blood Flow Metab. 21, 1430-1435 (2001)
Non-patent document 8:
   J Rheumatol, 1999, Vol. 26, p. 1992
Non-patent document 9:
   J Immunol, 1994, Vol. 152, p. 2060
Non-patent document 10:
   Clin Immunol Immunopathol, 1995, Vol. 77, p. 307
Non-patent document 11:
   Arthritis Rheum, 2001, Vol. 44, p. 1633
Non-patent document 12:
   J Immunol, 2001, Vol.167, p. 5381)
Non-patent document 13:
   J. Clin. Invest. 1998, Vol 101, p. 2910)
Non-patent document 14:
   Immunol. Lett., 1997, Vol 57, p. 117)
Non-patent document 15:
   Arthritis Rheum., 1999, Vol. 42, p. 989)
Non-patent document 16:
   Lancet, 1994, vol. 344, p. 1383)
Non-patent document 17:
   N Eng J Med, 1999, Vol. 340, p. 115)
Non-patent document 18:
   Circulation, 1998, vol. 97, p. 75)
Non-patent document 19:
   Blood, 1997, vol. 90, p. 909)
Non-patent document 20:
   Nature, 1998, vol. 394, p. 894)
Non-patent document 21:
   Transplantation, 2001, Vol. 72, p. 1195)
Non-patent document 22:
   FASEB, 1999, Vol. 13, p.1371)
Non-patent document 23:
   Lancet, 2001, Vol. 357, p. 1758)
Patent publication 1:
   WO 99/32100
Patent publication 2:
   WO 00/10965
Patent publication 3:
   WO 00/37455
Patent publication 4:
   WO 00/68203
Patent publication 5:
   WO 00/76993
Patent publication 6:
   WO 00/66551
Patent publication 7:
   WO 01/25200
Patent publication 8:
   WO 01/25199
Patent publication 9:
   WO 98/27815
Patent publication 10:
   WO 00/56729
Patent publication 11:
   WO 00/59497
Patent publication 12:
   WO 00/59498
Patent publication 13:
   WO 00/66558
Patent publication 14:
   WO 00/66559
Patent publication 15:
   WO 00/76511
Patent publication 16:
   WO 00/76512
Patent publication 17:
   WO 00/76514
Patent publication 18:
   WO 00/76973
Patent publication 19:
   WO 01/42208
Patent publication 20:
   WO 01/64213

### Disclosure of the Invention

As a result of various studies of compounds having a CCR antagonist effect, the present inventors found specific compounds having a CCR antagonistic effect that are effective in the prevention and treatment of graft-versus-host disease and rejection reactions during organ transplantation, and also effective in the prevention and treatment of rheumatoid arthritis, autoimmune diseases, allergic disorders, ischemic brain cell damage, myocardial infarction, chronic nephritis, and arteriosclerosis, and completed the present invention. In other words, the present invention provides agents that prevent and treat these diseases.

The present invention relates to:
(1) An agent for the prevention or treatment of graft-versus host disease and/or rejection reactions during organ or bone marrow transplantation which comprises a compound having a CCR antagonistic effect represented by the formula: 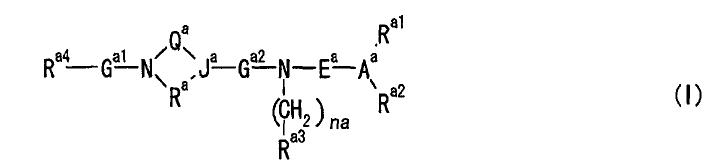 wherein R^{a1} is a hydrogen atom, a hydrocarbon group which may be substituted, a non-aromatic heterocyclic group which may be substituted, R^{a2} is a hydrocarbon group which may be substituted, a non-aromatic heterocyclic group which may be substituted, or R^{a1} and R^{a2} may combine with each other together with A^{a} to form a heterocyclic group which may be substituted, A^{a} is N or N⁺-R^{a5} · Y^{a-} (R^{a5} is a hydrocarbon group, Y^{a-} is a counter anion), R^{a3} is a cyclic hydrocarbon group which may be substituted or a heterocyclic group which may be substituted, na is 0 or 1, R^{a4} is a hydrogen atom, a hydrocarbon group which may be substituted, a heterocyclic group which may be substituted, an alkoxy group which may be substituted, an aryloxy group which may be substituted, or an amino group which may be substituted, E^{a} is a divalent aliphatic hydrocarbon group which may be substituted by a group other than an oxo group, G^{a1} is a bond, CO or SO₂, G^{a2} is CO, SO₂, NHCO, CONH or OCO, J^{a} is methine or a nitrogen atom, and each of Q^{a} and R^{a} is a bond or a divalent C₁₋₃ aliphatic hydrocarbon which may be substituted, with the proviso that J^{a} is methine when G^{a2} is OCO, one of Q^{a} and R^{a} is not a bond when the other is a bond, and each of Q^{a} and R^{a} is not substituted by an oxo group when G^{a1} is a bond, the formula: 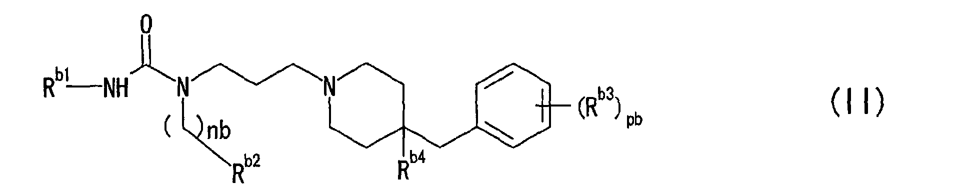 wherein R^{b1} is a hydrocarbon group which may be substituted; R^{b2} is a cyclic hydrocarbon group which may be substituted or a heterocyclic group which may be substituted; R^{b3} is a halogen atom, a carbamoyl group which may be substituted, a sulfamoyl group which may be substituted, an acyl group derived from a sulfonic acid, a C₁₋₄ alkyl group which may be substituted, a C₁₋₄ alkoxy group which may be substituted, an amino group which may be substituted, a nitro group or a cyano group; R^{b4} is a hydrogen atom or a hydroxy group; nb is an integer of 0 or 1; pb is an integer of 0 or 1 to 4, the formula: 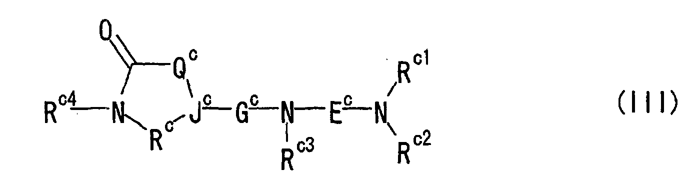 wherein R^{c1} is a hydrocarbon group, R^{c2} is a hydrocarbon group having 2 or more carbon atoms, or R^{c1} and R^{c2} may be bound together with the adjacent nitrogen atom to form a ring which may have a substituent or substituents, R^{c3} is a hydrocarbon group which may have a substituent or substituents or a heterocyclic group which may have a substituent or substituents, R^{c4} is a hydrogen atom, a hydrocarbon group which may have a substituent or substituents or a heterocyclic group which may have a substituent or substituents, E^{c} is a divalent aliphatic hydrocarbon group which may have a substituent or substituents other than an oxo group, G^{c} is CO or SO₂, J^{c} is a nitrogen atom or a methine group which may have a substituent or substituents, and Q^{c} and R^{c} are each a bond or a divalent aliphatic C₁₋₃ hydrocarbon group which may have a substituent or substituents, the formula: 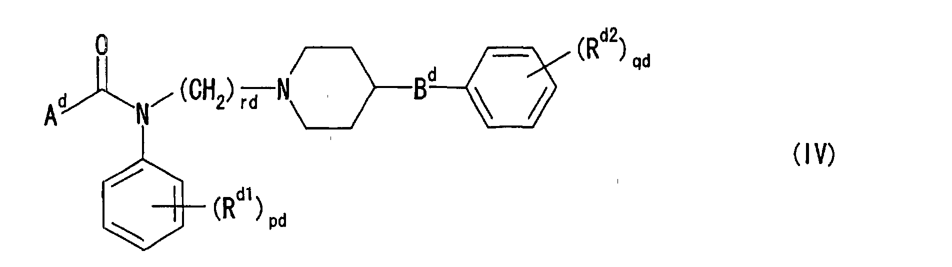 wherein A^{d} is a group represented by the formula: 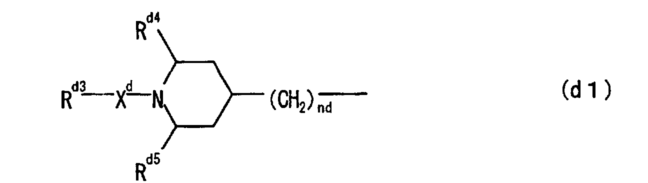 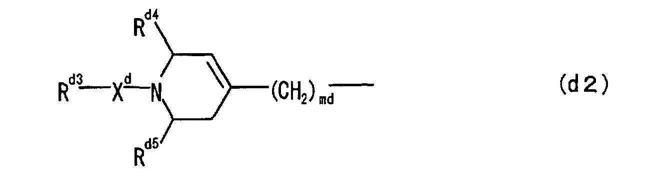 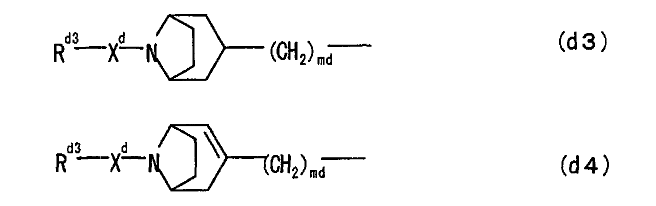 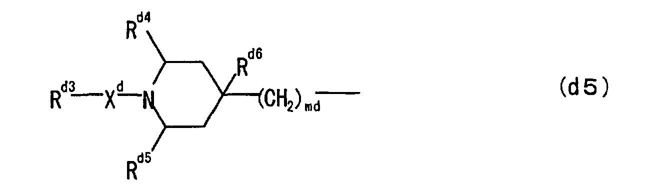 or 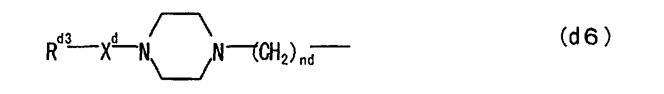 wherein, R^{d3} is (1) a hydrocarbon group which may be substituted, (2) a C₁₋₄ alkoxy group which may be substituted or (3) an amino group which may be substituted; X^{d} is a bond, -SO₂- or -CO-; nd is an integer of 1 to 3; md is 0 or an integer of 1 to 3; R^{d4} and R^{d5} are the same or different and each of which is a hydrogen atom or a C₁₋₆ alkyl group; R^{d6} is a hydroxyl group, a C₁₋₆ alkyl group or a C₂₋₆ alkenyl group; rd is an integer of 2 to 4; B^{d} is a bond, -CH₂-, -SO₂-, -SO-, -S-, -O-, -CO-, -NR^{da}-SO₂- or -NR^{da}-CO- (wherein, R^{da} is a hydrogen atom, a C₁₋₆ alkyl group, a C₂₋₆ alkenyl group or a C₃₋₈ cycloalkyl group); each of pd and qd is 0 or an integer of 1 to 4; R^{d1} is a halogen atom, a C₁₋₆ alkyl group, a C₂₋₄ alkenyl group, a C₁₋₄ alkanoyl group, a C₁₋₄ alkoxy group, a cyano group, a trifloromethyl group, a nitro group, a hydroxyl group, an amino group or an amidino group; R^{d2} is 1) a halogen, 2) a C₁₋₆ alkyl which may be substituted by a halogen or a C₁₋₄ alkoxy, 3) a C₁₋₄ alkoxy which may be substituted by a halogen or a C₁₋₄ alkoxy, 4) nitro, 5) cyano, 6) hydroxyl, 7) a C₁₋₄ alkanoylamino, 8) SO₂NR^{db}R^{dc}, 9) SO₂Rd^{dd}, 10) CONR^{db}R^{dc}, 11) NR ^{db}R^{dc} or 12) NR^{da}-SO₂R^{dd} (wherein, R^{da} has the meaning given above, and R^{db} and R^{dc} may be the same or different, and are (1) a hydrogen atom, (2) a C₁₋₆ alkyl group which may be substituted by a halogen or a C₁₋₄ alkoxy, or (3) a C₃₋₈ cycloalkyl group which may be substituted by a halogen or a C₁₋₄ alkoxy, or R^{db} and R^{dc} may bond with a nitrogen atom to form a cyclic amino group and R^{dd} is a C₁₋₆ alkyl group or a C₃₋₈ cycloalkyl group), each R^{d1} may be the same or different from each other when pd is two or more, and each R^{d2}, may be the same or different from each other when qd is two or more, or
   the formula: 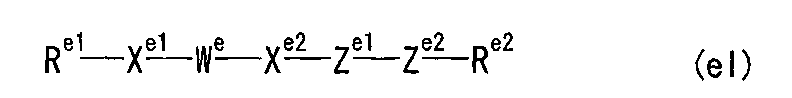 wherein R^{e1} represents a 5 to 6-membered cyclic ring group which may be substituted, X ^{e1} represents a bond or a bivalent group, in which the number of atoms constituting the straight-chain portion is 1 to 4, W^{e} represents a bivalent group represented by formula: 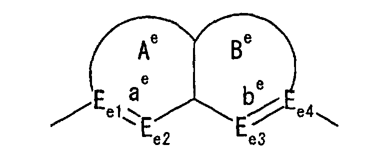 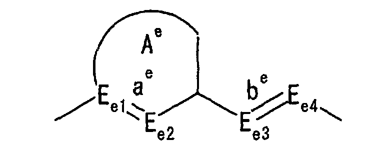 or 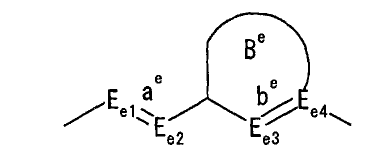 wherein each of ring A^{e} and ring B^{e} represents a 5- to 7-membered cyclic group which may be substituted, each of Ee₁ and Ee₄ is a carbon atom which may be substituted or a nitrogen atom which may be substituted, each of Ee₂ and Ee₃ is a carbon atom which may be substituted, a nitrogen atom which may be substituted, or a sulfur atom which may be oxidized or an oxygen atom, each of a^{e} and b^{e} is a single bond or a double bond), X^{e2} is a bivalent group in which the number of atoms constituting the straight-chain portion is 1 to 4, Z^{e1} is a bond or a bivalent cyclic ring group, Z^{e2} is a bond or a bivalent cyclic ring group in which the number of atoms constituting the straight-chain portion is 1 to 4, and R^{e2} is (1) an amino group which may be substituted, and the nitrogen atom may be converted into a quaternary ammonium or an N-oxide, (2) a nitrogen-containing heterocyclic ring group which may be substituted, may contain sulfur atom or an oxygen atom as a ring-constituting atom, and the nitrogen atom may be converted into a quaternary ammonium or a N-oxide, (3) a group which is bonded via the sulfur atom, (4) a group represented by formula: 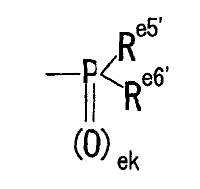 wherein ek is 0 or 1, the phosphorus atom may form a phosphonium salt when ek is 0, and each of R^{e5}' and R^{e6'} is a hydrocarbon atom which may be substituted, a hydroxyl group which may be substituted, or an amino group which may be substituted, and R^{e5'} and R^{e6'} may bond to each other to form a cyclic ring group together with the adjacent phosphorus atom, (5) an amidino group which may be substituted or (6) a guanidino group which may be substituted, or a salt thereof;
(2) An agent for the prevention or treatment of chronic rheumatoid arthritis, autoimmune diseases, allergic disorders, ischemic brain cell damage, myocardial infarction, chronic nephritis, and arteriosclerosis which comprises a compound having a CCR antagonistic effect represented by the formula: 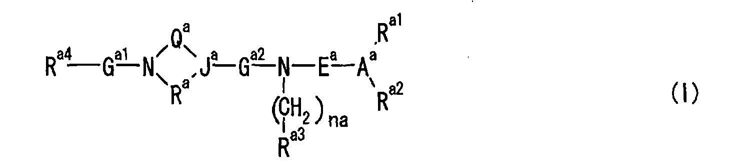 wherein R^{a1} is a hydrogen atom, a hydrocarbon group which may be substituted, a non-aromatic heterocyclic group which may be substituted, R^{a2} is a hydrocarbon group which may be substituted, a non-aromatic heterocyclic group which may be substituted, or R^{a1} and R^{a2} may combine with each other together with A^{a} to form a heterocyclic group which may be substituted, A^{a} is N or N⁺-R^{a5} · Y^{a-} (R^{a5} is a hydrocarbon group, Y^{a-} is a counter anion), R^{a3} is a cyclic hydrocarbon group which may be substituted or a heterocyclic group which may be substituted, na is 0 or 1, R^{a4} is a hydrogen atom, a hydrocarbon group which may be substituted, a heterocyclic group which may be substituted, an alkoxy group which may be substituted, an aryloxy group which may be substituted, or an amino group which may be substituted, E^{a} is a divalent aliphatic hydrocarbon group which may be substituted by a group other than an oxo group, G^{a1} is a bond, CO or SO₂, G^{a2} is CO, SO₂, NHCO, CONH or OCO, J^{a} is methine or a nitrogen atom, and each of Q^{a} and R^{a} is a bond or a divalent C₁₋₃ aliphatic hydrocarbon which may be substituted, with the proviso that J^{a} is methine when G^{a2} is OCO, one of Q^{a} and R^{a} is not a bond when the other is a bond, and each of Q^{a} and R^{a} is not substituted by an oxo group when G^{a1} is a bond,
   the formula: 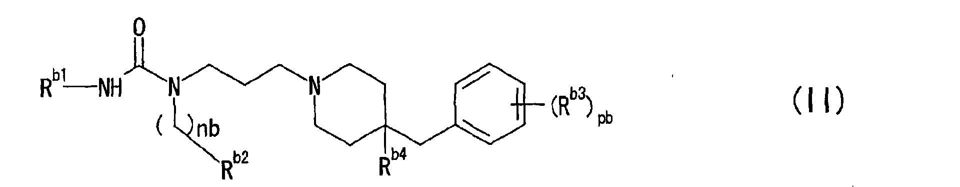 wherein R^{b1} is a hydrocarbon group which may be substituted; R^{b2} is a cyclic hydrocarbon group which may be substituted or a heterocyclic group which may be substituted; R^{b3} is a halogen atom, a carbamoyl group which may be substituted, a sulfamoyl group which may be substituted, an acyl group derived from a sulfonic acid, a C₁₋₄ alkyl group which may be substituted, a C₁₋₄ alkoxy group which may be substituted, an amino group which may be substituted, a nitro group or a cyano group; R^{b4} is a hydrogen atom or a hydroxy group; nb is an integer of 0 or 1; pb is an integer of 0 or 1 to 4,
   the formula: 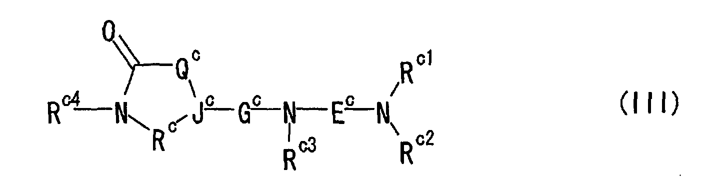 wherein R^{c1} is a hydrocarbon group, R^{c2} is a hydrocarbon group having 2 or more carbon atoms, or R^{c1} and R^{c2} may be bound together with the adjacent nitrogen atom to form a ring which may have a substituent or substituents, R^{c3} is a hydrocarbon group which may have a substituent or substituents or a heterocyclic group which may have a substituent or substituents, R^{c4} is a hydrogen atom, a hydrocarbon group which may have a substituent or substituents or a heterocyclic group which may have a substituent or substituents, E^{c} is a divalent aliphatic hydrocarbon group which may have a substituent or substituents other than an oxo group, G^{c} is CO or SO₂, J^{c} is a nitrogen atom or a methine group which may have a substituent or substituents, and Q^{c} and R^{c} are each a bond or a divalent aliphatic C₁₋₃ hydrocarbon group which may have a substituent or substituents,
   the formula: 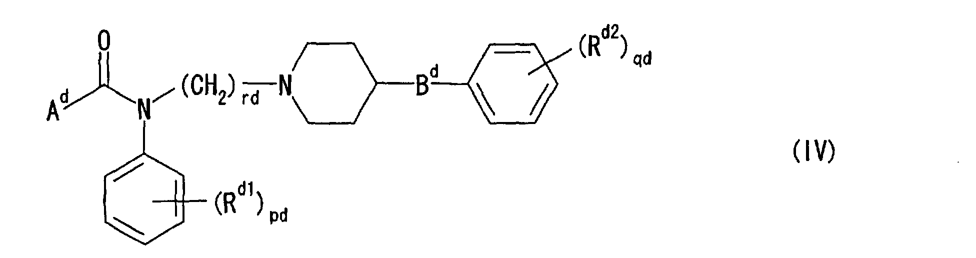 wherein A^{d} is a group represented by the formula: 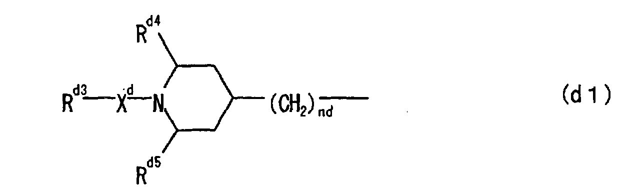 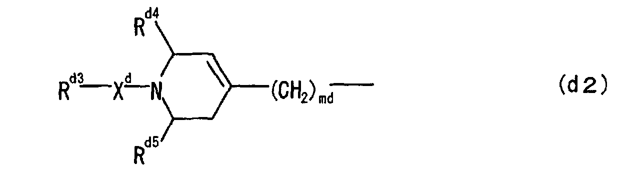 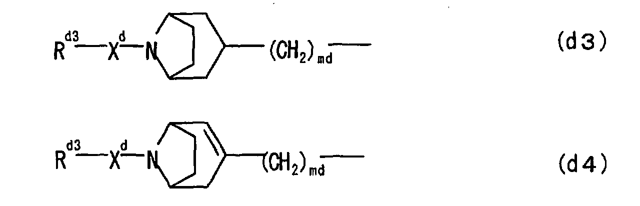 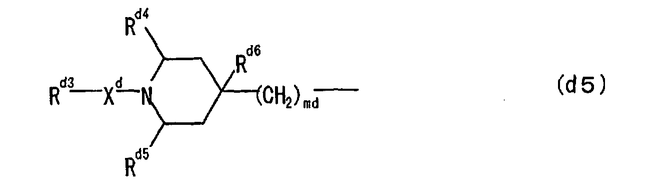 or 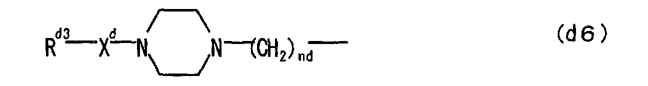 wherein, R^{d3} is (1) a hydrocarbon group which may be substituted, (2) a C₁₋₄ alkoxy group which may be substituted or (3) an amino group which may be substituted; X^{d} is a bond, -SO₂- or -CO-; nd is an integer of 1 to 3; md is 0 or an integer of 1 to 3; R^{d4} and R^{d5} are the same or different and each of which is a hydrogen atom or a C₁₋₆ alkyl group; R^{d6} is a hydroxyl group, a C₁₋₆ alkyl group or a C₂₋₆ alkenyl group; rd is an integer of 2 to 4; B^{d} is a bond, -CH₂-, -SO₂-, -SO-, -S-, -O-, -CO-, -NR^{da}-SO₂- or -NR^{da}-CO- (wherein, R^{da} is a hydrogen atom, a C₁₋₆ alkyl group, a C₂₋₆ alkenyl group or a C₃₋₈ cycloalkyl group); each of pd and qd is 0 or an integer of 1 to 4; R^{d1} is a halogen atom, a C₁₋₆ alkyl group, a C₂₋₄ alkenyl group, a C₁₋₄ alkanoyl group, a C₁₋₄ alkoxy group, a cyano group, a trifloromethyl group, a nitro group, a hydroxyl group, an amino group or an amidino group; R^{d2} is 1) a halogen, 2) a C₁₋₆ alkyl which may be substituted by a halogen or a C₁₋₄ alkoxy, 3) a C₁₋₄ alkoxy which may be substituted by a halogen or a C₁₋₄ alkoxy, 4) nitro, 5) cyano, 6) hydroxyl, 7) a C₁₋₄ alkanoylamino, 8) SO₂NR^{db}R^{dc}, 9) SO₂R^{dd}, 10) CONR^{db}R^{dc}, 11) NR ^{db}R^{dc} or 12) NR^{da}-SO₂R^{dd} (wherein, R^{da} has the meaning given above, and R^{db} and R^{dc} may be the same or different, and are (1) a hydrogen atom, (2) a C₁₋₆ alkyl group which may be substituted by a halogen or a C₁₋₄ alkoxy, or (3) a C₃₋₈ cycloalkyl group which may be substituted by a halogen or a C₁₋₄ alkoxy, or R^{db} and R^{dc} may bond with a nitrogen atom to form a cyclic amino group and R^{dd} is a C₁₋₆ alkyl group or a C₃₋₈ cycloalkyl group), each R^{d1} may be the same or different from each other when pd is two or more, and each R^{d2}, may be the same or different from each other when qd is two or more, or
   the formula: 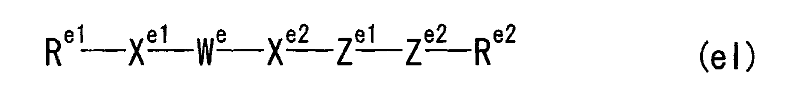 wherein R^{e1} represents a 5 to 6-membered cyclic ring group which may be substituted, X ^{e1} represents a bond or a bivalent group, in which the number of atoms constituting the straight-chain portion is 1 to 4, W^{e} represents a bivalent group represented by formula: 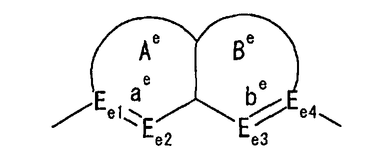 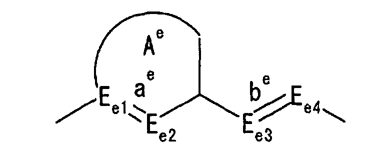 or 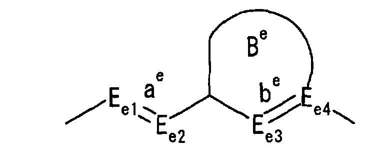 wherein each of ring A^{e} and ring B^{e} represents a 5- to 7-membered cyclic group which may be substituted, each of Ee₁ and Ee₄ is a carbon atom which may be substituted or a nitrogen atom which may be substituted, each of Ee₂ and Ee₃ is a carbon atom which may be substituted, a nitrogen atom which may be substituted, or a sulfur atom which may be oxidized or an oxygen atom, each of a^{e} and b^{e} is a single bond or a double bond), X^{e2} is a bivalent group in which the number of atoms constituting the straight-chain portion is 1 to 4, Z^{e1} is a bond or a bivalent cyclic ring group, Z^{e2} is a bond or a bivalent cyclic ring group in which the number of atoms constituting the straight-chain portion is 1 to 4, and R^{e2} is (1) an amino group which may be substituted, and the nitrogen atom may be converted into a quaternary ammonium or an N-oxide, (2) a nitrogen-containing heterocyclic ring group which may be substituted, may contain sulfur atom or an oxygen atom as a ring-constituting atom, and the nitrogen atom may be converted into a quaternary ammonium or a N-oxide, (3) a group which is bonded via the sulfur atom, (4) a group represented by formula: 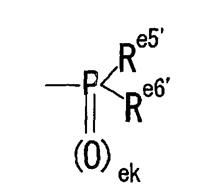 wherein ek is 0 or 1, the phosphorus atom may form a phosphonium salt when ek is 0, and each of R^{e5'} and R^{e6'} is a hydrocarbon atom which may be substituted, a hydroxyl group which may be substituted, or an amino group which may be substituted, and R^{e5}' and R^{e6}' may bond to each other to form a cyclic ring group together with the adjacent phosphorus atom), (5) an amidino group which may be substituted or (6) a guanidino group which may be substituted, or a salt thereof;
(3) The agent for the prevention or treatment according to the above (1) or (2), wherein the compound having a CCR antagonistic effect or a salt thereof is N-(3,4-dichlorophenyl)-1-(methylsulfonyl)-N-{3-[4-({4-[(methylsulfonyl)-amino]phenyl}sulfonyl)-1-piperidinyl]propyl}-4-piperidinecarboxamide, N-(3-chlorophenyl)-1-(methylsulfonyl)-N-(3-{4-[4-(methylsulfonyl)benzyl]-1-piperidinyl}propyl)-4-piperidinecarboxamide, N-(3-{4-[4-(aminocarbonyl)benzyl]-1-piperidinyl}propyl)-N-(3,4-dichlorophenyl)-1-(methylsulfonyl)-4-piperidinecarboxamide, 1-acetyl-N-(3-{4-[4-(aminocarbonyl)benzyl]-1-piperidinyl}propyl)-N-(3-chloro-4-methylphenyl)-4-piperidinecarboxamide, N-(3,4-dichlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-1-(methylsulfonyl)-N-(3-{4-[4-(methylsulfonyl)benzyl]-1-piperidinyl}propyl)-4-piperidinecarboxamide, N-(3-{4-[4-(aminocarbonyl)benzyl]-1-piperidinyl}propyl)-N-(3-chloro-4-methylphenyl)-1-(methylsulfonyl)-4-piperidinecarboxamide, N-[3-(4-benzyl-1-piperidinyl)propyl]-N'-(4-chlorophenyl)-N-phenylurea, N'-(4-chlorophenyl)-N-{3-[4-(4-fluorobenzyl)-1-piperidinyl]propyl}-N-phenylurea, N'-(4-chlorophenyl)-N-(3-{4-[4-(4-morpholinylsulfonyl)benzyl]-1-piperidinyl}propyl)-N-phenylurea, N'-(4-chlorophenyl)-N-(3-{4-[4-(4-methylsulfonyl)benzyl]-1-piperidinyl}propyl)-N-phenylurea, 9-{[1-(3-{[(9-chloroanilino)carbonyl]anilino}propyl)-4-piperidinyl]methyl}benzamide, N-[3-(4-benzyl-1-piperidinyl)propyl]-N-(3,4-dichlorophenyl)-1-methyl-5-oxo-3-pyrrolidinecarboxamide, 1-benzyl-N-[3-(4-benzyl-1-piperidinyl)propyl]-5-oxo-N-phenyl-3-pyrrolidinecarboxamide, N-[3-(4-benzyl-1-piperidinyl)propyl]-1-(2-chlorobenzyl)-5-oxo-N-phenyl-3-pyrrolidinecarboxamide, N-(3,4-dichlorophenyl)-N-{3-[4-(4-fluorobenzyl)-1-piperidinyl]propyl}-1-methyl-5-oxo-3-pyrrolidinecarboxamide, N-[3-(4-benzyl-1-piperidinyl)propyl]-5-oxo-N-phenyl-1-(2,2,2-trifluoroethyl)-3-pyrrolidinecarboxamide, N-(3,4-dichlorophenyl)-N-(3-{4-[4-(methylsulfonyl)benzyl]-1-piperidinyl}propyl)-2-[1-(methylsulfonyl)-4-piperidinyl]acetamide, N-(3,4-dichlorophenyl)-N-(3-{4-[4-(isopropylsulfonyl)benzyl]-1-piperidinyl}propyl)-2-[1-(methylsulfonyl)-4-piperidinyl]acetamide, 3-(1-acetyl-4-piperidinyl)-N-(3,4-dichlorophenyl)-N-(3-{4-[4-(methylsulfonyl)benzyl]-1-piperidinyl}propyl)propanamide, or N-(3,4-dichlorophenyl)-4-hydroxy-1-(methylsulfonyl)-N-(3-(4-[4-(methylsulfonyl)benzyl]-1-piperidinyl}propyl)-4-piperidinecarboxamide or a salt thereof;
(4) The prevention or treatment agent according to the above (1) or (2), wherein the compound having a CCR antagonistic effect or a salt thereof is N-methyl-N-[4-[[[2-(4-methylphenyl)-6,7-dihydro-5H-benzocyclohepten-8-yl]carbonyl]amino]benzyl]piperidinium iodide, N-methyl-N-[4-[[[7-(4-methylphenyl)-2,3-dihydro-1-benzoxepin-4-yl]carbonyl]amino]benzyl]piperidinium iodide, N-[4-[N-methyl-N-(tetrahydropyran-4-yl)aminomethyl]phenyl]-7-(4-methylphenyl)-2,3-dihydro-1-benzoxepin-4-carboxamide, N-[4-[N-methyl-N-(tetrahydropyran-4-yl)aminomethyl]phenyl]-7-(4-morpholinophenyl)-2,3-dihydro-1-benzoxepin-4-carboxamide, 7-(4-ethoxyphenyl)-N-[4-[N-methyl-N-(tetrahydropyran-4-yl)aminomethyl]phenyl]-2,3-dihydro-1-benzoxepin-4-carboxamide, N,N-dimethyl-N-[4-[[[2-(4-methylphenyl)-6,7-dihydro-5H-benzocyclohepten-8-yl]carbonyl]amino]benzyl]-N-(tetrahydropyran-4-yl)ammonium iodide, N-methyl-N-[4-[[[7-(4-methylphenyl)-3,4-dihydronaphthalen-2-yl]carbonyl]amino]benzyl]piperidinium iodide, N,N-dimethyl-N-(4-(((2-(4-methylphenyl)-6,7-dihydro-5H-benzocyclohepten-8-yl)carbonyl)amino)benzyl)-N-(4-tetrahydropyranyl)ammonium chloride, N,N-dimethyl-N-(((7-(4-methylphenyl)-2,3-dihydro-1-benzoxepin-4-yl)carbonyl)amino)benzyl)-N-(4-oxocyclohexyl)ammonium chloride, N-(4-(((7-(4-ethoxyphenyl)-2,3-dihydro-1-benzoxepin-4-yl)carbonyl)amino)benzyl)-N,N-dimethyl-N-(4-tetrahydropyranyl)ammonium chloride, N-[4-[N-methyl-N-(tetrahydropyran-4-yl)aminomethyl]phenyl]-7-(4-propoxyphenyl)-1,1-dioxo-2,3-dihydro-1-benzothiepin-4-carboxamide, 7-(4-butoxyphenyl)-N-[4-[N-methyl-N-(tetrahydropyran-4-yl)aminomethyl]phenyl]-1,1-dioxo-2,3-dihydro-1-benzothiepin-4-carboxamide, 7-[4-[N-methyl-N-(2-propoxyethyl)amino]phenyl]-N-[4-[[N-methyl-N-(tetrahydropyran-4-yl)amino]methyl]phenyl]-1,1-dioxo-2,3-dihydro-1-benzothiepin-4-carboxamide, 7-[4-(2-ethoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, N-[4-[[N-methyl-N-(tetrahydropyran-4-yl)amino]methyl]phenyl]-7-[4-(2-propoxyethoxy)phenyl]-1,1-dioxo-2,3-dihydro-1-benzothiepin-4-carboxamide, 7-[4-(2-butoxyethoxy)phenyl]-N-[4-[[N-methyl-N-(tetrahydropyran-4-yl)amino]methyl)phenyl]-1,1-dioxo-2,3-dihydro-1-benzothiepin-4-carboxamide, 7-[4-(2-ethoxyethoxy)-3,5-dimethylphenyl]-N-[4-[[N-methyl-N-(tetrahydro-2H-pyran-4-yl)amino]methyl]phenyl]-1,1-dioxo-2,3-dihydro-1-benzothiepin-4-carboxamide, 7-[2-chloro-4-(2-propoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, 7-(3-methyl-4-propoxyphenyl)-N-[4-[[N-methyl-N-(tetrahydropyran-4-yl)amino]methyl]phenyl]-1,1-dioxo-2,3-dihydro-1-benzothiepin-4-carboxamide, 7-(3,4-dipropoxyphenyl)-N-(4-((N-methyl-N-(tetrahydro-2H-pyran-4-yl)amino)methyl)phenyl)-1,1-dioxo-2,3-dihydro-1-benzothiepin-4-carboxamide, 7-[4-(2-ethoxyethoxy)phenyl]-1-ethyl-N-[4-[[N-methyl-N-(tetrahydropyran-4-yl)amino]methyl]phenyl]-2,3-dihydro-1-benzoazepin-4-carboxamide, 1-ethyl-7-[4-(2-propoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-ethyl-N-[4-[[N-methyl-N-(tetrahydropyran-4-yl)amino]methyl]phenyl]-2,3-dihydro-1-benzoazepin-4-carboxamide, 7-[4-(2-ethoxyethoxy)phenyl]-1-formyl-N-[4-[[N-methyl-N-(tetrahydropyran-4-yl)amino]methyl]phenyl]-2,3-dihydro-1-benzoazepin-4-carboxamide, 1-formyl-7-[4-(2-propoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-formyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-5-yl)amino]methyl]phenyl]-1-propyl-2,3-dihydro-1-benzoazepin-4-carboxamide, N-[4-[[N-methyl-N-(tetrahydropyran-5-yl)amino]methyl]phenyl]-7-[4-(2-propoxyethoxy)phenyl]-1-propyl-2,3-dihydro-1-benzoazepin-4-carboxamide, 1-benzyl-7-[4-(2-butoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-cyclopropylmethyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-phenyl-2,3-dihydro-1-benzoazepin-4-carboxamide, 7-[4-(2-butoxyethoxy)phenyl]-1-(3,4-methylenedioxy)phenyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(2-methyloxazol-5-yl)-N-[4-[[N-methyl-N-(tetrahydropyran-4-yl)amino]methyl]phenyl]-2,3-dihydro-1-benzoazepin-4-carboxamide, 1-allyl-7-[4-(2-butoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(3-thienyl)methyl-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(thiazol-2-yl)methyl-2,3-dihydro-1-benzoazepin-4-carboxamide, 7-[4-(2-butoxyethoxy)phenyl]-1-(1-methylpyrazol-4-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(3-methylisothiazol-5-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(1-ethylpyrazol-4-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-isobutyl-N-[4-[[N-methyl-N-(tetrahydropyran-5-yl)amino]methyl]phenyl]-2,3-dihydro-1-benzoazepin-4-carboxamide, 1-isobutyl-N-[4-[[N-methyl-N-(tetrahydropyran-5-yl)amino]methyl]phenyl]- 7-[4-(2-propoxyethoxy)phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(thiazol-5-yl)methyl-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(1-methyltetrazol-5-yl)methyl-2,3-dihydro-1-benzoazepin-4-carboxamide, or 7-[4-(2-butoxyethoxy)phenyl]-N-[4-[[N-methyl-N-(tetrahydropyran-4-yl)amino]methyl]phenyl]-1-(2-methyltetrazol-5-yl)methyl-2,3-dihydro-1-benzoazepin-4-carboxamide or a salt thereof;
(5) A method of preventing or treating graft-versus host disease and/or rejection reactions during organ or bone marrow transplantation, which comprises the step of administering an effective amount of a compound having a CCR antagonist effect to a mammal;
(6) A method of preventing or treating chronic rheumatoid arthritis, autoimmune diseases, allergic disorders, ischemic brain cell damage, myocardial infarction, chronic nephritis, and arteriosclerosis, which comprises the step of administering an effective amount of a compound having a CCR antagonist effect to a mammal;
(7) Use of a compound having a CCR antagonist effect for manufacturing an agent for the prevention or treatment of graft-versus host disease and/or rejection reactions during organ or bone marrow transplantation;
(8) Use of a compound having a CCR antagonist effect for manufacturing an agent for the prevention or treatment of chronic rheumatoid arthritis, autoimmune diseases, allergic disorders, ischemic brain cell damage, myocardial infarction, chronic nephritis, and arteriosclerosis; and the like.

### Detailed Description of the Invention

The following compounds are examples of the compound represented by the above formula (eI) or salt thereof.
1) the prevention and treatment agent disclosed in (1) above that is a compound represented by the formula: 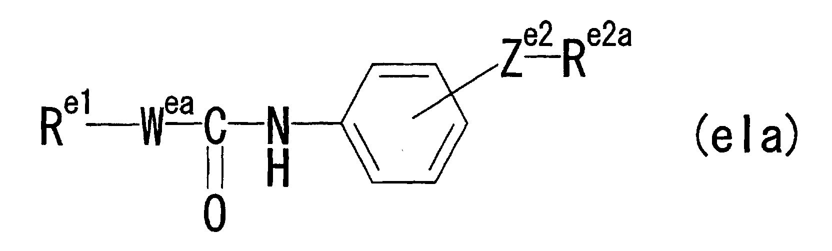 wherein R^{e1} is an optionally substituted 5- to 6-membered ring, W^{ea} is a divalent group represented by the formula: 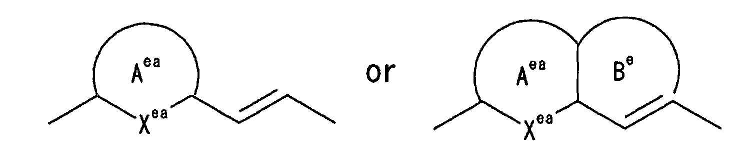 wherein the ring A ^{ea} is an optionally substituted 5- to 6-membered aromatic ring, X ^{ea} is an optionally substituted carbon atom, an optionally substituted nitrogen atom, a sulfur atom or an oxygen atom, the ring B^{e} is an optionally substituted 5- to 7-membered ring; Z ^{e2} is a bond or a divalent group in which the number of carbon atoms constituting the straight-chain portion is 1 to 4, R ^{e2a} is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium, (3) a group binding through a sulfur atom or (4) a group of the formula: 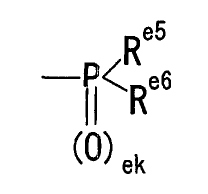 wherein ek is 0 or 1, and when ek is 0, a phosphorus atom may form a phosphonium; and R^{e5} and R^{e6} are independently an optionally substituted hydrocarbon group or an optionally substituted amino group, and R^{e5} and R^{e6} may bind to each other to form a cyclic group together with the adjacent phosphorus atom, or salt thereof,
2) the prevention and treatment agent disclosed in (1) above that is a compound represented by the formula: 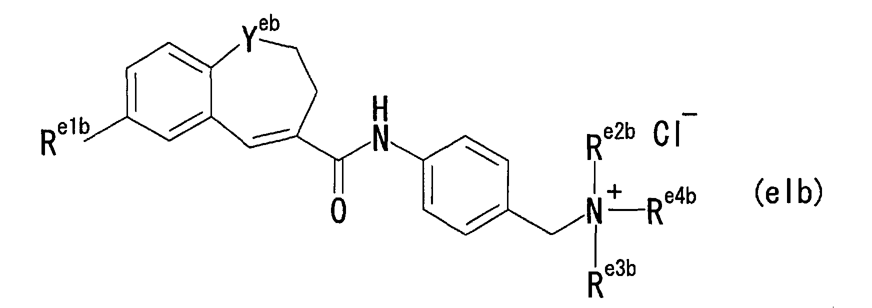 wherein R^{e1b} is an optionally substituted phenyl group or an optionally substituted thienyl group; Y^{eb} is -CH₂-, -O- or -S-; and R^{e2b}, R^{e3b} and R^{e4b} are independently an optionally substituted aliphatic hydrocarbon group or an optionally substituted alicyclic heterocyclic ring group, or salt thereof,
3) the prevention and treatment agent disclosed in (1) above that is a compound represented by the formula: 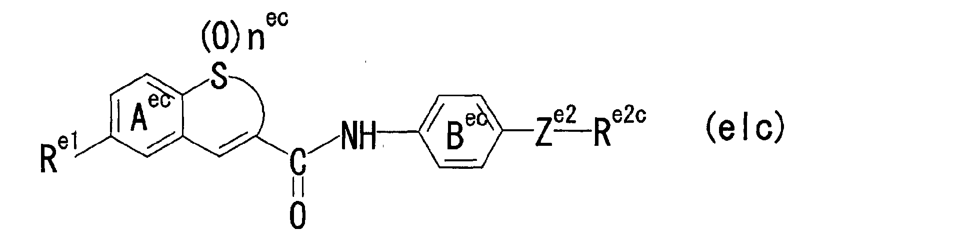 wherein R^{e1} is an optionally substituted 5- to 6-membered ring; the ring A^{ec} is an optionally substituted 6- to 7-membered ring; the ring B^{ec} is an optionally substituted benzene ring; n^{ec} is an integer of 1 or 2; Z^{e2} is a bond or a divalent group in which the number of carbon atoms constituting the straight -chain is 1 to 4; R^{e2c} is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium, (3) a group binding through a sulfur atom or (4) a group of the formula: 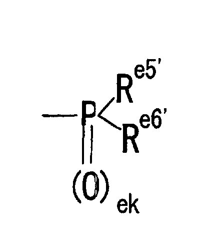 wherein ek is 0 or 1, and when ek is 0, a phosphorus atom may form a phosphonium; and R^{e5'} and R^{e6'} are independently an optionally substituted hydrocarbon group, an optionally substituted hydroxy group or an optionally substituted amino group, and R^{e5'} and R^{e6'} may bind to each other to form a cyclic group together with the adjacent phosphorus atom, or a salt thereof,
4) a compound represented by the formula: 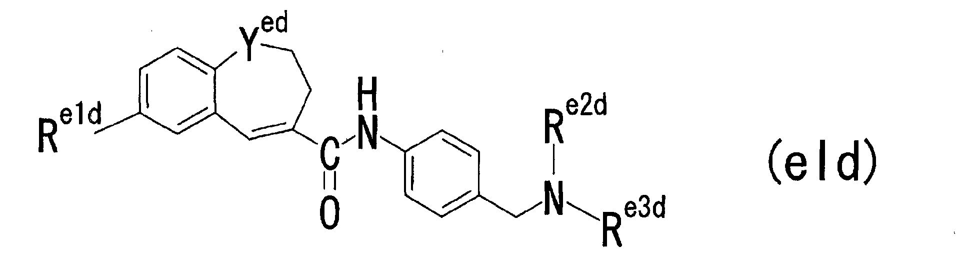
wherein R^{e1d} is a 5- to 6- membered aromatic ring which has a group of the formula: R^{ed}-Z^{e1d}-X^{ed}-Z^{e2d}- wherein R^{ed} is a hydrogen atom or an optionally substituted hydrocarbon group, X^{ed} is an optionally substituted alkylene chain, and Z^{e1a} and Z ^{e2d} are respectively hetero-atoms, and which may have a further substituent, the group R^{ed} may bind to the 5-6 membered aromatic ring to form a ring, Y^{ed} is an optionally substituted imino group, R^{e2d} and R^{e3d} are respectively an optionally substituted aliphatic hydrocarbon group or an optionally substituted alicyclic heterocyclic group, or a salt thereof.

In the formula (I), the "hydroxarbon group" in the "hydrocarbon group which may be substituted" represented by R^{a1} includes e.g. an aliphatic chain hydrocarbon group, an alicyclic hydrocarbon group and an aryl group, preferably, an aliphatic chain hydrocarbon group and an alicyclic hydrocarbon group.

Examples of the aliphatic hydrocarbon group include e.g. a straight-chain or branched aliphatic hydrocarbon group such as an alkyl group, an alkenyl group, an alkynyl group, etc., preferably an alkyl group. Examples of the alkyl group include e.g. a C₁₋₁₀ alkyl group (preferably a C₁₋₆ alkyl, etc.) such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, 1-methylheptyl, 1-ethylhexyl, n-octyl, 1-methyl-heptyl, nonyl, etc. Examples of the alkenyl group include e.g. a C₂₋₆ alkenyl group such as vinyl, allyl, isopropenyl, 2-methyl-allyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc. Examples of the alkynyl group include e.g. a C₂₋₆ alkynyl group such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, etc.

Examples of the alicyclic hydrocarbon group include e.g. a saturated or unsaturated alicyclic hydrocarbon group such as a cycloalkyl group, a cycloalkenyl group, a cycloalkanedienyl group, etc., preferably cycloalkyl group. Examples of the cycloalkyl group include e.g. a C₃₋₉ cycloalkyl (preferably a C₃₋₈ cycloalkyl) such as cyclopropyl, cyclobutyl, cyclopentyl, cyclohexyl, cycloheptyl, cyclooctyl, cyclononyl, etc., and a fused ring such as 1-indanyl, 2-indanyl, etc. Examples of the cycloalkenyl group include e.g. a C₃₋₆ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl, etc. Examples of the cycloalkanedienyl group include e.g. a C₄₋₆ cycloalkanedienyl group such as 2,4-cyclopentadien-1-yl, 2,4-cyclohexadien-1-yl, 2,5-cyclohexadien-1-yl, etc.

Examples of the aryl group include e.g. a monocyclic or fused polycyclic aromatic hydrocarbon group. Among others, a C₆₋₁₄ aryl group such as phenyl, naphthyl, anthryl, phenathryl, acenaphthylenyl, 4-indanyl, 5-indanyl, etc.. are preferable. In particular, phenyl, 1-naphthyl, 2-naphthyl, etc. are preferable.

Examples of the "non-aromatic heterocyclic group" in the "optionally substituted non-aromatic heterocyclic group" represented by R^{a1} include a 3- to 8-membered (preferably 5- or 6-membered) saturated or unsaturated (preferably saturated) non-aromatic heterocyclic group (alicyclic heterocyclic group) such as oxiranyl, azetidinyl, oxetanyl, thiethanyl, pyrrolidinyl, tetrahydrofuryl, thiolanyl, piperidinyl, tetrahydropyranyl, morpholinyl, thiomorpholinyl, piperazinyl, etc.

Examples of the substituent of the "optionally substituted hydrocarbon group" and "optionally substituted non-aromatic heterocyclic group" represented by R^{a1} include an optionally substituted alkyl group, an optionally substituted alkenyl group, an optionally substituted alkynyl group, an optionally substituted aryl group, an optionally substituted cycloalkyl or cycloalkenyl group, an optionally substituted heterocyclic group, an optionally substituted amino group, an optionally substituted imidoyl group, an optionally substituted amidino group, an optionally substituted hydroxyl group, an optionally substituted thiol group, an optionally esterified carboxyl group, an optionally substituted carbamoyl group, an optionally substituted thiocarbamoyl group, an optionally substituted sulfamoyl group, a halogen atom (e.g. fluorine, chlorine, bromine, iodine, etc., preferably chlorine, bromine, etc.), a cyano group, a nitro group, an acyl group derived from a sulfonic acid, an acyl group derived from an carboxylic acid, an optionally substituted alkyl-sulfinyl group, an optionally substituted aryl-sulfinyl group, etc. The optionally substituted hydrocarbon group and optionally substituted non-aromatic heterocyclic group may have 1 to 5 substituents as described above (preferably 1 to 3 substituents) at any possible position.

Examples of the aryl group in the "optionally substituted aryl group" as the substituent include a C₆₋₁₄ aryl group such as phenyl, naphthyl, anthryl, phenathryl, acenaphthylenyl, etc. Examples of the substituent of the aryl group include a lower alkoxy group which may be substituted by halogen (e.g. a C₁₋₆ alkoxy group such as methoxy, ethoxy, propoxy, etc., a C₁₋₄ alkoxy group substituted by halogen such as fluoromethoxy, difluoromethoxy, trifluoromethoxy, 1,1-difluoroethoxy, 2,2-difluoroethoxy, 3,3-difluoropropoxy, 2,2,3,3,3-pentafluoropropoxy, etc.), an aryloxy which may be substituted (e.g., phenoxy, 4-fluorophenoxy, 2-carbamoylphenoxy, etc.), a halogen atom (e.g., fluorine, chlorine, bromine, iodine, etc.), a lower alkyl group which may be substituted (an unsubstituted C₁₋₆ alkyl group such as methyl, ethyl, propyl, etc., a C₁₋₄ alkyl group substituted by halogen such as fluoromethyl, difluoromethyl, trifluoromethyl, 1,1-difluoroethyl, 2,2-difluoroethyl, 3,3-difluoropropyl, 2,2,3,3,3-pentafluoropropyl, etc.), a C₃₋₈ cycloalkyl (e.g., cyclopropyl, cyclobutyl, cyclopentyl, cyclohexyl, cycloheptyl, etc.), an amino group, a mono-substituted amino (e.g., carbamoylamino, methylsulfonylamino, methylamino, ethylamino, propylamino, etc.), di-substituted amino (e.g., dimethylamino, diethylamino, N-methyl-N-methylsulfonylamino, di(methylsulfonyl)amino, etc.), a carbamoyl group which may be substituted by a C₁₋₆ alkyl (e.g., butylcarbamoyl, etc.), formyl, a C₂₋₆ alkanoyl group (e.g., a C₂₋₆ alkanoyl such as acetyl, propionyl, butyryl, etc.), a C₆₋₁₄ aryl group (e.g., phenyl, naphthyl, etc.), a C₆₋₁₄ aryl carbonyl (e.g., benzoyl, naphthoyl, etc.), a C₇₋₁₃ aralkyl carbonyl (e.g., benzylcarbonyl, naphthylmethylcarbonyl, etc.), a hydroxyl group, an alkanoyloxy (a C₂₋₅ alkanoyloxy such as acetyloxy, propionyloxy, butyryloxy, etc.), a C₇₋₁₃ aralkyl-carbonyloxy (e.g., benzylcarbonyloxy, etc.), a nitro group, a sulfamoyl group which may be substituted (e.g., unsubstituted sulfamoyl group, N-methylsulfamoyl, etc.), an arylthio group which may be substituted (e.g., phenylthio, 4-methylphenylthio, etc.), -N=N-phenyl, a cyano group, an amidino group, a carboxyl group which may be esterified (free carboxyl group, and a C₁₋₄ alkoxy carbonyl such as methoxycarbonyl, ethoxycarbonyl, t-butoxycarbonyl, etc., etc.), a C₁₋₆ alkylthio, a C₁₋₆ alkylsulfinyl, a C₁₋₆ alkylsulfonyl, a C₆₋₁₄ arylthio, a C₆₋₁₄ arylsulfinyl, a C₆₋₁₄ arylsulfonyl, a heterocyclic group which may be substituted (e.g., pyridyl, thienyl, tetrazolyl, morpholinyl, oxazolyl, etc. and those as mentioned below for the definition of heterocyclic group which may be substituted shown as R^{a3}), etc. One or two of these substituents, may be present at any substitutable position.

Examples of the cycloalkyl group in the "optionally substituted cycloalkyl group" as the substituent include e.g.a C₃₋₇ cycloalkyl group such as cyclopropyl, cyclobutyl, cyclopentyl, cyclohexyl, cycloheptyl, etc. Examples of the substitutent of the cycloalkyl groups may have the same number and kind of substituents as those of the above described "optionally substituted aryl group".

Examples of the cycloalkenyl group in the "optionally substituted cycloalkenyl group" as the substituent include e.g. a C₃₋₆ cycloalkenyl group such as cyclopropenyl, cyclobutenyl, cyclopentenyl, cyclohexenyl, etc. Example of the substitutent of the cycloalkenyl groups which may be substituted may have the same number and kind of substituents as those of the above described "optionally substituted aryl group".

Examples of the alkyl group in the "optionally substituted alkyl group" as the substituent include e.g. a C₁₋₆ alkyl etc. such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, etc. Examples of the susbstitutent of the alkyl groups may have the same number and kind of substituents as those of the above described "optionally substituted aryl group".

Examples of the alkenyl group in the "optionally substituted alkenyl group" as the substituent include e.g. a C₂₋₆ alkenyl group such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc. Examples of the substitutents of the alkenyl groups may have the same number and kind of substituents as those of the above described "optionally substituted aryl group".

Examples of the alkynyl group in the "optionally substituted alkynyl group" as the substituent include e.g. a C₂₋₆ alkynyl group such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, etc. Examples of the substituent of the alkynyl groups may have the same kind and number of substituents as those of the above described "optionally substituted aryl group".

Examples of the heterocyclic group in the "optionally substituted heterocyclic group" as the substituent include e.g. an aromatic heterocyclic group, saturated or unsaturated non-aromatic heterocyclic group (alicyclic heterocyclic group) etc., which contains at least one hetero-atom (preferably 1 to 4 hetero-atoms, more preferably 1 to 2 hetero-atoms) consisting of 1 to 3 kinds of hetero-atoms (preferably 1 to 2 kinds of hetero-atoms) selected from an oxygen atom, a sulfur atom, a nitrogen atom, etc.

Examples of the "aromatic heterocyclic group" include an aromatic monocyclic heterocyclic group such as a 5- to 6-membered aromatic monocyclic heterocyclic group (e.g. furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.); an aromatic fused heterocyclic group such as a 8- to 12-membered aromatic fused heterocyclic group (e.g. benzofuranyl, isobenzofuranyl, benzothienyl, indolyl, isoindolyl, 1H-indazolyl, benzindazolyl, benzoxazolyl, 1,2-benzoisooxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.); etc., preferably, a heterocyclic group consisting of the above-mentioned 5- or 6-membered aromatic monocyclic heterocyclic group fused with a benzene ring or a heterocyclic group consisting of the above-mentioned 5- or 6-membered aromatic monocyclic heterocyclic group fused with the same or different above-mentioned 5- or 6-membered aromatic monocyclic heterocyclic group, etc.

Examples of the "non-aromatic heterocyclic group" include e.g. a 3- to 8-membered (preferably 5- or 6-membered) saturated or unsaturated (preferably saturated) non-aromatic heterocyclic group (alicyclic heterocyclic group) such as oxiranyl, azetidinyl, oxetanyl, thiethanyl, pyrrolidinyl, tetrahydrofuryl, thiolanyl, piperidinyl, tetrahydropyranyl, morpholinyl, thiomorpholinyl, piperazinyl, etc.

Examples of the substituent of the "optionally substituted heterocyclic group" as the substituent include a lower alkyl group (e.g. a C₁₋₆ alkyl group such as methyl, ethyl, propyl, etc.), an acyl group (e.g. a C₁₋₆ alkanoyl such as formyl, acetyl, propionyl, pivaloyl, etc., e.g. an aryl carbonyl such as benzoyl, etc., e.g. a C₁₋₆ alkylsulfonyl such as methylsulfonyl, ethylsulfonyl, etc., e.g. a substituted sulfonyl such as aminosulfonyl, methylaminosulfonyl, dimethylaminosulfonyl, etc.), a lower alkyl substituted by a halogen (e.g., trifluoromethyl, 1,1-difluoroethyl, etc.), etc.

Examples of the substituent in the "optionally substituted amino group", "optionally substituted imidoyl group", "optionally substituted amidino group", "optionally substituted hydroxyl group" and "optionally substituted thiol group" as the substituent include e.g. a lower alkyl group (e.g. a C₁₋₆ alkyl group such as methyl, ethyl, propyl, isopropyl, butyl, isobutyl, t-butyl, pentyl, hexyl, etc.), aryl group (e.g., phenyl, 4-methylphenyl, etc.), acyl group (C₁₋₆ alkanoyl (e.g., formyl, acetyl, propionyl, pivaloyl, etc.), e.g. aryl-carbonyl (e.g. benzoyl, etc.), C₁₋₆ alkylsulfonyl (e.g., methylsulfonyl, ethylsulfonyl, etc.), C₆₋₁₄ aryl-sulfonyl (e.g., para-toluenesulfonyl, etc.), etc., substituted-sulfonyl such as aminosulfonyl, methylaminosulfonyl, dimethylaminosulfonyl, etc.), an optionally halogenated C₁₋₆ alkoxy-carbonyl (e.g. trifluoromethoxycarbonyl, 2,2,2-trifluoroethoxycarbonyl, trichloromethoxycarbonyl, 2,2,2-trichloroethoxycarbonyl, etc.), etc. In addition, the "amino group" in the "optionally substituted amino group" as the substituent may be substituted with an optionally substituted imidoyl group (e.g., a C₁₋₆ alkylimidoyl, formimidoyl, amidino, etc.), etc. and two substituents of the "amino group" may form a cyclic amino group together with a nitrogen atom. Examples of the cyclic amino group include e.g. 3- to 8-membered (preferably 5- to 6-membered) cyclic amino group such as 1-azetidinyl, 1-pyrrolidinyl, piperidinol-piperidinyl, morpholino4-morpholinyl, 1-piperazinyl and 1-piperazinyl which may have at the 4-position a lower alkyl group (e.g. a C₁₋₆ alkyl group such as methyl, ethyl, propyl, isopropyl, butyl, t-butyl, pentyl, hexyl, etc.), an aralkyl group (e.g. a C₇₋₁₀ aralkyl group such as benzyl, phenethyl, etc.), an aryl group (e.g. a C₆₋₁₀ aryl group such as phenyl, 1-naphthyl, 2-naphthyl, etc.), etc.

Examples of the "optionally substituted carbamoyl group" include unsubstituted carbamoyl, as well as a N-mono-substituted carbamoyl group and a N,N-di-substituted carbamoyl group.

The "N-mono-substituted carbamoyl group" is a carbamoyl group having one substituent on the nitrogen atom and the substituent include e.g. a lower alkyl group (e.g. a C₁₋₆ alkyl group such as methyl, ethyl, propyl, isopropyl, butyl, isobutyl, t-butyl, pentyl, hexyl, etc.), a cycloalkyl group (e.g. a C₃₋₆ cycloalkyl group such as cyclopropyl, cyclobutyl, cyclopentyl, cyclohexyl, etc.), an aryl group (e.g. a C₆₋₁₀ aryl group such as phenyl, 1-naphthyl, 2-naphthyl, etc.), an aralkyl group (e.g. a C₇₋₁₀ aralkyl group such as benzyl, phenethyl, etc., preferably a phenyl-C₁₋₄ alkyl group etc.), a heterocyclic group (e.g. the same substituent as those in the above described "heterocyclic group" as the substituent of the "optionally substituted hydrocarbon group" represented by R^{a1}, etc.), etc. The the lower alkyl group, the cycloalkyl group, the aryl group, the aralkyl group and the heterocyclic group may have a substituent and examples of the substituent include e.g. a hydroxyl group, an optionally substituted amino group [the amino group may have 1 to 2 substituents (e.g. a lower alkyl group (e.g. a C₁₋₆ alkyl group such as methyl, ethyl, propyl, isopropyl, butyl, isobutyl, t-butyl, pentyl, hexyl, etc.), an acyl group (e.g. a C₁₋₆ alkanoyl such as formyl, acetyl, propionyl, pivaloyl, etc., an arylcarbonyl such as benzoyl, etc., a C₁₋₆ alkylsulfonyl such as methylsulfonyl, ethylsulfonyl, etc.), etc.)], a halogen atom (e.g. fluorine, chlorine, bromine, iodine, etc.), a nitro group, a cyano group, a lower alkyl group optionally substituted with 1 to 5 halogen atoms (e.g. fluorine, chlorine, bromine, iodine, etc.), a lower alkoxy group optionally substituted with 1 to 5 halogen atoms (e.g. fluorine, chlorine, bromine, iodine, etc.), etc. The lower alkyl group includes e.g. a C₁₋₆ alkyl group such as methyl, ethyl, n-propyl, isopropyl, n-butyl, isobutyl, sec-butyl, tert-butyl, pentyl, hexyl, etc. and in particular methyl, ethyl, etc. are preferable. The lower alkoxy group includes e.g. a C₁₋₆ alkoxy group such as methoxy, ethoxy, n-propoxy, isopropoxy, n-butoxy, isobutoxy, sec-butoxy, tert-butoxy, etc. and in particular methoxy, ethoxy, etc. are preferable. It is preferable that one, two or three substituents, more preferably two substituents are present.

The "N,N-di-substituted carbamoyl group" is a carbamoyl group having two substituents on the nitrogen atom. Examples of one of the substituents include the same as those of the above described "N-mono-substituted carbamoyl group" and examples of the other substituent include e.g. a lower alkyl group (e.g. a C₁₋₆ alkyl group such as methyl, ethyl, propyl, isopropyl, butyl, t-butyl, pentyl, hexyl, etc.), a C₃₋₆ cycloalkyl group (e.g. cyclopropyl, cyclobutyl, cyclopentyl, cyclohexyl, etc.), a C₇₋₁₀ aralkyl group (e.g. benzyl, phenethyl, etc., preferably phenyl-C₁₋₄ alkyl group, etc.), etc. In addition, two substituents of the "N,N-di-substituted carbamoyl group" may form a cyclic amino group together with a nitrogen atom. Examples of the cyclic amino-carbamoyl group include e.g. 3- to 8-membered (preferably 5- to 6-membered) cyclic amino-carbamoyl group such as 1-azetidinylcarbonyl, 1-pyrrolidinylcarbonyl, 1-piperidinylcarbonyl, 4-morpholinylcarbonyl, 1-piperazinylcarbonyl and 1-piperazinylcarbonyl which may have at the 4-position a lower alkyl group (e.g. a C₁₋₆ alkyl group such as methyl, ethyl, propyl, isopropyl, butyl, t-butyl, pentyl, hexyl, etc.), an aralkyl group (e.g. a C₇₋₁₀ aralkyl group such as benzyl, phenethyl, etc.), an aryl group (e.g. a C₆₋₁₀ aryl group such as phenyl, 1-naphthyl, 2-naphthyl, etc.), etc.

Examples of the substituent in the "optionally substituted thiocarbamoyl group" include the same substituent as those in the above described "optionally substituted carbamoyl group".
Examples of the "sulfamoyl group which may be substituted" include an unsubstituted-sulfamoyl group, a N-mono-substituted sulfamoyl group and a N,N-di-substituted sulfamoyl group.

The "N-mono-substituted sulfamoyl group" is a sulfamoyl group having one substituent at the nitrogen atom, and examples of the substituent include those mentioned as the substituent of N-mono-substituted carbamoyl group. The "N,N-di-substituted sulfamoyl group" is a sulfamoyl group having two substituents at the nitrogen atom, and examples of the substituent include those mentioned as the substituent of the N,N-di-substituted carbamoyl group.

Examples of the "optionally esterified carboxyl group" include a free carboxyl group as well as a lower alkoxycarbonyl group, an aryloxycarbonyl group, an aralkyloxycarbonyl group, etc.

Examples of the "lower alkoxycarbonyl group" include e.g. a C₁₋₆ alkoxy-carbonyl group such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl, etc. Among them, a C₁₋₃ alkoxy-carbonyl group such as methoxycarbonyl, ethoxycarbonyl, propoxycarbonyl, etc. is preferable.

Examples of the "aryloxycarbonyl group" include e.g. a C₇₋₁₂ aryloxy-carbonyl group such as phenoxycarbonyl, 1-naphthoxycarbonyl, 2-naphthoxycarbonyl, etc.

Examples of the "aralkyloxycarbonyl group" include e.g. a C₇₋₁₀ aralkyloxy-carbonyl group, etc. (preferably, a C₆₋₁₀ aryl-C₁₋₄ alkoxy-carbonyl, etc.) such as benzyloxycarbonyl, phenethyloxycarbonyl, etc.

The "aryloxycarbonyl group" and "aralkyloxycarbonyl group" may be substituted. Examples of the substituent include the same kind and number of the substituents of the aryl group and aralkyl group as examples of the substituent for the above described N-mono-substituted carbamoyl group.

Examples of the "acyl group derived from a sulfonic acid" as the substituent include a sulfonyl group substituted by a hydrocarbon group, and preferably include an acyl group such as C₁₋₁₀ alkyl-sulfonyl, C₂₋₆ alkenyl-sulfonyl, C₂₋₆ alkynyl-sulfonyl, C₃₋₉ cyclo-alkyl-sulfonyl, C₃₋₉ cyclo-alkenyl-sulfonyl, C₆₋₁₄ aryl-sulfonyl, C₇₋₁₀ aralkyl-sulfonyl etc. Examples of the C₁₋₁₀ alkyl include, for example, methyl, ethyl, propyl, isopropyl, butyl, isobutyl, t-butyl, pentyl, hexyl, heptyl, octyl, etc. Examples of the C₂₋₆ alkenyl include, for example, vinyl, allyl, 1-propenyl, isopropenyl, 2-butenyl, 3-butenyl, 2-hexenyl, etc. Examples of C₂₋₆ alkynyl include, for example, ethynyl, 2-propynyl, 2-butynyl, 5-hexynyl, etc. Examples of the C₃₋₉ cyclo-alkyl include, for example, cyclopropyl, cyclobutyl, cyclopentyl, cyclohexyl, cyclooctyl, etc. Examples of the C₃₋₉ cyclo-alkenyl include, for example, 1-cyclopenten-1-yl, 2-cyclopenten-1-yl, 3-cyclopenten-1-yl, 3-cyclohexen-1-yl, 3-cycloocten-1-yl, etc. Examples of the C₆₋₁₄ aryl include, for example, phenyl, 1-naphthyl, 2-naphthyl, etc. Examples of the C₇₋₁₀ aralkyl-sulfonyl include, for example, benzyl, phenethyl, etc.

These hydrocarbon groups bonded to the sulfonyl may be substituted. Examples of the substituent include, for example, hydroxyl, amino which may be substituted [the amino may be substituted with 1 or 2 substituents. (examples of the substituent include lower alkyl group (e.g. C₁₋₆ alkyl etc. such as methyl, ethyl, propyl, isopropyl, butyl, isobutyl, t-butyl, pentyl, hexyl, etc.), an acyl group (e.g., C₁₋₆ alkanoyl such as formyl, acetyl, propionyl, pivaloyl, etc., aryl carbonyl such as benzoyl, etc., C₁₋₆ alkyl-sulfonyl such as methylsulfonyl, ethylsulfonyl, etc.)), halogen atom (for example, fluorine, chlorine, bromine, iodine, etc.), nitro group, cyano group, lower alkyl group which may be substituted by 1 to 5 halogen atom (for example, fluorine, chlorine, bromine, iodine, etc.), lower alkyl group which may be substituted by 1 to 5 halogen atom (for example, fluorine, chlorine, bromine, iodine, etc.) etc.. Examples of the lower alkyl group include, for example, C₁₋₆ alkyl group such as methyl, ethyl, n-propyl, isopropyl, n-butyl, isobutyl, sec-butyl, tert-butyl, pentyl, hexyl, etc., and preferably include methyl, ethyl, etc. Examples of the lower alkoxy group include, for example, C₁₋₆ alkoxy group such as methoxy, ethoxy, n-propoxy, isopropoxy, n-butoxy, isobutoxy, sec-butoxy, tert-butoxy, etc, and preferably include methoxy, ethoxy, etc. These substituents may be the same or different from each other, and one, two or three substituents, preferably one or two substituents may be present.

Examples of the "acyl group derived from a carboxylic acid" as the substituent include a carbonyl group having a hydrogen atom or one substituent which the above described "N-mono-substituted carbamoyl group" have on the nitrogen atom, etc., preferably, a C₁₋₆ alkanoyl such as formyl, acetyl, trifluoroacetyl, propionyl, butyryl, isobutyryl, pivaloyl, etc., an acyl of an aryl-carbonyl such as benzoyl etc.

Examples of the alkyl in "alkyl-sulfinyl group which may be substituted" as a substituent include a lower alkyl, for example, a C₁₋₆ alkyl such as methyl, ethyl, propyl, isopropyl, butyl, isobutyl, t-butyl, pentyl, hexyl, etc. Examples of the aryl in "aryl-sulfinyl group which may be substituted" as a substituent include, for example, a C₆₋₁₄ aryl group etc., such as phenyl, naphthyl, anthryl, phenanthryl, acenaphthylenyl, etc. Examples of the substituent of the alkyl group or the aryl group include a lower alkoxy group (e.g. a C₁₋₆ alkoxy group etc, such as methoxy, ethoxy, propoxy, etc.), a halogen atom (e.g., fluorine, chlorine, bromine, iodine, etc.), a lower alkyl group (a C₁₋₆ alkyl group etc., such as methyl, ethyl, propyl, etc.), amino, hydroxyl, cyano, amidino, etc. One or two of these substituents may be present at any substitutable position.

Examples of "hydrocarbon group which may be substituted" and "non-aromatic heterocyclic group which may be substituted" shown as R^{a2} include the same ones as "hydrocarbon groups which may be substituted" and "non-aromatic heterocyclic group which may be substituted" shown by R^{a1}, respectively. Among them, a C₂₋₆ alkyl which may be substituted and a C₃₋₈ cycloalkyl which may be substituted are preferable.

When R^{a1} and R^{a2} are bind to each other together with the adjacent nitrogen atom to form a heterocyclic ring group which may be substituted, the heterocyclic ring contains one nitrogen atom, and may further contain a nitrogen atom, an oxygen atom and a sulfur atom. Examples of the ring include, for example, a cyclic amino group such a monocyclic ring as 1-azetidinyl, 1-pyrrolidinyl, 1-piperidinyl, 1-homopiperidinyl, heptamethyleneimino, 1-piperazinyl, 1-homopiperazinyl, 4-morpholinyl, 4-thiomorpholinyl, etc., such a fused ring as 2-isoindolinyl, 1,2,3,4-tetrahydro-2-isoquinolyl, 1,2,4,5-tetrahydro-3H-3-benzoazepine-3-yl, etc., such a spiro ring as inden-1-spiro-4'-piperidin-1'-yl, etc. The cyclic amino group may have 1 to 5 substituents, preferably 1 to 3 substituents at chemically substitutable positions on the ring.

Examples of the substituent include a hydroxyl group, a cyano group, a nitro group, an amino group, an oxo group, a halogen atom and a group represented by the formula: -YR^{aa}, wherein R^{aa} is a hydrocarbon group which may be substituted or a heterocyclic group which may be substituted, and Y is a bond (a single bond), -CR^{ab} R^{ac}-, -COO-, -CO-, -CR^{ab}(OH)-, -CO-NR^{ab}-, -CS-NR^{ab}-, -CO-S-, -CS-S-, - CO-NR^{ab}-CO-NR^{ac}-, -C (=NH) -NR^{ab}-, -NR^{ab}-, -NR^{ab}-CO-, -NR^{ab}-CS-, - NR^{ab}-CO-NR^{ac}-, -NR^{ab}-CS-NR^{ac}-, -NR^{ab}-CO-O-, -NR^{ab}-CS-O-, -NR^{ab}-CO-S-, -NR^{ab}-CS-S-, -NR^{ab}-C (=NH) -NR^{ac}-, -NR^{ab}-SO₂-, -NR^{ab}-NR^{ac}-, -O-, -O-CO-, -O-CS-, -O-CO-O, -O-CO-NR^{ab}-, -O-C(=NH)-NR^{ab}-, -S-, -SO-, -SO₂-, -CR^{ab}R^{ac}-S-, CR^{ab}R^{ac}- SO₂-, -SO₂-NR^{ab}-, - S-CO-, -S-CS-, -S-CO-NR^{ab}-, -S-CS-NR^{ab}-, -S-C (=NH) -NR^{ab}-,wherein each of R^{ab} and R^{ac} is a hydrogen atom, an alkyl group which may be substituted, an alkenyl group which may be substituted, an alkynyl group which may be substituted, an aryl group which may be substituted, a cycloalkyl group which may be substituted, a cycloalkenyl group which may be substituted, a heterocyclic group which may be substituted, an acyl group derived from sulfonic acid, an acyl group derived from carboxylic acid, etc.

Examples of the "hydrocarbon group" in the "optionally substituted hydrocarbon group" represented by R^{aa} include e.g. an aliphatic hydrocarbon group, an alicyclic hydrocarbon group and an aryl group, etc. Examples of the aliphatic hydrocarbon group, the alicyclic hydrocarbon group and the aryl group include those represented by R^{a1}. Examples of the substituent of the "hydrocarbon group optionally substituted" include the same substituent as those in the above described "hydrocarbon group which may be substituted" represented by R^{a1}.

Examples of the heterocyclic group in the "heterocyclic group which may be substituted" represented by R^{aa} include the same heterocyclic group as those of "heterocyclic group which may be substituted" represented by R^{a3} mentioned below. Examples of the "substituent" in the "heterocyclic group that may be substituted" include those mentioned as the "substituent" in a "non-aromatic heterocyclic group that may be substituted" represented by R^{a1}.

Examples of the "alkyl group which may be substituted", "alkenyl group which may be substituted", "alkynyl group which may be substituted", "aryl group which may be substituted", "cyclo-alkyl group which may be substituted", "cyclo-alkenyl group which may be substituted", "heterocyclic group which may be substituted", "acyl group derived from sulfonic acid", and "acyl group derived from carboxylic acid", each of which is represented by R^{ab} and R^{ac}, include those mentioned as the substituent in the "hydrocarbon group which may be substituted" represented by R^{a1}.

R^{a1} and R^{a2} preferably bind to each other together with the nitrogen atom to form a heterocyclic ring that may be substituted. More preferably, NR^{a1}R^{a2} is a group represented by the formula: 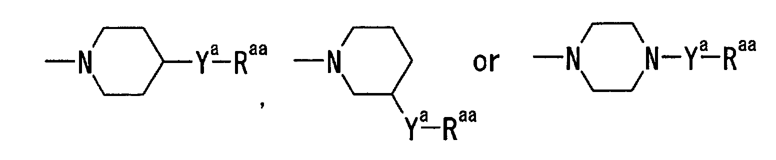 wherein Y^{a} and R^{aa} have the same meanings given above. In the above, while Y^{a} and R^{aa} have the same meanings given above, R^{aa} is more preferably an aryl group which may be substituted or a heterocyclic group which may be substituted.

Examples of a "cyclic hydrocarbon group" in the "cyclic hydrocarbon group which may be substituted" represented by R^{a3} include e.g. an alicyclic hydrocarbon group, an aryl group, etc.

Examples of the "alicyclic hydrocarbon group" include e.g. a saturated or unsaturated alicyclic hydrocarbon group such as a cycloalkyl group, a cycloalkenyl group, a cycloalkanedienyl group, etc., preferably a cycloalkyl group.

Examples of the "cycloalkyl group" include e.g. a C₃₋₉ cycloalkyl, (preferably a C₃₋₈ acycloalkyl, etc.) such as cyclopropyl, cyclobutyl, cyclopentyl, cyclohexyl, cycloheptyl, cyclooctyl, cyclononyl, etc., and a fused ring such as 1-indanyl, 2-indanyl, etc. Examples of the "cycloalkenyl group" include e.g. a C₃₋₆ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl, etc. Examples of the "cycloalkanedienyl group" include e.g. a C₄₋₆ cycloalkanedienyl group such as 2,4-cyclopentadien-1-yl, 2,4-cyclohexadien-1-yl, 2,5-cyclohexadien-1-yl, etc.

Examples of the "aryl group" include e.g. a monocyclic or fused polycyclic aromatic hydrocarbon group. Among them, a C₆₋₁₄ aryl group such as phenyl, naphthyl, anthryl, phenathryl, acenaphthyl, etc. is preferable. In particular, phenyl, 1-naphthyl, 2-naphthyl, etc. are preferable.

Examples of the substituent in the "cyclic hydrocarbon group which may be substituted" represented by R^{a3} include those mentioned as the substituent in the "hydrocarbon group which may be substituted" represented by R^{a1}. The substituent include, for example, a phenyl group, a phenyl group which may be substituted by a C₁₋₆ alkyl group such as a tolyl group, etc., a naphthyl group, etc., when the cyclic hydrocarbon group is alicyclic hydrocarbon group, and is preferably, for example a halogen atom (e.g., chlorine atom, fluorine atom, etc.), a C₁₋₆ alkyl group (methyl, ethyl, propyl, isopropyl, butyl, isobutyl, t-butyl, pentyl, hexyl, etc.), a C₁₋₆ alkoxy group (e.g., methoxy, ethoxy, n-propoxy, isopropoxy, n-butoxy, etc.), a C₃₋₆ cyclo-alkyl group (e.g., cyclopropyl, cyclobutyl, cyclopentyl, cyclohexyl, etc.), a halogenated-C₁₋₆ alkyl group (trifluoromethyl, etc.), a halogenated-C₁₋₆ alkoxy group (trifluoromethyloxy, etc.), a C₁₋₆ alkyl-thio group (methylthio, ethylthio, etc.), a C₁₋₆ alkyl-sulfonyl group (methylsulfonyl, ethylsulfonyl, etc.), cyano group, nitro group, etc., when the cyclic hydrocarbon group is an aryl group.

Examples of the heterocyclic group in the "optionally substituted heterocyclic group" represented by R^{a3} include e.g. an aromatic heterocyclic group, a saturated or unsaturated non-aromatic heterocyclic group (an alicyclic heterocyclic group) etc., which contains at least one hetero-atom (preferably 1 to 4 hetero-atoms, more preferably 1 to 2 hetero-atoms) consisting of 1 to 3 kinds of hetero-atoms (preferably 1 to 2 kinds of hetero-atoms) selected from an oxygen atom, a sulfur atom, a nitrogen atom, etc. as atom(s) which form a ring (ring atoms).

Examples of the "aromatic heterocyclic group" include an aromatic monocyclic heterocyclic group such as a 5- to 6-membered aromatic monocyclic heterocyclic group, etc. (e.g. furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.); an aromatic fused heterocyclic group such as a 8- to 12-membered aromatic fused heterocyclic group (e.g. benzofuranyl, isobenzofuranyl, benzothienyl, indolyl, isoindolyl, 1H-indazolyl, benzindazolyl, benzoxazolyl, 1,2-benzoisooxazolyl, benzothiazolyl, benzopyranyl, 1,2-benzoisothiazolyl, benzodioxolyl,benxoimidazolyl, 2,1,1-benzoxadiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, pyrazolo[3,4-b]piridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.); etc., preferably, a heterocyclic group consisting of the above-mentioned 5- or 6-membered aromatic monocyclic heterocyclic group fused with a benzene ring or a heterocyclic group consisting of the above-mentioned 5- or 6-membered aromatic monocyclic heterocyclic group fused with the same or different above-mentioned 5- or 6-membered aromatic monocyclic heterocyclic group, etc.

Examples of the "non-aromatic heterocyclic group" include a 3- to 8-membered (preferably 5- or 6-membered) saturated or unsaturated (preferably saturated) non-aromatic heterocyclic group (alicyclic heterocyclic group) such as oxiranyl, azetidinyl, oxetanyl, thiethanyl, pyrrolidinyl, tetrahydrofuryl, thiolanyl, piperidinyl, tetrahydropyranyl, morpholinyl, thiomorpholinyl, piperazinyl, etc.

Examples of the substituent in the "heterocyclic group which may be substituted" represented by R^{a3} include those mentioned as the substituent "non-aromatic heterocyclic group which may be substituted" represented by R^{a1}. R^{a3} is preferably a phenyl group that may be substituted.

Examples of the "hydrocarbon group which may be substituted" represented by R^{a4} include the same "hydrocarbon group which may be substituted" represented by R^{a1}. Examples of the "heterocyclic group which may be substituted" represented by R^{a4} include the same "heterocyclic group which may be substituted" represented by R^{a3}.

Examples of the "alkoxy group" in "alkoxy group which may be substituted" represented by R^{a4} preferably include, for example, a C₁₋₆ alkoxy such as methoxy, ethoxy, n-propoxy, isopropoxy, n-butoxy, isobutoxy, sec-butoxy, tert-butoxy, etc. Examples of the "substituent" include, for example, a cycloalkyl group (e.g. a C₃₋₆ cycloalkyl group such as cyclopropyl, cyclobutyl, cyclopentyl, cyclohexyl, etc.), an aryl group (e.g.a C₆₋₁₀ aryl group, etc., such as phenyl, 1-naphthyl, 2-naphthyl, etc.), an aralkyl group (e.g.a C₇₋₁₀ aralkyl group for example, such as benzyl, phenethyl, etc., preferably a phenyl-C₁₋₄ alkyl group, etc.), a heterocyclic group (e.g., a "heterocyclic group" mentioned as the substituent in the "hydrocarbon group which may be substituted" represented by R^{a1}), etc. Each of the lower alkyl group, the cycloalkyl group, the aryl group, the aralkyl group and the heterocyclic group may be substituted. Examples of the substituents include, for example, a hydroxyl group, an amino group which may be substituted [the amino group may have 1 or 2 substituents such as a lower alkyl group (a C₁₋₆ alkyl group such as methyl, ethyl, propyl, isopropyl, butyl, isobutyl, t-butyl, pentyl, hexyl, etc.), an acyl group (a C₁₋₆ alkanoyl such as formyl, acetyl, propionyl, pivaloyl, etc., an aryl carbonyl such as benzoyl, etc. for example, a C₁₋₆ alkyl-sulfonyl etc. such as methyl-sulfonyl, ethyl-sulfonyl etc.)], a halogen atom (e.g. fluorine, chlorine, bromine, iodine etc.), a nitro group, a cyano group, a lower alkyl group (which may be substituted by 1 to 5 halogen atoms (e.g. fluorine, chlorine, bromine, iodine etc.)), a lower alkoxy group (which may be substituted by 1 to 5 halogen atoms (e.g. fluorine, chlorine, bromine, iodine etc.)), etc. Examples of the lower alkyl group include a C₁₋₆ alkyl group such as methyl, ethyl, n-propyl, isopropyl, n-butyl, isobutyl, sec-butyl, tert-butyl, pentyl, hexyl, etc., and in particular methyl, ethyl, etc. are preferable. Examples of the lower alkoxy group include, for example, a C₁₋₆ alkoxy group etc. such as methoxy, ethoxy, n-propoxy, isopropoxy, n-butoxy, isobutoxy, sec-butoxy, tert-butoxy, etc., and in particular methoxy, ethoxy, etc. are preferable. The above described lower alkoxy group may have 1 or 2 to 3 (preferably 1 or 2) substituents. When the alkoxy group has 2 or 3 substituents, these substituents may be the same or different.

Examples of the "aryl group" in "aryloxy group which may be substituted" represented by R^{a4} include, for example, a C₆₋₁₄ aryl group such as phenyl, naphthyl, anthryl, phenanthryl, acenaphthylenyl, etc. Examples of the substituent include, for example, a lower alkoxy group (e.g. a C₁₋₆ alkoxy group such as methoxy, ethoxy, propoxy, etc.), a halogen atom (e.g., fluorine, chlorine, bromine, iodine, etc.), a lower alkyl group (for example a C₁₋₆ alkyl group such as methyl, ethyl, propyl, etc.), an amino group, a hydroxyl group, a cyano group, an amidino group, etc. These optional aryloxy groups may have 1 or 2 at any possible position.

Examples of the "substituent" in "amino group which may be substituted" represented by R^{a4} include, for example, a lower alkyl group (e.g., a C₁₋₆ alkyl group such as methyl, ethyl, propyl, isopropyl, butyl, isobutyl, t-butyl, pentyl, hexyl, etc.), an acyl group (a C₁₋₆ alkanoyl (e.g., formyl, acetyl, propionyl, pivaloyl, etc.), benzoyl, etc.), a C₁₋₆ alkoxy-carbonyl which may be halogenated (e.g., trifluoromethoxycarbonyl, 2,2,2-trifluoroethoxycarbonyl, trichloromethoxy carbonyl, 2,2,2-trichloroethoxy carbonyl, etc.), etc. In addition, the "amino group" in the "optionally substituted amino group" as the substituent may be substituted with an optionally substituted imidoyl group (e.g., a C₁₋₆ alkylimidoyl, formimidoyl, amidino, etc.), etc. and two substituents of the "amino group" may form a cyclic amino group together with a nitrogen atom. Examples of the cyclic amino group include e.g. a 3- to 8-membered (preferably 5- to 6-membered) cyclic amino group such as 1-azetidinyl, 1-pyrrolidinyl, 1-piperidinyl, 4-morpholinyl, 1-piperazinyl and 1-piperazinyl which may have at the 4-position a lower alkyl group (e.g. a C₁₋₆ alkyl group etc. such as methyl, ethyl, propyl, isopropyl, butyl, t-butyl, pentyl, hexyl, etc.), an aralkyl group (e.g. a C₇₋₁₀ aralkyl group etc. such as benzyl, phenethyl, etc.), an aryl group (e.g. a C₆₋₁₀ aryl group etc. such as phenyl, 1-naphthyl, 2-naphthyl, etc.), etc. R^{a4} is preferably a C₁₋₃ alkyl, a phenyl which may be substituted, 3-pyridyl, 4-pyridyl, etc.

Examples of the hydrocarbon group represented by R^{a5} include those mentioned as a "hydrocarbon group" in the "hydrocarbon group that may be substituted" represented by R^{a1}. The preferable examples of the hydrocarbon group include a lower alkyl group having 1 to 4 carbon atoms such as methyl, ethyl, n-propyl, isopropyl, butyl, n-butyl, isobutyl, tert-butyl, etc.

Examples of the counter anion represented by Y^{a}- include, for example, Cl⁻, Br⁻, I⁻, NO₃⁻, SO₄²⁻, PO₄³⁻, CH₃SO₃⁻, etc.

Examples of the divalent aliphatic hydrocarbon group in the divalent alipahatic hydrocarbon group which may be substituted by group other than an oxo group represented by E^{a} include, for example, a C₁₋₆ alkylene such as methylene, ethylene, etc., a C₂₋₆ alkenylene such as ethenylene, etc., a C₂₋₆ alkynylene such as ethynylen, etc., and among them, a C₂₋₅ alkylene is more preferable and trimethylene is the most preferable.

The substituent of the divalent hydrocarbon group is a substituent other than an oxo group, and examples of the substituents include, for example, an alkyl group which may be substituted, an aryl group which may be substituted, a cycloalkyl group or a cycloalkenyl group which may be substituted, a carboxyl group which may be esterified, a carbamoyl group or a thiocarbamoyl group which may be substituted, an amino group which may be substituted, a hydroxyl group which may be substituted, a thiol (i.e. mercapto) group which may be substituted, an acyl group derived from carboxylic acid, an acyl group derived from sulfonic acid, a halogen (e.g., fluorine, chlorine, bromine, etc.), nitro, cyano, etc. The divalent hydrocarbon group may have 1 to 3 substituents. Each of the alkyl group or the aryl group which may be substituted, the cycloalkyl group or the cycloalkenyl group which may be substituted, the carboxyl group which may be esterified, the carbamoyl group which may be substituted, the thiocarbamoyl group which may be substituted, the amino group which may be substituted, the hydroxyl group which may be substituted, the thiol group (i.e. mercapto group) which may be substituted, the acyl group derived from carboxylic acid and the acyl group derived from sulfonic acid include those mentioned as the substituent in "heterocyclic group which may be substituted" represented by R^{a3}.

Examples of the C₁₋₃ aliphatic hydrocarbon group in "divalent C₁₋₃aliphatic hydrocarbon group which may be substituted" represented by Q^{a} and R^{a} inclde a divalent aliphatic hydrocarbon group having 1 to 3 carbon atoms in the divalent aliphatic ydrocarbon group in divalent aliphatic hydrocarbon group which may be substituted by a group other than an oxo group represented by E^{a}.

Examples of the substituent in the "divalent C₁₋₃ aliphatic ydrocarbon group which may be substituted" represented by Q^{a} and R^{a} include those mentioned as the substituent in divalent aliphatic ydrocarbon group which may be substituted by a group other than an oxo group represented by E^{a}.

J^{a} is a methine or a nitrogen atom, and methine is preferable.

G^{a1} is a bond, CO or SO₂, and CO or SO₂ is preferable.

G^{a2} is CO, SO₂, NHCO, CONH or OCO, and among them, CO, NHCO and OCO are preferable. The compound of the formula (I) or a salt thereof may be a hydrate.

The following compounds are preferable.

(I-1) A compound as shown in the above (1), wherein R^{a1} is a hydrogen atom, a hydrocarbon group selected from Group a2 which may be substituted by member(s) selected from Group a1, a 3- to 8-membered saturated or unsaturated non-aromatic heterocyclic group which may be substituted by member(s) selected from Group a1; R^{a2} is a hydrocarbon group selected from Group a2 which may be substituted by member(s) selected from Group a1 or a 3- to 8-membered saturated or unsaturated non-aromatic heterocyclic group which may be substituted by member(s) selected from Group a1, or R^{a1} and R^{a2} may combine each other together with A^{a} to form a heterocyclic group selected from Group a4 which may be substituted by member(s) selected from Group a3; A^{a} is N or N⁺-R^{a5} · Y^{a-} (Y^{a}- is Cl⁻, Br⁻,. I⁻, NO₃⁻, SO₄²⁻, PO₄³⁻ or CH₃SO₃⁻; R^{a5} is a hydrocarbon group selected from Group a2); R^{a3} is a cyclic hydrocarbon group selected from Group a5 which may be substituted by member(s) selected from Group a1 or a heterocyclic group selected from Group a6 which may be substituted by member(s) selected from Group a1; R^{a4} is a hydrogen atom, a hydrocarbon group selected from Group a2 which may be substituted by member(s) selected from Group a1, a heterocyclic group, selected from Group a6 which may be substituted by member(s) selected from Group a1, a C₁₋₆ alkoxy group which may be substituted by member(s) selected from Group a7, a C₆₋₁₄ aryloxy group which may be substituted by member(s) selected from Group a8, an amino group which may be substituted by member(s) selected from Group a9 or a cyclic-amino group selected from Group a10; E^{a} is a divalent aliphatic hydrocarbon group selected from Group a12 which may be substituted by member(s) other than oxo group(s) and selected from Group a11; each of Q^{a} and R^{a} is a bond or a divalent C₁₋₃ aliphatic ydrocarbon group selected from Group a13 which may be substituted by member(s) selected from Group a11.

### Group a1

(1) a C₁₋₆ alkyl group which may be substituted by member(s) selected from Group a14, (2) a C₂₋₆ alkenyl group which may be substituted by member(s) selected from Group a14, (3) a C₂₋₆ alkynyl group which may be substituted by member(s) selected from Group a14, (4) a C₆₋₁₄ aryl group which may be substituted by member(s) selected from Group a14, (5) a C₃₋₇ cycloalkyl group which may be substituted by member(s) selected from Group a14, (6) a C₃₋₆ cycloalkenyl group which may be substituted by member(s) selected from Group a14, (7) a heterocyclic group selected from Group a16 which may be substituted by member(s) selected from Group a15, (8) an amino group which may be substituted by a C₁₋₆ alkyl-imidoyl(s), formyl-imidoyl(s), amidino(s) or member(s) selected from Group a17, (9) a cyclic-amino group selected from Group a10, (10) an imidoyl group which may be substituted by member(s) selected from Group a17, (11) an amidino group which may be substituted by member(s) selected from Group a17, (12) a hydroxyl group which may be substituted bya member selected from Group a17, (13) a thiol group which may be substituted by a member selected from Group a17, (14) a carboxyl group, (15) a C₁₋₆ alkoxy-carbonyl group which may be substituted by member(s) selected from Group a18, (16) a C₇₋₁₂ aryloxy-carbonyl group which may be substituted by member (s) selected from Group a18, (17) a C₇₋₁₀ aralkyl-oxy-carbonyl group which may be substituted by member(s) selected from Group a18, (18) a carbamoyl group, (19) a mono-substituted carbamoyl group which may be substituted by a member selected from Group a19, (20) a di-substituted carbamoyl group substituted by a member selected from Group a19 and a member selected from Group a20, (21) a cyclic-aminocarbamoyl group selected from Group a21, (22) a thiocarbamoyl group, (23) a mono-substituted thiocarbamoyl group which may be substituted by a member selected from Group a19, (24) a di-substituted thiocarbamoyl group substituted by a member selected from Group a19 and a member selected from Group a20, (25) a cyclic-aminothiocarbamoyl group selected from Group a21, a sulfamoyl group, (26) a N-mono-substituted sulfamoyl group substituted by a member selected from Group a19, (27) a N,N-di-substituted sulfamoyl group substituted by a member selected from Group a19 and a member selected from Group a20, (28) a cyclic-amino-sulfonyl group selected from Group a22, (29) a halogen atom, (30) a cyano group, (31) a nitro group, (32) an acyl group derived from a sulfonic acid selected from Group a22, (33) a formyl group, (34) a C₂₋₆ alkanoyl group, (35) a C₇₋₁₂ aryl-carbonyl group, (36) a C₁₋₆ alkyl-sulfinyl group which may be substituted by member(s) selected from Group a23 and (37) a C₆₋₁₄ aryl-sulfinyl group which may be substituted by member(s) selected from Group a23

### Group a2

(1) a C₁₋₁₀ alkyl group, (2) a C₂₋₆ alkenyl group, (3) a C₂₋₆ alkynyl group, (4) a C₃₋₉ cycloalkyl group which may be condensed with benzene, (5) a C₃₋₆ cycloalkenyl group, (6) a C₄₋₆ cycloalkadienyl group and (7) a C₆₋₁₄ aryl group

### Group a3

(1) a hydroxy group, (2) a cyano group, (3) a nitro group, (4) an amino group, (5) an oxo group, (6) a halogen atom and (7) a group represented by the formula:-B¹R^{aa} [wherein R^{aa} is a hydrocarbon group selected from Group a2 which may be substituted by member(s) selected from Group a1, or a heterocyclic group selected from Group a6 which may be substituted by member(s) selected from Group a1, B¹ is a bond (a single bond), -CR^{ab}R^{ac}-, -COO-, -CO-, -CR^{ab}(OH) -, -CR^{ab}R^{ac}-S-, -CR^{ab}R^{ac}-SO₂-, -CO-NR^{ab}-, -CS-NR^{ab}-, -CO-S-, -CS-S-, -CO-NR^{ab}-CO-NR^{ac}-, -C (=NH) -NR^{ab}-, -NR^{ab}-, -NR^{ab}-CO-, -NR^{ab}-CS-, -NR^{ab}-CO-NR^{ac}-, -NR^{ab}-CS-NR^{ac}-, -NR^{ab}-CO-O-, -NR^{ab}-CS-O-, - NR^{ab}-CO-S-, -NR^{ab}-CS-S-, -NR^{ab}-C (=NH) -NR^{ac}-, -NR^{ab}-SO₂-, -NR^{ab}-NR^{ac}-, -O-, -O-CO-, -O-CS-, -O-CO-O-, -O-CO-NR^{ab}-, -O-C(=NH)-NR^{ab}-, -S-, -SO-, -SO₂-, -SO₂-NR^{ab}-, -S-CO-, -S-CS-, -S-CO-NR^{ab}-, -S-CS-NR^{ab}-and-S-C (=NH)-NR^{ab}- (wherein each of R^{ab} and R^{ac} is a hydrogen atom, a C₁₋₆ alkyl group which may be substituted by member (s) selected from Group a14, a C₂₋₆ alkenyl group which may be substituted by member(s) selected from Group a14, a C₂₋₆ alkynyl group which may be substituted by member(s) selected from Group a14, a C₆₋₁₄ aryl group which may be substituted by member(s) selected from Group a14, a C₃₋₇ cycloalkyl group which may be substituted by member(s) selected from Group a14, a C₃₋₆ cycloalkenyl group which may be substituted by member(s) selected from Group a14, a heterocyclic group selected from Group a6 which may be substituted by member(s) selected from Group a1, an acyl group derived from a sulfonic acid selected from Group a22, a C₁₋₆ alkanoyl, and a C₇₋₁₂ aryl-carbonyl group)]

### Group a4

(1) a monocyclic heterocyclic group, (2) a heterocyclic group condensed with benzene and (3) a heterocyclic spiro compound, each of which contains one nitrogen atom and may further contain one or more atoms selected from the group consisting of a nitrogen atom, a oxygen atom and a sulfur atom

### Group a5

(1) a C₃₋₉ cycloalkyl which may be condensed with benzene, (2) a C₃₋₆ cycloalkenyl group, (3) a C₄₋₆ cycloalkadienyl group and (4) a C₆₋₁₄ aryl group

### Group a6

(1) a 5- to 6-membered aromatic monocyclic heterocyclic group selected from Group a24, (2) a 8- to 12-membered aromatic condensed heterocyclic group selected from Group a26 and (3) a 3- to 8-membered saturated or unsaturated non-aromatic heterocyclic group (aliphatic heterocyclic group) selected from Group a25, each of which contains at least one hetero atom of one to three hetero atoms selected from the group consisting of an oxygen atom, a sulfur atom and a nitrogen atom etc. as a ring formed of atoms (ring atoms).

### Group a7

a C₃₋₆ cycloalkyl group which may be substituted by member (s) selected from Group a18, a C₆₋₁₀ aryl group which may be substituted by member(s) selected from Group a18, a C₇₋₁₀ aralkyl group which may be substituted by member (s) selected from Group a18 and a heterocyclic group selected from Group a16 which may be substituted by member(s) selected from Group a18

### Group a8

a C₁₋₆ alkoxy group, a halogen atom, a C₁₋₆ alkyl group, an amino group, a hydroxyl group, a cyano group and an amidino group

### Group a9

(1) a C₁₋₆ alkyl group, (2) a C₁₋₆ alkanoyl, (3) benzoyl, (4) a C₁₋₆ alkoxy-carbonyl which may be substituted by halogen(s), (5) a C₁₋₆ alkyl-imidoyl, (6) formyl-imidoyl and (7) amidino

### Group a10

(1) 1-azetidinyl, (2) 1-pyrrolidinyl, (3) 1-piperidinyl, (4) 4-morpholinyl and (5) a 1-piperazinyl which may be substituted by member(s) selected from Group a27

### Group a11

(1) a C₁₋₆ alkyl group which may be substituted by member(s) selected from Group a14, (2) a C₆₋₁₄ aryl group which may be substituted by member(s) selected from Group a14, (3) a C₃₋₇ cycloalkyl group which may be substituted by member(s) selected from Group a14, (4) a C₃₋₆ cycloalkenyl group which may be substituted by member(s) selected from Group a14, (5) a carboxyl group, (6) a C₁₋₆ alkoxy-carbonyl group which may be substituted by member(s) selected from Group a18, (7) a C₇₋₁₂ aryloxy-carbonyl group which may be substituted by member(s) selected from Group a18, (8) a C₇₋₁₀ aralkyl-oxy-carbonyl group which may be substituted by member(s) selected from Group a18, (9) a carbamoyl group, (10) a mono-substituted carbamoyl group substituted by a member selected from Group a19, (11) a di-substituted carbamoyl group substituted by a member selected from Group a19 and a member selected from Group a20, (12) a cyclic-aminocarbamoyl group selected from Group a21, (13) a thiocarbamoyl group, (14) a mono-substituted thiocarbamoyl group substituted by a member selected from Group a19, (15) a di-substituted thiocarbamoyl group substituted by a member selected from Group a19 and a member selected from Group a20, (16) a cyclic-aminothiocarbamoyl group selected from Group a21, (17) an amino group which may be substituted by a C₁₋₆ alkyl-imidoyl(s), formyl-imidoyl(s), amidino(s) or member(s) selected from Group a17, (18) a cyclic-amino group selected from Group a10, (19) a hydroxyl group which may be substituted by a member selected from Group a17, (20) a thiol group which may be substituted by a member selected from Group a17, (21) a C₁₋₆ alkanoyl group, (22) a C₇₋₁₂ aryl-carbonyl group, (23) an acyl group derived from a sulfonic acid selected from Group a22, (24) a halogen, (25) nitro and (26) cyano

### Group a12

a C₁₋₆ alkylene, a C₂₋₆ alkenylene and a C₂₋₆ alkynylene

### Group a13

a C₁₋₃ alkylene, a C₂₋₃ alkenylene and a C₂₋₃ alkynylene

### Group a14

(1) a C₁₋₆ alkoxy group which may be substituted by halogen(s), (2) a phenoxy which may be substituted by halogen(s) or carbamoyl(s), (3) a halogen atom, (4) a C₁₋₆ alkyl group, (5) a C₁₋₄ alkyl group substituted by halogen (s), (6)C₃₋₈ cycloalkyl, (7) an amino group, (8) an amino group substituted by one or two members selected from the group consisting of carbamoyl, C₁₋₄ alkyl and C₁₋₄ alkyl-sulfonyl, (9) a carbamoyl group which may be substituted by C₁₋₆ alkyl(s), (10) formyl, (11) a C₂₋₆ alkanoyl group, (12) a C₆₋₁₄ aryl group, (13) a C₆₋₁₄ aryl-carbonyl, (14) a C₇₋₁₃ aralkyl-carbonyl, (15) a hydroxyl group, (16) a C₂₋₅ alkanoyl-oxy, (17) a C₇₋₁₃ aralkyl-carbonyloxy, (18) a nitro group, (19) a sulfamoyl group, (20) a N-C₁₋₄ alkyl-sulfamoyl, (21) a phenyl-thio, (22) a C₁₋₄ alkyl-phenylthio, (23) -N=N-phenyl, (24) a cyano group, (25) an oxo group, (26) an amidino group, (27) a carboxyl group, (28) a C₁₋₄ alkoxy-carbonyl group, (29) a C₁₋₆ alkyl-thio, (30) a C₁₋₆ alkyl-sulfinyl, (31) a C₁₋₆ alkyl-sulfonyl, (32) a C₆₋₁₄ aryl-thio, (33) a C₆₋₁₄ aryl-sulfinyl, (34) a C₆₋₁₄ aryl-sulfonyl and (35) a heterocyclic group selected from Group a6

### Group a15

a C₁₋₆ alkyl group, a C₁₋₆ alkanoyl, a C₇₋₁₃ aryl-carbonyl, a C₁₋₆ alkyl-sulfonyl, an aminosulfonyl, a mono-C₁₋₆ alkyl-aminosulfonyl, a di-C₁₋₆ alkyl-aminosulfonyl and a C₁₋₄ alkyl group substituted by halogen

### Group a16

(1) an aromatic heterocyclic group selected from Groups 24 and 26, and (2) a saturated or unsaturated non-aromatic heterocyclic group selected from Group a25, each of which contains at least one hetero atom of one to three hetero atoms selected from the group consisting of an oxygen atom, a sulfur atom and a nitrogen atom as ring constituting atom(s) (a ring atom)

### Group a17

(1) a C₁₋₆ alkyl group which may be substituted by halogen or a C₁₋₆ alkoxy, (2) a C₆₋₁₂ aryl group, (3) a C₆₋₁₂ aryl group substituted by C₁₋₄ alkyl (s), (4) a C₃₋₈ cycloalkyl group which may be substituted by halogen(s) or C₁₋₆ alkoxy (s), (5) a C₁₋₆ alkoxy group, (6) a C₁₋₆ alkanoyl, (7) a C₇₋₁₃ aryl-carbonyl, (8) a C₇₋₁₃ aryl-carbonyl substituted by C₁₋₄ alkyl(s), (9) a C₁₋₆ alkyl-sulfonyl, (10) a C₆₋₁₄ aryl-sulfonyl, (11) a aminosulfonyl, (12) a mono- or di-substituted aminosulfonyl substituted by C₁₋₄ alkyl(s) and (13) a C₁₋₆ alkoxy-carbonyl which may be substituted by halogen(s)

### Group a18

(1) a hydroxyl group, (2) an amino group, (3) a mono or di-substituted amino group substituted by member(s) selected from Group a28, (4) a halogen atom, (5) a nitro group, (6) a cyano group, (7) a C₁₋₆ alkyl group which may be substituted by halogen atom(s) and (8) a C₁₋₆ alkoxy group which may be substituted by halogen atom(s)

### Group a19

a C₁₋₆ alkyl group which may be substituted by member(s) selected from Group a18, a C₃₋₆ cycloalkyl group which may be substituted by member(s) selected from Group a18, a C₆₋₁₀ aryl group which may be substituted by member(s) selected from Group a18, a C₇₋₁₀ aralkyl group which may be substituted by member(s) selected from Group a18, a C₁₋₆ alkoxy group which may be substituted by member(s) selected from Group a18 and a heterocyclic group selected from Group a16 which may be substituted by member(s) selected from Group a18

### Group a20

a C₁₋₆ alkyl group, a C₃₋₆ cycloalkyl group and a C₇₋₁₀ aralkyl group

### Group a21

a 1-azetidinyl-carbonyl, a 1-pyrrolidinyl-carbonyl, a 1-piperidinyl-carbonyl, a 4-morpholinyl-carbonyl and a 1-piperazinyl-carbonyl which may be substituted by member(s) selected from Group a27

### Group a22

a C₁₋₁₀ alkyl-sulfonyl which may be substituted by member(s) selected from Group a18, a C₂₋₆ alkenyl-sulfonyl which may be substituted by member(s) selected from Group a18, a C₂₋₆ alkynyl-sulfonyl which may be substituted by member(s) selected from Group a18, a C₃₋₉ cycloalkylsulfonyl which may be substituted by member(s) selected from Group a18, a C₃₋₉ cycloalkenyl-sulfonyl which may be substituted by member(s) selected from Group a18, a C₆₋₁₄ aryl-sulfonyl which may be substituted by member(s) selected from Group a18 and a C₇₋₁₀ aralkyl-sulfonyl group which may be substituted by member(s) selected from Group a18

### Group a23

a C₁₋₆ alkoxy group, a halogen atom, a C₁₋₆ alkyl group, an amino group, a hydroxyl group, a cyano group and an amidino group

### Group a24

furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl

### Group a25

oxylanyl, azetidinyl, oxetanyl, thietanyl, pyrrolidinyl, tetrahydro furyl, thiolanyl, piperidinyl, tetrahydropyranyl, morpholinyl, thiomorpholinyl and piperazinyl

### Group a26

benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, benzodioxolyl, benzimidazolyl, 2,1,1-benzoxadiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenathrolinyl, indolizinyl, pyrrolo[1,2-b]pyridazinyl, pyrazolo[1,5-a]pyridyl, pyrazolo[3,4-b]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl

### Group a27

a C₁₋₆ alkyl group, a C₇₋₁₀ aralkyl group and a C₆₋₁₀ aryl group

### Group a28

a C₁₋₆ alkyl group, a C₁₋₆ alkanoyl, a C₇₋₁₃ aryl-carbonyl and a C₁₋₆ alkyl-sulfonyl

(I-2) a compound as shown in the above (I-1), wherein the 3- to 8-membered saturated or unsaturated nonaromatic heterocyclic group which may be substituted by member(s) selected from Group a1, represented by each of R^{a1} and R^{a2} is a 3- to 8-membered saturated or unsaturated nonaromatic heterocyclic group selected from Group a25 which may be substituted by member(s) selected from Group a1, and the heterocyclic group selected from Group a4 which may be substituted by member(s) selected from Group a3 formed by combining R^{a1} and R^{a2} together with A^{a} is a cyclic-amino group selected from Group a29 which may be substituted by member(s) selected from Group a3.

### Group a29

1-azetidinyl, 1-pyrrolidinyl, 1-piperidinyl, 1-homopiperidinyl, heptamethylenimino, 1-piperazinyl, 1-homopiperazinyl, 4-morpholinyl, 4-thiomorpholinyl, 2-isoindolinyl, 1,2,3,4-tetrahydro-2-isoquinolyl, 1,2,4,5-tetrahydro-3H-3-benzazepin-3-yl and indene-1-spiro-4'-piperidine-1'-yl

(I-3) a compound as shown in the above (I-1) wherein R^{a1} and R^{a2} combine each other together with A^{a} to form a 3-to 8-membered saturated or unsaturated non-aromatic heterocyclic group selected from Group a4, which may be substituted by member(s) selected from Group a3.

(I-4) a compound as shown in the above (I-1) wherein R^{a1} and R^{a2} combine each other together with A^{a} to form a 3-to 8-membered saturated or unsaturated non-aromatic heterocyclic group containing one or two nitrogen atoms, which ring may be substituted by member(s) selected from Group a3.

(I-5) a compound as shown in the above (I-3), wherein the group represented by -A^{a}R^{a1}R^{a2} is (1) a piperidinyl or (2) a piperazinyl group, each of which may be substituted by member(s) selected from Group a3.

(I-6) a compound as shown in the above (I-3), wherein the group represented by -A^{a}R^{a1}R^{a2} is a group represented by the formula: 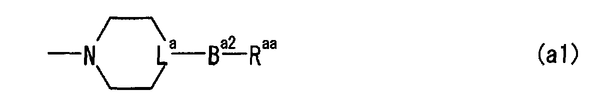 wherein L^{a} is methine or a nitrogen atom, B^{a2} is a bond, -CH₂-, -SO₂-, -SO-, -S-, -O-, -CO-, -NR^{ab1}-SO₂- (wherein R^{ab1} is a hydrogen atom, a C₁₋₆ alkyl group, a C₂₋₆ alkenyl group, a C₂₋₆ alkynyl group, a C₃₋₆ cycloalkyl group), -CH (OH)-, -NR^{ab2}- (wherein R^{ab2} is a hydrogen atom or a C₂₋₄ alkanoyl group), -NR^{ab1}-CO- (wherein R^{ab1} has the same meaning given above), -NR^{ab1}-CO-O- (wherein R^{ab1} has the same meaning given above), -CH₂SO₂- or -CH₂S-, R^{aa} is a hydrocarbon group selected from Group a2 which may be substituted by member(s) selected from Group a1 or a heterocyclic group selected from Group a6 which may be substituted by member(s) selected from Group a1.

(I-7) a compound as shown in the above (I-3), wherein the group represented by the formula-A^{a}R^{a1}R^{a2} is a group represented by the formula: 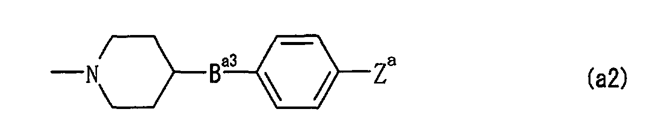 wherein B^{a3} is-CH₂-, -SO₂, -SO-, -S-, -O-, -CO-, -NR^{ab1}-SO₂ -(wherein R^{ab1} is a hydrogen atom, a C₁₋₆₄ alkyl group, a C₂₋₆ alkenyl group, a C₂₋₆ alkynyl or a C₃₋₆ cycloalkyl group), -NR^{ab1}-CO-, -NR^{ab1}-CO-O- (wherein R^{ab1} has the same meaning given above), Z^{a} is a halogen, SO₂NR^{ab3}R^{ab4} (wherein each of R^{ab3} and R^{ab4} is (1) a C₁₋₆ alkyl which may be substituted by halogen (s), hydroxyl (s) or C₁₋₆ alkoxy (s) *,* (2) a C₃₋₈ cycloalkyl which may be substituted by halogen(s) or C₁₋₆ alkoxy (s), (3) a C₁₋₆ alkoxy or (4) a hydrogen atom or, R^{ab3} and R^{ab4} are combine each other together with nitrogen to form a cyclic-amino group), SO₂R^{ab5}, (wherein R^{ab5} is (1) a C₁₋₆ alkyl group which may be substituted by halogen(s), hydroxyl(s) or C₁₋₆ alkoxy (s), (2) a C₃₋₈ cycloalkyl group which may be substituted by halogen(s) or C₁₋₆ alkoxy(s)), a CONR^{ab3}R^{ab4} (wherein each of R^{ab3} and R^{ab4} has the meaning given above) or -NR^{ab7}-SO₂R^{ab6} (wherein R^{ab6} is (1) a C₁₋₆ alkyl group which may be substituted by halogen(s) or C₁₋₆ alkoxy(s), (2) a C₃₋₈ cycloalkyl group which may be substituted by halogen(s) or C₁₋₆ alkoxy (s), R^{ab7} is (1) a C₁₋₆ alkyl group which may be substituted by halogen (s) or C₁₋₆ alkoxy (s), (2) a C₃₋₈ cycloalkyl group which may be substituted by halogen(s) or C₁₋₆ alkoxy(s) or (3) a hydrogen atom), a C₁₋₆ alkoxy group, an amino group which may be substituted by C₂₋₄ alkanoyl(s), nitro(s), cyano(s), tetrazolyl(s) or morpholinyl(s).

(I-8) a compound as shown in the above (I-1), wherein R^{a3} is a C₆₋₁₄ aryl group which may be substituted by member(s) selected from Group a1.

(I-9) a compound as shown in the above (I-1) , wherein R^{a3} is a phenyl group which may be substituted by member(s) selected from Group a1.

(I-10) a compound in which E^{a} is -CH₂CH₂-, -CH₂CH₂CH₂-, - CH₂CH₂CH₂CH₂- or -CH₂CH₂CH₂CH₂CH₂-.

(I-11) a compound in which E^{a} is-CH₂CH₂CH₂-.

(I-12 ) a compound in which G^{a2} is CO, SO₂, CONH or OCO.

(I-13) a compound in which G^{a2} is CO or NHCO.

(I-14) a compound in which G^{a2} is CO.

(I-15) a compound in which J^{a} is methine.

(I-16) a compound in which G^{a1} is CO or SO₂.

(I-17) a compound as shown in the above (I-1), wherein R^{a4} is a hydrocarbon group selected from Group a2 which may be substituted by member(s) selected from Group a1, a heterocyclic group selected from Group a6 which may be substituted by member(s) selected from Group a1, a C₁₋₆ alkoxy group which may be substituted by member(s) selected from Group a7, or an amino group which may be substituted by member(s) selected from Group a9.

(I-18) a compound in which R^{a4} is a C₁₋₃ alkyl.

(I-19) a compound in which R^{a4} is methyl.

(I-20) a compound in which each of Q^{a} and R^{a} is -CH₂CH₂- .

(I-21) a compound in which na is zero.

(1-22) a compound represented by the formula: 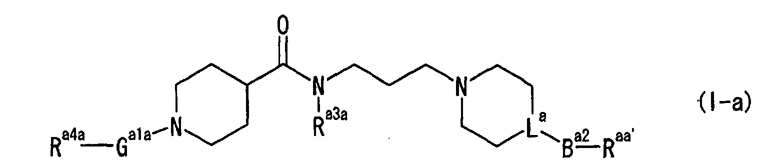 wherein R^{a4a} is (1) a C₁₋₆ alkyl group which may be substituted by halogen(s), C₁₋₆ alkoxy(s), oxo(s), amino(s), phenyl (s), pyridyl (s) or tetrazolyl (s), (2) a C₁₋₆ alkenyl group, (3) a C₃₋₈ cycloalkyl group which may be substituted by halogen (s), C₁₋₆ alkyl (s) or C₁₋₆ alkoxy (s), (4) a phenyl group which may be substituted by halogen(s), C₁₋₆ alkyl(s), C₁₋₆ alkoxy (s), nitro (s), cyano (s), hydroxyl (s), C₁₋₄ alkanoyl-amino(s), carbamoyl (s) or sulfamoyl(s), (5) an amino group which may be substituted by C₁₋₆ alkyl (s), (6) a C₁₋₆ alkoxy group which may be substituted by phenyl(s), (7) a C₃₋₈ cycloalkyl-oxy group (8) a heterocyclic group which may be substituted by halogen (s) , C₁₋₆ alkyl (s) or hydroxyl (s), G^{a1a} is CO or SO₂, R^{a3a} is a C₆₋₁₀ aryl group which may be substituted by (1) halogen(s), (2) C₁₋₆ alkyl(s) which may be substituted by halogen(s), (3) C₁₋₆ alkoxy(s) which may be substituted by halogen(s), (4) C₁₋₆ alkyl-thio(s), or (5) C6-10 aryl group which may be substituted by C₁₋₆ alkyl-sulfonyl(s), L^{a} is methine or a nitrogen atom, B^{a2} is a bond, -CH₂-, -SO₂-, -SO-, -S-, -O-, -CO-, -NR^{ab1}-SO₂- (wherein R^{ab1} has the same meaning given above), -CH(OH)-, -NR^{ab2}- (wherein R^{ab2} is a hydrogen atom or a C₂₋₄ alkanoyl group), -NR^{ab1}-CO- (wherein R^{ab1} has the same meaning given above), -NR^{ab1}-CO-O- (wherein R^{ab1} has the same meaning given above), -CH₂SO₂- or -CH₂S-, R^{aa}' is ① an aromatic hydrocarbon group which may be substituted by halogen (s) , SO₂NR^{ab3}R^{ab4} (wherein each of R^{ab3} and R^{ab4} has the same meaning given above), SO₂R^{ab5} (wherein R^{ab5} is (1) a C₁₋₆ alkyl group which may be substituted by halogen(s), hydroxyl(s) or C₁₋₆ alkoxy(s), (2) a C₃₋₈ cycloalkyl group which may be substituted by halogen(s) or C₁₋₆ alkoxy(s)), CONR^{ab3}R^{ab4} (wherein R^{ab3} and R^{ab4} have the same meanings given above) or-NR^{ab7}-SO₂R^{ab6} (wherein R^{ab6} has the same meaning given above), a C₁₋₆ alkoxy, an amino which may be substituted by a C₂₋₄ alkanoyl(s), a nitro, a cyano, s tetrazolyl or a morpholinyl or ②(2) an aromatic heterocyclic group which may be substituted by substituent(s) selected from the above mentioned substituents of aromatic hydrocarbon group, or salt thereof.

(I-23) a compound as shown in the above (I-22), wherein R^{a3a} is a phenyl group which may be substituted by halogen(s), trifluoromethyl(s) or C₁₋₆ alkyl(s).

(I-24) a compound as shown in the above (1-22), wherein L^{a} is methine.

(I-25) a compound as shown in the above (I-22), wherein B^{a2} is-CH₂-, -SO₂-, -SO-, -S-, -O-, -CO-, -NR^{ab1}- SO₂-, -NR^{ab1}-CO- or NR^{ab1}-CO-O- (wherein R^{ab1} has the same meaning given above).

(I-26) a compound as shown in the above (I-22), wherein R^{aa'} is a phenyl group which may be substituted by (1) halogen(s), (2) SO₂R^{ae} (wherein R^{ae} is a C₁₋₆ alkyl group or a C₃₋₈ cycloalkyl group), (3) N (R^{ad}) SO₂R^{ae} (wherein R^{ad} is a hydrogen atom or a C₁₋₉ alkyl group, R^{ae} has the same meaning given above), (4) SO₂NR^{af}R^{ag} (wherein each of R^{af} and R^{ag} is a hydrogen atom or a C₁₋₆ alkyl group or R^{af} and R^{ag} may combine each other together with a nitrogen atom to form a cyclic-amino group) or (5) CONR^{af}R^{ag} (wherein R^{af} and R^{ag} are the same or different and independently a hydrogen atom or a C₁₋₆ alkyl group or, R^{af} and R^{ag} combine each other together with a nitrogen atom to form a cyclic-amino group).

(I-27) a compound as shown in the above (I-22), wherein B² is SO₂, CH₂ or N(R^{ad})-SO₂ (wherein R^{ad} is a hydrogen atom or a C₁₋₄ alkyl) ; R^{aa'} is a phenyl which may be substituted by (1) halogen(s), (2) SO₂R^{ae} (wherein R^{ae} is a C₁₋₆ alkyl or a C₃₋₈ cycloalkyl) , (3) N(R^{ad}) SO₂R^{ae} (wherein R^{ad} is a hydrogen atom or a C₁₋₄ alkyl, and R^{ae} has the same meaning given above), (4) SO₂NR^{af}R^{ag} (wherein each of R^{af} and R^{ag} is a hydrogen atom or a C₁₋₆ alkyl or R^{af} and R^{ag} may combine each other together with a nitrogen atom to form a cyclic-amino group) or (5) CONR^{af}R^{ag} (wherein each of R^{af} and R^{ag} is a hydrogen atom or a C₁₋₆ alkyl group or R^{af} and R^{ag} may combine each other together with a nitrogen atom to form a cyclic-amino group); R^{a3a} is a phenyl group substituted by one or two members selected from the group of halogen atom and a C₁₋₄ alkyl.

(I-28) a compound as shown in the above (I-22), wherein G^{a1a} is SO₂ or CO, L^{a} is methine, B^{a 2} is SO₂ or CH₂, R^{aa}' is a group represented by the formula: 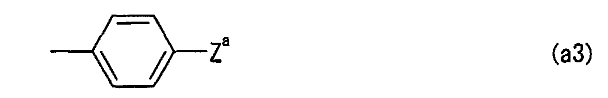 wherein Z^{a} is a C₁₋₄ alkyl-sulfonyl group, a sulfamoyl group which may be substituted by a C₁₋₄ alkyl or a carbamoyl group; R^{a3a} is a phenyl group which may be substituted by one or two members selected from the group consisting of halogen atom(s) or C₁₋₄ alkyl(s); and R^{a4a} is methyl.

Examples of the "hydrocarbon group" in the "a hydrocarbon group which may be substituted" represented by R^{b1} preferably include, for example, an aliphatic chain hydrocarbon group, an alicyclic hydrocarbon group and an aryl group etc. Examples of the aliphatic chain hydrocarbon group include a C₁₋₆ alkyl group, for example, methyl, ethyl, n-propyl, isopropyl, n-butyl, isobutyl, sec-butyl, tert-butyl, n-pentyl, isopentyl, neopentyl, 1-methylpropyl, n-hexyl, isohexyl etc. Examples of the "alicyclic hydrocarbon group" preferably include a C₃₋₈ cycloalkyl group such as cyclopropyl, cyclobutyl, cyclopentyl, cyclohexyl, cycloheptyl, cyclooctyl, etc.

Examples of the aryl group preferably include a C₆₋₁₄ aryl group such as phenyl, naphthyl (1-naphthyl, 2-naphthyl), etc.

Examples of the "substituent(s)" in the "hydrocarbon group which may be substituted" represented by R^{b1} include a hydrocarbon group which may be substituted, a heterocyclic group which may be substituted, a halogen atom (e.g., fluorine, chlorine, bromine, iodine), a C₁₋₄ alkoxy group which may be substituted, a C₁₋₄ alkylthio group which may be substituted, a C₂₋₆ alkoxycarbonyl group which may be substituted, a C₁₋₆ alkanoyl group which may be substituted, an amino group which may be substituted, a nitro group, a cyano group, a carbamoyl group which may be substituted, a sulfamoyl group which may be substituted, an acyl group derived from a sulfonic acid, etc.

Examples of the "hydrocarbon group(s)" in the "hydrocarbon group which may be substituted" are those similar to the "hydrocarbon group" of the "hydrocarbon group which may be substituted", which is represented by R^{b1}. Among these substituents, a C₁₋₆ alkyl group, a C₃₋₈ cycloalkyl group, a C₆₋₁₄ aryl group are preferred. These examples may include the substituents as mentioned above for R^{b1}. Examples of the "substituents" in the "hydrocarbon group which may be substituted" include, for example, a lower alkoxy group (e.g., a C₁₋₆ alkoxy group such as methoxy, ethoxy, propoxy, etc.), a halogen atom (e.g., fluorine, chlorine, bromine, iodine etc.), a lower alkyl group (e.g., a C₁₋₆ alkyl group such as methyl, ethyl, propyl, etc.), a lower alkynyl group (e.g., a C₁₋₄ alkynyl group such as vinyl, 1-propenyl, 2-propenyl, isopropenyl, butenyl, isobutenyl, etc.), an amino group, a hydroxy group, a cyano group, an amidino group etc. The hydrocarbon in "hydrocarbon that may be substituted" may have 1 to 3 substituent(s) as described above at any possible position. Examples of the heterocyclic group in the "heterocyclic group which may be substituted" (the substituent in the "hydrocarbon group which may be substituted, which is represented by R^{b1}") include, for example, an aromatic heterocyclic group, saturated or unsaturated non-aromatic heterocyclic group (alicyclic heterocyclic group) etc., which contains at least one heteroatom(s) (preferably 1 to 4 heteroatom(s), more preferably, 1 to 2 heteroatom(s)) consisting of 1 to 3 kind(s) of heteroatom(s) (preferably 1 to 2 kinds of heteroatom(s)) selected from an oxygen atom, a sulfur atom, a nitrogen atom, etc. as ring constituting atom(s).

Examples of the "aromatic heterocyclic group" include an aromatic monocyclic heterocyclic group such as a 5 or 6-membered aromatic monocyclic heterocyclic group (e.g., furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.); an aromatic fused heterocyclic group such as a 8 to 12-membered aromatic fused heterocyclic group (e.g., benzofuranyl, isobenzofuranyl, benzothienyl, indolyl, isoindolyl, 1H-indazolyl, benzindazolyl, benzoxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, irnidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.); etc., preferably, a heterocyclic group consisting of the above-mentioned 5- or 6-membered aromatic monocyclic heterocyclic group fused with a benzene ring or heterocyclic group consisting of the above-mentioned 5- or 6-membered aromatic monocyclic heterocyclic group fused with the same or different above-mentioned 5- or 6-membered aromatic monocyclic heterocyclic group, etc.

Examples of the "non-aromatic heterocyclic group" include a 3 to 8-membered (preferably 5 or 6-membered) saturated or unsaturated (preferably saturated) non-aromatic heterocyclic group (aliphatic heterocyclic group) such as oxiranyl, azetidinyl, oxetanyl, thiethanyl, pyrrolidinyl, tetrahydrofuryl, thiolanyl, piperidinyl, tetrahydropyranyl, morpholinyl, thiomorpholinyl, piperazinyl, etc.

Examples of the "substituent(s)" of the "heterocyclic group which may be substituted" (substituent(s) of the hydrocarbon group which may be substituted, which is represented by R^{b1}) are those similar to the "substituent(s)" of the "hydrocarbon group which may be substituted" that is(are) the "substituent(s)" of the hydrocarbon group which may be substituted, which is represented by R^{b1}.

Examples of "C₁₋₄ alkoxy group" in the "C₁₋₄ alkoxy group which may be substituted" include, for example, methoxy, ethoxy, n-propoxy, isopropoxy, n-butoxy, isobutoxy, tert-butoxy, etc. Example of "C₁₋₄ an alkylthio group" in the "C₁₋₉. an alkylthio group which may be substituted" include, for example, methylthio, ethylthio, n-propylthio, isopropylthio, n-butylthio, isobutylthio, tert-butylthio, etc. Example of the "C₂₋₆ alkoxycarbonyl group" in "C₂₋₆ alkoxycarbonyl group which may be substituted" include, for example, methoxycarbonyl, ethoxycarbonyl, n-propoxycarbonyl, isopropoxycarbonyl, n-butoxycarbonyl, isobutoxycarbonyl, tert-butoxycarbonyl, n-pentyloxycarbonyl, etc.

Examples of the "C₁₋₆ alkanoyl group" in the "C₁₋₆ alkanoyl group which may be substituted" include, for example, formyl, acetyl, propionyl, pivaloyl etc. Examples of the substituent in the "C₁₋₄ alkoxy group which may be substituted", "C₁₋₄ alkylthio group which may be substituted", and "C₁₋₆ alkoxycarbonyl group which may be substituted", "C₁₋₆ alkanoyl group which may be substituted" are those similar to the substituent(s) of the "hydrocarbon group which may be substituted", which are the substituent(s) of the "hydrocarbon group which may be substituted" represented by R^{b1}.

Examples of the substituent(s) of the "amino group which may be substituted" include, for example, a lower alkyl group (e.g., a C₁₋₆ alkyl group such as methyl, ethyl, propyl, isopropyl, butyl, isobutyl, t-butyl, pentyl, hexyl, etc.), an acyl group derived from a carboxylic acid (e.g., a C₁₋₆ alkanoyl such as formyl, acetyl, propionyl, pivaloyl, etc.), a C₇₋₁₅ arylcarbonyl such as benzoyl, etc., an acyl group derived from a sulfonic acid (e.g., a C₁₋₆ alkylsulfonyl such as methylsulfonyl, ethylsulfonyl, etc.), an optionally halogenated C₂₋₆ alkoxycarbonyl (e.g., trifluoromethoxycarbonyl, 2,2,2-trifluoroethoxycarbonyl, trichloromethoxycarbonyl, 2,2,2-trichloroethoxycarbonyl, etc.), etc. In addition, the "amino group" in the "amino group that may be substituted" may be substituted with an imidoyl group that may be substituted (e.g., a C₁₋₆ alkylimidoyl, formylimidoyl, amidino, etc.), etc. Alternatively, two substituents of the amino group may form a cyclic amino group together with a nitrogen atom. Examples of the cyclic amino group include e.g. 3 to 8-membered (preferably 5 or 6-membered) cyclic amino group such as 1-azetidinyl, 1-pyrrolidinyl, 1-piperidinyl, 4-morpholinyl, 1-piperazinyl and 1-piperazinyl which may have at the 4-position a lower alkyl group (e.g., a C₁₋₆ alkyl group such as methyl, ethyl, propyl, isopropyl, butyl, t-butyl, pentyl, hexyl, etc.), an aralkyl group (e.g. a C₇₋₁₀ aralkyl group such as benzyl, phenethyl, etc.), an aryl group (e.g. a C₆₋₁₀ aryl group such as phenyl, 1-naphthyl, 2-naphthyl, etc.), etc.

Examples of the "carbamoyl group which may be substituted" include unsubstituted carbamoyl, a N-mono-substituted carbamoyl group and a N,N-di-substituted carbamoyl group.

The "N-mono-substituted carbamoyl group" is. a carbamoyl group having one substituent on the nitrogen atom and the substituent include, for example, a lower alkyl group (e.g., a C₁₋₆ alkyl group such as methyl, ethyl, propyl, isopropyl, butyl, isobutyl, t-butyl, pentyl, hexyl, etc.), a cycloalkyl group (e.g., a C₃₋₆ cycloalkyl group such as cyclopropyl, cyclobutyl, cyclopentyl, cyclohexyl, etc.), an aryl group (e.g., a C₆₋₁₀ aryl group such as phenyl, 1-naphthyl, 2-naphthyl, etc.), an aralkyl group (e.g., a C₇₋₁₀ aralkyl group, preferably a phenyl-C₁₋₄ alkyl group such as benzyl, phenethyl, etc.), a heterocyclic group (e.g., the above described "heterocyclic group" as the substituent of the "hydrocarbon group which may be substituted" represented by R^{b1}, etc.), etc. The lower alkyl group, the cycloalkyl group, the aryl group, the aralkyl group and the heterocyclic group as described above may have substituent(s), and the substituent(s) include, for example, a hydroxy group, an amino group which may be substituted [the amino group may have 1 or 2 substituent(s) (e.g. a lower alkyl group (e.g., a C₁₋₆ alkyl group such as methyl, ethyl, propyl, isopropyl, butyl, isobutyl, t-butyl, pentyl, hexyl, etc.), an acyl group (e.g., a C₁₋₆ alkanoyl such as formyl, acetyl, propionyl, pivaloyl, etc., an arylcarbonyl such as benzoyl, etc., a C₁₋₆ alkylsulfonyl such as methylsulfonyl, ethylsulfonyl, etc.), etc.)], a halogen atom (e.g., fluorine, chlorine, bromine, iodine, etc.), a nitro group, a cyano group, a lower alkyl group which may be substituted with 1 to 5 halogen atom(s) (e.g., fluorine, chlorine, bromine, iodine, etc.), a lower alkoxy group which may be substituted with 1 to 5 halogen atom(s) (e.g., fluorine, chlorine, bromine, iodine, etc.), etc. The lower alkyl group includes, e.g. a C₁₋₆ alkyl group such as methyl, ethyl, n-propyl, isopropyl, n-butyl, isobutyl, sec-butyl, tert-butyl, pentyl, hexyl, etc. and in particular methyl, ethyl, etc. are preferable. The lower alkoxy group include e.g. a C₁₋₆ alkoxy group such as methoxy, ethoxy, n-propoxy, isopropoxy, n-butoxy, isobutoxy, sec-butoxy, tert-butoxy, etc. and in particular methoxy, ethoxy, etc. are preferable. The above described lower alkyl group, cycloalkyl group, aryl group, aralkyl group and heterocyclic group may have 1, 2 or 3 (preferably 1 or 2) substituent(s).

The "N,N-di-substituted carbamoyl group" is a carbamoyl group having two substituents on the nitrogen atom. Examples of one of the substituents include the same as those of the above described "N-mono-substituted carbamoyl group" and examples of the other substituent include e.g. a lower alkyl group (e.g., a C₁₋₆ alkyl group such as methyl, ethyl, propyl, isopropyl, butyl, t-butyl, pentyl, hexyl, etc.), a C₃₋₆ cycloalkyl group (e.g., cyclopropyl, cyclobutyl, cyclopentyl, cyclohexyl, etc.), a C₇₋₁₀ aralkyl group (e.g., benzyl, phenethyl, etc., preferably phenyl-C₁₋₄ alkyl group, etc.), etc. In addition, two substituents of the "N,N-di-substituted carbamoyl group" may form a cyclic amino group together with a nitrogen atom. Examples of the cyclic aminocarbonyl group include, e.g., 3 to 8-membered (preferably 5 or 6-membered) cyclic aminocarbonyl group such as 1-azetidinylcarbonyl, 1-pyrrolidinylcarbonyl, 1-piperidinylcarbonyl, 4-morpholinylcarbonyl, 1-piperazinylcarbonyl and 1-piperazinylcarbonyl which may have a lower alkyl group (e.g., a C₁₋₆ alkyl group such as methyl, ethyl, propyl, isopropyl, butyl, t-butyl, pentyl, hexyl, etc.), an aralkyl group (e.g., a C₇₋₁₀ aralkyl group such as benzyl, phenethyl, etc.), an aryl group (e.g., a C₆₋₁₀ aryl group such as phenyl, 1-naphthyl, 2-naphthyl, etc.), etc. at the 4-position. Examples of the "sulfamoyl group which may be substituted" include an unsubstituted sulfamoyl group, a N-mono-substituted sulfamoyl group and a N,N-di-substituted sulfamoyl group.

The "N-mono-substituted sulfamoyl group". is a sulfamoyl group having one substituent at the nitrogen atom, and examples of the substituent include those mentioned for the substituents of N-mono-substituted carbamoyl group.

The "N,N-di-substituted sulfamoyl group" is a sulfamoyl group having two substituents at the nitrogen atom, and examples of the substituents include those mentioned as the substituents of the N,N-di-substituted carbamoyl group.

Examples of the "acyl group derived from a sulfonic acid" include a sulfonyl group substituted by a hydrocarbon group, and preferably, include an acyl group such as C₁₋₁₀ alkylsulfonyl, C₂₋₆ alkenylsulfonyl, C₂₋₆ alkynylsulfonyl, C₃₋₉ cycloalkylsulfonyl, C₃₋₉ cycloalkenylsulfonyl, C₆₋₁₄ arylsulfonyl, C₇₋₁₀ aralkylsulfonyl. Examples of the C₁₋₁₀ alkyl include, for example, methyl, ethyl, propyl, isopropyl, butyl, isobutyl, t-butyl, pentyl, hexyl, heptyl, octyl, etc. Examples of the C₂₋₆ alkenyl include, for example, vinyl, allyl, 1-propenyl, isopropenyl, 2-butenyl, 3-butenyl, 2-hexenyl, etc. Examples of C₂₋₆ alkynyl include, for example, ethynyl, 2-propynyl, 2-butynyl, 5-hexynyl, etc. Examples of the C₃₋₉ cycloalkyl include, for example, cyclopropyl, cyclobutyl, cyclopentyl, cyclohexyl, cyclooctyl, etc. Examples of the C₃₋₉ cycloalkenyl include, for example, 1-cyclopenten-1-yl, 2-cyclopenten-1-yl, 3-cyclopenten-1-yl, 3-cyclohexen-1-yl, 3-cycloocten-1-yl, etc. Examples of the C₆₋₁₄ aryl include, for example, phenyl, 1-naphthyl, 2-naphthyl, etc. Examples of the C₇₋₁₀ aralkyl-sulfonyl include, for example, benzyl, phenethyl, etc. These hydrocarbon groups that are the substituents of the sulfonyl may be substituted. Examples of these substituents include, for example, hydroxy group, amino group which may be substituted [the amino group may be substituted by one or two lower alkyl group (e.g., C₁₋₆ alkyl such as methyl, ethyl, propyl, isopropyl, butyl, isobutyl, t-butyl, pentyl, hexyl, etc.), an acyl group (e.g., a C₁₋₆ alkanoyl such as formyl, acetyl, propionyl, pivaloyl, etc., an aryl carbonyl such as benzoyl, etc., a C₁₋₆ alkylsulfonyl such as methylsulfonyl, ethylsulfonyl, etc.)], a halogen atom (for example, fluorine, chlorine, bromine, iodine, etc.), nitro group, cyano group, a lower alkyl which may be substituted by 1 to 5 halogen atom(s) (e.g. fluorine, chlorine, bromine, iodine, etc.), a lower alkoxy group which may be substituted by 1 to 5 halogen atom(s) (e.g. fluorine, chlorine, bromine, iodine, etc.). Examples of the lower alkyl group include, for example, a C₁₋₆ alkyl group such as methyl, ethyl, n-propyl, isopropyl, n-butyl, isobutyl, sec-butyl, tert-butyl, pentyl, hexyl, etc., and preferably include methyl, ethyl, etc. The lower alkoxy group includes, for example, a C₁₋₆ alkoxy group such as methoxy, ethoxy, n-propoxy, isopropoxy, n-butoxy, isobutoxy, sec-butoxy, tert-butoxy, etc, and preferably includes methoxy, ethoxy, etc. Preferably, one, two or three (preferably one or two) from these substituents are used, wherein the substituents may be the same or different. Examples of the "cyclic hydrocarbon group" in the "cyclic hydrocarbon group which may be substituted" represented by R^{b2} include alicyclic hydrocarbon group and aryl group.

Examples of the "alicyclic hydrocarbon group" include, for example, a saturated or unsaturated alicyclic hydrocarbon group such as a cycloalkyl group, a cycloalkenyl group, a cycloalkanedienyl group, etc. Examples of the "cycloalkyl group" include, for example, a C₃₋₉ cycloalkyl (preferably, a C₃₋₈ cycloalkyl etc.) such as cyclopropyl, cyclobutyl, cyclopentyl, cyclohexyl, cycloheptyl, cyclooctyl, cyclononyl, etc., and a fused ring such as 1-indanyl, 2-indanyl, etc. Examples of the "cycloalkenyl group" include, for example, a C₃₋₆ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl, etc. Examples of the "cycloalkanedienyl group" include, for example, a C₄₋₆ cycloalkanedienyl group such as 2,4-cyclopentanedien-1-yl, 2,4-cyclohexanedien-1-yl, 2,5-cyclohexanedien-1-yl, etc. In particular, a C₃₋₈ cyrloalkyl group such as cyclohexyl is preferable.

Examples of the "aryl group" include a monocyclic or fused polycyclic aromatic hydrocarbon group. Among others, a C₆₋₁₄ aryl group such as phenyl, naphthyl, anthryl, phenanthryl, acenaphthylenyl, 4-indanyl, 5-indanyl, etc. is preferable. In particular, phenyl, 1-naphthyl, 2-naphthyl, etc. are preferable.

Examples of the "substituent(s)" in the "cyclic hydrocarbon group which may be substituted" represented by R^{b2} are those similar to the "substituent" of the "hydrocarbon group which may be substituted" described as the substituent(s) of the "hydrocarbon group which may be substituted", which are represented by R^{b1}.

Examples of the "heterocyclic group which may be substituted" of R^{b2} are those similar to the "heterocyclic group which may be substituted" described as the substituent(s) of the "hydrocarbon group which may be substituted", which are represented by R^{b1}.
The halogen atom represented by R^{b3} includes, for example, fluorine, chlorine, bromine, iodine, etc.

The "carbamoyl group which may be substituted", "sulfamoyl group which may be substituted" and "acyl group derived from a sulfonic acid" represented by R^{b3} are those similar to the "carbamoyl group which may be substituted", "sulfamoyl group which may be substituted" and "acyl group derived from a sulfonic acid", which are represented by R^{b1}. Examples of the "C₁₋₄ alkyl group" of the "C₁₋₄ alkyl group which may be substituted" represented by R^{b3} include, for example, methyl, ethyl, n-propyl, isopropyl, n-butyl, isobutyl, tert-butyl. Examples of the "C₁₋₄ alkoxy group" of the "C₁₋₄ alkoxy group which may be substituted" represented R^{b3} include, for example, methoxy, ethoxy, propoxy, n-butoxy, isobutoxy, tert-butoxy.

Example of the substituent(s) in the "C₁₋₄ alkyl group which may be substituted" and "C₁₋₄ alkoxy group which may be substituted", which is represented by R^{b3} are those similar to the "substituent(s)" of the "hydrocarbon group which may be substituted" which is(are) the "substituent (s)" of "the hydrocarbon group which may be substituted", which is represented by R^{b1}.

Examples of the substituents of "amino group which may be substituted" represented by R^{b3} include, for example, a lower alkyl group (e.g., C₁₋₆ alkyl group such as methyl, ethyl, propyl, isopropyl, butyl, isobutyl, t-butyl, pentyl, hexyl, etc.), an acyl group derived from carboxylic acid (e.g., C₁₋₆ alkanoyl such as formyl, acetyl, propionyl, pivaloyl, etc.), for example, C₇₋₁₅ arylcarbonyl such as benzoyl, etc, an acyl group derived from sulfonic acid (e.g., C₁₋₆ alkylsulfonyl such as methylsulfonyl, ethylsulfonyl, etc.), an optionally halogenated C₁₋₆ alkoxy-carbonyl (e.g., trifluoromethoxycarbonyl, 2,2,2-trifluoroethoxycarbonyl, trichloromethoxycarbonyl, 2,2,2-trichloroethoxycarbonyl, etc.), etc. In addition, the "amino group" of the "amino group that may be substituted" may be substituted with an imidoyl group that may be substituted (e.g., a C₁₋₆ alkylimidoyl, formylimidoyl, amidino, etc.), etc. and two substituents of the "amino group" may form a cyclic amino group together with a nitrogen atom. Examples of the cyclic amino group include, for example, 3 to 8-membered (preferably, 5 or 6-membered) cyclic amino group such as 1-azetidinyl, 1-pyrrolidinyl, 1-piperidinyl, 4-morpholinyl, 1-piperazinyl and 1-piperazinyl which may have a lower alkyl group (e.g., a C₁₋₆ alkyl group such as methyl, ethyl, propyl, isopropyl, butyl, t-butyl, pentyl, hexyl, etc.), an aralkyl group (e.g., a C₇₋₁₀ aralkyl group such as benzyl, phenethyl, etc.), an aryl group (e.g., a C₆₋₁₀ aryl group such as phenyl, 1-naphthyl, 2-naphthyl, etc.), etc. at the 4-position.

Examples of the leaving group represented by X include, for example, a halogen atom (e.g., a chlorine atom, a bromine atom, an iodine atom, etc.), an alkyl or aryl sulfonyloxy group (e.g., methanesulfonyloxy, trifluoromethanesulfonyloxy, ethanesulfonyloxy, benzenesulfonyloxy, p-toluenesulfonyloxy, etc.), etc.

Examples of the salt of a compound of the formula (II) of the present invention include a salt with an acid, for example, a salt with inorganic acid (e.g., hydrochloric acid salt, sulfuric acid salt, hydrobromic acid salt, phosphoric acid salt, etc.), a salt of an organic acid (e.g., acetic acid salt, trifluoroacetic acid salt, succinic acid salt, maleic acid salt, fumaric acid salt, propionic acid salt, citric acid salt, tartaric acid salt, lactic acid salt, oxalic acid salt, methanesulfonic acid salt, p-toluenesulfonic acid salt, etc.), etc., a salt with a base (e.g., an alkali metal salt such as potassium salt, sodium salt, lithium salt, etc., an alkaline earth metal salt such as calcium salt, magnesium salt, etc., ammonium salt, a salt with an organic base such as ammonium salt, trimethylamine salt, triethylamine salt, tert-butyl dimethyl amine salt, dibenzyl methylamine salt, benzyl dimethylamine salt, N,N-dimethylaniline salt, pyridine salt, quinoline salt, etc.).

The compound of the formula (II) or salt thereof may also be hydrated. Hereinafter the compound of the formula (II), its salt and its hydrate are referred to as Compound (II).

Among the compounds represented by the formula (II) or salts thereof (hereinafter referred to as. Compounds (II)), the following compounds are preferable.

(II-1) the compound wherein R^{b3} is a halogen atom, a C₁₋₄ alkyl group which may be substituted, a C₁₋₄ alkoxy group which may be substituted, an amino group which may be substituted, a nitro group or a cyano group,

(II-2) the compound wherein R^{b1} is an alicyclic hydrocarbon group which may be substituted or an aryl group which may be substituted,

(II-3) the compound wherein R^{b1} is a hydrocarbon group which may be substituted by 1 to 4 substituent(s) selected from 1) a hydrocarbon group which may be substituted, 2) an heterocyclic group which may be substituted, 3) a C₁₋₄, alkoxy group which may be substituted, 4) a C₁₋₄ alkylthio group which may be substituted, 5) a C₂₋₆ alkoxycarbonyl group which may be substituted, 6) a C₁₋₆ alkanoyl group which may be substituted, 7) an amino group which may be substituted, 8) a cyclic amino group, 9) a halogen atom, 10) a nitro group, 11) a cyano group, 12) a carbamoyl group which may be substituted, 13) a sulfamoyl group which may be substituted and 14) an acyl group derived from a sulfonic acid,

(II-4) the compound wherein R^{b1} is a hydrocarbon group which may be substituted by 1 to 4 substituent(s) selected from 1) a hydrocarbon group which may be substituted, 2) a heterocyclic group which may be substituted, 3) a C₁₋₄ alkoxy group which may be substituted, 4) a C₁₋₄ alkylthio group which may be substituted, 5) a C₂₋₆ alkoxycarbonyl group which may be substituted, 6) an amino group which may be substituted, 7) a halogen atom, 8) a nitro group and 9) a cyano group,

(II-5) the compound wherein R^{b1} is a hydrocarbon group which may be substituted by 1 to 4 substituent(s) selected from 1) a hydrocarbon group which may be substituted, 2) a heterocyclic group which may be substituted, 3) a C₁₋₄ alkylthio group which may be substituted, 4) a C₂₋₆ alkoxycarbonyl group which may be substituted, 5) an amino group which may be substituted, 6) a halogen atom and 7) a nitro group,

(II-6) the compound wherein R^{b2} is an cyclic hydrocarbon group which may be substituted,

(II-7) the compound wherein R^{b3} is a halogen, a carbamoyl group which may be substituted, a sulfamoyl group which may be substituted or an acyl group derived from a sulfonic acid,

(II-8) the compound wherein R^{b3} is a halogen,

(II-9) the compound wherein R^{b4} is a hydrogen atom,

(II-10) the compound wherein n is 0,

(II-11) the compound wherein R^{b1} is a hydrocarbon group selected from Group 3 which may be substituted by member(s) selected from Group 1; R^{b2} is a cyclic hydrocarbon group selected from Group 10 which may be substituted by member(s) selected from Group 2, or a heterocyclic group selected from Group 4 which may be substituted by member(s) selected from Group 2; R^{b3} is a halogen atom, a carbamoyl group, a N-mono-substituted carbamoyl group which may be substituted by a member selected from Group 11, a N,N-di-substituted carbamoyl group which may be substituted by a member selected from Group 11 and a member selected from Group 14, a cyclic aminocarbonyl group selected from Group 17, a sulfamoyl group, N-mono-substituted sulfamoyl group which may be substituted by a member selected from Group 11, a N,N-di-substituted sulfamoyl group which may be substituted by a member selected from Group 11 and a member selected from Group 14, a cyclic aminosulfonyl group selected from Group 20, an acyl group derived from a sulfonic acid selected from Group 15, a C₁₋₄ alkyl group which may be substituted by member(s) selected from Group 2, a C₁₋₄ alkoxy group which may be substituted by member(s) selected from Group 2, an amino group which may be substituted by member(s) selected from Group 8, a cyclic amino group selected from Group 9, a nitro group or a cyano group.

### Group 1

1) a hydrocarbon group selected from Group 3 which may be substituted by member(s) selected from Group 2, 2) a heterocyclic group selected from Group 4 which may be substituted by member(s) selected from Group 2, 3) a C₁₋₄ alkoxy group which may be substituted by member(s) selected from Group 2, 4) a C₁₋₄ alkylthio group which may be substituted by member(s) selected from Group 2, 5) a C₂₋₆ alkoxycarbonyl group which may be substituted by member(s) selected from Group 2, 6) a C₁₋₆alkanoyl group, 7) an amino group which may be substituted by member(s) selected from Group 8, 8) a cyclic amino group selected from Group 9, 9) a halogen atom, 10) a nitro group, 11) a cyano group, 12) a carbamoyl group, 13) a mono-substituted carbamoyl group which is substituted by a member selected from Group 11, 14) di-substituted carbamoyl group which is substituted by a member selected from Group 11 and a member selected Group 14, 15) a cyclic amino carbamoyl group selected from Group 17, 16) a sulfamoyl group, 17) a N-mono substituted sulfamoyl group which is substituted by a member selected from Group 11, 18) a N,N-di-substituted sulfamoyl group which is substituted by a member selected from Group 11 and a member selected Group 14, 19) an acyl group derived from a sulfonic acid selected from Group 19

### Group 2

1) a C₁₋₆ alkoxy group, 2) a halogen atom, 3) a C₁₋₆ alkyl group, 4) a C₁₋₄ alkynyl group, 5) an amino group, 6) a hydroxy group, 7) a cyano group and 8) an amidino group

### Group 3

1) a C₁₋₆ alkyl group, 2) a C₃₋₈ cycloalkyl group and 3) a C₆₋₁₄ aryl group

### Group 4

1) an aromatic monocyclic heterocyclic group selected from Group 5, 2) an aromatic condensed heterocyclic group selected from Group 6 and 3) a saturated or unsaturated non-aromatic heterocyclic group selected from Group 7

### Group 5

furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl

### Group 6

benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl

### Group 7

oxylanyl, azetidinyl, oxetanyl, thietanyl, pyrrolidinyl, tetrahydrofuryl, thiolanyl, piperidinyl, tetrahydropyranyl, morpholinyl, thiomorpholinyl and piperazinyl

### Group 8

1) a C₁₋₆ alkyl, 2) a C₁₋₆ alkanoyl, 3) a C₇₋₁₃ arylcarbonyl, 4) an optionally halogenated C₂₋₆ alkoxycarbonyl, 5) a C₁₋₆ alkylimidoyl, 6) a formylimidoyl and 7) an amidino

### Group 9

1) 1-azetidinyl, 2) 1-pyrrolidinyl, 3) 1-piperidinyl, 4) 4-morpholinyl, 5) 1-piperazinyl and 6) 1-piperazinyl which may have a C₁₋₆ alkyl, a C₇₋₁₀ aralkyl and a C₆₋₁₀ aryl at 4-position

### Group 10

C₃₋₉ cycloalkyl, 1-indanyl, 2-indanyl, C₃₋₆ cycloalkenyl, C₄₋₆ cycloalkanedienyl and C₆₋₁₄ aryl

### Group 11

1) a C₁₋₆ alkyl group which may be substituted by member(s) selected from Group 12, 2) a C₃₋₆ cycloalkyl group which may be substituted by member (s) selected from Group 12, 3) a C₆₋₁₀ aryl group which may be substituted by member(s) selected from Group 12, 4) a C₇₋₁₀ aralkyl group which may be substituted by member(s) selected from Group 12, 5) a C₁₋₆ alkoxy group which may be substituted by member(s) selected from Group 12 and 6) a heterocyclic group selected from Group 13 which may be substituted by member(s) selected from Group 12

### Group 12

1) a hydroxy group, 2) an amino group, 3) an amino group which is mono or di-substituted by member(s) selected from Group 16, 4) a halogen atom, 5) a nitro group, 6) a cyano group, 7) a C₁₋₆ alkyl group which may be substituted by halogen atom(s) and 8) a C₁₋₆ alkoxy group which may be substituted by halogen atom(s)

### Group 13

1) an aromatic heterocyclic group selected from Group 5 and Group 6 and 2) a saturated or unsaturated non-aromatic heterocyclic group selected from Group 7, each of which contains at least one heteroatom(s) selected from the group consisting of an oxygen atom, a sulfur atom and a nitrogen atom as ring-constituting atom(s)

### Group 14

a C₁₋₆ alkyl group, a C₃₋₆ cycloalkyl group and a C₇₋₁₀ aralkyl group

### Group 15

1) a C₁₋₁₀ alkylsulfonyl which may be substituted by member(s) selected from Group 12, 2) a C₂₋₆ alkenylsulfonyl which may be substituted by member(s) selected from Group 12, 3) a C₂₋₆ alkynylsulfonyl which may be substituted by member (s) selected from Group 12, 4) a C₃₋₉ cycloalkylsulfonyl which may be substituted by member(s) selected from Group 12, 5) a C₃₋₉ cycloalkenylsulfonyl which may be substituted by member(s) selected from Group 12, 6) a C₆₋₁₄ arylsulfonyl which may be substituted by member(s) selected from Group 12 and 7) a C₇₋₁₀ aralkylsulfonyl which may be substituted by member(s) selected from Group 12

### Group 16

a C₁₋₆ alkyl group, a C₁₋₆ alkanoyl, a C₇₋₁₃ arylcarbonyl and a C₁₋₆ alkylsulfonyl

### Group 17

1-azetidinylcarbonyl, 1-pyrrolidinylcarbonyl, 1-piperidinylcarbonyl, 4-morpholinylcarbonyl and 1-piperazinylcarbonyl which may be substituted by member(s) selected from Group 18

### Group 18

a C₁₋₆ alkyl group, a C₇₋₁₀ aralkyl group and a C₆₋₁₀ aryl group

### Group 19

a C₁₋₁₀ alkylsulfonyl which may be substituted by member(s) selected from Group 12, a C₂₋₆ alkenylsulfonyl which may be substituted by member (s) selected from Group 12, a C₂₋₆ alkynylsulfonyl which may be substituted by member(s) selected from Group 12, a C₃₋₉ cycloalkylsulfonyl which may be substituted by member(s) selected from Group 12, a C₃₋₉ cycloalkenylsulfonyl which may be substituted by member(s) selected from Group 12, a C₆₋₁₄ arylsulfonyl which may be substituted by member(s) selected from Group 12, and a C₇₋₁₀ aralkylsulfonyl which may be substituted by member(s) selected from Group 12

### Group 20

1-azetidinylsulfonyl, 1-pyrrolidinylsulfonyl, 1-piperidinylsulfonyl, 4-morpholinylsulfonyl and 1-piperazinylsulfonyl which may be substituted by member(s) selected from Group 18
(II-12) the compound wherein R^{b1} is a C₃₋₈ cycloalkyl group which may be substituted by member(s) selected from Group 1 or a C₆₋₁₄ aryl group which may be substituted by member(s) selected from Group 1, (II-13) the compound as shown in the above (II-12), wherein R^{b1} is 1) a C₆₋₁₄ aryl group which may be substituted by a halogen atom, a C₁₋₆ alkyl which may be substituted by halogen(s), a C₁₋₄ alkylthio, a nitro, a carbamoyl, a sulfamoyl or a C₁₋₆ alkylsulfonyl, 2) a C₁₋₆ alkyl group which may be substituted by (i) a C₂₋₆ alkoxycarbonyl group or (ii) a phenyl which may be substituted by C₁₋₆ alkyl (s) or 3) a C₃₋₈ cycloalkyl group which may be substituted by (i) a halogen atom, (ii) a C₁₋₆ alkyl(s) which may be substituted by halogen(s) or (iii) a C₁₋₆ alkoxy group which may be substituted by halogen(s);
R^{b2} is a phenyl group which may be substituted by a halogen atom, a C₁₋₆ alkyl, a C₁₋₄ alkoxy or a cyano, a C₃₋₈ cycloalkyl group or a pyridyl group;
R^{b3} is (i) a halogen atom, (ii) a carbamoyl group, (iii) a sulfamoyl group which may have one or two C₁₋₆ alkyl (s) or C₃₋₆ cycloalkyl (s) at N-atoms, a cyclic aminosulfonyl group selected from Group 20, a C₁₋₆ alkylsulfonyl group or C₃₋₆ cycloalkylsulfonyl group;
R^{b4} is a hydrogen atom;
nb is 0 or 1 and
pb is 0 or 1,
(II-14) the compound as shown in the above (II-12), wherein R^{b1} is 1) a phenyl which may be substituted by a halogen atom, a C₁₋₃ alkyl, trifluoromethyl, methoxy, trifluoromethoxy, methylthio or nitro, 2) a naphthyl, 3) a C₁₋₆ alkyl group which may be substituted by (i) a C₂₋₃ alkoxycarbonyl, (ii) phenyl or (iii) 3-isopropenylphenyl or 4) cyclohexyl group;
R^{b2} is a phenyl group which may be substituted by a halogen atom, methyl, methoxy or cyano, a cyclohexyl group or a 3-pyridyl group;
R^{b3} is (i) a halogen atom, (ii) a carbamoyl group, (iii) a 4-morpholinylsulfonyl group or (iv) a methylsulfonyl group,
R^{b4} is a hydrogen atom;
nb is 0 or 1; and
pb is 0 or 1,
(II-15) the compound as shown in the above (II-12), wherein R^{b1} is a phenyl group which may be substituted by a halogen atom or a C₁₋₃ alkyl; R^{b2} is a phenyl group which may be substituted by a halogen atom and methyl(s);
R^{b3} is (i) a halogen atom, (ii) a carbamoyl group, (iii) a sulfamoyl group which may be substituted by one or two members selected from the group consisting of C₁₋₆ alkyl and C₃₋₆ cycloalkyl at N-atoms, a cyclic aminosulfonyl group selected from Group 20, a C₁₋₆ alkylsulfonyl group or a C₃₋₆ cycloalkyl sulfonyl group, R^{b4} is a hydrogen atom, nb is 0, and pb is 0 or 1.

The hydrocarbon group represented by R^{c1} includes, for example, an aliphatic chain hydrocarbon group, an alicyclic hydrocarbon group, an aryl group and the like. Preferably, it is an aliphatic chain hydrocarbon group or an alicyclic hydrocarbon group.

The aliphatic chain hydrocarbon group includes, for example, a straight or branched aliphatic hydrocarbon group such as alkyl group, alkenyl group, alkynyl group and the like, with preference given to alkyl group. Examples of the alkyl group include C₁₋₁₀ alkyl groups (preferably C₁₋₆ alkyl etc.), such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, 1-methylheptyl, 1-ethylhexyl, n-octyl, 1-methylheptyl, nonyl and the like.

Examples of the alkenyl group include C₂₋₆ alkenyl groups, such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like. Examples of the alkynyl group include C₂₋₆ alkynyl groups, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like.

Examples of the alicyclic hydrocarbon group include saturated or unsaturated alicyclic hydrocarbon groups, such as cycloalkyl group, cycloalkenyl group, cycloalkanedienyl group and the like, with preference given to cycloalkyl group. Examples of the cycloalkyl group include C₃₋₉ cycloalkyl (preferably C₃₋₈ cycloalkyl etc.), such as cyclopropyl, cyclobutyl, cyclopentyl, cyclohexyl, cycloheptyl, cyclooctyl, cyclononyl and the like, and fused rings such as 1-indanyl, 2-indanyl and the like.

Examples of the cycloalkenyl group include C₃₋₆ cycloalkenyl groups, such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl and the like.

Examples of the cycloalkanedienyl group include C₄₋₆ cycloalkanedienyl groups, such as 2,4-cyclopentanedien-1-yl, 2,4-cyclohexanedien-1-yl, 2,5-cyclohexanedien-1-yl and the like.

Examples of the aryl group include monocyclic or fused polycyclic aromatic hydrocarbon groups, which are preferably C₆₋₁₄ aryl groups, such as phenyl, naphthyl, anthryl, phenanthryl, acenaphthylenyl, 4-indanyl, 5-indanyl etc., and the like, with particular preference given to phenyl, 1-naphthyl, 2-naphthyl and the like.

The hydrocarbon group having 2 or more carbon atoms represented by R^{c2} includes, for example, the hydrocarbon groups having 2 or more carbon atoms among those represented by R^{c1}. Of those recited with regard to R^{c1}, preferred are C₂₋₆ alkyl and C₃₋₈ cycloalkyl.

When R^{c1} and R^{c2} in combination form, together with an adjacent nitrogen atom, a ring optionally having a substituent or substituents, the ring may contain, besides one nitrogen atom, a different nitrogen atom, an oxygen atom and a sulfur atom. Examples thereof include monocyclic groups, such as 1-azetidinyl, 1-pyrrolidinyl, 1-piperidinyl, 1-homopiperidinyl, heptamethyleneimino, 1-piperazinyl, 1-homopiperazinyl, morpholino, thiomorpholino and the like, fused rings such as 2-isoindolinyl, 1,2,3,4-tetrahydro-2-isoquinolyl, 1,2,4,5-tetrahydro-3H-3-benzodiazepin-3-yl and the like, cyclic amino groups such as spiro ring and the like (e.g., indene-1-spiro-4'-piperidin-1'-yl etc.), the cyclic amino group optionally having 1 to 5, preferably 1 to 3, substituent(s) at a chemically permitted position on the ring.

Examples of the substituent include hydroxy group, cyano group, nitro group, oxo group, halogen atom (e.g., fluorine atom, chlorine atom, bromine atom, iodine atom etc.), a group of the formula: -Y^{c}R^{ca} (wherein R^{ca} is a hydrocarbon group optionally having a substituent or substituents or a heterocyclic group optionally having a substituent or substituents, Y^{c} is a bond (single bond), -CR^{cb}R^{cc}-, -COO-, -CO-, -CO-NR^{cb}-, -CS-NR^{cb}-, -CO-S-, -CS-S-, -CO-NR^{cb}-CO-NR^{cc}-, -C (=NH) -NR^{cb}-, -NR^{b}-, -NR^{cb}-CO-, -NR^{cb}-CS-, -NR^{-cb}-CO-NR^{cc}-, -NR^{cb}-CS-NR^{cc}-, -NR^{cb}-CO-O-, -NR^{cb}-CS-O-, -NR^{cb}-CO-S-, -NR^{cb}-CS-S-, -NR^{cb}-C (=NH) -NR^{cc}-, -NR^{cb}-SO₂-, -NR^{cb}-NR^{cc}-, -O-, -O-CO-, -O-CS-, -O-CO-O, -O-CO-NR^{cb}-, -O-C(=NH)-NR^{cb}-, -S-, -SO-, -SO,-, -SO₂NR^{cb}-, -S-CO-, - S-CS-, -S-CO-NR^{cb}-, -S-CS-NR^{cb}-, -S-C (=NH) -NR^{cb}- and the like, wherein R^{cb} and R^{cc} are each a hydrogen atom, alkyl group optionally having a substituent or substituents, alkenyl group optionally having a substituent or substituents, alkynyl group optionally having a substituent or substituents, an aryl group optionally having a substituent or substituents, cycloalkyl group or cycloalkenyl group optionally having a substituent or substituents, a heterocyclic group optionally having a substituent or substituents, acyl group derived from carboxylic acid, alkylsulfonyl group optionally having a substituent or substituents, an aryl sulfonyl group optionally having a substituent or substituents, etc.), and the like.

The "hydrocarbon group" of the hydrocarbon group optionally having a substituent or substituents represented by R^{ca} includes an aliphatic chain hydrocarbon group, alicyclic hydrocarbon group, aryl group and the like.

Examples of these aliphatic chain hydrocarbon group, alicyclic hydrocarbon group and aryl group is the aliphatic chain hydrocarbon group, alicyclic hydrocarbon group and aryl group represented by R^{c1} respectively. Examples of the substituent of the hydrocarbon group include those mentioned as the substituents for the "hydrocarbon group optionally having a substituent or substituents" represented by R^{c3} to be mentioned later.

Examples of the "heterocyclic group optionally having a substituent or substituents" at the aforementioned R^{ca} include those mentioned as the "heterocyclic group optionally having a substituent or substituents" represented by R^{c3} to be mentioned later.

Examples of the alkyl group optionally having a substituent or substituents, alkenyl group optionally having a substituent or substituents, alkynyl group optionally having a substituent or substituents, aryl group optionally having a substituent or substituents, cycloalkyl group or cycloalkenyl group optionally having a substituent or substituents, heterocyclic group optionally having a substituent or substituents, acyl group derived from carboxylic acid, alkyl sulfonyl group optionally having a substituent or substituents and arylsulfonyl group optionally having a substituent or substituents, represented by the aforementioned R^{cb} and R^{cc} include those mentioned as the substituent of the hydrocarbon group optionally having a substituent represented by R^{c3} to be mentioned later.

It is preferable that R^{c1} and R^{c2} in combination forms, together with an adjacent nitrogen atom, a heterocyclic ring optionally having a substituent or substituents. More preferably, NR^{c1}R^{c2} is a group of the formula: 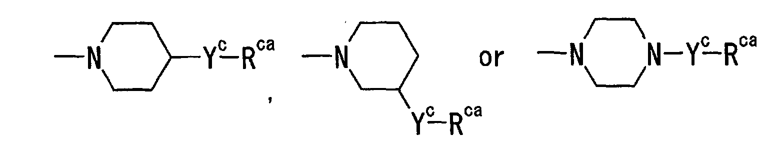 wherein Y^{c} and R^{ca} are as defined above. As used here, Y^{c} and R^{ca} are as defined above, and R^{ca} is particularly preferably an aryl group optionally having a substituent or substituents or a heterocyclic group optionally having a substituent or substituents.

Y^{c}R^{ca} is particularly preferably a benzyl group optionally having a substituent or substituents.

NR^{c1}R^{c2} is particularly preferably a 4-benzyl-1-piperidinyl group optionally having a substituent or substituents.

Examples of the hydrocarbon group of the hydrocarbon group optionally having a substituent or substituents represented by R^{c3} include those similar to the hydrocarbon groups represented by R^{c1}, with particular preference given to C₁₋₆ alkyl group, C₃₋₈ cycloalkyl group and aryl group. These are exemplified by those recited for R^{c1}.

The heterocyclic group of the heterocyclic group optionally having a substituent or substituents represented by R^{c3} is, for example, an aromatic heterocyclic group, a saturated or unsaturated non-aromatic heterocyclic group (aliphatic heterocyclic group) and the like, containing, as an atom constituting the ring system, at least one (preferably 1 to 4, more preferably 1 or 2) of 1 to 3 kinds (preferably 1 or 2 kinds) of the hetero atom selected from an oxygen atom, a sulfur atom, a nitrogen atom and the like.

Examples of the aromatic heterocyclic group include aromatic monocyclic heterocyclic group (e.g., 5- or 6-membered aromatic monocyclic heterocyclic group such as furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.); condensed aromatic heterocyclic group [e.g., 8 to 12-membered condensed aromatic heterocyclic group (preferably a heterocycle wherein the aforementioned 5- or 6-membered aromatic monocyclic heterocyclic group is condensed with a benzene ring or a heterocycle wherein the same or different two heterocycles of the aforementioned 5- or 6-membered aromatic monocyclic heterocyclic group are condensed), such as benzofuranyl, isobenzofuranyl, benzothienyl, indolyl, isoindolyl, 1H-indazolyl, benzindazolyl, benzooxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrro[1,2-b]pyridazinyl, pyrrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc.] and the like.

Examples of the non-aromatic heterocyclic group include 3 to 8-membered (preferably 5- or 6-membered) saturated or unsaturated (preferably saturated) non-aromatic heterocyclic group (aliphatic heterocyclic group), such as oxiranyl, azetidinyl, oxetanyl, thietanyl, pyrrolidinyl, tetrahydrofuryl, thioranyl, piperidinyl, tetrahydropyranyl, morpholinyl, thiomorpholinyl, piperazinyl etc., and the like.

Examples of the substituent of the hydrocarbon group optionally having a substituent or substituents as represented by R^{c3} and the substituent of the heterocyclic group optionally having a substituent or substituents as represented by R^{c3} include alkyl group optionally having a substituent or substituents, alkenyl group optionally having a substituent or substituents, alkynyl group optionally having a substituent or substituents, an aryl group optionally having a substituent or substituents, cycloalkyl group or cycloalkenyl group optionally having a substituent or substituents, a heterocyclic group optionally having a substituent or substituents, amino group optionally having a substituent or substituents, imidoyl group optionally having a substituent or substituents, amidino group optionally having a substituent or substituents, hydroxy group optionally having a substituent or substituents, thiol group optionally having a substituent or substituents, optionally esterified carboxyl group, carbamoyl group optionally having a substituent or substituents, thiocarbamoyl group optionally having a substituent or substituents, sulfamoyl group optionally having a substituent or substituents, halogen atom (e.g., fluorine, chlorine, bromine, iodine etc., preferably chlorine, bromine etc.), cyano group, nitro group, acyl group derived from carboxylic acid, alkyl sulfinyl group optionally having a substituent or substituents, alkyl sulfonyl group optionally having a substituent or substituents, arylsulfinyl group optionally having a substituent or substituents, arylsulfonyl group optionally having a substituent or substituents and the like, wherein 1 to 5 (preferably 1 to 3) of these optional substituents may be present at a substitutable position.

The aryl group of the "aryl group optionally having a substituent or substituents" as a substituent may be, for example, C₆₋₁₄ aryl group such as phenyl, naphthyl, anthryl, phenanthryl, acenaphthylenyl etc., and the like. Here, the substituent of the aryl group includes, for example, lower alkoxy group (e.g., C₁₋₆ alkoxy group such as methoxy, ethoxy, propoxy etc., and the like), halogen atom (e.g., fluorine, chlorine, bromine, iodine etc.), lower alkyl group (e.g., C₁₋₆ alkyl group such as methyl, ethyl, propyl etc., etc.), amino group, hydroxy group, cyano group, amidino group and the like, wherein one or two of these optional substituents may be present at a substitutable position.

The cycloalkyl group of the "cycloalkyl group optionally having a substituent or substituents" as a substituent may be, for example, C₃₋₇ cycloalkyl group, such as cyclopropyl, cyclobutyl, cyclopentyl, cyclohexyl, cycloheptyl etc., and the like. As used herein, examples of the substituent of the "cycloalkyl group" are similar in the kind and the number to those exemplified for the substituent of the aforementioned "aryl group optionally having a substituent or substituents". The cycloalkenyl group of the "cycloalkenyl group optionally having substituents" as a substituent may be, for example, C₃₋₆ cycloalkenyl group such as cyclopropenyl, cyclobutenyl, cyclopentenyl, cyclohexenyl etc., and the like. As used herein, examples of the substituent of the "cycloalkenyl group optionally having a substituent or substituents" are similar in the kind and the number to those exemplified for the substituent of the aforementioned "aryl group optionally having a substituent or substituents".

The alkyl group of the "alkyl group optionally having a substituent or substituents" as a substituent may be, for example, C₁₋₆ alkyl such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl etc., and the like. As used herein, examples of the substituent of the alkyl group are similar in the kind and the number to those exemplified for the substituent of the aforementioned "aryl group optionally having a substituent or substituents".

The alkenyl group of the "alkenyl group optionally having a substituent or substituents" as a substituent may be, for example, C₂₋₆ alkenyl group such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl etc., and the like. As used herein, examples of the substituent of the alkenyl group are similar in the kind and the number to those exemplified for the substituent of the aforementioned "aryl group optionally having a substituent or substituents".

The alkynyl group of the "alkynyl group optionally having a substituent or substituents" as a substituent may be, for example, C₂₋₆ alkynyl group, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. As used herein, examples of the substituent of the alkynyl group are similar in the kind and the number to those exemplified for the substituent of the aforementioned "aryl group optionally having a substituent or substituents". The heterocyclic group of the "heterocyclic group optionally having a substituent or substituents" as a substituent may be, for example, an aromatic heterocyclic group, a saturated or unsaturated non-aromatic heterocyclic group (aliphatic heterocyclic group) and the like, containing, as an atom (cyclic atom) constituting the ring system, at least one (preferably 1 to 4, more preferably 1 or 2) of 1 to 3 kinds (preferably 1 or 2 kinds) of the hetero atom selected from an oxygen atom, a sulfur atom and a nitrogen atom, and the like.

Examples of the "aromatic heterocyclic group" include aromatic monocyclic heterocyclic group (e.g., 5- or 6-membered aromatic monocyclic heterocyclic group, such as furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.) and condensed aromatic heterocyclic group [e.g., 8 to 12-membered condensed aromatic heterocycle (preferably a heterocycle wherein the aforementioned 5- or 6-membered aromatic monocyclic heterocyclic group is condensed with a benzene ring or a heterocycle wherein the same or different two heterocycle of the aforementioned 5- or 6-membered aromatic monocyclic heterocyclic group are condensed), such as benzofuranyl, isobenzofuranyl, benzothienyl, indolyl, isoindolyl, 1H-indazolyl, benzindazolyl, benzooxazolyl, 1,2-benzoisooxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrro[1,2-b]pyridazinyl, pyrrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc.] and the like.

Examples of the "non-aromatic heterocyclic group" include 3 to 8-membered (preferably 5- or 6-membered) saturated or unsaturated (preferably saturated) non-aromatic heterocyclic group (aliphatic heterocyclic group), such as oxiranyl, azetidinyl, oxetanyl, thietanyl, pyrrolidinyl, tetrahydrofuryl, thioranyl, piperidinyl, tetrahydropyranyl, morpholinyl, thiomorpholinyl, piperazinyl etc., and the like.

The substituent that the "heterocyclic group optionally having a substituent or substituents" as a substituent may have is exemplified by lower alkyl group (e.g., C₁₋₆ alkyl group, such as methyl, ethyl, propyl etc., and the like), acyl group (e.g., C₁₋₆ alkanoyl, such as formyl, acetyl, propionyl, pivaloyl etc., benzoyl etc.), and the like. The substituent of the "amino group optionally having a substituent or substituents", "imidoyl group optionally having a substituent or substituents", "amidino group optionally having a substituent or substituents", "hydroxy group optionally having a substituent or substituents" and "thiol group optionally having a substituent or substituents" as a substituent may be, for example, lower alkyl group (e.g., C₁₋₆ alkyl group, such as methyl, ethyl, propyl, isopropyl, butyl, isobutyl, t-butyl, pentyl, hexyl etc., and the like), acyl group (e.g., C₁₋₆ alkanoyl (e.g., formyl, acetyl, propionyl, pivaloyl etc.), benzoyl etc.), C₁₋₆ alkyl sulfonyl (e.g., methanesulfonyl, ethanesulfonyl etc.), C₃₋₁₄ arylsulfonyl (e.g., benzenesulfonyl, p-toluenesulfonyl etc.), optionally halogenated C₁₋₆ alkoxy-carbonyl (e.g., trifluoromethoxycarbonyl, 2,2,2-trifluoroethoxycarbonyl, trichloromethoxycarbonyl, 2,2,2-trichloroethoxycarbonyl etc.), and the like. The "amino group" of the "amino group optionally having a substituent or substituents" as the substituent may be substituted by imidoyl group optionally having a substituent or substituents (e.g., C₁₋₆ alkyl imidoyl, formylimidoyl, amidino etc.), and the like. In addition, two substituents may form a cyclic amino group together with a nitrogen atom. In this case, examples of the cyclic amino group include 3 to 8-membered (preferably 5- or 6-membered) cyclic amino, such as 1-azetidinyl, 1-pyrrolidinyl, 1-piperidinyl, morpholino, 1-piperazinyl and 1-piperazinyl optionally having, at the 4-position, lower alkyl group (e.g., C₁₋₆ alkyl group, such as methyl, ethyl, propyl, isopropyl, butyl, t-butyl, pentyl, hexyl etc., and the like), aralkyl group (e.g., C₇₋₁₀ aralkyl group, such as benzyl, phenethyl etc., and the like), aryl group (e.g., C₆₋₁₀ aryl group, such as phenyl, 1-naphthyl, 2-naphthyl etc., and the like), and the like.

Examples of the "carbamoyl group optionally having a substituent or substituents" include unsubstituted carbamoyl, N-monosubstituted carbamoyl group and N,N-disubstituted carbamoyl group.

The "N-monosubstituted carbamoyl group" is a carbamoyl group having one substituent on the nitrogen atom.

Examples of the substituent include lower alkyl group (e.g., C₁₋₆ alkyl group, such as methyl, ethyl, propyl, isopropyl, butyl, isobutyl, t-butyl, pentyl, hexyl etc., and the like), cycloalkyl group (e.g., C₃₋₆ cycloalkyl group, such as cyclopropyl, cyclobutyl, cyclopentyl, cyclohexyl etc., and the like), aryl group (e.g., C₆₋₁₀ aryl group, such as phenyl, 1-naphthyl, 2-naphthyl etc., and the like), aralkyl group (e.g., C₇₋₁₀ aralkyl group, such as benzyl, phenethyl etc., preferably phenyl-C₁₋₄ alkyl group etc.), heterocyclic group (e.g., those exemplified as the "heterocyclic group" as a substituent of "hydrocarbon group optionally having a substituent or substituents" represented by R^{c3} and the like). The lower alkyl group, cycloalkyl group, aryl group, aralkyl group and heterocyclic group may have substituents, which substituents are, for example, hydroxy group, amino group optionally having a substituent or substituents [which amino group optionally having 1 or 2 from lower alkyl group (e.g., C₁₋₆ alkyl group such as methyl, ethyl, propyl, isopropyl, butyl, isobutyl, t-butyl, pentyl, hexyl etc., and the like), acyl group (e.g., C₁₋₆ alkanoyl such as formyl, acetyl, propionyl, pivaloyl etc., benzoyl, etc.), and the like as substituents], halogen atom (e.g., fluorine, chlorine, bromine, iodine etc.), nitro group, cyano group, lower alkyl group optionally having 1 to 5 halogen atoms as substituents (e.g., fluorine, chlorine, bromine, iodine etc.) lower alkoxy group optionally having 1 to 5 halogen atoms as substituents (e.g., fluorine, chlorine, bromine, iodine etc.), and the like. Examples of the lower alkyl group include C₁₋₆ alkyl group, such as methyl, ethyl, n-propyl, isopropyl, n-butyl, isobutyl, sec-butyl, tert-butyl, pentyl, hexyl etc., and the like, particularly preferably methyl, ethyl and the like. Examples of the lower alkoxy group include C₁₋₆ alkoxy group, such as methoxy, ethoxy, n-propoxy, isopropoxy, n-butoxy, isobutoxy, sec-butoxy, tert-butoxy etc., and the like, particularly preferably methoxy, ethoxy and the like. One, two or three of these (preferably 1 or 2) are the same or different and may be present.

The "N,N-disubstituted carbamoyl group" is a carbamoyl group having 2 substituents on a nitrogen atom. Examples of one of the substituents are those similar to the substituents of the aforementioned "N-monosubstituted carbamoyl group" and examples of the other include lower alkyl group (e.g., C₁₋₆ alkyl group, such as methyl, ethyl, propyl, isopropyl, butyl, t-butyl, pentyl, hexyl etc., and the like), C₃₋₆ cycloalkyl group (e.g., cyclopropyl, cyclobutyl, cyclopentyl, cyclohexyl etc.), C₇₋₁₀ aralkyl group (e.g., benzyl, phenethyl etc., preferably phenyl-C₁₋₄ alkyl group etc.) and the like. Two substituents may form a cyclic amino group together with a nitrogen atom. In this case, examples of the cyclic aminocarbamoyl group include 3 to 8-membered (preferably 5- or 6-membered) cyclic amino-carbomoyl such as 1-azetidinylcarbonyl, 1-pyrrolidinylcarbonyl, 1-piperidinylcarbonyl, morpholinocarbonyl, 1-piperazinylcarbonyl and 1-piperazinylcarbonyl optionally having, at the 4-position, lower alkyl group (e.g., C₁₋₆ alkyl group, such as methyl, ethyl, propyl, isopropyl, butyl, t-butyl, pentyl, hexyl etc., and the like), aralkyl group (e.g., C₇₋₁₀ aralkyl group, such as benzyl, phenethyl etc., and the like), aryl group (e.g., C₆₋₁₀ aryl group, such as phenyl, 1-naphthyl, 2-naphthyl etc., and the like), and the like.

Examples of the substituent of the "thiocarbamoyl group optionally having a substituent or substituents" are similar to those exemplified for the substituent of the aforementioned "carbamoyl group optionally having a substituent or substituents".

Examples of the "sulfamoyl group optionally having a substituent or substituents" include unsubstituted sulfamoyl, N-monosubstituted sulfamoyl group and N,N-disubstituted sulfamoyl group.

The "N-monosubstituted sulfamoyl group" means sulfamoyl group having one substituent on a nitrogen atom. Examples of the substituent are those similar to the substituent of the "N-monosubstituted carbamoyl group". The "N,N-disubstituted sulfamoyl group" means sulfamoyl group having 2 substituents on a nitrogen atom. Examples of the substituent are those similar to the substituent of the "N,N-disubstituted carbamoyl group". Examples of the "optionally esterified carboxyl group" include, besides free carboxyl group, lower alkoxy carbonyl group, aryloxycarbonyl group, aralkyloxycarbonyl group and the like.

Examples of the "lower alkoxy carbonyl group" include C₁₋₆ alkoxy-carbonyl group, such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl etc., and the like. Of these, C₁₋₃ alkoxy-carbonyl group, such as methoxycarbonyl, ethoxycarbonyl, propoxycarbonyl etc., and the like are preferable.

Examples of the "aryloxycarbonyl group" preferably include C₇₋₁₂ aryloxy-carbonyl group, such as phenoxycarbonyl, 1-naphthoxycarbonyl, 2-naphthoxycarbonyl etc., and the like.

Examples of the "aralkyloxycarbonyl group" preferably include C₇₋₁₀ aralkyloxy-carbonyl group (preferably C₆₋₁₀ aryl-C₁₋₄ alkoxy-carbonyl etc.) such as benzyloxycarbonyl, phenethyloxycarbonyl etc., and the like.

The "aryloxycarbonyl group" and "aralkyloxycarbonyl group" may have substituents. Examples of the substituent are similar in the kind and the number to those exemplified for the substituent of aryl group and aralkyl group as the substituents of the aforementioned N-monosubstituted carbamoyl group.

The "acyl group derived from carboxylic acid" as the substituent is exemplified by one in which a hydrogen atom or the single substituent that the aforementioned "N-monosubstituted carbamoyl group" has on a nitrogen atom is bonded to carbonyl, and the like. Preferably, C₁₋₆ alkanoyl such as formyl, acetyl, propionyl, pivaloyl etc. and acyl such as venzoyl etc., and the like.

The alkyl of the "alkyl sulfinyl group optionally having a substituent or substituents" and "alkyl sulfonyl group optionally having a substituent or substituents" as the substituent may be, for example, lower alkyl group such as C₁₋₆ alkyl group (e.g., methyl, ethyl, propyl, isopropyl, butyl, isobutyl, t-butyl, pentyl, hexyl etc.), and the like.

The aryl of the "arylsulfinyl group optionally having a substituent or substituents" and "arylsulfonyl group optionally having a substituent or substituents" as the substituent may be, for example, C₆₋₁₄ aryl group, such as phenyl, naphthyl, 'anthryl, phenanthryl, acenaphthylenyl etc., and the like.

The substituent of these alkyl and aryl may be, for example, lower alkoxy group (e.g., C₁₋₆ alkoxy group, such as methoxy, ethoxy, propoxy etc., and the like), halogen atom (e.g., fluorine, chlorine, bromine, iodine etc.), lower alkyl group (e.g., C₁₋₆ alkyl group, such as methyl, ethyl, propyl etc., and the like), amino group, hydroxy group, cyano group, amidino group and the like, wherein one or two of these optional substituents may be present at a substitutable position.

The hydrocarbon group optionally having a substituent or substituents represented by R^{c4} is exemplified by those shown with regard to hydrocarbon group optionally having a substituent or substituents represented by R^{c3}, and the heterocyclic group optionally having a substituent or substituents represented by R^{c4} is exemplified by those shown with regard to the heterocyclic group optionally having a substituent or substituents represented by R^{c3}.

The divalent chain hydrocarbon group of the divalent chain hydrocarbon group optionally having a substituent or substituents other than oxo group, represented by E^{c}, is exemplified by C₁₋₆ alkylene, such as methylene, ethylene etc., C₂₋₆ alkenylene, such as ethenylene etc., C₂₋₆ alkynylene, such as ethynylene etc., and the like. Preferred is C₁₋₅ alkylene and more preferred is trimethylene.

The substituent of the divalent hydrocarbon group may be any as long as it is not an oxo group. Examples thereof include alkyl group optionally having a substituent or substituents, an aryl group optionally having a substituent or substituents, cycloalkyl group or cycloalkenyl group optionally having a substituent or substituents, optionally esterified carboxyl group, carbamoyl group or thiocarbamoyl group optionally having a substituent or substituents, amino group optionally having a substituent or substituents, hydroxy group optionally having a substituent or substituents, thiol (mercapto) group optionally having a substituent or substituents, acyl group derived from carboxylic acid, alkyl sulfonyl group optionally having a substituent or substituents, arylsulfonyl group optionally having a substituent or substituents, halogen (e.g., fluorine, chlorine, bromine etc.), nitro, cyano and the like. The number of the substituents may be 1 to 3. The alkyl group optionally having a substituent or substituents, the aryl group optionally having a substituent or substituents, the cycloalkyl group or cycloalkenyl group optionally having a substituent or substituents, the optionally esterified carboxyl group, the carbamoyl group or thiocarbamoyl group optionally having a substituent or substituents, the amino group optionally having a substituent or substituents, the hydroxy group optionally having a substituent or substituents, the thiol (mercapto) group optionally having a substituent or substituents, the acyl group derived from carboxylic acid, the alkyl sulfonyl group optionally having a substituent or substituents, the arylsulfonyl group optionally having a substituent or substituents are those similar to the substituent of the heterocyclic group optionally having a substituent or substituents represented by the aforementioned R^{c3}.

Examples of the substituent of the methine group optionally having a substituent or substituents represented by J^{c} are those similar to the substituent of the heterocyclic group optionally having a substituent or substituents represented by the aforementioned R^{c3}.

The divalent chain C₁₋₃ hydrocarbon group of the divalent chain C₁₋₃ hydrocarbon group optionally having a substituent or substituents, represented by Q^{c} and R^{c}, is exemplified by one having 1 to 3 carbon atoms from the divalent chain hydrocarbon group of the divalent chain hydrocarbon group optionally having a substituent or substituents other than oxo group, represented by E^{c}.

The substituent of the divalent chain C₁₋₃ hydrocarbon group optionally having a substituent or substituents, represented by Q^{c} and R^{c}, is exemplified by those exemplified as the substituent of the divalent chain hydrocarbon group optionally having a substituent or substituents other than oxo group, represented by E^{c}. The salt of the carboxyl group or sulfonic acid group, as represented by R^{c5}, is exemplified by salts with alkali metal, such as sodium, potassium, lithium etc., salts with alkaline earth metal, such as calcium, magnesium, strontium etc., ammonium salt and the like.

Among the compounds represented by the formula (III) or salts thereof (hereinafter referred to as Compound (III)), the following compounds are preferable.

(III-1) The compound wherein R^{c1} is a C₁₋₆ alkyl group or a C₃₋₈ cycloalkyl group; R^{c2} is a C₂₋₆ alkyl group or a C₃-₈ cycloalkyl group, or R^{c1} and R^{c2} in combination form, together with an adjacent nitrogen atom, a ring optionally having a substituent or substituents; R^{c3} is a C₁₋₆ alkyl group optionally having a substituent or substituents, a C₃₋₈ cycloalkyl group optionally having a substituent or substituents, an aryl group optionally having a substituent or substituents or a heterocyclic group optionally having a substituent or substituents; R^{c4} is a hydrogen atom, alkyl group optionally having a substituent or substituents, a C₃₋₈ cycloalkyl group optionally having a substituent or substituents, an aryl group optionally having a substituent or substituents or a heterocyclic group optionally having a substituent or substituents; E^{c} is a C₂₋₅ alkylene group optionally having a substituent or substituents other than oxo group; G^{c} is CO or SO₂; J^{c} is a nitrogen atom or a methine group optionally having a substituent or substituents; and Q^{c} and R^{c} are each a bond or a C₁₋₃ alkylene group optionally having a substituent or substituents.

(III-2) The compound wherein R^{c1} and R^{c2} in combination form, together with an adjacent nitrogen atom, a ring optionally having a substituent or substituents.

(III-3) The compound as shown in the above (III-2), wherein the ring optionally having a substituent or substituents is a 1-piperidinyl group or a 1-piperazinyl group each optionally having a substituent or substituents.

(III-4) The compound as shown in the above (III-3), wherein the substituent of the 1-piperidinyl group or 1-piperazinyl group is (1) phenyl-C₁₋₄ alkyl optionally having halogen on a benzene ring, (2) diphenylmethyl optionally having hydroxy, (3) benzoyl optionally having halogen on a benzene ring, (4) 2-phenylethen-1-yl, (5) phenyl optionally having halogen, (6) hydroxy, (7) phenoxy or (8) benzyloxy.

(III-5) The compound as shown in the above (III-2), wherein the ring optionally having a substituent or substituents is a 1-piperidinyl group optionally having a substituent or substituents.

(III-6) The compound as shown in the above (III-5), wherein the substituent of the 1-piperidinyl group is a benzyl group optionally having halogen on a benzene ring.

(III-7) The compound wherein R^{c3} is (1) a C₁₋₆ alkyl group, (2) a C₃₋₈ cycloalkyl group, (3) a benzyl group optionally having a hydroxy group, (4) a naphthylmethyl group, (5) a phenyl group optionally having, as a substituent, (a) C₁₋₄ alkyl optionally having halogen, (b) C₁₋₄ alkoxy optionally having halogen, (c) phenyl, (d) cyano, (e) benzyloxy or (f) a halogen atom, (6) a naphthyl group, (7) an indanyl group or (8) a tetrahydronaphthyl group.

(III-8) The compound wherein R^{c3} is a phenyl group optionally having, as a substituent, C₁₋₄ alkyl or halogen.

(III-9) The compound wherein E^{c} is C₂₋₆ polymethylene optionally having hydroxy.

(III-10) The compound wherein R^{c4} is (1) a hydrogen atom, (2) C₁₋₆ alkyl optionally having (a) halogen, (b) pyridyl, (c) morpholino, (d) furyl, (e) ethynyl or (f) C₃₋₈ cycloalkyl, (3) phenyl-C₁₋₄ alkyl optionally having (a) halogen, (b) C₁₋₄ alkyl, (c) halogeno-C₁₋₄ alkyl or (d) C₁₋₄ alkoxy on a benzene ring, or (4) C₃₋₈ cycloalkyl.

(III-11) The compound wherein R^{c4} is (a) C₁₋₄ alkyl group optionally having, as a substituent, halogen or furyl or (b) a benzyl group optionally having halogen on a benzene ring.

(III-12) The compound wherein -N(R^{c1})R^{c2} is a 1-piperidinyl group optionally having a substituent or substituents, E^{c} is a trimethylene group, R^{c3} is a phenyl group optionally having a substituent or substituents, G^{c} is CO, J^{c} is CH, and Q^{c} and R^{c} are each a methylene group. In the above formulas, examples of the C₁₋₆ alkyl group shown by R^{da} in groups -NR^{da}-SO₂- and -NR^{da}-CO-, which are represented by B^{d} include e.g. methyl, ethyl, propyl, isopropyl, butyl, isobutyl, t-butyl, pentyl, hexyl, etc., examples of the C₂₋₆ alkenyl group include e.g. vinyl, allyl, 1-propeny, isopropeny, 2-butenyl, 3-butenyl, 2-hexenyl, etc., and examples of the C₃₋₈ cycloalkyl group include e.g. cyclopropyl, cyclobutyl, cyclopentyl, cyclohexyl, cyclooctyl, etc.

Examples of the halogen atom represented by R^{d1} include e.g.fluorine, chlorine, bromine, iodine, etc., examples of the C₁₋₆ alkyl group include e.g. methyl, ethyl, propyl, isopropyl, butyl, isobutyl, t-butyl, pentyl, hexyl, etc., examples of the C₂₋₄ alkenyl group include e.g. vinyl, 1-propeny, 2-propeny, isopropeny, butenyl, isobutenyl, etc., example of C₁₋₄ alkanoyl group include e.g. formyl, acetyl, propionyl, butyryl, etc., and example of C₁₋₄ alkoxy group include e.g. methoxy, ethoxy, propoxy, etc.

Examples of halogen represented by R^{d2} include e.g.fluorine, chlorine, bromine, iodine, etc.

Examples of the C₁₋₆ alkyl in the "C₁₋₆ alkyl which may be substituted by a halogen or C₁₋₄ alkoxy" represented by R^{d2} include e.g. methyl, ethyl, propyl, isopropyl, butyl, isobutyl, t-butyl, pentyl, hexyl, etc., examples of the halogen as the substituent includes e.g. fluorine, chlorine, bromine, iodine, etc., and examples of C₁₋₄ alkoxy as the substituent include e.g. methoxy, ethoxy, n-propoxy, isopropoxy, n-butoxy, isobutoxy, sec-butoxy, tert-butoxy, etc.

Examples of the C₁₋₄ alkoxy group in the C₁₋₄ alkoxy which may be substituted by a halogen or C₁₋₄ alkoxy" represented by R^{d2} include e.g. methoxy, ethoxy, n-propoxy, isopropoxy, n-butoxy, isobutoxy, sec-butoxy, tert-butoxy, etc., examples of the halogen as the substituent includes e.g. fluorine, chlorine, bromine, iodine, etc., and examples of C₁₋₄ alkoxy as the substituent include e.g. methoxy, ethoxy, n-propoxy, isopropoxy, n-butoxy, isobutoxy, sec-butoxy, tert-butoxy, etc.

Examples of C₁₋₄ alkanoylamino represented by R^{d2} include e.g. formylamino, acetylamino, propionylamino, butyrylamino, etc.

In the groups SO₂NR^{db}R^{dc}, CONR^{db}R^{dc} and NR^{db}R^{dc} each of which is represented by R^{d2}, examples of the "C₁₋₆ alkyl group which may be substituted by a halogen or C₁₋₄ alkoxy" represented by R^{db} and R^{dc} include those mentioned for the "C₁₋₆ alkyl which may be substituted by a halogen or C₁₋₄ alkoxy" represented by R^{d2}; examples of the "C₃₋₈ cycloalkyl group which may be substituted by a halogen or C₁₋₄ alkoxy" include e.g. cyclopropyl, cyclobutyl, cyclopentyl, cyclohexyl, cyclooctyl, etc.; the halogen which is the substituent include e.g. fluorine, chlorine, bromine, iodine, etc., and the C₁₋₄ alkoxy which is the substituent include e.g. methoxy, ethoxy, n-propoxy, isopropoxy, n-butoxy, isobutoxy, sec-butoxy, tert-butoxy, etc.

In the groups SO₂NR^{db}R^{dc}, CONR^{db}R^{dc} and NR^{db}R^{dc} each of which is represented by R^{dc}, examples of the cyclic amino group which forms by combining R^{db} and R^{dc} together with the nitrogen atom include e.g. 1) 1-azetidinyl, 2) 1-pyrrolidinyl, 3) 1-piperidinyl, 4) 4-morpholinyl, 5) 1-piperazinyl and 6) 1- piperazynyl which may be substituted by a C₁₋₆ alkyl (e.g.methyl, ethyl, propyl, isopropyl, butyl, isobutyl, t-butyl, pentyl, hexyl, etc.), a C₇₋₁₀ aralkyl (e.g. benzyl, phenethyl, etc) or a C₆₋₁₀ aryl (e.g. phenyl, 1-naphthyl, 2-naphthyl, etc.) at 4-position. In the group NR^{da}-SO₂R^{dd} represented by R^{d2}, the definition of R^{da} is the same as that of R^{da} in groups -NR^{da}-SO₂- and -NR^{da}-CO- each of which represented by B^{d}.

In the groups SO₂R^{dd} and -NR^{da}-SO₂R^{dd}represented by R^{d2}, examples of the C₁₋₆alkyl group represented by R^{dd} include e.g. methyl, ethyl, propyl, isopropyl, butyl, isobutyl, t-butyl, pentyl, hexyl, etc. and examples of the C₃₋₈cycloalkyl group include, e.g. cyclopropyl, cyclobutyl, cyclopentyl, cyclohexyl, cyclooctyl, etc.

In the formula (IV), (IIId) and (IVd), examples of the hydrocarbon group in the hydrocarbon group which may be substituted represented by R^{d3} in the groups shown by the formulas (d1), (d2), (d3), (d4), (d5) and (d6) includes e.g. a C₁₋₆alkyl (e.g. methyl, ethyl, propyl, isopropyl, butyl, isobutyl, t-butyl, pentyl, hexyl, etc.), a C₂₋₆alkenyl (e.g. vinyl, allyl, 1-propeny, isopropeny, 2-butenyl, 3-butenyl, 2-hexenyl, etc.), a C₂₋₆alkynyl (e.g. ethynyl, 2-propynyl, 2-butynyl, 5-hexynyl, etc.), a C₃₋₈cycloalkyl (e.g. cyclopropyl, cyclobutyl, cyclopentyl, cyclohexyl, cyclooctyl, etc.), a C₆₋₁₀aryl (e.g. phenyl, naphthyl, etc.), etc.

Examples of the substituent of the hydrocarbon group include e.g. a halogen (e.g. fluorine, chlorine, bromine, iodine, etc), a C₁₋₄alkoxy( e.g. methoxy, ethoxy, n-propoxy, isopropoxy, n-butoxy, isobutoxy, sec-butoxy, tert-butoxy, etc.), phenyl, a C₁₋₆alkyl (e.g. methyl, ethyl, n-propyl, isopropyl, n-butyl, isobutyl, sec-butyl, tert-butyl, pentyl, hexyl, etc.), nitro, cyano, hydroxy, a C₁₋₄alkanoylamino (e.g. formylamino, acetylamino, propionylamino, etc.), carbamoyl, sulfamoyl, etc.

Among them, when the hydrocarbon group is a C₁₋₆alkyl, a C₂₋₆alkenyl or C₂₋₆alkynyl, preferable examples of the substituent include a halogen, a C₁₋₄alkoxy and phenyl; when the hydrocarbon group is a C₃₋₈cycloalkyl, preferable examples of the substituent include a halogen, a C₁₋₆alkyl and a C₁₋₄alkoxy; when the hydrocarbon group is a C₆₋₁₀aryl, preferable example include a halogen, a C₁₋₆alkyl, a C₁₋₄alkoxy, nitro, cyano, hydroxy, a C₁₋₄alkanoylamino, carbamoyl and sulfamoyl.

Examples of the C₁₋₄alkoxy in C₁₋₄alkoxy which may be substituent represented by R^{d3} include e.g. methoxy, ethoxy, propoxy, etc, and example of the substituent in C₁₋₄alkoxy which may be substituent represented by R^{d3} include e.g. a halogen (e.g. fluorine, chlorine, bromine, iodine, etc.), phenyl, etc.

Examples of the amino group which may be substituted represented by R^{d3} include not only an unsubstituted amino group but also, e.g. a C₁₋₆alkylamino (e.g. methylamino, ethylamino, propylamino, isopropylamino, butylamino, isobutylamino, t-butylamino, pentylamino, hexylamino, etc.), a di (C₁₋₆alkyl) amino (e.g. dimethylamino, diethylamino, dipropylamino, dibutylamino, etc.), a C₁₋₄alkoxyamino (e.g. methoxyamino, ethoxyamino, n-propoxyamino, isopropoxyamino, n-butoxyamino, isobutoxyamino, sec-butoxyamino, tert-butoxyamino, etc.), etc.

Examples of the C₁₋₆ alkyl group represented by R^{d4} and R^{d5} include e.g. methyl, ethyl, propyl, isopropyl, butyl, isobutyl, t-butyl, pentyl, hexyl, etc.

Examples of the C₁₋₆ alkyl group represented by R^{d6} include e.g. methyl, ethyl, propyl, isopropyl, butyl, isobutyl, t-butyl, pentyl, hexyl, etc. and examples of the C₂₋₆ alkenyl group include e.g. vinyl, allyl, 1-propeny, isopropeny, 2-butenyl, 3-butenyl, 2-hexenyl, etc.

Among the compounds of the formula (IV) or salts thereof (hereinafter referred to as Compound (IV)), the following compounds are preferable.

(IV-1) a compound wherein R^{d3} is 1) C₁₋₆ alkyl group which may be substituted by a halogen, a C₁₋₄ alkoxy or phenyl, 2) a C₂₋₆alkenyl group, 3) a C₂₋₆alkynyl group, 4) aC₃₋₈cycloalkylgroup which may be substituted by a halogen, a C₁₋₆alkyl or a C₁₋₄alkoxy, 5) a C₆₋₁₀aryl group which may be substituted by a halogen, a C₁₋₆alkyl, a C₁₋₄alkoxy, nitro, cyano, hydroxy, a C₁₋₄alkanoylamino, carbamoyl or sulfamoyl, 6) a C₁₋₄alkoxy group which may be substituted by a halogen or phenyl, or 7) an amino group which may be substituted by one or two groups selected from the group consisting of a C₁₋₆alkyl and a C₁₋₄alkoxy;

(IV-2) a compound wherein R^{d3} is 1) C₁₋₆ alkyl group, 2) a C₂₋₆alkenyl group, 3) a C₃₋₈cycloalkylgroup, 4) a C₁₋₄alkoxy group or 5) an amino group which may be substituted by a C₁₋₆alkyl or a C₁₋₄alkoxy, X^{d} is -SO₂- or -CO-, nd is 1 or 2, md is 0, 1 or 2, R^{d4} and R^{d5} are the same or different, and each of which is a hydrogen atom or a methyl group, R^{d6} is a hydroxy group, methyl group or a C₂₋₆alkenyl group, and rd is 3.

(IV-3) a compound wherein A^{d} is a group of the formula: 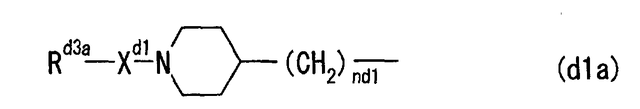 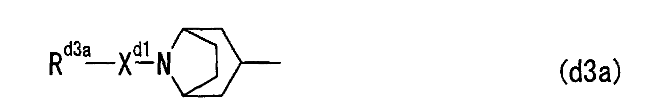 or 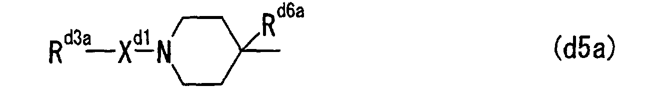 wherein, R^{d3a} is a C₁₋₆alkyl group, X^{d1} is -SO₂- or -CO-, nd1 is 1 or 2, R^{d6a} is a hydroxy group or a methyl group; rd is 3, B^{d} is -CH₂-, each of pd and qd is 0, 1 or 2, R^{d1} is a halogen atom, a methylgroup, R^{d2} is a halogen, a C₁₋₄alkoxy, nitro, a C₁₋₄alkanoylamino, SO₂NR^{db}R^{dc}, SO₂R^{dd}, CONR^{db}R^{dc}, NR^{db}R^{dc} or NR^{da}-SO₂R^{dd}, wherein R^{da} is a hydrogen atom, a C₁₋₆alkyl group, a C₂₋₆alkenyl group or a C₃₋₈cycloalkyl group, R^{db} and R^{dc} are the same or different, and each of which is a hydrogen atom, a C₁₋₆alkyl group or a C₃₋₈cycloalkyl group, or R^{db} and R^{dc} may combine each other together with the nitrogen atom to form a cyclic amino group, R^{dd} is a C₁₋₆alkylgroup or a C₃₋₈cycloalkyl group.

"A 5- to 6-membered cyclic ring" of "a 5- to 6-membered cyclic ring group which may be substituted", which is represented by R^{e1} in the above-mentioned formula (eI), is exemplified by a group which is formed by removing one hydrogen atom from a 6-membered aromatic hydrocarbon such as benzene or the like, a 5- to 6-membered aliphatic hydrocarbon such as cyclopentane, cyclohexane, cyclopentene, cyclohexene, cyclopentadiene, cyclohexadiene or the like, a 5- to 6-membered aromatic heterocyclic ring, which contains 1 to 4 heteroatoms of 1 to 2 kinds selected from the nitrogen atom, the sulfur atom and the oxygen atom, such as furan, thiophene, pyrrole, imidazole, pyrazole, thiazole, oxazole, isothiazole, isoxazole, tetrazole, pyridine, pyrazine, pyrimidine, pyridazine, triazole or the like, a 5- to 6-membered, non-aromatic heterocyclic ring, which contains 1 to 4 heteroatoms of 1 to 2 kinds selected from the nitrogen atom, the sulfur atom and the oxygen atom, such as tetrahydrofuran, tetrahydrothiophene, dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran or the like, or the like, where as for "a 5- to 6-membered cyclic ring" is preferably benzene, furan, thiophene, pyridine, cyclopentane, cyclohexane, pyrrolidine, piperidine, piperazine, morpholine, thiomorpholine, tetrahydropyran (preferably, a 6-membered cyclic ring) or the like, particularly benzene.

Examples of "a substituent" which may be possessed by "a 5- to 6-membered cyclic ring" of "a 5- to 6-membered cyclic ring group which may be substituted", which is represented by R^{e1}, to be used include a halogen atom, nitro, cyano, an alkyl which may be substituted, a cycloalkyl which may be substituted, the hydroxyl group which may be substituted, the thiol group which may be substituted (the sulfur atom may be oxidized to form a sulfinyl group which may be substituted or a sulfonyl group which may be substituted), an amino group which may be substituted, an acyl which may be substituted, the carboxyl group which may be esterified, an aromatic group which may be substituted and the like.

A halogen as a substituent of R^{e1} is exemplified by fluorine, chlorine, bromine, iodine or the like, where fluorine and chlorine are particularly preferable.

An alkyl for an alkyl which may be substituted as a substituent of R^{e1} is exemplified by a straight-chain or branched alkyl having a carbon number of 1 to 10, for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl. A substituent in the alkyl which may be substituted is exemplified by a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxyl which may be halogenated (for example, methoxy, ethoxy, propoxy, butoxy, trifluoromethoxy, trifluoroethoxy or the like), a C₁₋₄ alkoxy-C₁₋₄ alkoxyl which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like) or the like, where the number of the substituents is preferably 1 to 3.

The cycloalkyl for a cycloalkyl which may be substituted as a substituent of R^{e1} is exemplified by, for example, a C₃₋₇ cycloalkyl such as cyclopropyl, cyclobutyl, cyclopentyl, cyclohexyl, cycloheptyl or the like. The substituent in the cycloalkyl which may be substituted is exemplified by a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxyl which may be halogenated (for example, methoxy, ethoxy, propoxy, butoxy, trifluoromethoxy, trifluoroethoxy or the like), a C₁₋₄ alkoxy-C₁₋₄ alkoxyl which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like) or the like, where the number of the substituents is preferably 1 to 3.

The substituent for the hydroxyl group which may be substituted as a substituent of R^{e1} is exemplified by a substituent such as (1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, etc.);
(2) a cycloalkyl which may be substituted and may contain heteroatoms (for example, a C₃₋₇ cycloalkyl such as cyclopropyl, cyclobutyl, cyclopentyl, cyclohexyl, cycloheptyl or the like; a saturated, 5- to 6-membered heterocyclic ring group containing 1 to 2 heteroatoms such as tetrahydrofuranyl, tetrahydrothienyl, pyrrolidinyl, pyrazolidinyl, piperidyl, piperazinyl, morpholinyl, thiomorpholinyl, tetrahydropyranyl, tetrahydrothiopyranyl or the like (preferably, tetrahydropyranyl or the like); or. the like is exemplified);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl 2-cyclopentenylmethyl, 2-cyclohexenylmethyl or the like, or the like is exemplified);
(5) an aralkyl which may be substituted (for example, a phenyl-C₁₋₄ alkyl (for example, benzyl, phenethyl or the like) or the like is exemplified);
(6) formyl or an acyl which may be substituted (for example, an alkanoyl which has the carbon number of 2 to 4 (for example, acetyl, propionyl, butyryl, isobutyryl or the like), an alkylsulfonyl which has the carbon number of 1 to 4 (for example, methanesulfonyl, ethanesulfonyl or the like) or the like is exemplified); and
(7) an aryl which may be substituted (for example, phenyl, naphthyl or the like is exemplified),
where examples of the substituents which may be possessed by (1) an alkyl which may be substituted, (2) a cycloalkyl which may be substituted, (3) an alkenyl which may be substituted, (4) a cycloalkenyl which may be substituted, (5) an aralkyl which may be substituted, (6) an acyl which may be substituted and (7) an aryl which may be substituted include a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₆ alkoxyl which may be halogenated (for example, methoxy, ethoxy, propoxy, butoxy, trifluoromethoxy, trifluoroethoxy or the like; preferably, a C₁₋₄ alkoxyl which may be halogenated), formyl, a C₂₋₄ alkanoyl (for example, acetyl, propionyl or the like), a C₁₋₄ alkylsulfonyl (for example, methanesulfonyl, ethanesulfonyl or the like), a 5- to 6-membered, aromatic heterocyclic ring which may be substituted [for example, an aromatic heterocyclic ring, which contains 1 to 4 heteroatoms of 1 to 2 kinds selected from the nitrogen atom, the sulfur atom and the oxygen atom, such as furan, thiophene, pyrrole, imidazole, pyrazole, thiazole, oxazole, isothiazole, isoxazole, tetrazole, pyridine, pyrazine, pyrimidine, pyridazine, triazole or the like; the substituent which may be possessed by the heteroaromatic ring is exemplified by a halogen (for example, fluorine, chlorine, bromine, iodine or the like), nitro, cyano, the hydroxyl group, the thiol group, an amino group, the carboxyl group, a C₁₋₄ alkyl which may be halogenated (for example, trifluoromethyl, methyl, ethyl or the like), a C₁₋₄ alkoxyl which may be halogenated (for example, methoxy, ethoxy, propoxy, butoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.], or the like, where the number of the substituents is preferably 1 to 3.

The substituent for the thiol group which may be substituted as a substituent of R^{e1} is exemplified by a substituent similar to "a substituent for the hydroxyl group which may be substituted as a substituent of R^{e1"}, among which preferable is
(1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like is exemplified);
(3) an aralkyl which may be substituted (for example, a phenyl-C₁₋₄ alkyl (for example, benzyl, phenethyl or the like) or the like is exemplified); and
(4) an aryl which may be substituted (for example, phenyl, naphthyl or the like is exemplified),
where examples of the substituents which may be possessed by (1) an alkyl which may be substituted, (2) a cycloalkyl which may be substituted, (3) an aralkyl which may be substituted and (4) an aryl which may be substituted, which are described above, include a halogen (for example,. fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxyl which may be halogenated (for example, methoxy, ethoxy, propoxy, butoxy, trifluoromethoxy, trifluoroethoxy or the like), a C₁₋₄ alkoxy-C₁₋₄ alcoxy which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

The substituent for the amino group which may be substituted as the substituent of R^{e1} is exemplified by an amino group which may have 1 to 2 substituents similar to "a substituent for the hydroxyl group which may be substituted as a substituent of R^{e1"}, among which preferable is (1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified);
(5) formyl, or an acyl which may be substituted (for example, an alkanoyl which has the carbon number of 2 to 4 (for example, acetyl, propionyl, butyryl, isobutyryl or the like), an alkylsulfonyl which has the carbon number of 1 to 4 (for example, methanesulfonyl, ethanesulfonyl or the like) or the like is exemplified); and
(6) an aryl which may be substituted (for example, phenyl, naphthyl or the like is exemplified),
where examples of the substituents which may be possessed by (1) an alkyl which may be substituted, (2) a cycloalkyl which may be substituted, (3) an alkenyl which may be substituted, (4) a cycloalkenyl which may be substituted, (5) an acyl which may be substituted and (6) an aryl which may be substituted, which are described above, include a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxy which may be halogenated (for example, methoxy, ethoxy, propoxy, butoxy, trifluoromethoxy, trifluoroethoxy or the like), C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy or the like) formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

In addition, as for the substituents for an amino group which may be substituted as a substituent of R^{e1}, the substituents for the amino group may bind together to form a cyclic ring amino group (for example, a cyclic ring amino group which is formed by removing one hydrogen from the nitrogen atom constituting the ring of a 5- to 6-membered cyclic ring such as tetrahydropyrrole, piperazine, piperidine, morpholine, thiomorpholine, pyrrole, imidazole or the like, and has a bond on the nitrogen atom). The cyclic ring amino group may have substituents, and such substituents are exemplified by a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxy which may be halogenated (for example, methoxy, ethoxy, propoxy, butoxy, trifluoromethoxy, trifluoroethoxy or the like), a C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

The acyl which may be substituted as a substituent of R^{e1} is exemplified by a group, wherein the carbonyl group or the sulfonyl group is bonded with
(1) hydrogen,
(2) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(3) a cycloalkyl which may be substituted (for example, a C₃₋₇ cycloalkyl such as cyclopropyl, cyclobutyl, cyclopentyl, cyclohexyl, cycloheptyl or the like);
(4) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(5) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified); or
(6) a 5- to 6-membered, monocyclic ring aromatic group which may be substituted (for example, phenyl, pyridyl or the like is exemplified), which is bonded with a carbonyl group or a sulfonyl group, (for example, acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl or the like), where examples of the substituents which may be possessed by (2) the alkyl which may be substituted, (3) the cycloalkyl which may be substituted, (4) the alkenyl which may be substituted, (5) the cycloalkenyl which may be substituted and (6) the 5- to 6-membered, monocyclic ring aromatic group which may be substituted, which are described above, include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), a carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxy which may be halogenated (for example, methoxy, ethoxy, propoxy, butoxy, trifluoromethoxy, trifluoroethoxy or the like), a C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

The carboxyl group which may be esterified as a substituent of R^{e1} is exemplified by a group, wherein the carbonyloxy group is bonded with
(1) hydrogen,
(2) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(3) a cycloalkyl which may be substituted and may contain heteroatoms (for example, a C₃₋₇ cycloalkyl such as cyclopropyl, cyclobutyl, cyclopentyl, cyclohexyl, cycloheptyl or the like);
(4) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(5) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified); or
(6) an aryl which may be substituted (for example, phenyl, naphthyl or the like is exemplified), preferably carboxyl, a lower (C₁₋₆) alkoxycarbonyl and an aryloxycarbonyl (for example, methoxycarbonyl, ethoxycarbonyl, propoxycarbonyl, phenoxycarbonyl, naphthoxycarbonyl or the like), where examples of the substituents which may be possessed by (2) an alkyl which may be substituted, (3) a cycloalkyl which may be substituted, (4) an alkenyl which may be substituted, (5) a cycloalkenyl which may be substituted and (6) an aryl which may be substituted, which are described above, include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxy which may be halogenated (for example, methoxy, ethoxy, propoxy, butoxy, trifluoromethoxy, trifluoroethoxy or the like), a C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

The aromatic group for an aromatic group which may be substituted as a substituent of R^{e1} is exemplified by a 5-to 6-membered, same element or heterocyclic ring, aromatic group such as phenyl, pyridyl, furyl, thienyl, pyrrolyl, imidazolyl, pyrazolyl, thiazolyl, oxazolyl, isothiazolyl, isooxazolyl, tetrazolyl, pirazinyl, pyrimidinyl, pyridazinyl, triazolyl or the like, a condensed, heterocyclic ring aromatic group such as benzofuran, indole, benzothiophene, benzoxazole, benzthiazole, indazole, benzimidazole, quinoline, isoquinoline, quinoxaline, phthalazine, quinazoline, cinnoline or the like, or the like. Examples of the substituents for these aromatic groups include a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, propoxy, butoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

One to four (preferably one or two) of these substituents of R^{e1} may be the same or different and present at any position. Also, in the case where "a 5- to 6-membered cyclic ring" of "a 5- to 6-membered cyclic ring which may be substituted", which is represented by R^{e1}, has 2 or more substituents, 2 substituents of them may be bonded together to form, for example, a lower (C₁₋₆) alkylene (for example, trimethylene, tetramethylene or the like), a lower (C₁₋₆) alkyleneoxy (for example, -CH₂-O-CH₂-, -O-CH₂-CH₂-, -O-CH₂-CH₂-CH₂-, -O-CH₂-CH₂-CH₂-CH₂-, -O-C (CH₃) (CH₃) -CH₂-CH₂- or the like) , a lower (C₁₋₆) alkylenethio (for example, -CH₂-S-CH₂-, -S-CH₂-CH₂-, -S-CH₂-CH₂-CH₂-, -S-CH₂-CH₂-CH₂-CH₂-, -S-C (CH₃) (CH₃)-CH₂-CH₂- or the like), a lower (C₁₋₆) alkylenedioxy (for example, -O-CH₂-O-, -O-CH₂-CH₂-O-, -O-CH₂-CH₂-CH₂-O- or the like), a lower (C₁₋₆) alkylenedithio (for example, -S-CH₂-S-, -S-CH₂-CH₂-S-, -S-CH₂-CH₂-CH₂-S- or the like), an oxy-lower (C₁₋₆) alkylenamino (for example, -O-CH₂-NH-, -O-CH₂-CH₂-NH- or the like), an oxy-lower (C₁₋₆) alkylenethio (for example, -O-CH₂-S-, -O-CH₂-CH₂-S- or the like), a lower (C₁₋₆) alkylenamino (for example, -NH-CH₂-CH₂-, -NH-CH₂-CH₂-CH₂- or the like) , a lower (C₁₋₆) alkylenediamino (for example, -NH-CH₂-NH-, -NH-CH₂-CH₂-NH- or the like), a thia-lower (C₁₋₆) alkylenamino (for example, -S-CH₂-NH-, -S-CH₂-CH₂-NH- or the like), a lower (C₂₋₆) alkenylene (for example, -CH₂-CH=CH-, -CH₂-CH₂-CH=CH-, -CH₂-CH=CH-CH₂-CH₂- or the like), a lower (C₄₋₆) alkadienylene (for example, -CH=CH-CH=CH- or the like) or the like.

Furthermore, the bivalent group which is formed by binding each other 2 substituents of R^{e1} may have 1 to 3 substituents similar to "the substituent" which may be possessed by "a 5- to 6-membered cyclic ring" of "a 5- to 6-membered cyclic ring which may be substituted", which is represented by R^{e1}, (a halogen atom, nitro, cyano, an alkyl which may be substituted, a cycloalkyl which may be substituted, the hydroxyl group which may be substituted, the thiol group which may be substituted (the sulfur atom may be oxidized to form a sulfinyl group which may be substituted or a sulfonyl group which may be substituted), an amino group which may be substituted, an acyl which may be substituted, the carboxyl group which may be esterified or amidated, an aromatic group which may be substituted and the like).

"The substituent" which may be possessed by "a 5- to 6-membered cyclic ring" of "a 5- to 6-membered cyclic ring which may be substituted", which is represented by R^{e1}, is exemplified particularly by a lower (C₁₋₄) alkyl which may be halogenated or alkoxylated with a lower (C₁₋₄) alkyl (for example, methyl, ethyl, t-butyl, trifluoromethyl, methoxymethyl, ethoxymethyl, propoxymethyl, butoxymethyl, methoxyethyl, ethoxyethyl, propoxyethyl, butoxyethyl, or the like), a lower (C₁₋₄) alkoxyl which may be halogenated or alkoxylated with a lower (C₁₋₄) alkyl (for example, methoxy, ethoxy, propoxy, butoxy, t-butoxy, trifluoromethoxy, methoxymethoxy, ethoxymethoxy, propoxymethoxy, butoxymethoxy, methoxyethoxy, ethoxymethoxy, propoxyethoxy, butoxyethoxy, methoxypropoxy, ethoxypropoxy, propoxypropoxy, butoxypropoxy or the like), a halogen (for example, fluorine, chlorine or the like), nitro, cyano, an amino group which may be substituted with 1 to 2 of a lower (C₁₋₄) alkyl, formyl or a lower (C₂₋₄) alkanoyl (for example, amino, methylamino, dimethylamino, formylamino, acetylamino or the like), a 5- to 6-membered cyclic ring amino group (for example, 1-pyrrolidinyl, 1-piperazinyl, 1-piperidinyl, 4-morpholino, 4-thiomorpholino, 1-imidazolyl, 4-tetrahydropyranyl or the like) or the like.

"A bivalent group, in which the number of atoms constituting the straight-chain portion is 1 to 4", represented by X^{e1} and X^{e2}, is exemplified by, for example, - (CH₂) ₑₐ,- [ea' represents an integer of 1 to 4 (preferably, an integer of 1 to 2)], -(CH₂)_{eb},-X^{e3}- [eb' represents an integer of 0 to 3 (preferably, an integer of 0 to 1) and X^{e3} represents an imino group which may be substituted (for example, an imino group which may be substituted with a lower (C₁₋₆) lower alkyl, a lower (C₃₋₇) cycloalkyl, formyl, a lower (C₂₋₇) lower alkanoyl, a lower (C₁₋₆) lower alkoxycarbonyl or the like), the carbonyl group, the oxygen atom, the sulfur atom which may be oxidized by the oxygen atom (for example, -S (O) ₑₘ- (em represents an integer of 0 to 2) or the like)], -CH=CH-, -C≡C-, -CO-NH-, -SO₂-NH- or the like. Although these groups may be bonded with W^{e} through either of the left or right bond, it is preferable to be bonded with W^{e} through the right hand in the case of X^{e1}, whereas it is preferable to be bonded with W^{e} through the left hand in the case of X^{e2}.

As for X^{e1}, a bond, - (CH₂) _{eb'}-O- [eb' is an integer of 0, 1 or 2 (preferably, an integer of 0 to 1), -C≡C- or the like is preferable, and a bond is more preferable.

As for X^{e2}, - (CH₂) _{ea"}- [ea" is an integer of 1 to 2], -(CH₂)_{eb"}-X^{e3}- [eb" is an integer of 0 to 1 and X^{e3} is an imino group which may be substituted, the carbonyl group, the oxygen atom, the sulfur atom which may be oxidized by the oxygen atom], -CH=CH-, -CO-NH-, -SO₂-NH- or the like is preferable, and -CO-NH- is more preferable.

In the above-mentioned formula (eI), a bivalent group which is represented by formula representd by W^{e} 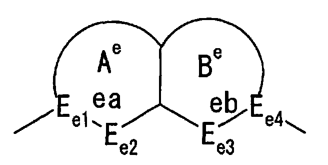 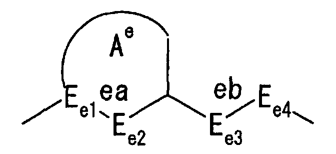 and 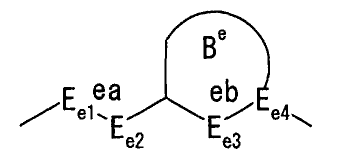 (wherein each of ring A^{e} and ring B^{e} is a 5- to 7-membered cyclic ring group which may be substituted, each of Eₑ₁ and Eₑ₄ represents the carbon atom which may be substituted or the nitrogen atom which may be substituted, each of Eₑ₂ and Eₑ₃ represents the carbon atom which may be substituted or the nitrogen atom which may be substituted, the sulfur atom which may be oxidized (for example, -S(O)ₑₘ- (em represents an integer of 0 to 2) or the like) or the oxygen atom and each of ea and eb represents to be a single bond or a double bond) represents which the bonding with adjacent X^{e1} and X^{e2} is made in a mode which is shown respectively by 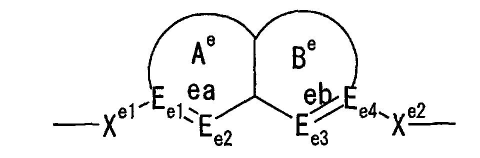 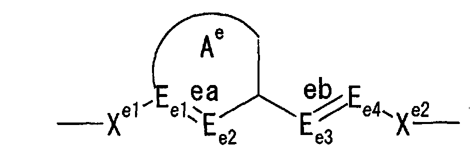 and 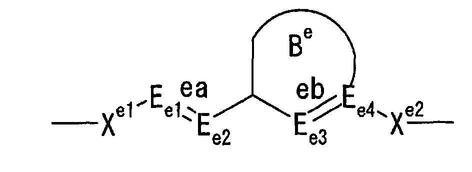 (wherein each of the symbols has the meaning defined above).

In the above-mentioned formula (eI), "a 5- to 7-membered cyclic ring" of "a 5- to 7-membered cyclic ring which may be substituted", which is represented by A^{e}, is exemplified by a 5- to 7-membered (preferably, 5- to 6-membered, saturated or unsaturated alicyclic hydrocarbon such as a C₅₋₇ cycloalkane (for example, cyclopentane, cyclohexane, cycloheptane or the like), a C₅₋₇ cycloalkene (for example, 1-cyclopentene, 2-cyclopentene, 3-cyclopentene, 2-cyclohexene, 3-cyclohexene or the like), a C₅₋₆ cycloalkadiene (for example, 2,4-cyclopentadiene, 2,4-cyclohexadiene, 2,5-cyclohexadiene or the like); a 6-membered aromatic hydrocarbon such as benzene; a 5- to 7-membered, aromatic heterocyclic ring, a saturated or unsaturated non-aromatic heterocyclic ring (aliphatic heterocyclic ring) or the like, which contains at least one (preferably, 1 to 4, more preferably 1 to 2) of heteroatoms of 1 to 3 kinds (preferably, 1 to 2 kinds) selected from the oxygen atom, the sulfur atom and the nitrogen atom; or the like.

Herein, "an aromatic heterocyclic ring" is exemplified by a 5- to 7-membered, aromatic monocyclic heterocyclic ring (for example, furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazane, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine or the like) or the like, and "a non-aromatic heterocyclic ring" is exemplified by, for example, a 5- to 7-membered (preferably, 5- to 6-membered), saturated or unsaturated non-aromatic heterocyclic ring (aliphatic heterocyclic ring) such as pyrrolidine, tetrahydrofuran, thiolan, piperidine, tetrahydropyran, morpholine, thiomorpholine, piperazine, pyran, oxepine, thiepin, azepine or the like, or a 5- to 6-membered, non-aromatic heterocyclic ring, where a part or all of the double bonds in the above-mentioned aromatic monocyclic heterocyclic ring are saturated, or the like.

As for "a 5- to 7-membered cyclic ring" of "a 5- to 7-membered cyclic ring which may be substituted", which is represented by A^{e}, a 5- to 6-membered, aromatic ring is preferable, and further benzene, furan, thiophene, pyrrole, pyridine (preferably, 6-membered) or the like is preferable, where benzene is particularly preferable.

"The substituent" which may be possessed by "a 5- to 7-membered cyclic ring" of "a 5- to 7-membered cyclic ring which may be substituted", which is represented by A^{e}, is exemplified by a group similar to "the substituent" which may be possessed by "a 5- to 6-membered cyclic ring" of "a 5- to 6-membered cyclic ring which may be substituted", which is represented by R^{e1}. Also, 1 to 4 (preferably, 1 to 2) of the substituent for A^{e} may be present at any positions of the same or different rings, and the substituent may be possessed at any substitutable position, whether it is any of the positions that are representd by Eₑ₁ and Eₑ₂ or any of other positions.

In the above-mentioned formula (eI), "a 5- to 7-membered cyclic ring" of "a 5- to 7-membered cyclic ring which may be substituted", which is represented by B, is exemplified by a 5- to 7-membered cyclic ring which may have substituents at optional, substitutable positions, which is represented by formula 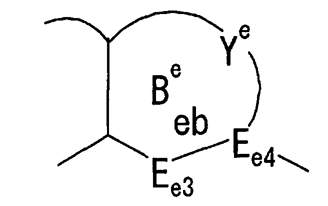 or the like.

In the above-mentioned formula (eI), the bivalent group which is represented by Y^{e} is a bivalent group, with which ring B^{e} forms a 5- to 7-membered cyclic ring which may be substituted, and is exemplified by a bivalent group such as, for example,
(1) -(CH₂)ₑₐ₁-O-(CH₂)ₑₐ₂- (each of ea1 and ea2 represents 0, 1 or 2 in the same or different manner. Hereupon, the sum of ea1 and ea2 is 2 or less), -O-(CH=CH)-, -(CH=CH)-O-,
(2) -(CH₂) _{eb1}-S(O) ₑₘ-(CH₂) _{eb2}- (em represents an integer of 0 to 2, and each of eb1 and eb2 represents 0, 1 or 2 in the same or different manner. Hereupon, the sum of b1 and b2 is 2 or less), -S(O) ₑₘ-(CH=CH)-, -(CH=CH)-S(O) ₑₘ-,
(3) -(CH₂) _{ed1}- (de1 represents 1, 2 or 3), -CH₂-(CH=CH)-, -(CH=CH)-CH₂-, -CH=CH-, -CH=,
(4) -(CH₂)ₑ₁-NH-(CH₂)ₑ₂- (each of e1 and e2 represents 0, 1 or 2 in the same or different manner. Hereupon, the sum of e1 and e2 is 2 or less), -NH-(CH=CH)-, -(CH=CH)-NH-, -(CH₂)ₑ₆-(N=CH)-(CH₂)ₑ₇-, -(CH₂)ₑ₇-(CH=N)-(CH₂)ₑ₆- (either of e6 and e7 represents 0, and the other represents 0 or 1), -(CH₂) ₑ₈- (N=N) - (CH₂)ₑ₉- (either of e8 and e9 represents 0, and the other represents 0 or 1) or the like. Specifically, the bivalent group is exemplified by, for example, a bivalent group such as -O-, -O-CH₂-, -O-CH₂-CH₂-, -O-CH=CH-, - S (O) ₑₘ- (em represents an integer of 0 to 2), -S(O) ₑₘ-CH₂- (em represents an integer of 0 to 2), -S(O) ₑₘ-CH₂-CH₂- (em represents an integer of 0 to 2), -S(O) ₑₘ-(CH=CH)-, (em represents an integer of 0 to 2), -CH₂-, -(CH₂)₂-, -(CH₂)₃-, - CH=, -CH=CH-, -CH=CH-CH₂-, -CH₂-CH=CH-, -NH-, -N=CH-, -CH=N-, -N=N- (respectively, the bonding represented starts from ring A) or the like.

In addition, the bivalent group may have a substituent, and the substituent is exemplified by a group similar to "the substituent" which may be possessed by "a 5- to 6-membered cyclic ring" of "a 5- to 6-membered cyclic ring which may be substituted", which is represented by R^{e1}, oxo and the like, where a lower (C₁₋₃) alkyl (for example, methyl, ethyl, propyl or the like), phenyl, oxo, a hydroxyl group or the like is particularly preferable. Furthermore, the bivalent group may be -O-C(O)- (the bonding represented starts from ring A) or the like. The substituent for such a bivalent group may be present at any same or different positions of 1 to 4 (preferably, 1 to 2). The substituent position may be any position that is substitutable to the bivalent group.

As the bivalent group which is represented by Y^{e}, a group such as -Y^{e'}-(CH₂)_{em'}- (Y^{e}' is -S (O)ₑₘ- (em is an integer of 0 to 2), -O-, -NH- or -CH₂-, and em' is an integer of 0 to 2), -CH=, -CH=CH-, -N=CH-, -(CH₂)ₑₘ'-Y^{e'}- (Y^{e}' is -S(O)ₑₘ- (em is an integer of 0 to 2), -O-, -NH- or - CH₂-, and em' is an integer of 0 to 2), -CH=N- or the like, with starting the bonding representd from ring A^{e}, is preferable, where a group such as -Y^{e'} - (CH₂) _{em'}- (Y^{e}' is -S(O)ₑₘ- (em is an integer of 0 to 2), -O-, -NH- or -CH₂-, and em' is an integer of 0 to 2), -CH=, -CH=CH-, -N=CH- or the like, with starting the bonding representd from ring A^{e}, is more preferable, and a group (ring B^{e} is a 5- to 7-membered ring which may be substituted) such as -Y^{e}'-(CH₂)₂- (Y^{e}' represents -S(O)ₑₘ- (em represents an integer of 0 to 2), -O-, -NH- or -CH₂-), with starting the bonding representd from ring A^{e}, is most preferable.

"The substituent" which may be possessed by "a 5- to 7-membered cyclic ring" of "a 5- to 7-membered cyclic ring which may be substituted", which is represented by B^{e}, is exemplified by a group similar to "the substituent" which may be possessed by "a 5- to 6-membered cyclic ring" of "a 5- to 6-membered cyclic ring which may be substituted", which is represented by R^{e1}. Also, such a substituent for B^{e} may be present at any positions of 1 to 4 (preferably, 1 to 2) of the same or different rings, but it is preferable that the position of Eₑ₃ is unsubstituted.

In the above-mentioned formula (eI), it is preferable that each of Eₑ₃ and Eₑ₄ is the carbon atom that may be substituted (preferably, the carbon atom which is not substituted), and eb is a double bond.

In the above-mentioned formula (eI), "a bivalent cyclic ring group" representd by Z^{e1} is exemplified by a group which is formed by removing two hydrogen atoms from a group similar to "a 5- to 6-membered cyclic ring" of "a 5-to 6-membered cyclic ring which may be substituted", or the like, where a bivalent cyclic ring group, which is formed by removing two hydrogen atoms from benzene, furan, thiophene, pyridine, cyclopentane, cyclohexane, pyrrolidine, piperidine, piperazine, morpholine, thiomorpholine, tetrahydropyran or the like, is more preferable, and a bivalent cyclic ring group, which is formed by removing two hydrogen atoms from benzene, cyclohexane or piperidine (preferably, benzene) is most preferably used.

"A bivalent cyclic ring group" representd by Z^{e1} may have a substituent similar to "a substituent" which is possessed by "a 5- to 6-membered cyclic ring" of "a 5- to 6-membered cyclic ring which may be substituted", but it is preferable not to have a substituent other than X^{e2} and Z^{e2}, and also it is preferable that the substitution position for Z^{e2} is para to X^{e2} in the case where Z^{e1} is a 6-membered, bivalent cyclic ring group (preferably, phenylene).

In the above-mentioned formula (eI), "a bivalent group, in which the number of hydrogen atoms constituting the straight-chain portion is 1 to 4", which is represented by Z^{e2}, is exemplified by a bivalent group which has a hydrocarbon chain having the carbon number of 1 to 4, which may be substituted (for example, a C₁₋₄ alkylene, a C₂₋₄ alkenylene or the like, preferably a C₁₋₃ alkylene, more preferably methylene), or the like.

A bivalent group, which is represented by Z^{e2}, may be any group having a bivalent chain, in which the number of atoms constituting the straight-chain portion is 1 to 4, and is exemplified by, for example, an alkylene chain represented by -(CH₂)ₑₖ₁- (ek1 is an integer of 1 to 4), an alkenylene chain represented by -(CH₂)ₑₖ₂-(CH=CH)-(CH₂)ₑₖ₃- (each of ek2 and ek3 represents an integer of 0, 1 or 2 in the same or different manner. Hereupon, the sum of ek2 and ek3 is 2 or less) or the like.

A bivalent group, which is represented by X^{e1}, X^{e2} and Z^{e2}, may have a substituent at an optional position (preferably, on the carbon atom), where such a substituent may be any one that is capable of being bonded to a bivalent chain constituting the straight-chain portion, is exemplified by, for example, a lower (C₁₋₆) alkyl (for example, methyl, ethyl, propyl, isopropyl, butyl, isobutyl, sec-butyl, tert-butyl, pentyl, isopentyl, neopentyl, hexyl or the like), a lower (C₃₋₇) cycloalkyl (for example, cyclopropyl, cyclobutyl, cyclopentyl, cyclohexyl, cycloheptyl or the like), formyl, a lower (C₂₋₇) alkanoyl (for example, acetyl, propionyl, butyryl or the like), a phosphono group which may be esterified, the carboxyl group which may be esterified, the hydroxyl group, oxo or the like, where preferably a lower alkyl having the carbon number of 1 to 6 (preferably a C₁₋₃ alkyl), the hydroxyl group, oxo or the like is exemplified.

The phosphono group which may be esterified is exemplified by a group represented by -P(O) (OR^{e7}) (OR^{e8}) [wherein each of R^{e7} and R^{e8} is hydrogen, an alkyl group having the carbon number of 1 to 6 or a cycloalkyl group having the carbon number of 3 to 7, and may be bonded together to form a 5- to 7-membered cyclic ring].

In the above-mentioned formula, an alkyl group having the carbon number of 1 to 6, which is represented by R^{e7} and R^{e8}, is exemplified by methyl, ethyl, propyl, isopropyl, butyl, isobutyl, sec-butyl, tert-butyl, pentyl, isopentyl, neopentyl, hexyl or the like, and a cycloalkyl having the carbon number of 3 to 7 is exemplified by cyclopropyl, cyclobutyl, cyclopentyl, cyclohexyl, cycloheptyl or the like), where a lower alkyl having the carbon number of 1 to 6 is exemplified preferably, and a lower alkyl having the carbon number of 1 to 3 is exemplified more preferably. R^{e7} and R^{e8} may be the same or different, but it is preferable to be the same. Also, in the case where R^{e7} and R^{e8} are bonded together to form a 5- to 7-membered cyclic ring, R^{e7} and R^{e8} are bonded together to form a straight-chained C₂₋₄ alkylene side chain represented by -(CH₂)₂-, -(CH₂)₃-, -(CH₂)₄-. The side chain may have substituents, and such a substituent is exemplified by, for example, the hydroxyl group, a halogen or the like.

The carboxyl group, which is esterified for the carboxyl group which may be esterified, is exemplified by a group, wherein the carboxyl group is bonded with an alkyl group having the carbon number of 1 to 6 or a cycloalkyl group having the carbon number of 3 to 7, such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl or the like.

A bivalent group, which is represented by Z^{e2}, is exemplified by a C₁₋₃ alkylene that may be substituted, where a C₁₋₃ alkylene that may be substituted with a C₁₋₃ alkyl, the hydroxyl group or oxo is more preferable.

Furthermore, as for a bivalent group, which is represented by Z^{e2}, a group represented by -Z^{e'}-(CH₂)en- or - (CH₂)en-Z^{e'} - (Z^{e'} is -CH (OH) -, -C (O) - or -CH₂-, en is an integer of 0 to 2 and each methylene group may have 1 to 2 groups of the same or different substituent), with starting from the benzene ring, is preferable, a group represented by -Z^{e'}-(CH₂)en- (Z^{e'} represents -CH (OH) -, -C (O) - or -CH₂-, en represents an integer of 0 to 2 (preferably, en is 0) and each methylene group may have 1 to 2 groups of the same or different substituents), with starting from the benzene ring, is more preferable and methylene is most preferable.

In the above-mentioned formula (eI), "an amino group" of "an amino group which may be substituted, where the nitrogen atom may be converted into a quaternary ammonium or the N-oxide" represented by R^{e2}, is exemplified by an amino group which may have 1 to 2 substituents, an amino group which has 3 substituents, where the nitrogen atom may be converted into a quaternary ammonium, or the like. In the case where the substituents on the nitrogen atom are 2 or more, these substituents may be the same or different, and in the case where the substituents on the nitrogen atom are 3, the amino group may be of any type of -N⁺ (R^{e}) ₃, -N⁺ (R^{e})₂R^{e'}, -N⁺R^{e}R^{e'}R^{e' '} (each of R^{e}, R^{e}' and R^{e' '} is hydrogen or a substituent in a different manner). In addition, a counter anion for the amino group, in which the nitrogen atom is converted into a quaternary ammonium, is exemplified by, in addition to an anion of a halogen atom (for example, Cl⁻, Br⁻, I⁻ or the like) or the like, an anion derived from an inorganic acid such as hydrochloric acid, hydrobromic acid, nitric acid, sulfuric acid, phosphoric acid and the like, an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like and an anion derived from an acidic amino acid such as aspartic acid, glutamic acid and the like, where Cl⁻, Br⁻, I⁻ or the like is more preferable.

Examples of substituents for the amino group include substituents such as,
(1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl or the like is exemplified);
(2) a cycloalkyl which may be substituted (for example, a C₃₋₈ cycloalkyl such as cyclopropyl, cyclobutyl, cyclopentyl, cyclohexyl, cycloheptyl, cyclooctyl or the like is exemplified);
   (2-1) the cycloalkyl contains one heteroatom selected from the sulfur atom, the oxygen atom and the nitrogen atom, and may form oxirane, thiolan, aziridine, tetrahydrofuran, tetrahydrothiophene, pyrrolidine, tetrahydropyran, tetrahydrothiopyran, tetrahydrothiopyran 1-oxide, piperidine or the like (preferably, a 6-membred ring such as tetrahydropyran, tetrahydrothiopyran, piperidine or the like), where the binding position with the amino group is preferably position 3 or position 4 (preferably, position 4);
   (2-2) also, the cycloalkyl may condensed with the benzene ring to form an indane (for example, indan-1-yl, indan-2-yl or the like), tetrahydronaphthalene, (for example, tetrahydronaphthalen-5-yl, tetrahydronaphthalen-6-yl or the like) or the like (preferably, indane, etc.);
   (2-3) furthermore, the cycloalkyl may be crosslinked via a straight-chained, atom chain having the carbon number of 1 to 2 to form a crosslinked, cyclic hydrocarbon residue such as bicyclo[2.2.1]heptyl, bicyclo[2.2.2]octyl, bicyclo[3.2.1]octyl, bicyclo[3.2.2]nonyl or the like (preferably, a cycloalkyl having a crosslink via a straight-chained, atom chain having the carbon number of 1 to 2, or the like, more preferably bicyclo[2.2.1]heptyl or the like);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified);
(5) an aralkyl which may be substituted (for example, a phenyl-C₁₋₄ alkyl (for example, benzyl, phenethyl or the like) or the like is exemplified);
(6) formyl or an acyl which may be substituted (for example, an alkanoyl which has the carbon number of 2 to 4 (for example, acetyl, propionyl, butyryl, isobutyryl or the like), an alkylsulfonyl which has the carbon number of 1 to 4 (for example, methanesulfonyl, ethanesulfonyl or the like), an alkoxycarbonyl which has the carbon number of 1 to 4 (methoxycarbonyl, ethoxycarbonyl, tert-butoxycarbonyl or the like), an aralkyloxycarbonyl which has the carbon number of 7 to 10 (for example, benzyloxycarbonyl or the like) or the like is exemplified);
(7) an aryl which may be substituted (for example, phenyl, naphthyl or the like is exemplified); and
(8) a heterocyclic ring group which may be substituted (a group which is formed by removing one hydrogen atom from a 5- to 6-membered, aromatic heterocyclic ring, which contains 1 to 4 heteroatoms of 1 to 2 kinds selected from the nitrogen atom, the sulfur atom and the oxygen atom, such as furan, thiophene, pyrrole, imidazole, pyrazole, thiazole, oxazole, isothiazole, isoxazole, tetrazole, pyridine, pyrazine, pyrimidine, pyridazine, triazole or the like, a group which is formed by removing one hydrogen atom from a 5- to 6-membered, non-aromatic heterocyclic ring, which contains 1 to 4 heteroatoms of 1 to 2 kinds selected from the nitrogen atom, the sulfur atom and the oxygen atom, such as tetrahydrofuran, tetrahydrothiophene, dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran or the like, or the like; preferably, a group, which is formed by removing one hydrogen atom from a 5- to 6-membered, non-aromatic heterocyclic ring, or the like; and more preferably, a group, which is formed by removing one hydrogen atom from a 5- to 6-membered, non-aromatic heterocyclic ring, which has one heteroatom, such as tetrahydrofuran, piperidine, tetrahydropyran, tetrahydrothiopyran or the like) and the like. In addition, the substituents of the amino group may be bonded each other to form a 5- to 7-membered, cyclic amino such as piperidine, piperazine, morpholine, thiomorpholine or the like.

Examples of the substituents which may be possessed by (1) an alkyl which may be substituted, (2) a cycloalkyl which may be substituted, (3) an alkenyl which may be substituted, (4) a cycloalkenyl which may be substituted, (5) an aralkyl which may be substituted, (6) an acyl which may be substituted, (7) an aryl which may be substituted and (8) a heterocyclic ring group which may be substituted, which are described above, include a halogen (for example, fluorine, chlorine, bromine, iodine or the like), a lower (C₁₋₄) alkyl which may be halogenated, a C₁₋₄ alkoxyl which may be halogenated (for example, methoxy, ethoxy, propoxy, butoxy, trifluoromethoxy, trifluoroethoxy or the like), a C₁₋₄ alkylenedioxy (for example, -O-CH₂-O-, -O-CH₂-CH₂-O- or the like), formyl, a C₂₋₄ alkanoyl (for example, acetyl, propionyl or the like), a C₁₋₄ alkylsulfonyl (for example, methanesulfonyl, ethanesulfonyl or the like), a phenyl-lower (C₁₋₄) alkyl, a C₃₋₇ cycloalkyl, cyano, nitro, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a lower (C₁₋₄) alkoxycarbonyl, a lower (C₇₋₁₀) aralkyloxycarbonyl, the oxo group (preferably, a halogen, a lower (C₁₋₄) alkyl which may be halogenated, a C₁₋₄ alkoxyl which may be halogenated, a phenyl-lower (C₁₋₄) alkyl, a C₃₋₇ cycloalkyl, cyano, a hydroxyl group or the like) and the like, where the number of the substituents is preferably 1 to 3.

In the above-mentioned formula (eI), "an amino group which may be substituted, where the nitrogen atom may be converted into a quaternary ammonium or the N-oxide" representd by R^{e2}, is preferably an amino group which may have 1 to 3 substituents selected from,
(1) a straight-chained or branched, lower (C₁₋₆) alkyl which may have 1 to 3 groups of a halogen, cyano, a hydroxyl group or a C₃₋₇ cycloalkyl;
(2) a C₅₋₈ cycloalkyl which may have 1 to 3 groups of a halogen, a lower (C₁₋₄) alkyl which may be halogenated or a phenyl-lower (C₁₋₄) alkyl, may contain one heteroatom selected from the sulfur atom, the oxygen atom and the nitrogen atom, may be condensed with the benzene ring and may be crosslinked via a straight-chained, atom chain having the carbon number of 1 to 2, (for example, cyclopentyl, cyclohexyl, cycloheptyl cyclooctyl, tetrahydropyranyl, tetrahydrothiapyranyl, piperidinyl, indanyl, tetrahydronaphthalenyl, bicyclo[2.2.1]heptyl or the like);
(3) a phenyl-lower (C₁₋₄) alkyl which may have 1 to 3 groups of a halogen, a lower (C₁₋₄) alkyl which may be halogenated or a C₁₋₄ alkoxyl which may be halogenated;
(4) a phenyl which may have 1 to 3 groups of a halogen, a lower (C₁₋₄) alkyl which may be halogenated or a C₁₋₄ alkoxyl which may be halogenated; and
(5) a 5- to 6-membered, aromatic heterocyclic ring which may have 1 to 3 groups of a halogen, a lower (C₁₋₄) alkyl which may be halogenated, a C₁₋₄ alkoxy which may be halogenated, a lower (C₁₋₄) alkoxy-(C₁₋₄) lower alkoxy which may be halogenated, a phenyl-lower (C₁₋₄) alkyl, cyano or the hydroxyl (for example, a group which is formed by removing one hydrogen atom from furan, thiophene, pyrrole, pyridine or the like).

In the above-mentioned formula (eI), "a nitrogen-containing, heterocyclic ring" of "a nitrogen-containing, heterocyclic group which may be substituted and may contain the sulfur atom or the oxygen atom as a ring-constituting atom, where the nitrogen atom may be converted into a quaternary ammonium or the N-oxide" is exemplified by a 5-to 6-membered, aromatic heterocyclic ring, which may contain, in addition to one nitrogen atom, 1 to 3 heteroatoms of 1 to 2 kinds selected from the nitrogen atom, the sulfur atom and the oxygen atom, such as pyrrole, imidazole, pyrazole, thiazole, oxazole, isothiazole, isoxazole, tetrazole, pyridine, pyrazine, pyrimidine, pyridazine, triazole or the like, a 5- to 8-membered, non-aromatic heterocyclic ring, which may contain, in addition to one nitrogen atom, 1 to 3 hetero atoms of 1 to 2 kinds selected from the nitrogen atom, the sulfur atom and the oxygen atom, such as pyrrolidine, pyrroline, imidazolidine, imidazoline, pyrazolidine, pyrazoline, piperidine, piperazine, oxazine, oxadiazine, thiazine, thiadiazine, morpholine, thiomorpholine, azacycloheptane, azacyclooctane (azokane) or the like, or the like, where each of these nitrogen-containing, heterocyclic rings may be crosslinked via a straight-chained, atom chain having the carbon number of 1 to 2 and may form a crosslinked, nitrogen-containing, heterocyclic ring such as azabicyclo[2.2.1]heptane, azabicyclo[2.2.2]octane (quinuclidine) or the like (preferably, piperidine, which may have a crosslink via a straight-chained, atom chain having the carbon number of 1 to 2, or the like).

Among the specific examples of the above-mentioned nitrogen-containing heterocyclic ring, pyridine, imidazole, pyrrolidine, piperidine, piperazine, morpholine, thiomorpholine and azabicyclo[2.2.2]octane (preferably, 6-membered, cyclic rings) are preferable.

The nitrogen atom of the "nitrogen-containingheterocyclic ring" may be converted into a quaternary ammonium, or may be oxidized. In the case where the nitrogen atom of the "nitrogen-containing heterocyclic ring" is converted into a quaternary ammonium, a counter anion for "the nitrogen-containing heterocyclic ring group, in which the nitrogen atom is converted into a quaternary ammonium", is exemplified by, in addition to an anion of a halogen atom (for example, Cl⁻, Br⁻, I⁻ or the like) or the like, an anion derived from an inorganic acid such as hydrochloric acid, hydrobromic acid, nitric acid, sulfuric acid, phosphoric acid and the like, an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like and an anion derived from an acidic amino acid such as aspartic acid, glutamic acid and the like, where Cl⁻, Br⁻, I⁻ or the like is more preferable.

The "nitrogen-containing heterocyclic ring" may be bonded with a bivalent group, which is represented by Z^{e2}, via either of the carbon atom or the nitrogen atom and may be bonded on the carbon atom constituting the cyclic ring, as in the case of 2-pyridyl, 3-pyridyl, 2-piperidyl or the like, whereas a bonding as in the case of 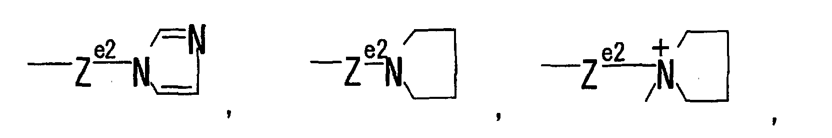 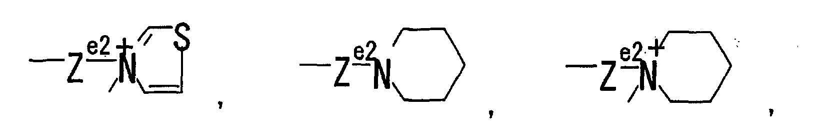 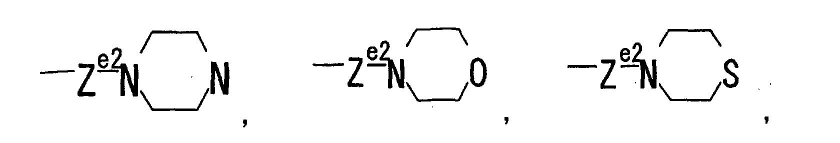 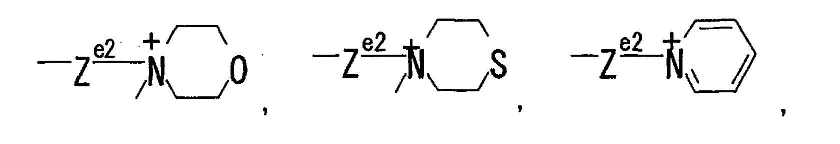 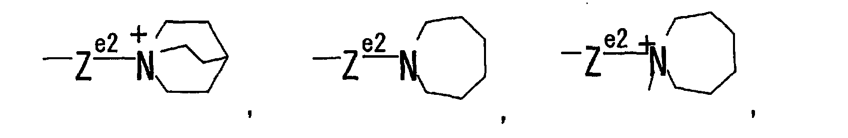 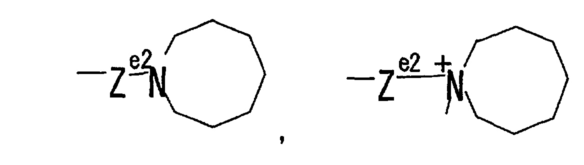 or the like is preferable.

Examples of substituents of the "nitrogen-containing heterocyclic ring" include a halogen (for example, fluorine, chlorine, bromine, iodine or the like), a lower (C₁₋₄) alkyl which may be substituted, a lower (C₁₋₄) alkoxyl which may be substituted, a phenyl which may be substituted, a mono- or diphenyl-lower (C₁₋₄) alkyl which may be substituted, a C₃₋₇ cycloalkyl which may be substituted, cyano, nitro, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a lower (C₁₋₄) alkoxycarbonyl, formyl, a (C₂₋₄) alkanoyl, a lower (C₁₋₄) alkylsulfonyl, a heterocyclic ring group which may be substituted (e.g. a group which is formed by removing one hydrogen atom from a 5- to 6-membered, aromatic heterocyclic ring, which contains 1 to 4 heteroatoms of 1 to 2 kinds selected from the nitrogen atom, the sulfur atom and the oxygen atom, such as furan, thiophene, pyrrole, imidazole, pyrazole, thiazole, oxazole, isothiazole, isoxazole, tetrazole, pyridine, pyrazine, pyrimidine, pyridazine, triazole or the like, a group which is formed by removing one hydrogen atom from a 5- to 6-membered, non-aromatic heterocyclic ring, which contains 1 to 4 heteroatoms of 1 to 2 kinds selected from the nitrogen atom, the sulfur atom and the oxygen atom, such as tetrahydrofuran, tetrahydrothiophene, dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran or the like, or the like, where the number of the substituents is preferably 1 to 3. Also, the nitrogen in the "nitrogen-containing heterocyclic ring" may be oxidized.

Examples of the substituents, which may be possessed by "a lower (C₁₋₄) alkyl which may be substituted", "a lower (C₁₋₄) alkoxyl which may be substituted", "a phenyl which may be substituted", "a mono- or diphenyl-lower (C₁₋₄) alkyl which may be substituted", "a C₃₋₇ cycloalkyl which may be substituted" and "a heterocyclic ring group which may be substituted", as the substituents which may be possessed by the "nitrogen-containing heterocyclic ring", include, for example, a halogen (for example, fluorine, chlorine, bromine, iodine or the like), a lower (C₁₋₄) alkyl which may be halogenated, a lower (C₃₋₁₀) cycloalkyl, a lower (C₃₋₁₀) cycloalkenyl, a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like), a C₁₋₃ alkylenedioxy (for example, methylenedioxy, ethylenedioxy or the like), cyano, nitro, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a lower (C₁₋₄) alkoxycarbonyl and the like, where the number of the substituents is preferably 1 to 3.

In the above-mentioned formula (eI), the substituent, which may be possessed by "a nitrogen-containing heterocyclic ring" of "a nitrogen-containing heterocyclic ring group which may be substituted and may contain the sulfur atom or the oxygen atom as a ring-constituting atom, where the nitrogen atom may be converted into a quaternary ammonium or the N-oxide" is exemplified preferably by (1) a halogen, (2) cyano, (3) a hydroxyl group, (4) a carboxyl group, (5) a lower (C₁₋₄) alkoxycarbonyl, (6) a lower (C₁₋₄) alkyl which may be substituted with a halogen, a hydroxyl group or a lower (C₁₋₄) alkoxy, (7) a lower (C₁₋₄) alkoxy which may be substituted with a halogen, the hydroxyl group or a lower (C₁₋₄) alkoxy, (8) a phenyl which may be substituted with a halogen, a lower (C₁₋₄) alkyl, a hydroxyl group, a lower (C₁₋₄) alkoxy or a C₁₋₃ alkylenedioxy, (9) a mono- or diphenyl-lower (C₁₋₄) alkyl which may be substituted with a halogen, a lower (C₁₋₄) alkyl, the hydroxyl group, a lower (C₁₋₄) alkoxy or a C₁₋₃ alkylenedioxy, (10) a group which is formed by removing one hydrogen atom from a 5- to 6-membered aromatic heterocyclic ring, such as furan, thiophene, pyrrole, pyridine or the like, or the like.

In the above-mentioned formula (eI), "a group which is bonded via the sulfur atom", which is represented by R^{e2}, is exemplified by a group represented by -S(O)em-R^{eS} (wherein em represents an integer of 0 to 2 and R^{eS} is a substituent). In the above-mentioned formula, examples of the substituent representd by R^{eS} include, for example,
(1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like is exemplified);
(3) an aralkyl which may be substituted (for example, a phenyl-C₁₋₄ alkyl (for example, benzyl, phenethyl or the like) or the like is exemplified); and
(4) an aryl which may be substituted (for example, phenyl, naphthyl or the like is exemplified), where examples of the substituents which may be possessed by (1) an alkyl which may be substituted, (2) a cycloalkyl which may be substituted, (3) an aralkyl which may be substituted and
(4) an aryl which may be substituted, which are described above, include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like) or the like,
where the number of the substituents is preferably 1 to 3.

In the above-mentioned formula (eI), "the hydrocarbon group" for the hydrocarbon group which may be substituted, which is represented by R^{e5'} and R^{e6'}, in "a group represented by formula 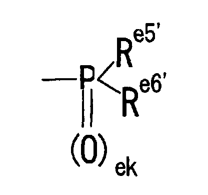 wherein ek represents 0 or 1, the phosphorus atom may form a phosphonium salt when ek is 0, and each of R^{e5'} and R^{e6'} is a hydrocarbon group which may be substituted, a hydroxyl group which may be substituted or an amino group which may be substituted, preferably, the hydrocarbon group which may be substituted or an amino group which may be substituted; more preferably the hydrocarbon group which may be substituted, and R^{e5'} and R^{e6'} may bind each other to form a cyclic ring group together with the adjacent phosphorus atom", which is represented by R^{e2}, is exemplified by,
(1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified);
(5) an alkinyl which may be substituted (for example, an alkinyl which has the carbon number of 2 to 10 such as ethinyl, 1-propinyl, 2-propinyl, 1-butinyl, 2-pentinyl, 3-hexinyl or the like, preferably a lower (C₂₋₆) alkinyl, or the like is exemplified);
(6) an aralkyl which may be substituted (for example, a phenyl-C₁₋₄ alkyl (for example, benzyl, phenethyl or the like) or the like is exemplified); and
(7) an aryl which may be substituted (for example, phenyl, naphthyl or the like is exemplified), where examples of the substituents which may be possessed by (1) an alkyl which may be substituted, (2) a cycloalkyl which may be substituted, (3) an alkenyl which may be substituted, (4) a cycloalkenyl which may be substituted, (5) an alkinyl which may be substituted, (6) an aralkyl which may be substituted and (7) an aryl which may be substituted, which are described above, include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like) or the like,
where the number of the substituents is preferably 1 to 3.

The hydroxyl group which may be substituted, which is represented by R^{e5}' and R^{e6}', is exemplified by a hydroxyl group which may be substituted by, for example, (1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified);
(5) an aralkyl which may be substituted (for example, a phenyl-C₁₋₄ alkyl (for example, benzyl, phenethyl or the like) or the like is exemplified);
(6) formyl or an acyl which may be substituted (for example, an alkanoyl which has the carbon number of 2 to 4 (for example, acetyl, propionyl, butyryl, isobutyryl or the like), an alkylsulfonyl which has the carbon number of 1 to 4 (for example, methanesulfonyl, ethanesulfonyl or the like) or the like is exemplified); and
(7) an aryl which may be substituted (for example, phenyl, naphthyl or the like is exemplified).

Examples of the substituents which may be possessed by (1) an alkyl which may be substituted, (2) a cycloalkyl which may be substituted, (3) an alkenyl which may be substituted, (4) a cycloalkenyl which may be substituted, (5) an aralkyl which may be substituted, (6) an acyl which may be substituted and (7) an aryl which may be substituted, which are described above, include a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like) and the like, where the number of the substituents is preferably 1 to 3.

Also, in the above-mentioned formula, R^{e5'} and R^{e6'} may bind each other to form a cyclic ring group together with the adjacent phosphorus atom (preferably, a 5- to 7-membered cyclic ring). Such a cyclic ring group may have substituents and examples of the substituents include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like) and the like, where the number of the substituents is preferably 1 to 3.

In the above-mentioned formula (eI), a counter anion in the case where the phosphorus atom forms a phosphonium salt is exemplified by, in addition to an anion of a halogen atom (for example, Cl⁻, Br⁻, I⁻ or the like) or the like, an anion derived from an inorganic acid such as hydrochloric acid, hydrobromic acid, nitric acid, sulfuric acid, phosphoric acid and the like, an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like and an anion derived from an acidic amino acid such as aspartic acid, glutamic acid and the like, where Cl⁻, Br⁻, I⁻ or the like is more preferable.

An amino group that may be substituted, which is represented by R^{e5'} and R^{e6'}, is exemplified by an amino group that may have 1 to 2 groups such as,
(1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified);
(5) formyl or an acyl which may be substituted (for example, an alkanoyl which has the carbon number of 2 to 4 (for example, acetyl,. propionyl, butyryl, isobutyryl or the like), an alkylsulfonyl which has the carbon number of 1 to 4 (for example, methanesulfonyl, ethanesulfonyl or the like) or the like is exemplified); and
(6) an aryl which may be substituted (for example, phenyl, naphthyl or the like is exemplified).

Examples of the substituents which may be possessed by (1) an alkyl which may be substituted, (2) a cycloalkyl which may be substituted, (3) an alkenyl which may be substituted, (4) a cycloalkenyl which may be substituted, (5) an acyl which may be substituted and (6) an aryl which may be substituted, which are described above, include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), a carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like) and the like, where the number of the substituents is preferably 1 to 3.

The substituents for "an amidino group which may be substituted" and "a guanidino group which may be substituted" each of which is represented by R^{e2} are exemplified by substituents similar to those for "an amino group which may be substituted, where the nitrogen atom may be converted into a quaternary ammonium or the N-oxide" which is represented by R^{e2}.

It is preferable that R^{e2} is (1) an amino group which may be substituted, where the nitrogen atom may be converted into a quaternary ammonium or the N-oxide, (2) a nitrogen-containing heterocyclic ring group which may be substituted and may contain the sulfur atom or the oxygen atom as a ring-constituting atom, where the nitrogen atom may be converted into a quaternary ammonium or the N-oxide, (3) an amidino group which may be substituted or (4) a guanidino group which may be substituted and it is more preferable that R^{e2} is an amino group which may be substituted, where the nitrogen atom may be converted into a quaternary ammonium or the N-oxide, or the like. Also, R^{e2} may be an amidino group which may be substituted or a guanidino group which may be substituted.

It is more preferable that R^{e2} is a group represented by -NR^{e}R^{e''} or -N⁺R^{e}R^{e}'R^{e''} (wherein each of R^{e}, R^{e'} and R^{e''} represents an aliphatic hydrocarbon group (an aliphatic-chain hydrocarbon group and an aliphatic, cyclic hydrocarbon group) which may be substituted or an alicyclic (non-aromatic) heterocyclic ring group which may be substituted).

In the above-mentioned formula, "an aliphatic hydrocarbon group which may be substituted" and "an alicyclic heterocyclic ring group which may be substituted", which are representd by R^{e}, R^{e'} and R^{e"}, are exemplified by substituents similar to "an alicyclic hydrocarbon group which may be substituted (for example, an alkyl, a cycloalkyl, an alkenyl, a cycloalkenyl or the like, each of which may be substituted)" and "an alicyclic, heterocyclic ring group which may be substituted (for example, a 5- to 6-membered non-aromatic heterocyclic ring which may be substituted or the like)", which are exemplified by as the substituents which may be possessed by "an amino group which may be substituted" representd by R^{e2}.

Especially, as for R^{e} and R^{e}', an aliphatic hydrocarbon group which may be substituted (for example, an alkyl, an alkenyl or the like, each of which may be substituted) is preferable, a C₁₋₆ alkyl group which may be substituted is more preferable and the methyl group which may be substituted is particularly preferable.

It is preferable that R^{e"} is an alicyclic hydrocarbon group which may be substituted (preferably, a C₃₋₈ cycloalkyl group which may be substituted; more preferably, a cyclohexyl which may be substituted) or an alicyclic heterocyclic ring group which may be substituted (preferably, a saturated alicyclic heterocyclic ring group which may be substituted (preferably, a 6-membered cyclic ring group); more preferably, tetrahydropyranyl which may be substituted, tetrahydrothiopyranyl which may be substituted or piperidyl which may be substituted; and particularly preferably, tetrahydropyranyl which may be substituted).

In the above formula (eIa) , R^{e1} and Z^{e2} have the same meanings given above.

In the above formula (eIa), a group of the formula: 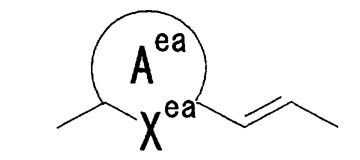 and 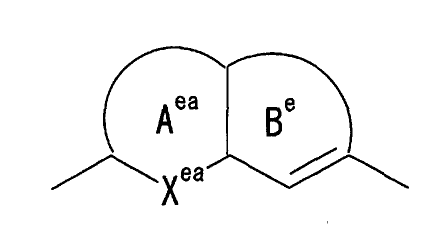 represented by W^{ea} binds to adjacent groups in the following manner: 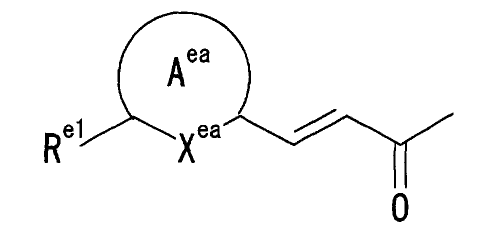 or

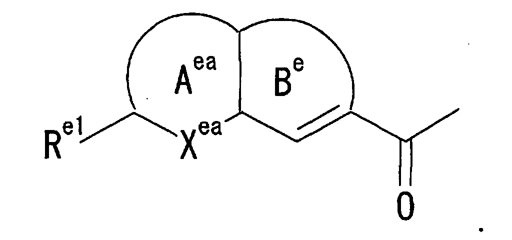

In the above formula, examples of the "5- to 6-membered aromatic ring" in the "optionally substituted 5-to 6-membered aromatic ring" represented by A^{ea} include 6-membered aromatic hydrocarbon such as benzene, etc.; 5- to 6-membered aromatic heterocyclic ring containing 1 to 3 hetero-atoms consisting of 1 to 2 kinds of hetero-atoms selected from oxygen atom, sulfur atom and nitrogen atom such as furan, thiophene, pyrrole, imidazole, pyrazole, thiazole, oxazole, isothiazole, isoxazole, pyridine, pyrazine, pyrimidine, pyridazine, triazole, etc.; etc. Among others, benzene, furan, thiophene, pyridine (preferably, 6-membered ring) etc. are preferable, and in particular benzene is preferable.

Examples of the "substituents", which the "5- to 6-membered aromatic ring" in the "optionally substituted 5-to 6-membered aromatic ring" represented by A^{ea} may have, are similar to the "substituents" which the "5- to 6-membered ring" in the "optionally substituted 5- to 6-membered ring" represented by R^{e1} may have. The number of the substituents for the ring A^{ea} is 1-4 (preferably 1-2), and they may be same or different and present at any possible position (e.g. the position of the group X^{ea} and the other positions) on the ring represented by A^{ea}.

In the above formula, examples of the "5- to 7-membered ring" in the "optionally substituted 5- to 7-membered ring" represented by B^{e} include a 5- to 7-membered ring group of the formula: 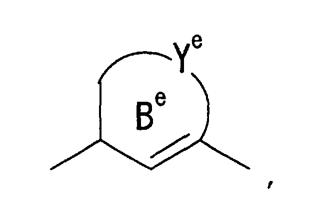 which may have a substituent at optionally substitutable positions, etc.

In the above formula, Y^{e} has the meaning given above. One to four (preferably one or two) of these substituents for A^{ea} may be the same or different from each other and present at any positions in the ring, and it is preferable that the carbon atom at the position ea in the group: 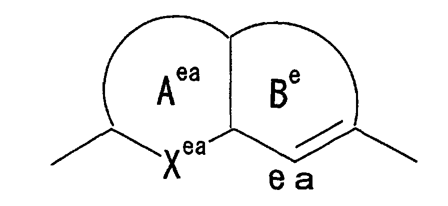 represented by W^{ea} is not substituted.

In the above formula (eIa), the amino group that may be substituted, where the nitrogen atom may be converted into a quaternary ammonium, represented by R^{e2a} include those mentioned as the amino group that may be substituted, where the nitrogen atom may be converted into a quaternary ammonium, represented by R^{e2}.

In the above formula (eIa), the group represented by the formula represented by R^{e2a}: 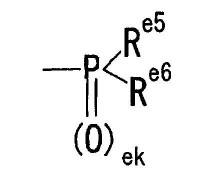 wherein ek represents 0 or 1, the phosphorus atom may form a phosphonium salt when ek is 0, and each of R^{e5} and R^{e6} represents the hydrocarbon atom that may be substituted or an amino group that may be substituted and R^{e5} and R^{e6} may bind each other to form a cyclic ring group together with the adjacent phosphorus atom, "hydrocarbon atom that may be substituted" or "an amino group that may be substituted" represented by R^{e5} or R^{e6} and a cyclic group in case of forming a cyclic ring group by combining R^{e5} and R^{e6} together with the adjacent phosphorus atom include those mentioned as examples for the above R^{e5'} and R^{e6'}.

In the above formula, a counter anion in case that the phosphorus atom is converted into a quaternary ammonium, is exemplified by, in addition to an anion of a halogen atom (for example, Cl⁻, Br⁻, I⁻ or the like) or the like, an anion derived from an inorganic acid such as hydrochloric acid, hydrobromic acid, nitric acid, sulfuric acid, phosphoric acid and the like, an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like and an anion derived from an acidic amino acid such as aspartic acid, glutamic acid and the like, where Cl⁻, Br⁻, I⁻ or the like is more preferable.

As the R^{e2a}, "an amino group that may be substituted, where the nitrogen atom may be converted into a quaternary ammonium and the group represented by the formula:

-N⁺R^{e}R^{e,} R^{e"}

wherein each of R^{e}, R^{e}' and R^{e"} is an aliphatic hydrocarbon group which may be substituted or an alicyclic heterocyclic group which may be substituted are more preferable.

As a compound of the formula (eIa), a compound of the formula: 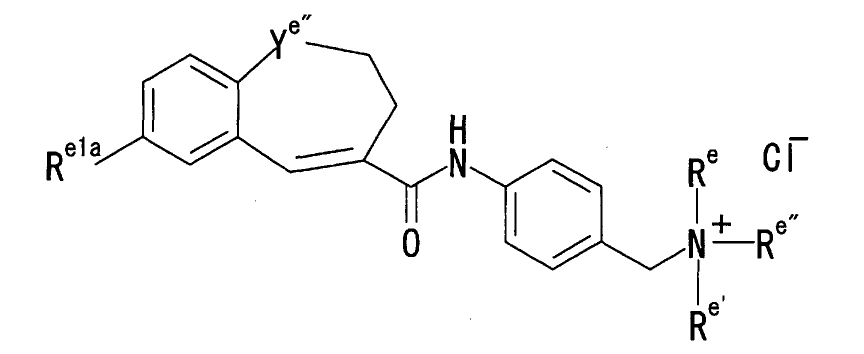 wherein R^{e1a} is an optionally substituted phenyl group or an optionally substituted thienyl group; Y^{e}" is -CH₂-, -O-, or -S-; and R^{e}, R^{e}' and R^{e}" are independently an optionally substituted aliphatic hydrocarbon group or an optionally substituted alicyclic heterocyclic ring group, is preferable.

In the above formula, the "substituent" which may be possessed by the "phenyl group" in the "optionally substituted phenyl group" and the "thienyl group" in the "potionally substituted thienyl group", each of which is represented by R^{e1a}, include those for the optionally substituted 5- or 6- membered cyclic ring group represented by the above R^{e1}.

Examples of the "optionally substituted aliphatic hydrocarbon group" and the "optionally substituted alicyclic heterocyclic ring group" represented by R^{e}, R^{e}' or R^{e}" include those exemplified by the substituents for the "optionally substituted amino group" represented by R^{e2a}.

Among them, as the group R^{e} or R^{e'}, an optionally substituted chain hydrocarbon group is preferable, an optionally substituted C₁₋₆ alkyl group is more preferable, and an optionally substituted methyl group is most preferable.

As the group R", an optionally substituted alicyclic hydrocarbon group (more preferably, an optionally substituted C₃₋₈ cycloalkyl group; more preferably, an optionally substituted cyclohexyl) or an optionally substituted alicyclic heterocyclic ring group (preferably, an optionally substituted saturated alicyclic heterocyclic ring group (preferably 6-membered ring group); more preferably, an optionally substituted tetrahydropyranyl, an optionally substituted tetrahydrothiopyranyl or an optionally substituted piperidyl; most preferably, an optionally substituted tetrahydropyranyl) is preferable. Among them, a compound of the formula: 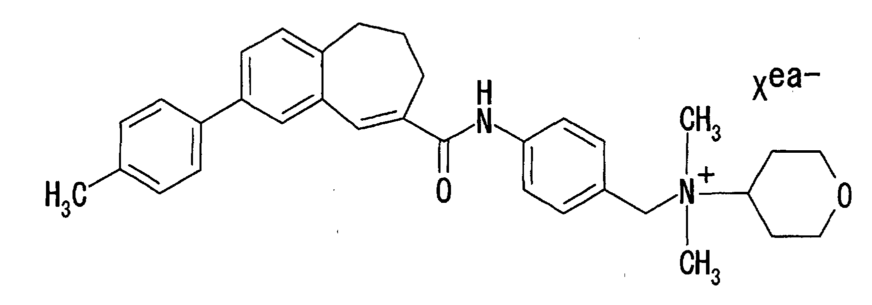 wherein X^{ea-} is an anion is preferable.

In the above formula, examples of the anion represented by X^{ea-} include an anion of a halogen atom; an anion derived from an inorganic acid such as hydrochloric acid, hydrobromic acid, nitric acid, sulfuric acid, phosphoric acid, etc.; an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; an anion derived from an acidic amino acid such as aspartic acid, glutamic acid, etc.; etc. Among them, an anion of a halogen atom is preferable.

In the above formula (eIb), the substituents of "phenyl group" in the "phenyl group which may be substituted" and the substituents of "thienyl group" in the "thienyl group which may be substituted" shown by R^{e1b} include those mentioned as the substituents in "5- to 6-membered cyclic ring group" shown by R^{e1}.

In the above formula (eIb), Y^{eb} is -CH₂-, -O- or -S-; and preferably -CH₂- or -O- .

In the above formula (eIb), R^{e2b}, R^{e3b} and R^{e4b} . are independently an "optionally substituted aliphatic hydrocarbon group" or an "optionally substituted alicyclic heterocyclic ring group".

Examples of the "aliphatic hydrocarbon group" in the "optionally substituted aliphatic hydrocarbon group" represented by R^{e2b}, R^{e3b} and R^{e4b} include
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably C₁₋₆ alkyl, etc.);
(2) an optionally substituted cycloalkyl (e.g. C₃₋₈ cycloalkyl, etc. such as cyclopropyl, cyclobutyl, cyclopentyl, cyclohexyl, cycloheptyl, cyclooctyl, etc.);
(3) an optionally substituted alkenyl (e.g. alkenyl which has a carbon number of 2 to 10 such as allyl, crotyl, 2-pentenyl, 3-hexenyl, etc., preferably alkenyl, etc.);
(4) an optionally substituted cycloalkenyl (e.g. cycloalkenyl which has a carbon number 3 to 7, such as 2-cyclopentenyl, 2-cyclohexenyl, 2-cyclopentenylmethyl, 2-cyclohexenylmethyl, etc.); etc.

Examples of the "alicyclic heterocyclic ring group" in the "optionally substituted alicyclic heterocyclic ring group" represented by R^{e2b}, R^{e3b} and R^{e4b} include group formed by removing a hydrogen atom from a 5- to 6-membered non-aromatic heterocyclic ring containing 1 to 4 hetero-atoms consisting of 1 to 2 kinds of hetero-atoms selected from nitrogen atom, sulfur atom and oxygen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, etc.; preferably a group formed by removing a hydrogen atom from a 5- to 6-membered saturated heterocyclic ring, containing 1 hetero-atom such as tetrahydrofuran, piperidine, tetrahydropyran, tetrahydrothiopyran, etc.

Examples of the substituents, which the "aliphatic hydrocarbon group" and the "alicyclic heterocyclic ring group" in the "optionally substituted aliphatic hydrocarbon group" and the "optionally substituted alicyclic heterocyclic ring group" represented by R^{e2b}, R^{e3b} and R^{e4b} may have, include halogen (e.g. fluorine, chlorine, bromine, iodine, etc.), an optionally halogenated C₁₋₄ alkyl, an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), phenyl, phenyl C₁₋₄ alkyl, C₃₋₇ cycloalkyl, cyano, nitro, oxo, hydroxy, mercapto, amino, carboxyl, C₁₋₄ alkoxy-carbonyl (preferably, halogen, an optionally halogenated C₁₋₄ alkyl, an optionally halogenated C₁₋₄ alkoxy, phenyl C₁₋₄ alkyl, C₃₋₇ cycloalkyl, cyano, oxo, hydroxy group, etc.), etc., and the number of the substituents are preferably 1 to 3.

In the above formula (eIb), as the group R^{e2b} or R^{e3b}, an optionally substituted chain hydrocarbon group is preferable, and an optionally substituted alkyl group is more preferable. In particular, the groups R^{e2b} and R^{e3b} are preferably the same and more preferably both of the groups R^{e2b} and R^{e3b} are methyl.

In the above formula (eIb), as the group R^{e4b}, an optionally substituted alicyclic hydrocarbon group or an optionally substituted alicyclic heterocyclic group is preferable and an optionally substituted cycloalkyl group or an optionally substituted saturated alicyclic heterocyclic ring group is more preferable. In particular, R^{e4b} is preferably an optionally substituted cyclohexyl or an optionally substituted 6-membered saturated alicyclic heterocyclic ring group and more preferably an optionally substituted cycloalkyl, an optionally substituted tetrahydropyranyl, an optionally substituted tetrahydrothiopyranyl or an optionally substituted piperidyl.
In the formula (eIc), R^{e1} has the same meaning given above.

In the above formula (eIc), the substituent of the 6-to 7-membered ring in a "6- to 7-membered ring which may be substituted" represented by A^{ec} include those which the "5-to 6-membered ring" in the "optionally substituted 5- to 6-membered ring" represented by R^{e1} may have. One to three (preferably one to two) of the substituents of A^{ec} are the same or different each other. and may be present at any position on the ring.

In the group of the formula: 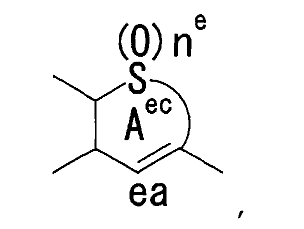 a carbon atom at the position ea is preferably unsubstituted.

Examples of the "optionally substituted 6- to 7-membered ring" represented by A^{ec} include a 6- to 7-membered ring group of the formula: 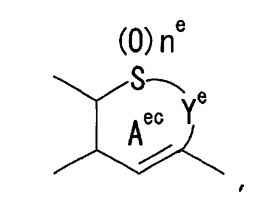 which may have a substituent at any possible position, etc.

In the above formula, Y^{e} has the same meaning given above.

Examples of the "substituents", which the "benzene ring" in the "optionally substituted benzene ring" represented by B^{ec} may have, include those for the "5- to 6-membered ring" in the "optionally substituted 5- to 6-membered ring" represented by R^{e1}, etc. Among them, halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc. are preferable and in particular, halogen, an optionally halogenated C₁₋₄ alkyl, an optionally halogenated C₁₋₄ alkoxy, etc. are preferable. The number of the substituents is preferably 1 to 3.

In the above formula (eIc), n is an integer of 1 or 2 (preferably, 2).

In the above formula (eIc), the divalent group represented by Z^{e2} has the same meaning given above.
In the above formula (eIc), examples of (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium, (3) a group binding through a sulfur atom and (4) a group of the formula: 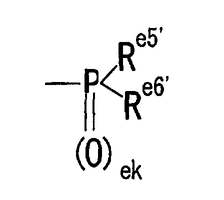 wherein each symbols has the same meanings given above, represented by R^{e2c} are the same as those of R^{e2} mentioned above.

In the above formula (eId), examples of the "5- to 6-membered aromatic ring" of the "5- to 6-membered aromatic ring which has a group of the formula: R^{ed}-Z^{e1d}-X^{ed}-Z^{e2d}- wherein R^{ed} is a hydrogen atom or an optionally substituted hydrocarbon group, X^{ed} is an optionally substituted alkylene chain, and Z^{e1a} and Z^{e2d} are respectively hetero-atoms, include a 6-membered aromatic hydrocarbon such as benzene, etc.; 5- to 6-membered aromatic heterocyclic ring containing 1 to 4 hetero-atoms consisting of 1 to 2 kinds of hetero-atoms selected from nitrogen atom, sulfur atom and oxygen atom such as furan, thiophene, pyrrole, imidazole, pyrazole, thiazole, oxazole, isothiazole, isoxazole, tetrazole, pyridine, pyrazine, pyrimidine, pyridazine, triazole, etc.; etc. Among them, benzene, furan, thiophene, pyridine, etc. are preferable, benzene, furan or thiophene is more preferable, and in particular, benzene is preferable.

Examples of the "hydrocarbon group" in the "optionally substituted hydrocarbon group" represented by R^{ed} include
(1) alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc., more preferably lower (C₁₋₄) alkyl, etc.);
(2) cycloalkyl (e.g. C₃₋₇ cycloalkyl, etc. such as cyclopropyl, cyclobutyl, cyclopentyl, cyclohexyl, cycloheptyl, etc.);
(3) alkenyl (e.g. alkenyl which has a carbon number of 2 to 10 such as allyl, crotyl, 2-pentenyl, 3-hexenyl, etc., preferably lower (C₂₋₆) alkenyl, etc.);
(4) cycloalkenyl (e.g. cycloalkenyl which has a carbon number of 3 to 7, etc. such as 2-cyclopentenyl, 2-cyclohexenyl, 2-cyclopentenylmethyl, 2-cyclohexenylmethyl, etc.);
(5) alkynyl (e.g. alkynyl which has a carbon number of 2 to 10 such as ethynyl, 1-propynyl, 2-propynyl, 1-butynyl, 2-pentynyl, 3-hexynyl, etc., preferably lower (C₂₋₆) alkynyl, etc.);
(6) aralkyl (e.g. phenyl-C₁₋₄ alkyl (e.g. benzyl, phenethyl, etc.), etc.);
(7) aryl (e.g. phenyl, naphthyl, etc.);
(8) cycloalkyl -alkyl (e.g. C₃₋₇ cycloalkyl-C₁₋₄ alkyl such as cyclopropylmethyl, cyclobutylmethyl, cyclopentylmethyl, cyclohexylmethyl, cycloheptylmethyl;), etc.

Examples of the substituents, which the above-mentioned (1) alkyl, (2) cycloalkyl, (3) alkenyl, (4) cycloalkenyl, (5) alkynyl, (6) aralkyl, (7) aryl and (8) cycloalkyl-alkyl may have, include halogen (e.g. fluorine, chlorine, bromine, iodine, etc.), nitro, cyano, hydroxy group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), C₁₋₄ alkylenedioxy (e.g. -O-CH₂-O-, -O-CH₂-CH₂-O-, etc.), an optionally substituted sulfonamide [a group formed by combining an optionally substituted amino group (e.g. amino, a mono-C₁₋₄ alkyklamino, di-C₁₋₄ alkylamino, 5- to 6-membered cyclic amino such as tetrahydropyrrole, piperazine, piperidine, morpholine, thiomorpholine, pyrrole, imidazole, etc. ) with -SO₂-, etc.], formyl, C₂₋₄ alkanoyl (e.g. acetyl, propionyl, etc.), C₁₋₄ alkylsulfonyl (e.g. methanesulfonyl, ethanesulfonyl, etc.), an optionally substituted heterocyclic group etc., and the number of the substituents are preferably 1 to 3.

The "heterocyclic group" in the "optionally substituted heterocyclic group" which is the substituent for the "optionally substituted hydrocarbon group" represented by R^{ed} is exemplified by a group which is formed by removing one hydrogen atom from an aromatic heterocyclic ring or non-aromatic heterocyclic ring. The aromatic heterocyclic ring include a 5- to 6-membered aromatic heterocyclic ring, which contains 1 to 4 hetero atoms of 1 to 2 kinds selected from the nitrogen atom, the sulfur atom and the oxygen atom, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole or the like, and the non-aromatic heterocyclic ring includes a 5- to 6-membered non-aromatic heterocyclic ring, which contains 1 to 4 heteroatoms of 1 to 2 kinds selected from the nitrogen atom, the sulfur atom and the oxygen atom, such as tetrahydrofuran, tetrahydrothiophene, dioxolan dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran or the like and non-aromatic heterocyclic ring group formed by being saturated all or a part of the bond of the ring (preferably, aromatic heterocyclic ring such as pyrazole, thiazole, oxazole tetrazole or the like).

Examples of "heterocyclic ring" of "heterocyclic group that may be substituted" which is the substituent of the "optionally substituted hydrocarbon group" represented by R^{ed}, may optionally have 1 to 3 substituents at any substitutable position. The substituent include a halogen atom (e.g. fluorine, chlorine, bromine, iodine, et.), nitro, cyano, hydroxyl, a thiol group which may be substituted (e.g. thiol, a C₁₋₄ alkylthio, etc.), an amino group which may be substituted (e.g. amino, a mono-C₁₋₄ alkylamino, a di-C₁₋₄ alkylamino, a 5- to 6- membered cyclic amino such as tetrahydropyrrole, piperazine, piperidine, morpholine, thiomorpholine, pyrrole, imidazole, etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), C₁₋₄ alkylenedioxy (e.g. -O-CH₂-O-, -O-CH₂-CH₂-O-, etc.), an optionally substituted sulfonamide [a group formed by combining an optionally substituted amino group (e.g. amino, a monoC₁₋₄ alkyklamino, diC₁₋₄ alkylamino, 5- to 6-membered cyclic amino such as tetrahydropyrrole, piperazine, piperidine, morpholine, thiomorpholine, pyrrole, imidazole, etc. ) with -SO₂-, etc.], formyl, C₂₋₄ alkanoyl (e.g. acetyl, propionyl, etc.), C₁₋₄ alkylsulfonyl (e.g. methanesulfonyl, ethanesulfonyl, etc.), (preferably C₁₋₄ alkyl etc.).

When the group of the formula: R^{ed}-Z^{e1d}-X^{ed}-Z^{e2d}- (wherein each symbol has the same meaning given above) is a monovalent group, it does not bind to the 5- to 6-membered aromatic ring to form a ring, an optionally substituted alkyl group is preferable as the group R^{ed}, an optionally halogenated lower alkyl group is more preferable, and in particular, an optionally halogenated C₁₋₄ alkyl group is preferable.

In the above formula (eId), examples of the "optionally substituted alkylene chain" represented by X^{ed} include an optionally substituted straight or branched C₁₋₆ alkylene, etc. In the alkylene chain, a straight portion is preferably formed by 1-4 carbon atoms, and in particular, an optionally substituted straight C₁₋₄ alkylene (preferably ethylene or propylene) is preferable as X^{ed}.

Examples of the substituent, which the "alkylene chain" in the "optionally substituted alkylene chain" represented by X^{ed} may have, include any one of which can bind to a divalent chain constituting the straight portion, for example, lower alkyl which has a carbon number of 1 to 6 (e.g. methyl, ethyl, propyl, isopropyl, butyl, isobutyl, sec-butyl, tert-butyl, pentyl, isopentyl, neopentyl, hexyl, etc.), lower (C₃₋₇) cycloalkyl (e.g. cyclopropyl, cyclobutyl, cyclopentyl, cyclohexyl, cycloheptyl, etc.), formyl, lower (C₂₋₇) alkanoyl (e.g. acetyl, propionyl, butyryl, etc.), an optionally esterified phosphono group, an optionally esterified carboxyl group, hydroxy group, oxo, etc., and more preferably lower alkyl which has a carbon number of 1 to 6 (preferably C₁₋₃ alkyl), hydroxy group, oxo, etc.

Examples of the optionally esterified phosphono group include a group of the formula: P(O) (OR^{e7d}) (OR^{e8d}) wherein R^{e7d} and R^{e8d} are independently hydrogen, a alkyl group which has a carbon number of 1 to 6 or a cycloalkyl group which has a carbon number of 3 to 7, and R^{e7d} and R^{e8d} may bind to each other to form a 5- to 7-membered ring.

In the above formula, examples of the alkyl group which has a carbon number of 1 to 6 represented by R^{e7d} and R^{e8d} include methyl, ethyl, propyl, isopropyl, butyl, isobutyl, sec-butyl, tert-butyl, pentyl, isopentyl, neopentyl, hexyl, etc., and examples of the cycloalkyl which has a carbon number of 3 to 7 include cyclopropyl, cyclobutyl, cyclopentyl, cyclohexyl, cycloheptyl, etc. Among them, a straight-chain lower alkyl which has a carbon number of 1 to 6 is preferable and lower alkyl which has a carbon number of 1 to 3 is more preferable. The groups R^{e7d} and R^{e8d} may be same or different, and preferably the groups R^{e7d} and R^{e8d} are same. When R^{e7d} and R^{e8d} may bind to each other to form a 5- to 7-membered ring, the groups R^{e7d} and R^{e8d} bind to each other to represent a straight-chain C₂₋₄ alkylene side chain of the formula: -(CH₂)₂-, -(CH₂)₃-, -(CH₂) ₄-, etc. The side chain may have a substituent, and examples of the substituent include hydroxy group, halogen, etc.

Examples of the optionally esterified carboxyl group include an ester group formed by binding a carboxyl group to an alkyl group which has a carbon number of 1 to 6 or a cycloalkyl group which has a carbon number of 3 to 7 (e.g. methoxycarbonyl, ethoxycarbonyl, propoxycarbonyl, isopropoxycarbonyl, butoxycarbonyl, isobutoxycarbonyl, sec-butoxycarbonyl, tert-butoxy-carbonyl, pentyloxycarbonyl, hexyloxycarbonyl, etc.).

As the group X^{ed}, an optionally substituted C₁₋₄ alkylene is preferable, C₁₋₄ alkylene which may be substituted with C₁₋₃ alkyl, hydroxy group or oxo is more preferable, and in particular, a group of the formula: -(CH₂)ₑₙ- (en is an integer of 1-4) is preferable.

Examples of the hetero-atom represented by Z^{e1d} and Z^{e2d} include -O-, -S(O)ₑₘ- (em is an integer of 0 to 2), -N (R^{e4a}) - (R^{e4d} is a hydrogen atom or an optionally substituted lower alkyl group), etc. As the group Z^{e1d}, -O- or -S(O)ₑₘ- (em is an integer of 0 to 2) is preferable, and - O- is more preferable. As the group Z^{e2d}, -O- or -N(R^{e4d)}- (R^{e4d} is a hydrogen atom or an optionally substituted lower alkyl group) is preferable, and -O- is more preferable.

Examples of the "optionally substituted lower alkyl group"represented by R^{e4d} are similar to the above "optionally substituted lower alkyl group" exemplified in the "optionally substituted hydrocarbon group" represented by R^{ed}.

Examples of the further substituent, which the "5- to 6-membered ring" in the "5- to 6- membered aromatic ring which has a group of the formula: R^{ed}-Z^{e1d}-X^{ed}-Z^{e2d}- wherein each symbol is the same meaning given above, and which may have a further substituent" represented by R^{e1d} may have, in addition to the group of the formula: R^{ed}-Z^{e1d}-X^{ed}-Z^{e2d}-, include a halogen atom, nitro, cyano, an optionally substituted alkyl, an optionally substituted cycloalkyl, an optionally substituted hydroxy group, an optionally substituted thiol group (wherein a sulfur atom may be oxidized to form an optionally substituted sulfinyl group or an optionally substituted sulfonyl group), an optionally substituted amino group, an optionally substituted acyl group, an optionally esterified or amidated carboxyl group, an optionally substituted aromatic group.

Examples of the halogen as the substituents for R^{e1d} include fluorine, chlorine, bromine, iodine, etc. Among them, fluorine and chlorine are preferable.

Examples of the alkyl in the optionally substituted alkyl as the substituents for R^{e1d} include a straight or branched alkyl that has a carbon number of 1 to 10 such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl.

Examples of the substituents in the optionally substituted alkyl include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkoxy (e.g. methoxy, ethoxy, propoxy, butoxy, trifluoromethoxy, trifluoroethoxy, etc.), an optionally halogenated C₁₋₄ alkoxy-C₁₋₉ alkoxy (e.g. methoxymethoxy, methoxyethoxy, ethoxyethoxy, trifluoromethoxyethoxy, trifluoroethoxyethoxy, etc.), formyl, C₂₋₄ alkanoyl (e.g. acetyl, propionyl, etc.), C₁₋₉ alkylsulfonyl (e.g. methanesulfonyl, ethanesulfonyl, etc.), etc., and the number of the substituents are preferably 1 to 3.

Examples of the cycloalkyl in the optionally substituted cycloalkyl as the substituents for R^{e1d} include C₃₋₇ cycloalkyl, etc. such as cyclopropyl, cyclobutyl, cyclopentyl, cyclohexyl, cycloheptyl, etc.

Examples of the substituents in the optionally substituted cycloalkyl include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the substituents in the optionally substituted hydroxy group as the substituents for R^{ed} include:
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl which may contain a hetero-atom (e.g. C₃₋₇ cycloalkyl such as cyclopropyl, cyclobutyl, cyclopentyl, cyclohexyl, cycloheptyl, etc.; a saturated 5- to 6-membered heterocyclic ring group containing 1 to 2 hetero-atoms such as tetrahydrofuranyl, tetrahydrothienyl, pyrrolidinyl, pyrazolidinyl, piperidyl, piperazinyl, morpholinyl, thiomorpholinyl, tetrahydropyranyl, tetrahydrothiopyranyl, etc (preferably, tetrahydropyranyl, etc.).; etc.);
(3) an optionally substituted alkenyl (e.g. alkenyl which has a carbon number of. 2 to 10 such as allyl, crotyl, 2-pentenyl, 3-hexenyl, etc., preferably lower (C₂₋₆)alkenyl, etc.);
(4) an optionally substituted cycloalkenyl (e.g. cycloalkenyl which has a carbon number of 3 to 7, etc. such as 2-cyclopentenyl, 2-cyclohexenyl, 2-cyclopentenylmethyl, 2-cyclohexenylmethyl, etc.);
(5) an optionally substituted aralkyl (e.g. phenyl-C₁₋₄ alkyl (e.g. benzyl, phenethyl, etc.), etc.);
(6) formyl or an optionally substituted acyl (e.g. alkanoyl which has a carbon number of 2 to 4 (e.g. acetyl, propionyl, butyryl, isobutyryl, etc.), alkylsulfonyl which has a carbon number of 1 to 4 (e.g. methanesulfonyl, ethanesulfonyl, etc.), etc.);
(7) an optionally substituted aryl (e.g. phenyl, naphthyl, etc.); etc.

Examples of the substituents which the above-mentioned (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl and (7) optionally substituted aryl may have include halogen (e.g., fluorine, chlorine, bromine, iodine, etc.), nitro, cyano, hydroxy group, an optionally substituted thiol group (e.g., thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₆ alkoxy (e.g. methoxy, ethoxy, propoxy, butoxy, trifluoromethoxy, trifluoroethoxy, etc.; preferably an optionally halogenated C₁₋₄ alkoxy), formyl, C₂₋₄ alkanoyl (e.g. acetyl, propionyl, etc.), C₁₋₄ alkylsulfonyl (e.g. methanesulfonyl, ethanesulfonyl, etc.), an optionally substituted 5- to 6-membered aromatic heterocyclic ring [e.g. 5- to 6-membered aromatic heterocyclic ring containing 1 to 4 hetero-atoms consisting of 1 to 2 kinds of hetero-atoms selected from nitrogen atom, sulfur atom and oxygen atom such as furan, thiophene, pyrrole, imidazole, pyrazole, thiazole, oxazole, isothiazole, isoxazole, tetrazole, pyridine, pyrazine, pyrimidine, pyridazine, triazole, etc.; Examples of the substituents which the heterocyclic ring may have include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3], etc., and the number of the substituents are preferably 1 to 3.

Examples of the substituents in the optionally substituted thiol group as the substituents for R^{e1d} are similar to the above-described substituents in the optionally substituted hydroxy group as the substituents for R^{e1d}, and among them,
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₇ cycloalkyl, etc. such as cyclopropyl, cyclobutyl, cyclopentyl, cyclohexyl, cycloheptyl, etc.);
(3) an optionally substituted aralkyl (e.g. phenyl-C₁₋₄ alkyl (e.g. benzyl, phenethyl, etc.), etc.);
(4) an optionally substituted aryl (e.g. phenyl, naphthyl, etc.); etc. are preferable.

Examples of the substituents which the above-mentioned (1) optionally substituted alkyl, (2) optionally substituted cycloalkyl, (3) optionally substituted aralkyl and (4) optionally substituted aryl may have include halogen (e.g., fluorine, chlorine, bromine, iodine, etc.), nitro, cyano, hydroxy group, an optionally substituted thiol group (e.g., thio, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the substituents in the optionally substituted amino group as the substituents for R^{e1d} include an amino group which may have one to two substituents similar to the above-described substituents in the optionally substituted hydroxy group as the substituents for R^{e1d} , etc. Among them,
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₇ cycloalkyl, etc. such as cyclopropyl, cyclobutyl, cyclopentyl, cyclohexyl, cycloheptyl, etc.);
(3) an optionally substituted alkenyl (e.g. alkenyl which has a carbon number of 2 to 10 such as allyl, crotyl, 2-pentenyl, 3-hexenyl, etc., preferably lower (C₂₋₆) alkenyl, etc.);
(4) an optionally substituted cycloalkenyl (e.g. cycloalkenyl which has a carbon number of 3 to 7, etc. such as 2-cyclopentenyl, 2-cyclohexenyl, 2-cyclopentenylmethyl, 2-cyclohexenylmethyl, etc.);
(5) formyl or an optionally substituted acyl (e.g. alkanoyl which has a carbon number of 2 to 4 (e.g. acetyl, propionyl, butyryl, isobutyryl, etc.), which has a carbon number of 1 to 4 alkylsulfonyl (e.g. methanesulfonyl, ethanesulfonyl, etc.), etc.);
(6) an optionally substituted aryl (e.g. phenyl, naphthyl, etc.); etc. are preferable.

Examples of the substituents, which each of the above-described (1) optionally substituted alkyl, (2) optionally substituted cycloalkyl,. (3) optionally substituted alkenyl, (4) optionally substituted cycloalkenyl, (5) optionally substituted acyl and (6) optionally substituted aryl may have, include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents is preferably 1 to 3.

The substituents in the optionally substituted amino group as the substituents for R^{e1d} may bind to each other to form a cyclic amino group (e.g. 5- to 6-membered cyclic amino, etc. such as tetrahydropyrrole, piperazine, piperidine, morpholine, thiomorpholine, pyrrole, imidazole, etc.). The cyclic amino group may have a substituent, and examples of the substituents include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents is preferably 1 to 3.

Examples of the optionally substituted acyl as the substituents for R^{e1d} include a carbonyl group or a sulfonyl group binding to
(1) hydrogen;
(2) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(3) an optionally substituted cycloalkyl (e.g. C₃₋₇ cycloalkyl, etc. such as cyclopropyl, cyclobutyl, cyclopentyl, cyclohexyl, cycloheptyl, etc.);
(4) an optionally substituted alkenyl (e.g. alkenyl which has a carbon number of 2 to 10 such as allyl, crotyl, 2-pentenyl, 3-hexenyl, etc., preferably lower (C₂₋₆) alkenyl, etc.);
(5) an optionally substituted cycloalkenyl (e.g. cycloalkenyl which has a carbon number of 3 to 7, etc. such as 2-cyclopentenyl, 2-cyclohexenyl, 2-cyclopentenylmethyl, 2-cyclohexenylmethyl, etc.);
(6) an optionally substituted 5- to 6-membered monocyclic aromatic group (e.g. phenyl, etc., 5- to 6-membeed aromatic heterocyclic group containing 1 to 4 hetero-atoms of 1 to 2 kinds selected from nitrogen atom, sulfur atom and oxygen atom, such as furyl, thienyl, pyrrolyl, imidazole, pyrazolyl, thiazolyl, oxazolyl, isothiazolyl, isoxazolyl, tetrazolyl, pyridyl, pyrazyl, pyrimidyl, pyridazinyl, triazolyl, etc., preferably, pyridyl, thienyl, etc.)
(7) an optionally substituted 5- to 6-membered monocyclic non-aromatic heterocyclic group ( a group formed by removing one hydrogen atom from a 5- to 6-membered monocyclic non-aromatic heterocyclic ring which contains 1 to 4 heteroatoms of 1 to 2 kinds selected from nitrogen atom, sulfur atom and oxygen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, etc., preferably dioxolanyl, etc.) with carbonyl or sulfonyl group (e.g. acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl, etc.).

Examples of the substituents, which the above-mentioned (2) optionally substituted alkyl, (3) optionally substituted cycloalkyl, (4) optionally substituted alkenyl, (5) optionally substituted cycloalkenyl, (6) optionally substituted 5- to 6-membered monocyclic aromatic group and (7) optionally substituted 5- to 6-membered non-aromatic heterocyclic group may have, include a halogen (e.g. fluorine, chlorine, bromine, iodine, etc.), nitro, cyano, hydroxyl group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, a 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), C₁₋₄ alkylenedioxy (e.g. -O-CH₂-O-, -O-CH₂-CH₂-O-, etc.), an optionally substituted sulfonamide [a group formed by combining an optionally substituted amino group (e.g. amino, a monoC₁₋₄ alkyklamino, diC₁₋₄ alkylamino, 5- to 6-membered cyclic amino such as tetrahydropyrrole, piperazine, piperidine, morpholine, thiomorpholine, pyrrole, imidazole, etc. ) with -SO₂-, etc.), formyl, a C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the optionally esterified carboxyl group as the substituents for R^{e1d} include a carbonyloxy group binding to
(1) hydrogen;
(2) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(3) an optionally substituted cycloalkyl (e.g. C₃₋₇ cycloalkyl, etc. such as cyclopropyl, cyclobutyl, cyclopentyl, cyclohexyl, cycloheptyl, etc.);
(4) an optionally substituted alkenyl (e.g. alkenyl which has a carbon number of 2 to 10 such as allyl, crotyl, 2-pentenyl,3-hexenyl, etc., preferably lower (C₂₋₆) alkenyl, etc.);
(5) an optionally substituted cycloalkenyl (e.g. cycloalkenyl which has a carbon number of 2 to 1, etc. such as 2-cyclopentenyl, 2-cyclohexenyl, 2-cyclopentenylmethyl, 2-cyclohexenylmethyl, etc.);
(6) an optionally substituted aryl (e.g. phenyl, naphthyl, etc.); etc., and preferably carboxyl, lower (C₁₋₆) alkoxycarbonyl, aryloxycarbonyl (e.g. methoxycarbonyl, ethoxycarbonyl, propoxycarbonyl, phenoxycarbonyl, naphthoxycarbonyl, etc.), etc.

Examples of the substituents, which the above-mentioned (2) optionally substituted alkyl, (3) optionally substituted cycloalkyl, (4) optionally substituted alkenyl, (5) optionally substituted cycloalkenyl and (6) optionally substituted aryl may have, include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the optionally amidated carboxyl group as the substituents for R^{e1d} include an carbonyl group binding to an optionally substituted amino group, etc. which is similar to the above-described "optionally substituted amino group as the substituents for R^{e1d} ", and among others, carbamoyl, mono-C₁₋₆ alkylcarbamoyl, di-C₁₋₆ alkylcarbamoyl, etc. are preferable.

Examples of the aromatic group in the optionally substituted aromatic group as the substituents for R^{e1d} include 5- to 6-membered aromatic homocyclic or heterocyclic ring such as phenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl, triazolyl, oxadiazolyl, thiadiazolyl, etc.; fused aromatic heterocyclic ring such as benzofuran, indole, benzothiophene, benzoxazole, benzothiazole, indazole, benzimidazole, quinoline, isoquinoline, quinoxaline, phthalazine, quinazoline, cinnoline, etc.; etc.

Examples of the substituents for these aromatic groups include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents is preferably 1 to 3.

The number of the above-mentioned substituents for R^{e1d} is 1-4 (preferably 1 to 2) and they may be same or different and present at any possible position on the ring.

When the group represented by R^{ed} binds to the 5 to 6 membered aromatic ring to form a ring, the group of the formula: R^{ed}-Z^{e1d}-X^{ed}-Z^{e2d}- wherein each symbol is as defined above (as the group R^{ed} is preferably a hydrogen atom) forms a divalent group such as lower (C₁₋₆) alkylenedioxy (e.g., -O-CH₂-O-, -O-CH₂-CH₂-O-, -O-CH₂-CH₂-CH₂-O-, etc.), oxy-lower (C₁₋₆) alkylene-amino (e.g., -O-CH₂-NH-, -O-CH₂-CH₂-NH-, etc.), oxy-lower (C₁₋₆) alkylenethio (e.g., -O-CH₂-S-, -O-CH₂-CH₂-S-, etc.), lower (C₁₋₆) alkylene-diamino (e.g., -NH-CH₂-NH-, -NH-CH₂-CH₂-NH-, etc.), thia-lower (C₁₋₆) alkylene-amino (e.g., -S-CH₂-NH-, -S-CH₂-CH₂-NH-, etc.), etc. Examples of the further substituent, which the "5 to 6 membered ring" in the "5 to 6 membered aromatic ring which has a group of the formula: R^{ed}-Z^{e1d}-X^{ed}-Z^{e2d}- wherein each symbol is as defined above, and which may have a further substituent" represented by R^{e1d} may have, in addition to the group of the formula: R^{ed}-Z^{e1d}-X^{ed}-Z^{e2d}-, include a lower (C₁₋₄) alkyl optionally substituted with a halogen or a lower (C₁₋₄) alkoxy (e.g. methyl, ethyl, t-butyl, trifluoromethyl, methoxymethyl, ethoxymethyl, propoxymethyl, butoxymethyl, methoxyethyl, ethoxyethyl, propoxyethyl, butoxyethyl, etc.), a lower (C₁₋₄) alkoxy optionally substituted with a halogen or a lower (C₁₋₄) alkoxy (e.g. methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy, etc.), halogen (e.g. fluorine, chlorine, etc.), nitro, cyano, an amino optionally substituted with 1-2 lower (C₁₋₄) alkyl, formyl or lower (C₂₋₄) alkanoyl (e.g. amino, methylamino, dimethylamino, formylamino, acetylamino, etc.), 5- to 6-membered cyclic amino (e.g. 1-pyrrolidinyl, 1-piperazinyl, 1-piperidinyl, 4-morpholino, 4-thiomorpholino, 1-imidazolyl, 4-tetrahydropyranyl, etc.), etc.

When R^{e1d} is a benzene, the "group of the formula: R^{ed}-Z^{e1d}-X^{ed}-Z^{e2d}-" is preferably present at para position and the further substituent, which the "5 to 6 membered aromatic ring which may have, in addition to the group of the formula: R^{ed}-Z^{e1d}-X^{ed}-Z^{e2d}-, is preferably present at meta position.

In the above formula, examples of the "optionally substituted imino group" represented by Y^{ed} include a divalent group of the formula: -N(R^{e5d})- wherein R^{e5d} is a hydrogen atom or a substituent, etc..

As R^{e5d}, a hydrogen atom, an optionally substituted hydrocarbon group, an optionally substituted heterocyclic group, an optionally substituted hydroxyl group, an optionally substituted thiol group (which may be oxidized, and form an optionally substituted sulfinyl group or an optionally substituted sulfonyl group), an optionally substituted amino group, an optionally esterified or amidated carboxyl group,and an optionally substituted acyl group etc., are preferable, and a hydrogen atom, an optionally substituted hydrocarbon group,an optionally substituted heterocyclic group, an optionally substituted acyl group, etc. are more preferable. Preferable examples of R^{e5d} include a hydrogen atom, an optionally substituted hydrocarbon group, an optionally substituted acyl group, etc., and C₁₋₄ alkyl, C₁₋₄ alkylsulfonyl, formyl, C₂₋₅ alkanoyl, etc. are more preferable, C₁₋₄ alkyl, formyl, C₂₋₅ alkanoyl, etc. are further more preferable, and in particular, formyl or ethyl is preferable. As examples of preferable embodiment of R^{ed}, a group represented by the formula : - (CH₂) ₑₖ-R^{e6d}, wherein ek is 0 or 1, and R^{e6d} represents an optionally substituted 5- to 6- membered monocyclic aromatic group (for example, those similar to "(6) an optionally substituted 5- to 6-membered monocyclic aromatic group" mentioned in the item "an optionally substituted acyl group" as the substitutent of R^{ed} are mentioned, and phenyl, pyrazolyl, thiazolyl, oxazolyl, tetrazolyl, each of which may be substituted by a halogen, an optionally halogenated C₁₋₄ alkyl, an optionally halogenated C₁₋₄ alkoxy, etc., etc. are preferable.) .

Specific examples of the "optionally substituted hydrocarbon group" as R^{e5d} are similar to the "optionally substituted hydrocarbon group" as R^{ed}. Specific examples of the "optionally substituted heterocyclic group" as R^{ed} are similar to the "optionally substituted heterocyclic group" which is a substituent of the "optionally substituted hydrocarbon group" represented by R^{ed}. Specific examples of the "optionally substituted hydroxyl group", the "optionally substituted thiol group, the "optionally substituted amino group", the "optionally esterified or amidated carboxyl group" and the "optionally substituted acyl group", each of which is represented by R^{e5d} , are similar to the "optionally substituted hydroxyl group", the "optionally substituted thiol group", the "optionally substituted amino group", the "optionally esterified or amidated carboxyl group" and the "optionally substituted acyl group", each of which is the substituent of R ^{e1d} , respectively.

In the above formula (eId), examples of the "optionally substituted aliphatic hydrocarbon group" (aliphatic chain hydrocarbon group and aliphatic cyclic hydrocarbon group) represented by R^{e2d} and R^{e3a} include
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₈ cycloalkyl, etc. such as cyclopropyl, cyclobutyl, cyclopentyl, cyclohexyl, cycloheptyl, cyclooctyl, etc.), provided that
   (2-1) the cycloalkyl may contain one hetero-atom selected from a sulfur atom, an oxygen atom and a nitrogen atom to form oxirane, thiorane, aziridine, tetrahydrofuran, tetrahydrothiophene, pyrrolidine, tetrahydropyran, tetrahydrothiopyran, tetrahydrothiopyran 1-oxide, piperidine, etc. (preferably, 6-membered ring such as tetrahydropyran, tetrahydrothiopyran, piperidine, etc.); that
   (2-2) the cycloalkyl may be fused with a benzene ring to form indane, tetrahydronaphthalene, etc. (preferably, indane, etc.); and that
   (2-3) the cycloalkyl may be clrosslinked via straight-chained, atom chain having a carbon number of 1 to 2, and may form a crosslinked cyclic hydrocarbon residue such as bicyclo[2.2.1]heptyl, bicyclo[2.2.2]octyl, bicyclo[3.2.1]octyl, bicyclo[3.2.2]nonyl, etc., preferably, a cyclohexyl group, etc. having a bridging comprising a straight chain formed by 1-2 carbon atoms, and more preferably bicyclo[2.2.1]heptyl, etc.;
(3) an optionally substituted alkenyl (e.g. C₂₋₁₀ alkenyl such as allyl, crotyl, 2-pentenyl,3-hexenyl, etc., preferably lower (C₂₋₆)alkenyl, etc.);
(4) an optionally substituted cycloalkenyl (e.g. C₃₋₇ cycloalkenyl, etc. such as 2-cyclopentenyl, 2-cyclohexenyl, 2-cyclopentenylmethyl, 2-cyclohexenylmethyl, etc.); etc.

Examples of the substituents, which the above-mentioned (1) optionally substituted alkyl, (2) optionally substituted cycloalkyl, (3) optionally substituted alkenyl and (4) optionally substituted cycloalkenyl may have, include halogen (e.g. fluorine, chlorine, bromine, iodine, etc.), an optionally halogenated lower (C₁₋₄) alkyl, an optionally halogenated C₁₋₄ alkoxy (e.g., methoxy, ethoxy, propoxy, butoxy, trifluoromethoxy, trifluoroethoxy, etc.), C₁₋₄ alkylenedioxy (e.g., -O-CH₂-O-, -O-CH₂-CH₂-O-, etc.), formyl, C₂₋₄ alkanoyl (e.g., acetyl, propionyl, etc.), C₁₋₄ alkylsulfonyl (e.g., methanesulfonyl, ethanesulfonyl, etc.), phenyl-lower (C₁₋₄) alkyl, C₃₋₇ cycloalkyl, cyano, nitro, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), a lower (C₁₋₄) alkoxy-carbonyl, oxo group (preferably, halogen, an optionally halogenated lower (C₁₋₄) alkyl, an optionally halogenated lower (C₁₋₄) alkoxy, phenyl-lower (C₁₋₄) alkyl, C₃₋₇ cycloalkyl, cyano, hydroxy group, etc.), etc., and the number of the substituents are preferably 1 to 3.
Examples of the "optionally substituted aliphatic hydrocarbon group" represented by R^{e2d} and R^{e3d} include
(1) a lower (C₁₋₆) straight or branched alkyl which may have 1-3 substituents selected from the class consisting of halogen, cyano, hydroxy group and C₃₋₇ cycloalkyl;
(2) C₅₋₈ cycloalkyl which may be substituted with 1-3 substituents selected from the class consisting of a halogen, an optionally halogenated lower (C₁₋₄) alkyl and a phenyl-lower (C₁₋₄) alkyl, which may contain a hetero-atom selected from the class consisting of a sulfur atom, an oxygen atom and a nitrogen atom, which may be fused with a benzene ring and which may be crosslinked via straight-chained, atom chain having the carbon number of 1 to 2 (e.g., cyclopentyl, cyclohexyl, cycloheptyl, cyclooctyl, tetrahydropyranyl, tetrahydrothiapyranyl, piperidinyl, indanyl, tetrahydronaphthalenyl, bicyclo[2.2.1]heptyl, etc., each of which may be substituted); etc.

In the above formula (eId), examples of the "optionally substituted alicyclic (non-aromatic) heterocyclic group" represented by Re^{2d} and R^{e3d} include 5-to 6-membered non-aromatic heterocyclic ring containing 1 to 4 hetero-atoms consisting of 1 to 2 kinds of hetero-atoms selected from nitrogen atom, sulfur atom and oxygen atom such as tetrahydrofuran, tetrahydrothiophene, dioxolane, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, etc., preferably, a 5- to 6-membered non-aromatic heterocyclic ring containing 1 hetero-atom such as tetrahydrofuran, piperidine, tetrahydropyran, etc., or the like.

Examples of the substituent, which the "alicyclic heterocyclic group" in the "optionally substituted alicyclic heterocyclic group" represented by R^{e2d} and R^{e3d} may have, include halogen (e.g. fluorine, chlorine, bromine, iodine, etc.), an optionally halogenated lower (C₁₋₄) alkyl, an optionally halogenated C₁₋₄ alkoxy (e.g., methoxy, ethoxy, propoxy, butoxy, trifluoromethoxy, trifluoroethoxy, etc.), C₁₋₄ alkylenedioxy (e.g., -O-CH₂-O-, -O-CH₂-CH₂-O-, etc.), formyl, C₂₋₄ alkanoyl (e.g., acetyl, propionyl, etc.), C₁₋₄ alkylsulfonyl (e.g., methanesulfonyl, ethanesulfonyl, etc.), phenyl-lower (C₁₋₄) alkyl, C₃₋₇ cycloalkyl, cyano, nitro, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), a lower (C₁₋₄) alkoxy-carbonyl, oxo group (preferably, halogen, an optionally halogenated lower (C₁₋₄) alkyl, an optionally halogenated lower (C₁₋₄) alkoxy, phenyl-lower (C₁₋₄) alkyl, C₃₋₇ cycloalkyl, cyano, hydroxy group, etc.), etc., and the number of the substituents are preferably 1 to 3.

Among them, as R^{e2d}, an optionally substituted acyclic hydrocarbon group (e.g., alkyl, alkenyl, etc., each of which may be substituted) is preferable, an optionally substituted lower C₁₋₆ alkyl group is more preferable, and in particular, an optionally substituted methyl group is preferable.

As R^{e3d}, an optionally substituted alicyclic hydrocarbon group (e.g., cycloalkyl, cycloalkenyl, etc., each of which may be substituted; preferably, an optionally substituted lower C₃₋₈ cycloalkyl group; and more preferably, an optionally substituted cyclohexyl) or an optionally substituted alicyclic heterocyclic group (preferably, an optionally substituted saturated alicyclic heterocyclic group (preferably, 6 membered ring group)); more preferably, an optionally substituted tetrahydropyranyl, an optionally substituted tetrahydrothiopyranyl or an optionally substituted piperidyl; and in particular, an optionally substituted tetrahydropyranyl) is preferable.

The compound represented by the formula (IV) may be hydrated, and the compound represented by the formula (IV), the salt thereof and the hydrate thereof are hereinafter referred to as Compound (IV).

Compound (IV) can be produced, for example, by the following manner.

### Production Method 1

As shown by the following formula, Compound (IV) can be produced by reacting a compound of the formula (IId) or a salt thereof (hereinafter referred to as Compound (IId)) with a compound represented by the formula (IIId), a salt thereof or a reactive derivative thereof at the carboxyl group hereinafter referred to as Compound (IIId). 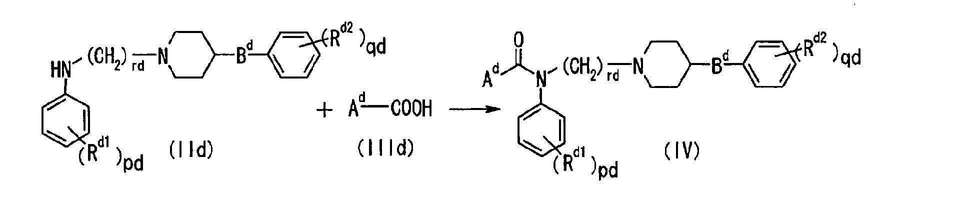 wherein each symbol has the same meaning given above.

Examples of the reactive derivative at the carboxyl group of a compound represented by the formula (IIId) which can be used in an acylating reaction include, for example, an acid halide, an acid azide, an acid anhydride, a mixed acid anhydride, an active amide, an active ester, an active thio ester, an isocyanate, etc. Examples of the acid halide include, for example, an acid chloride, an acid bromide, etc.; examples of the mixed acid anhydrides include a mono-C₁₋₆ alkyl-carbonic acid mixed acid anhydride (e.g. a mixed acid anhydride of free acid and monomethylcarbonic acid, monoethylcarbonic acid, mono-isopropylcarbonic acid, mono-isobutylcarbonic acid, mono-tert-butylcarbonic acid, mono-benzylcarbonic acid, mono-(p-nitrobenzyl)carbonic acid, mono-allylcarbonic acid, etc.), a C₁₋₆ aliphatic carboxylic acid mixed acid anhydride (e.g. a mixed acid anhydride of free acid and acetic acid, trichloroacetic acid, cyanoacetic acid, propionic acid, butyric acid, isobutyric acid, valeric acid, isovaleric acid, pivalic acid, trifluoroacetic acid, trichloroacetic acid, acetoacetic acid, etc.), a C₇₋₁₂ aromatic carboxylic acid mixed acid anhydride (e.g. a mixed acid anhydride of free acid and benzoic acid, p-toluic acid, p-chloro benzoic acid, etc.), an organic sulfonic acid mixed acid anhydride (e.g. mixed acid anhydride of free acid and methanesulfonic acid, ethanesulfonic acid, benzenesulfonic acid, p-toluenesulfonic acid, etc.) etc.; examples of the active amide include an amide with a nitrogen-containing heterocyclic compound (an acid amide of a free acid and, for example, pyrazole, imidazole, benzotriazole, etc., these nitrogen-containing heterocyclic compound may be substituted with a C₁₋₆ alkyl group (e.g., methyl, ethyl, etc.), a C₁₋₆ alkoxy group (e.g., methoxy, ethoxy, etc.), a halogen atom (e.g., fluorine, chlorine, bromine, etc.), an oxo group, a thioxo group, a C₁₋₆ alkylthio group (e.g., methylthio, ethylthio, etc.), etc.), etc.

As an active ester, all the active esters used in the field of the synthesis of β-lactam and peptide may be used. Examples of the active ester include, for example, an organic phosphoric acid ester (e.g. diethoxyphosphoric acid ester, diphenoxyphosphoric acid ester, etc.), p-nitrophenyl ester, 2,4-dinitrophenyl ester, cyanomethyl ester, pentachlorophenyl ester, N-hydroxysuccinimide ester, N-hydroxyphthalimide ester, 1-hydroxybenzotriazole ester, 6-chloro-1-hydroxybenzotriazole ester, 1-hydroxy-1H-2-pyridone ester, etc. Examples of the active thio ester include an ester of the acid with an aromatic heterocyclic thiol compound (e.g. 2-pyridylthiol ester, 2-benzothiazolylthiol ester, etc., which heterocyclics may be substituted with a C₁₋₆alkyl group (e.g. methyl, ethyl, etc.), a C₁₋₆alkoxy group (e.g., methoxy, ethoxy, etc.), a halogen atom (e.g., fluorine, chlorine, bromine, etc.), a C₁₋₆alkyl-thio group (e.g., methylthio, ethylthio, etc.), etc.).

Examples of the leaving group represented by L include, for example, a halogen atom (e.g., a chlorine atom, a bromine atom, an iodine atom, etc.), an alkyl- or arylsulfonyloxy group (e.g., methanesulfonyloxy, trifluoromethanesulfonyloxy, ethanesulfonyloxy, benzenesulfonyloxy, p-toluenesulfonyloxy, etc.), etc.

The reaction is usually carried out in a solvent inert to the reaction. Examples of the use for the solvent include an ether solvent (e.g., ethyl ether, diisoprpyl ether, dimethoxy ethane, tetrahydrofuran, dioxane, etc.), a halogenated solvent (e.g., dichloromethane, dicholoroethane, chloroform, etc.), an aromatic solvent (e.g., toluene, chlorobenzene, xylene, etc.), acetonitrile, N,N-dimethylformamide (DMF), acetone, methylethyl ketone, dimethylsulfoxide (DMSO), water, etc., or a mixed solvent thereof. Among them, acetonitrile, dichloromethane, chloroform, etc. are preferable. The reaction is usually carried out by using 1 to 5 equivalent, preferably 1 to 3 equivalents of Compound (IIId) relative to 1 equivalent of Compound (IId). The reaction temperature ranges from -20 °C to 50 °C, preferably 0 °C to room temperature, and reaction time is usually 5 minutes to 100 hours. The reaction may smoothly proceed by using a base. As the base, an inorganic base and an organic base can be used effectively. Examples of the inorganic base include a hydroxide, a hydride, a carbonate, a bicarbonate of alkaline metal or alkaline earth metal. Among them, potassium carbonate, sodium carbonate, sodium hydroxide, potassium hydroxide, sodium hydrogencarbonate, potassium hydrogencarbonate are preferable. Examples of the organic base preferably include a tertiary amine such as triethylamine. Examples of the reactive derivative at the carboxyl group of the formula (IIId) are as mentioned above, and among them, an acid halide is preferable. The used amount of the base is usually 1 to 10 equivalents, preferably 1 to 3 equivalents relative to 1 equivalent of Compound (IId).

The acylation reaction in which a carboxylic acid is used is carried out in an inert solvent (e.g., a halogenated solvent, acetonitrile) by reacting one equivalent of Compound (IId) with 1 to 1.5 equivalent of carboxylic acid (A^{d}-CO₂H) in the presence of 1 to 1.5 equivalent of dehydrating condensation agent such as dicyclohexyl carbodiimide (DCC), etc. The reaction is usually carried out at room temperature, and the reaction time is 0.5 to 24 hours.

Compound (IIId) used as the starting material in the reaction can be produced by a conventional manner using a compound described in Heterocycles, 43(10) 2131-2138 (1996) etc. as a starting compound. Compound (IId) used in the method can be produced by a manner described in Chem. Pharm. Bull. 47(1) 28-36 (1999) or Japanese unexamined patent publication No. 53654/1981 or a similar manner thereof.

Compound (IId) wherein rd is 3 can be produced, for example, by a method described in Synthetic Comm., 1991, 20, 3167-3180.

That is, the above compound can be produced by the following method by applying an addition reaction of amines or amides to unsaturated bond. 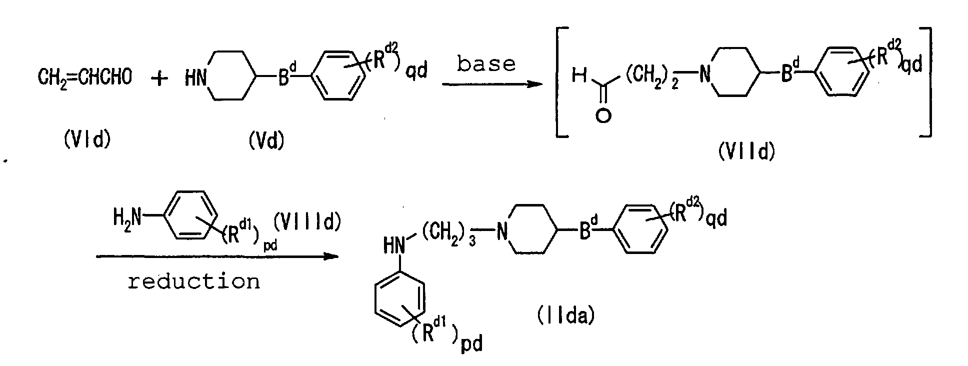 wherein each symbol has the same meaning as defined above.

The compound can be produced by reacting acrolein derivatives (VId) with Compound (Vd), followed by reacting the resulting compound (VIId) with Compound (VIIId) under a condition of reduction. The reaction of Compound (VId) with Compound (Vd) is usually carried out in a solvent inert to the reaction in the presence of a base. Examples of the base include 1) a strong base such as hydride of alkali metal or alkaline earth metal (e.g., lithium hydride, sodium hydride, potassium hydride, calcium hydride, etc.), an amide of an alkali metal or an alkaline earth metal (e.g., lithium amide, sodium amide, lithium diisopropylamide, lithium dicyclohexylamide, lithium hexamethyldisilazide, sodium hexamethyldisilazide, potassium hexamethyldisilazide, etc.), a lower alkoxide of alkali metal or alkaline earth metal (e.g., sodium methoxide, sodium ethoxide, potassium t-butoxide, etc.), etc., 2) an inorganic base such as a hydroxide of an alkali metal or an alkaline earth metal (e.g., sodium hydroxide, potassium hydroxide, lithium hydroxide, barium hydroxide, etc.), a carbonate of an alkali metal or an alkaline earth metal (e.g., sodium carbonate, potassium carbonate, cesium carbonate, etc.), a bicarbonate of alkali metal or alkaline earth metal (e.g., sodium hydrogencarbonate, potassium hydrogencarbonate, etc.), etc., 3) an organic base etc., such an amine as triethylamine, diisopropylethylamine, N-methylmorpholine, dimethylaminopyridine, DBU(1,8-diazabicyclo[5.4.0]-7-undecene), DBN(1,5-diazabicyclo[4.3.0]non-5-ene), etc., etc., and such basic heterocyclic Compound , etc., as pyridine, imidazole, 2,6-lutidine, etc. Examples of the solvent include those mentioned in the reaction of Compound (IId) with Compound (IIId). These solvents can be used solely or in combination. Compound (VIId) can be obtained in the reaction.

In the reaction between Compound (VIId) and Compound (VIIId), examples of the reducing agent include sodium borohydride, lithium borohydride, sodium borocyanohydride, sodium borotriacetooxyhydride, etc. The amount of the reducing agent used is usually 1 to 10 equivalent relative to 1 equivalent of Compound (VIId), preferably 1 to 4 equivalent. The reaction temperature is -20 to 50 °C, preferably 0 °C to ambient temperature, and the reaction time is 0.5 to 24 hours.

The reaction can also be carried out by a catalytic reduction reaction.
The catalytic reduction reaction is carried out in the presence of a catalytic amount of a metal catalyst such as Raney nickel, platinum oxide, metallic palladium, palladium-carbon, etc., in an inert solvent (e.g., an alcohol solvent such as methanol, ethanol, isopropanol, t-butanol, etc.), at room temperature to 100 °C, under a hydrogen pressure of 1 to 100 atm for 1 to 48 hours to produce Compound (IIda).

### Production Method 2

As shown below, Compound (IV) can be produced by reacting a compound (IVd) or its salt (herein after referred to as Compound (IVd)) with a compound (Vd) or its salt (herein after referred to as Compound (Vd)). 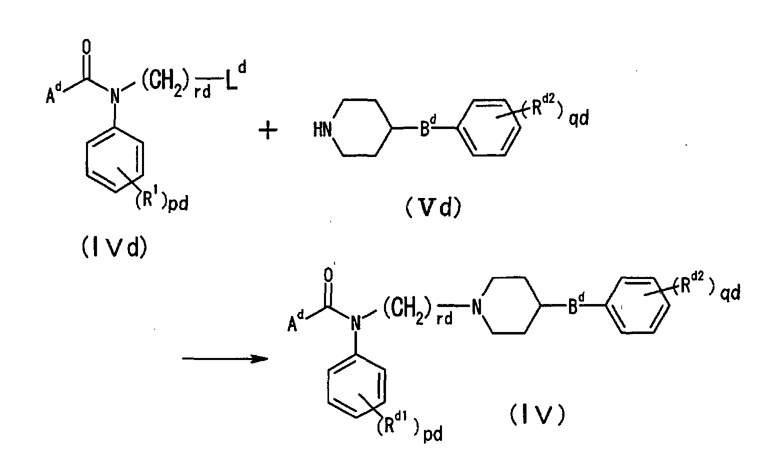 wherein L^{d} is a leaving group, and the other symbols have the meaning given above.

Examples of the leaving group represented by L^{d} include, for example, a halogen atom (e.g., a chlorine atom, a bromine atom, an iodine atom, etc.), an alkyl or arylsulfonyloxy group (e.g., methanesulfonyloxy, trifluoromethanesulfonyloxy, ethanesulfonyloxy, benzenesulfonyloxy, p-toluenesulfonyloxy, etc.), etc.

The reaction can be carried out by a manner similar to that described in Organic Functional Group Preparations 2nd ed., (Academic Press, Inc.).

The reaction is usually carried out in a solvent inert to the reaction. Examples of the solvent include an alcohol solvent, an ether solvent, a halogenated solvent, an aromatic solvent, acetonitrile, N,N-dimethylformamide (DMF), acetone, methylethyl ketone, dimethylsulfoxide (DMSO), etc. These solvents can be used solely or in combination. Among them, acetonitrile, dimethylformamide, acetone, ethanol, etc., are preferable. The reaction temperature ranges usually from room temperature to 100 °C, preferably from room temperature to 50 °C, and the reaction time is usually 0.5 to 1 day. In this reaction, a base is usually added in an amount of 1 to 3 equivalents relative to 1 equivalent of Compound (IVd), but it is not essential. Examples of the base include those mentioned in the reaction of Compound (IId) with Compound (IIId).

Compound (IVd) used as the starting material in this reaction can be produced by a known conventional manner with the use of Compound (IIId) as a starting material. Examples of the salt of a compound of the formulas (I). (II), (III), (IV) and (eI) of the present invention include an acid addition salt such as a salt of an inorganic acid (e.g., hydrochloric acid salt, sulfuric acid salt, hydrobromic acid salt, phosphoric acid salt, etc.), a salt of an organic acid (e.g., acetic acid salt, trifluoroacetic acid salt, succinic acid salt, maleic acid salt, fumaric acid salt, propionic acid salt, citric acid salt, tartaric acid salt, lactic acid salt, oxalic acid salt, methanesulfonic acid salt, p-toluenesulfonic acid salt, etc.), etc., a salt with a base (e.g. an alkali metal salt such as potassium salt, sodium salt, lithium salt, etc., an alkaline earth metal salt such as calcium salt, magnesium salt, etc., ammonium salt, a salt with an organic base such as trimethylamine salt, triethylamine salt, tert-butyl dimethylamine salt, dibenzyl methylamine salt, benzyl dimethylamine salt, N,N-dimethylaniline salt, pyridine salt, quinoline salt, etc.).

Compounds represented by the aforementioned formulas (I), (II), (III), (IV) and (eI) or salts thereof have a CCR antagonist effect, and particularly a CCR 5 antagonist effect, a CXCR4 antagonist effect, a CXCR3 antagonist effect, a CCR2 antagonist effect, and a CCR3 antagonist effect, and in order to reduce toxicity or side effects, can be administered orally or non-orally alone, or combined with a pharmaceutically acceptable carrier to form a pharmaceutical preparation such as a solid preparation such as tablets, capsules, granules and powders; or a liquid preparation such as a syrups and injectable preparations.

Examples of non-oral forms of administration include injections, intravenous drips, suppositories, rectal suppositories, vaginal suppositories, and the like.

Commonly used organic or inorganic substances may be employed as ingredients of the pharmaceutically acceptable carrier, and may be combined with excipients, lubricants, binders, and disintegrators in solid preparations, and solvents, solubilizers, suspending agents, isotonizing agents, buffers, analgesic agents and the like in liquid preparations. Additives such as preservatives, antioxidants, colorants, sweeteners and the like may also be added according to need. Examples of suitable excipients include lactose, sugar, D-mannitol, starch, corn starch, crystalline cellulose, light silicic anhydride and the like. Examples of suitable lubricants include magnesium stearate, calcium stearate, talc, colloidal silica, and the like. Examples of suitable binders include crystalline cellulose, sugar, D-mannitol, dextrin, hydroxypropyl cellulose, hydroxypropylmethyl cellulose, polyvinyl pyrrolidone, and the like. Examples of suitable disintegrators include starch, carboxymethyl cellulose, calcium carboxymethyl cellulose, croscarmellose sodium, sodium carboxymethyl starch, and the like. Examples of suitable solvents include water for injection, alcohol, propylene glycol, sesame oil, corn oil, and the like. Examples of suitable solubilizers include polyethylene glycol, propylene glycol, D-mannitol, benzyl benzoate, ethanol, trisaminomethane, cholesterol, triethanolamine, sodium carbonate, sodium citrate, and the like. Examples of suitable suspending agents include surfactants such as stearyltriethanolamine, sodium lauryl sulfate, laurylaminopropionic acid, lecithin, benzalkonium chloride, benzethonium chloride, glycerol monostearate, and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose, and the like. Examples of suitable isotonizing agents include sodium chloride, glycerol, D-mannitol, and the like. Examples of suitable buffers include buffer solutions of phosphate, acetate, carbonate, citrate, and the like. Examples of suitable analgesic agents include benzyl alcohol and the like. Examples of suitable preservatives include para-hydroxybenzoate esters, chlorobutanol, benzyl alcohol, phenethyl alcohol, dehydroacetic acid, sorbic acid, and the like. Examples of suitable antioxidants include sulfite salts, ascorbate salts, and the like.

Pharmaceutical compositions containing a compound represented by the aforementioned formulas (I), (II), (III), (IV) and (eI) or a salt thereof can be employed as an agent for the prevention and treatment of a variety of diseases, such as an agent for the prevention and treatment of graft-versus-host disease during organ transplantation and/or rejection reactions, an agent for the prevention and treatment for rheumatoid arthritis, autoimmune disease, allergy disorders, ischemic brain cell damage, myocardial infarction, chronic nephritis, and arteriosclerosis, and the like. Examples of the diseases that are targeted by the preventative and therapeutic agent of the present invention include transplant rejection reactions (post-transplant rejection reactions, post-transplant polycythemia/hypertension/organ damage/vascular thickening, graft-versus-host disease, and the like), arthritic bone diseases including osteomyelitis and the like (chronic rheumatoid arthritis, deforming arthritis, rheumatoid myelitis, osteoporosis, abnormal growth of cells and the like, bone fractures, refractures, osteomalacia, Behcet's syndrome of bone, ankylosing spondylitis, joint tissue destruction caused by deformation gonarthritis and related diseases, and the like), autoimmune diseases (collagen disease, systemic lupus erythematosus, scleroderma, polyarteritis, myasthenia gravis, multiple sclerosis, and the like), allergic disorders (allergic rhinitis, conjunctivitis, gastrointestinal tract allergies, pollinoisis, anaphylaxis, atopic dermatitis, bronchial asthma, and the like), inflammatory bowel diseases (ulcerative colitis, Crohn's disease, gastritis, gastric ulcer, post-operative gastric injury, dyspepsia, esophageal ulcer, pancreatisis, stress-induced gastric ulcer, colon polyps, cholelithiasis, hemorrhoidial disease, peptic ulcer, regional ileitis, and the like), inflammatory diseases (retinopathy, post-surgical/injury inflammation, reduction of swelling, pharyngitis, cystitis, meningitis, inflammatory ophthalmopathy, and the like), respiratory diseases (colds, pneumonia, asthma, pulmonary hypertension, pulmonary thrombosis, pulmonary embolism, pulmonary sarcoidosis, tuberculosis, interstitial pneumonia, silicosis, adult respiratory distress syndrome, chronic obstructive pulmonary disease, and the like), infectious diseases (viral infections such as cytomegalovirus, influenza virus, herpes virus, rickettsia infection, bacterial infection, sexually transmitted disease, pneumocystis carini pneumonia, Helicobactor pylori infection, systemic mycosis, tuberculosis, invasive staphylococcal infection, acute viral infection, acute bacterial infection, AIDS encephalopathy, septicemia, sepsis, severe sepsis, septic shock, endotoxic shock, toxic shock syndrome, and the like), carcinoma and cachexia accompanying the same, metastatic cancer (bladder cancer, breast cancer, uterine and cervical cancer, ovarian cancer, chronic lymphocytic leukemia, chronic myelogenous leukemia, colon cancer, rectal cancer, colorectal cancer, multipule myeloma, malignant myeloma, prostate cancer, lung cancer, stomach cancer, Hodgkin's disease, malignant melanoma, malignant lymphoma, and the like), non-Hodgkin's lymphoma, non-small cell lung cancer, malignant melanoma, neurodegenerative diseases (Alzheimer's disease, Parkinson's disease, amyotrophic lateral sclerosis (ALS), Huntington's chorea, diabetic neuropathy, Creutzfeldt-Jakob disease, and the like), psychiatric diseases (depression, epilepsy, dipsomania, and the like), schizophrenia, vascular insufficiency, central nervous system injury (injury due to cerebral hemorrhage and cerebral infarction as well as aftereffects/complications thereof, head injury, spinal cord injury, cerebral edema, injury to sensory functions, abnormal sensory functions, injury to the autonomic nervous system, abnormal autonomic nervous system, and the like), central injury (heat injury, spinal injury, whiplash, and the like), vascular dementia (multiinfarct dementia, Binswanger's disease, and the like), cerebrovascular injury (asymptomatic cerebrovascular injury, transient cerebral ischemic attack, cerebral accident, multiinfarct dementia, hypertensive encephalopathy, and the like), reoccurrence and after-effects of cerebrovascular injury (neurotic disorders, psychological disorders, subjective symptoms, disruptions to daily living activities, and the like), decline in central functions after multiinfarct dementia and cerebrovascular blockage, injury or abnormalities to the auto-modulation of cerebral circulation/renal circulation, injury to the blood-brain barrier, anxiety, acute coronary artery disease syndrome such as unstable angina, dysphoric psychological states, amnesia, trigeminal neuralgia, otorhinological diseases (Meniere's syndrome, tinnitus, injuries to the gustatory sense, dysphagia, and the like), migraine headache, chronic pain (keloid, hemangioma, psoriasis, and the like), arteriosclerosis obliterans, thromboangiitis obliterans, peripheral arterial occlusion, postischemic reperfusion injury, Raynaud's disease, Buerger disease, myocarditis, cardiac ischemia, cardiac infarction, 'progressive cardiac insufficiency after cardiac infarction, cardiomyopathy, cardiomegaly, chronic cardiac insufficiency including acute cardiac insufficiency and congestive heart failure, angina, arrhythmia, tachycardia, abnormal diurnal variation in blood pressure, abnormal blood/blood cell properties (platelet hyperagglutination, abnormally deformed red blood cells, white blood cell hyperadhesion, increase in blood viscosity, polycythemia, vascular peliosis, autoimmune hemolytic anemia, disseminated intravascular coagulation syndrome, multiple myeloma, and the like), athero and arteriosclerosis (aneurism, coronary artery sclerosis, cerebral artery sclerosis, peripheral arteriosclerosis, and the like), post-bypass surgery vascular reocclusion/restenosis, post-intervention (percutaneous transluminal coronary angioplasty, stent placement, coronary artery endoscope, endovascular ultrasound, coronary thrombolysis therapy, and the like) vascular thickening or occlusion and organ damage, production and hyperactivity of vasoactive substances and thrombosis inducers (endothelin, thromboxane A2, and the like), angiogenesis (including abnormal angiogenesis during abnormal capillary network formation in atherosclerotic lesions), thrombosis, fat deposit promotion, ophthalmopathy (glaucoma, high ocular pressure, and the like), hypertension, hypertensive tinnitus, dialytic hypotension, endothelial cell and organ derangement, endocrine diseases (Addison's disease, Cushing's disease, pheochromoctyoma, primary hyperaldosteronism, and the like), nephritis, renal diseases (nephritis, glomerulonephritis, glomerulosclerosis, renal insufficiency, thrombotic microangiopathy, dialysis complications, organopathy including nephropathy due to radiation exposure, diabetic nephropathy, and the like), diabetic diseases (insulin dependent diabetes, diabetic complications, diabetic retinopathy, diabetic microangiopathy, diabetic neuropathy, and the like), glucose tolerance abnormalities, liver diseases (hepatitis and chronic hepatitis, hepatocirrhosis, and the like), interstitial liver disease, chronic pancreatitis, portal hypertension, obesity, male infertility, gynecological diseases (menopausal disorders, gestosis, endometriosis, uterine myoma, ovarian diseases, mammary diseases, and the like), swelling, chronic fatigue syndrome, prostatomegaly, Behcet disease, Hodgkin's disease, Lacunar blockages, impaired consciousness, psoriasis, diseases due to environmental/vocational causes (radiation damage, damage caused by ultravioletrays/infrared rays/laser light, altitude sickness, and the like), claudicatio intermittens, and the like.

Pharmaceutical compositions that contain the compounds represented by the aforementioned formulas (I), (II), (III), (IV) and (eI) or salts thereof will differ according to the type of disease being targeted, but may be employed in combination with other drugs. Examples of those other drugs include HDL enhancers [squalene synthase inhibitors, CEPT inhibitors, LPL activators, and the like], agents for the prevention and treatment of HIV infection [nucleoside reverse transcriptase inhibitors such as zidovudine, didanosine, zalcitabine, lamivudine, stavudine, abacavir, adefovir, adefovir dipivoxil, fozivudine tidoxil, and the like, non-nucleoside reverse transcriptase inhibitors such as nevirapine, delavirdine, efavirenz, loviride, immunocal, oltipraz, and the like, and protease inhibitors such as saquinavir, ritonavir, indinavir, nelfinavir, amprenavir, palinavir, lasinavir, lopinavir, and the like], HMG-CoA reductase inhibitors: cerivastatin, atorvastatin, pravastatin, simvastatin, itavastatin, lovastatin, fluvastatin, (+)-3R,5S-7-[4-(4-fluorophenyl)-6-isopropyl-2-(N-methyl-N-methanesulfonylamino)pyrimidin-5-yl]3,5-dihydroxy-6(E)-heptenoic acid, and the like, atopic dermatitis therapeutic agents [sodium chromoglycate and the like], allergic rhinitis therapeutic agents [sodium chromoglycate, chlorphenylamine maleate, alimemazine tartrate, clemasine fumarate, homochlorocyclizine hydrochloride, terfenadine, mequitazine, and the like], imipenem cilastatin sodium, endotoxin antagonists or antibodies, oxidosqualene-lanosterol cylase [e.g., decalin derivatives, azadecalin derivatives, and indane derivatives], calcium antagonists (diltiazem and the like), glycerol, choline esterase inhibitors (e.g., Aricept (donepezil) and the like), compounds which suppress cholesterol absorption (e.g., sitosterol, neomycin, and the like), compounds which inhibit cholesterol production [e.g., HMG-CoA reductase inhibitors such as lovastatin, simvastatin, pravastatin, and the like], cyclooxygenase suppressors [(Cox-I, Cox-II suppressors) e.g., celecoxib, rofrcoxib, salicylic acid derivatives such as aspirin or the like, diclofenac, indomethacin, loxoprofen, and the like], signal transduction inhibitors, squalene epoxidase inhibitors [e.g., NB-598, related compounds and the like], steroids [e.g., dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclometasone dipropionate, estriol, and the like], diacerin, niacin, derivatives thereof and analogues thereof [e.g., acipimox and probucol], nicerogolin, therapeutic agents for nephrotic syndrome: prednisolone (Predonine), prednisolone sodium succinate (Predonine), sodium methylprednisolone succinate (Solu-Medrol), betamethasone (Rinderon), dipyridamole (Persantine), dilazep hydrochloride (Comelian), ticlopidine, clopidogrel, antiplatelet drugs such as FXa inhibitors and the like and anticoagulant drugs, barbital type anticonvulsant drugs or anesthetics (phenobarbital, mephobarbital, metharbital, and the like), therapeutic agents for Parkinson's disease (e.g., L-dopa and the like), histamine receptor blocking agents (cimetidine, famotidine, and the like), hydantoin type anticonvulsant drugs (phenytoin, mephenytoin, ethotoin, and the like), hydroxycam, fibrates (e.g, clofibrate, benzafibrate, gemfibrozil, and the like), prostaglandins, megestrol acetate, therapeutic agents for gastric and duodenal ulcers: antacids [e.g., histamine H2 antagonists (cimetidine and the like), proton pump inhibitors (lansoprazole and the like), and the like], inflammatory mediator effect suppressants, coronary vasodilators: nifedipine, diltiazem, nicorandil, nitrous acid agents, and the like, therapeutic agents for infection: [e.g., antibiotics (cefotiam dihydrochloride, cefozopran hydrochloride, ampicillin, and the like), chemotherapeutic agents (sulfa drugs, synthetic antibacterial drugs, antiviral drugs, and the like), biological preparations (vaccines, blood preparations such as immunoglobulin and the like) and the like] and the like, therapeutic agents for liver disease: glycyrrhizin preparations [e.g., stronger minophagen and the like], liver hydrolyzate, SH compounds [e.g., glutamines and the like], special amino acid preparations [e.g., aminoleban and the like], phosphatides [e.g., polyenephosphatidylcholine and the like], vitamins [e.g., vitamins B1, B2, B6, B12, C and the like], adrenal cortical hormones [e.g., dexamethasone, betamethasone, and the like], interferon [e.g., interferon α, interferon β, and the like], therapeutic agents for hepatic encephalopathy [e.g., lactulose and the like], hemostatic agents employed during esophageal and gastric varix ruptures [e.g., vasopressin, somatostatin, and the like], and the like, therapeutic agents for arthritis, muscle relaxants [pridinol, tubocurarine, pancuronium, tolperisone hydrochloride, chlorphenesin carbamate, baclofen, chlormezanone, mephenesin, chlorzoxazone, eperisone, tizanidine, and the like], vasodilators [oxyphedrin, diltiazem, tolazoline, hexobendine, bamethan, clonidine, methyldopa, guanabenz, and the like], vasoconstrictors [dopamine, dobutamine denopamine, and the like], blood platelet aggregant suppressors (ozagrel and the like), agents for preventing or treating thrombus formation: anticoagulants [e.g., heparin sodium, heparin calcium, warfarin calcium (warfarin), Xa inhibitors], thrombolytic agents [e.g., tPA, urokinase], antiplatelet agents [e.g., aspirin, sulfinpyrazone (Anturan), dipyridamole (Persantin), ticlopidine (Panaldine), cilostazol (Pletal), GPIIb/IIIa antagonists (Rheopro)], antidepressant drugs [imipramine, chlomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride, and the like], antiepileptic drugs [gabapentin, phenytoin, ethosuximide, acetazolamide, chlordiazepoxide, trimethadione, carbamazepine, phenobarbital, primidone, sulthiame, sodium valproate, clonazepam, diazepam, nitrazepam, and the like], antiallergic drugs [diphenhydramine, chlorpheniramine, tripelennamine, methdilamine, clemizole, diphenylpyraline, methoxyphenamine, sodium cromoglycate, tranilast, Repriinast, Amlexanox, ibudilast,, ketotifen, terfenadine, mequitazin, azelastine, epinasine, ozagrel hydrochloride, pranlkast hydrate, seratrodast, fexofenadine, ebastine, Bucillamine, Oxatomide, Stronger Neo-Minophagen, tranexamic acid, ketotifen fumarate, and the like], cholilytic drugs (e.g., ipratropium bromide, flutropium bromide, oxyphenonium bromide, and the like), anti-Parkinson's drugs (dopamine, levodopa, and the like), anti-rheumatic drugs, anti-inflammation agents (e.g., aspirin, acetaminophen, diclofenac sodium, ibuprofen, indomethacin, loxoprofen sodium, dexamethasone, and the like), anticoagulant drugs and anti-platelet drugs [sodium citrate, activated protein C, tissue factor path inhibitors, antithrombin III, dalteparin sodium, argatroban, gabexate, ozagrel sodium, ethyl icosapenate, beraprost sodium, alprostadil, pentoxyfylline, tisokinase, streptokinase, heparin, and the like], anticoagulation therapy agents [dipyridamole (Persatine), dilazep hydrochloride (Comelian), ticlopidine, clopidogrel, Xa inhibitors], antibacterial drugs [(i) sulfa drugs [sulfamethizole, sulfisoxazole, sulfamonomethoxine, sulfamethizole, salazosulfapyridine, sulfadiazine silver, and the like], (ii) quinoline type antibiotics [nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin, and the like], (iii) antituberculosis drugs [isoniazid, ethambutol ethambutol hydrochloride]]], p-aminosalicylic acid (calcium paraaminosalicylate), pyrazinamide, ethionamide, protionamide, rifampicin, streptomycin sulfate, kanamycin sulfate, cyclocerine, and the like], (iv) antiacidfast bacterium drugs [diaphenylsulfone, rifampicin, and the like], (v) antiviral drugs [idoxuridine, aciclovir, aminopterine, vidarabine, ganciclovir, and the like], (vi) anti-HIV drugs [zidovudine, didanosine, zalcitabine, indinavir sulfate ethanol adduct, ritonavir, and the like], (vii) antispirochetic drugs, (viii) antibiotics [tetracycline hydrochloride, ampicillin, piperacillin, gentamicin, dibekacin, kanendomycin, lividomycin, tobramycin, amikacin, fradiomycin, sisomicin, tetracycline, oxytetracycline, rolitetracycline, doxycycline, ampicillin, piperacillin, ticarcillin, cefalothin, cephapirin, cephaloridine, cefaclor, cephalexin, cefroxadine, cefadroxil, cefamandole, cefotiam, cefuroxime, cefotiam, cefotiam hexetyl, cefuroxime axetil, cefdinir, cefditoren pivoxil, ceftazidime, cefpiramide, cefsulodin, cefmenoxime, cefpodoxime proxetil, cefpirome, cefozopran, cephem, cefsulodin, cefmetazole, cefminox, cefoxitin, cefbuperazone, latamoxef, flomoxef, cefazolin, cefotaxime, cefoperazone,ceftizoxime, moxalactam, thienamycin, sulfazecin, azthreonam, or salts thereof, griseofulvin, lankacidins [Journal of Antibiotics, 38, 877-885 (1985)] and the like], cefixime, levofloxacin], antithrombosis agents (argatroban and the like), antiprotozoan drugs [metronidazole, tinidazole, diethylcarbamazine citrate, quinine hydrochloride, quinine sulfate, and the like], antineoplastic drugs [6-0-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, hexoprenaline sulfate, vincristine sulfate, vinblastine sulfate, irinotecan hydrochloride, cyclophosphamide, melphalan, busulphan, thiotepa, procarbazine hydrochloride, cisplatin, azathioprine, mercaptopurine, tegafur, carmofur, cytarabine, methyltestosterone, testosterone propionate, testosterone enanthnate, mepitiostane, fosfestrol, chlormadinone acetate, leuprolide acetate, buserelin acetate, and the like], antifungal drugs [(i) polyethylene type antibiotics (e.g., amphotericin B, nystatin, trichomycin), (ii) griseofulvin, pyrrolnitrin, and the like, (iii) cytosine antimetabolites (e.g., flucytosine), (iv) imidazole derivatives (e.g., econazole, clotrimazole, miconazole nitrate, bifonazole, cloconazole), (v) triazole derivatives (e.g., fluconazole, itraconazole, azole compounds [2-[(1R,2R)-2-(2,4-difluorophenyl)-2-hydroxy-1-methyl-3-(1H-1,2,4-triazol-1-yl)propyl]-4-[4-(2,2,3,3-tetrafluorobenzene)phenyl-3-(2H,4H)-1,2,4-triazolam], (vi) thiocarbamic acid derivatives (e.g., trinaphthol), (vii) echinocandin derivatives (e.g., caspfungin, FK-463, V-echinocandin) and the like], antipsychotic drugs [chlorpromazine hydrochloride, prochloroperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, recerpine, clocapramine hydrochloride, sulpiride, zotepine, and the like], antiulcer drugs [metoclopramide, histidine hydrochloride, lansoprazole, metoclopramide, pirenzepine, cimetidine, ranitidine, famotidine, urogastrine, oxethazaine, proglumide, omeprazole, sucralfate, sulpiride, cetraxate, gefarnate, aldioxa, teprenone, prostaglandin, and the like], anti-diabetes agents (e.g., pioglitazone, nateglinide, voglibose, acarbose, and the like), antiobestic drug (mazindol and the like), antirheumatic drugs and the like, antianxiety drugs [diazepam, lorazepam, oxazepam, chlordiazepoxide, medazepam, oxazolam, cloxazolam, clotiazepam, bromazepam, etizolam, fludiazepam, hydroxyzine, and the like], antiarrhythmic drugs: disopyramide, lidocaine, quinidine sulfate, flecainide acetate, mexiletine hydrochloride, amitriptyline hydrochloride, and β-blockers, Ca antagonists and the like, antiasthmatic drugs [isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, ipratropium bromide, oxyphenonium bromide, flutropium bromide, theophyline, aminophyllin, sodium cromoglycate, tranilast, repirinast, Amlexanox, ibudilast, ketotifen, terfenadine, mequitazine, azelastine, epinastine, ozagrel hydrochloride, pranlkast hydrate, seratrodast, dexamethasone, prednisolone, hydrocortisone, beclomethasone propionate, fluticasone propionate, beclomethasone dipropionate, procaterol, and the like], therapeutic agents for hypothyroidism [desiccated thyroid (Thyreoid), levothyroxine sodium (Thyroxine S), liothyronine sodium (Thyronine, Thyromin); therapeutic agents for nephrotic syndrome: prednisolone (Predonine), prednisolone sodium succinate (Predonine), sodium methylprednisolone succinate (Solu-Medrol), betamethasone (Rinderon)], therapeutic drugs for hypertension [(i) sympathetic nerve suppressants [ α 2 stimulators (e.g., clonidine, guanabenz, guanfacine, methyldopa, and the like), ganglion blocking drugs (e.g., hexamethonium, trimethaphan, and the like), presynapse blocking agents (e.g., alseroxylon, dimethylamino reserpinate, Rescinnamine, Reserpine, Syrosingopine, and the like), neuron blocking drugs (e.g., betanidine, guanethidine, and the like), α1 blocking drugs (e.g., bunazosin, doxazosin, prazosin, terazosin, urapidil, and the like), β blocking drugs (e.g., propranolol, nadolol, timolol, nipradilol, bunitrolol, indenolol, penbutolol, carteolol, carvedilol, pindolol, acebutolol, atenolol, bisoprolol, metopro'lol, labetalol, amoslalol, arotinolol, and the like), and the like, (ii) vasodilator drugs [calcium channel antagonists (e.g., manidipine, nicardipine, nilvadipine, nisoldipine, nitrendipine, benidipine, amlodipine, aranidipine, and the like), phthalazine derivatives (e.g., budralazine, cadralazine, ecarazine, hydralazine, todralazine, and the like), and the like], (iii) ACE inhibitors [alacepril, captopril, cilazapril, Delapril, enalapril, lisinopril, temocapril, trandolapril, quinapril, imidapril, benazepril, perindopril, and the like], (iv) AII antagonists [losartan, candesartan, valsartan, termisartan, irbesartan, forasartan, and the like], (v) diuretics (e.g., the aforementioned diuretics and the like)], therapeutic agents for hypertension: diuretics [e.g, furosemide (Lasix), bumetanide (Luneoron), azosemide (Diart)] hypotensive drugs [e.g., ACE inhibitors, (enalapril maleate (Renivace) and the like) and Ca antagonists (manidipine, amlodipine, and the like), α or β receptor blocking drugs and the like], therapeutic agents for hyperlipemia [HMG-CoA reductase inhibitors (e.g., fluvastatin, cerivastatin, atorvastatin, and the like), fibrate drugs (e.g., simfibrate, aluminium clofibrate, clinofibrate, fenofibrate, and the like), anion exchange resins (e.g., cholestyramide and the like), nicotinic acid preparations (e.g., nicomol, niceritrol, tocopherol nicotinate, and the like), polyunsaturated fatty acid derivatives (e.g., ethyl icosapenate, polyenephosphatidylcholine, melinamide, and the like), plant sterols (e.g., gamma-oryzanol, soysterol, and the like), elastase, dextran sulfate sodium, squalene synthase inhibitors, CEPT inhibitors, ethyl 2-chloro-3-[4-(2-methyl-2-phenylpropyloxy)phenyl]propionate (Chemical and Pharmaceutical Bulletin, 38, 2792-2796 (1990)) and the like], drugs for treating bone diseases: calcium preparations (e.g., calcium carbonate and the like), calcitonine preparations, activated vitamin D₃ preparations (e.g., alfacarcidol (Alfarol and the like), calcitrol (Rocaltrol) and the like), sex hormones (e.g., estrogen, estradiol and the like), hormone preparations [e.g., conjugated estrogen (Premarin) and the like], ipriflavone preparations (Osten), vitamin K₂, vitamin K₂ preparations [e.g., menatetrenone (Glakay) and the like], bisphosphonic acid preparations (etidronate and the like), prostaglandin E2, fluorine compounds (e.g., sodium fluoride and the like), bone morphogenetic protein (BMP), fibroblast growth factor (FGF), platelet derived growth factor (PDGF), transforming growth factor (TGF-β), insulin-like growth factors 1 and 2 (IGF-1, -2), parathyroid hormone (PTH), compounds described in European Patent Application Publication No. EP-A1-376197, EP-A1-460488 and EP-A1-719782 (e.g.. (2R,4S)-(-)-N-[4-(diethoxyphosphorylmethyl)phenyl)-1,2,4,5-tetrahydro-4-methyl-7,8-methylenedioxy-5-oxo-3-benzothiepin-2-carboxamide, and the like) and the like), and the like, fat soluble vitamins [(i) vitamin A: vitamin A₁, vitamin A₂, and retinol palmitate, (ii) vitamin D: vitamins D₁, D₂, D₃, D₄, D₅, (iii) vitamin E: α -tocopherol, β -tocopherol, γ - tocopherol, δ -tocopherol, dl- α -tocopherol nicotinate), (iv) vitamin K: vitamins K₁, K₂, K₃, K₄, (v) folic acid (vitamin M), and the like, vitamin derivatives [derivatives of each type of vitamin, e.g., vitamin D₃ derivatives such as 5,6-trans-cholecalciferol, 2,5-hydroxycholecalciferol, 1-α-hydroxycholecalciferol, and the like, vitamin D₂ derivatives such as 5,6-trans-ergocalciferol, and the like], disease modulating antirheumatic drugs and immunosuppressive drugs [e.g., methotrexate, leflunomide, prograf, sulphasalazine, D-penicillamine, oral gold agents], vasopressor drugs [dopamine, dobutamine, denopamine, digitoxin, digoxin, methyldigoxin, lanatoside C, G-strophantin, and the like], myocardiac protection drugs: drugs for opening heart ATP-K, Na-H exchange inhibitors, endothelin antagonists, urotensin antagonists, and the like, drugs for treating cardiac insufficiency [cardiac restoratives (e.g., digitoxin, digoxin, methyldigoxin, lanatoside C, proscillaridin, and the like), α, β stimulators (e.g., epinephrine, norepinephrine, isoproterenol, dopamine, docarpamine, dobutamine, denopamine, and the like), phosphodiesterase inhibitors (e.g., amrinone, milrinone, olprinone hydrochloride, and the like), calcium channel sensitizers (e.g., pimobendan and the like), nitric acid drugs (e.g., nitroglycerin, isosorbide dinitrate, and the like), ACE inhibitors (e.g., the aforementioned ACE inhibitors and the like), diuretics (e.g., the aforementioned diuretics and the like), carperitide, ubidecarenone, vesnarinone, aminophylline, and the like], neurotrophic factors, drugs for treating renal insufficiency/nephropathy, biological preparations [e.g., monoclonal antibodies, (e.g., anti TNF-α antibodies, anti IL-12 antibodies, anti IL-6 antibodies, anti ICAM-I antibodies, anti CD4 antibodies, and the like), soluble receptors (TNF-α receptors and the like), protein ligands (IL-I receptor antagonist and the like)], bile acid binding resins [e.g., cholestyramine, colestipol, and the like], drugs for treating biliary tract disease: choleratic drugs [e.g., dehydrocholic acid and the like], cholekinetic agents [e.g., magnesium sulfate and the like] and the like, central nervous system agents: antianxiety drugs, sedative hypnotic drugs, anesthetic drugs, antispasmodic drugs, autonomic drugs, antiparkinsonic drugs and other psychiatric drugs and the like, antitussives and expectorants [ephedrine hydrochloride, noscapine hydrochloride, codeine phosphate, dihydrocodeine phosphate, isoproterenol hydrochloride, ephedrine hydrochloride, methylephedrin hydrochloride, noscapine hydrochloride, alloclamide, chlorphezianol, picoperidamine, cloperastine, protokylol, isoproterenol, salbutamol, terbutaline, oxymethebanol, morphine hydrochloride, dextromethorphan hydrobromide, oxycodone hydrochloride, dimorphan phosphate, tipepidine hibenzate, pentoxyverine citrate, clofedanol hydrochloride, benzonatate, guaifenesin, bromhexine hydrochloride, ambroxol hydrochloride, acetylcysteine, ethylcysteine hydrochloride, carbocysteine, and the like], sedative drugs [chlorpromazine hydrochloride, atropine sulfate, phenobarbital, barbital, amobarbital, pentobarbital, sodium thiopental, thiamylal sodium, nitrazepam, estazolam, flurazepam, haloxazolam, triazolam, flunitrazepam, bromvalerylurea, chloral hydrate, triclofos sodium, and the like], analgesic and antiphlogistic drugs [e.g., centrally acting analgesics (e.g., morphine, codeine, pentazocine, and the like), steroids (e.g., prednisolone, dexamethasone, betamethasone, antiphlogistic enzymes (e.g., promercin, lysozyme, proctase, and the like)], drugs for treating diabetes [sulfonylureas (e.g., tolbutamide, chlorpropamide, glyclopyramide, acetohexamide, tolazamide, glibenclamide, glybuzole, and the like), biguanide agents (e.g., metformin hydrochloride, buformine hydrochloride, and the like), α-glucosidase inhibitors (e.g., voglibose, acarbose, and the like), drugs for improving insulin resistance (e.g., pioglitazone, troglitazone, and the like), insulin, glucagon, drugs for treating diabetic complications (e.g., epalrestat, thioctic acid, and the like), Actos, rosiglitazone, Kinedak, Penfill, Humulin, Euglucon, Glimicron, Daonil, Novolin, Monotard, insulin, Glucobay, Dimelin, Rastinon, , Deamelin-S, Iszilin, and the like], cerebral function activation drugs (e.g., Idevenone, vinpocetine, and the like), drugs for treating urinary system and male sex organ diseases:[e.g., drugs for treating prostatomegaly (tamsulosin hydrochloride, prazosin hydrochloride, chlormadinone acetate, and the like)] and the like, non-steroidal antiinflammatory drugs [acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenburfen, pranoprofen, floctafenine, epirizole, tiaramide hydrochloride, zaltoprofen, gabexate mesilate, camostat mesilate, urinastatin, colchicine, probenecid, sulfinpyrazone, benzbromarone, allopurinol, sodium aurothiomalate, sodium hyaluronate, sodium salicylate, morphine hydrochloride, salicylic acid, atropine, scopolamine, morphine, pethidine, levorphanol, ketoprofen, naproxen, oxymorphone, or salts thereof and the like], drugs for treating pollakiuria/incontinence [flavoxate hydrochloride and the like], unstable plaque stabilizers [MMP inhibitors, chymase inhibitors, and the like], antiarrhythmic drugs [sodium channel blocking drugs (e.g., chinidine, procaineamide, disopyramide, ajmaline, cibenzoline, lidocaine, diphenylhydantoin, mexiletine, propafenone, flecainide, pilsicainide, phenytoin, and the like), β-blocking drugs (e.g., propranolol, alprenolol, bufetolol, oxprenolol, atenolol, acebuolol, metoprolol, bisoprolol, pindolol, carteolol, arotinolol, and the like), potassium channel blocking drugs (e.g., amiodarone and the like), calcium channel blocking drugs (e.g., verapamil, diltiazem, and the like) and the like], drugs for treating gynecological diseases: [e.g., drugs for treating menopausal disorders (conjugated estrogen, estradiol, testosterone enanthnate/estradiol valerate, and the like), drugs for treating breast tumors (tamoxifen citrate, and the like), drugs for treating endometriosis/hysteromyoma (leuprorelin acetate, danazol, and the like)] and the like, anesthetics [a. local anesthetics [cocaine hydrochloride, procaine hydrochloride,. lidocaine, dibucaine hydrochloride, tetracaine hydrochloride, mepivacaine hydrochloride, bupivacaine hydrochloride, oxybuprocaine hydrochloride, ethyl aminobenzoate, oxethazaine] and the like], b. general anesthetics [(i) inhalational anesthetics (e.g., ether, halothane, nitrous oxide, influrane, enflurane), (ii) intravenous anesthetics (e.g., ketamine hydrochloride, droperidol, thiopentone sodium, thiamylal sodium, pentobarbital) and the like]], narcotic antagonists [levallorphan, nalorphine, naloxone, or salts thereof and the like], drugs for treating chronic heart failure: cardiac stimulants [cardiac glycosides (digoxin and the like), β receptor stimulants (catecholamine preparations such as denopamine and dobutamine) and PDE inhibitors and the like], diuretics [e.g., furosemide (Lasix), spironolactone (Aldactone), bumetanide (Luneoron), azosemide (Diart) and the like], ACE inhibitors, [e.g., enalapril maleate (Renivace) and the like], Ca antagonists [e.g., amlodipine, manidipine, and the like], and β receptor blocking drugs and the like, immunomodulators [cyclosporin, tacrolimus, gusperimus, azathioprine, antilymphocyte serum, dried sulfonated immunoglobulin, erythropoietin, colony stimulating factor, interleukin, interferon, and the like], diuretics [thiazide diuretics (benzylhydrochlorothiazide, cyclopenthiazide, ethiazide, hydrochlorothiazide, hydroflumethiazide, methyclothiazide, penflutizide, polythiazide, trichlormethiazide, and the like), loop diuretics (chlorthalidone, clofenamide, indapamide, mefruside, meticrane, metolazone, tripamide, quinethazone, metolazone, furosemide, mefruside, and the like), potassium sparing diuretics (spironolactone, triamterene, and the like)], and impotence drugs (Viagra, apomorphine, and the like), and the like.

These drugs can be formulated by mixing individually or simultaneously with pharmaceutically acceptable carriers, excipients, binders, diluents or the like, and can be administered orally or non-orally. When the drugs are formulated individually, they can be mixed with a diluent or the like at the time of use and then administered, however individually formulated preparations may be administered simultaneously or over a period of time to the same subject. A kit in which a diluent or the like is mixed with the individually formulated drugs at the time of use and administered (e.g. an injection kit which contains ampoules each containing a powdered drug and a diluent for mixing and dissolving two or more drugs at the time of use, and the like), a kit for administering individually formulated preparations simultaneously or over a period of time to the same subject (e.g. a tablet kit for administering tablets simultaneously or over a period of time, the kit having two or more tablets each containing a drug and placed in the same or separate bags and, if necessary, a time table for administering the drugs, and the like), and the like are also included in the pharmaceutical compositions of the present invention.

In the event that the aforementioned pharmaceutical compositions are employed as agents for the prevention and treatment of graft-versus-host disease and/or rejection reactions when organs such as the heart, kidney, liver, bone marrow, and the like are transplanted, they can be administered from 3 days before transplantation, and can be continuously administered after transplantation. The dose per day of the present pharmaceutical compositions will differ depending on the condition and body weight of a patient and the method of administration, however with oral administration, about 5 to 1000 mg, preferably about 10 to 600 mg, more preferably about 10 to 300 mg, and in particular about 15 to 150 mg, of the active ingredient [Compounds (I), (II), (III), (IV), (Ie)] can be administered per adult patient (body weight 50 kg) once or 2 to 3 times per day. In addition, in these cases, other agents for suppressing graft-versus-host disease and/or rejection reactions during organ transplant may be employed in combination. Specific examples of the agents that are employed to suppress graft-versus-host disease and/or rejection reactions during organ transplant in combination with the compounds represented by the aforementioned formulas (I), (II), (III), (IV), and (eI) or salts thereof include cyclosporin, tacrolimus, rapamycin, steroids, axathioprine, mycophenolate mofetil, mizoribine, and the like. In the event that these drugs are employed in combination, the dosage of each drug can be suitably adjusted when one drug has an impact on the metabolism of another drug, however the dosage that is generally employed is that when each drug is administered alone.

The daily dosage of the compounds represented by the aforementioned formulas (I), (II), (III), (IV), and (eI) or salts thereof that are employed for diseases other than the suppression of graft-versus-host disease and/or rejection reactions during organ transplant will differ depending on the type of disease, the condition and body weight of a patient, and the method of administration, however with oral administration, about 5 to 1000 mg, preferably about 10 to 600 mg, more preferably about 10 to 300 mg, and in particular about 15 to 150 mg, of the active ingredient [Compounds (I), (II), (III), (IV), (Ie)] can be administered per adult patient (body weight 50 kg) once or 2 to 3 times per day. In addition, in the event that other drugs are used in combination, the dosage of the other drugs can, for example, be suitably selected in a range between about 1/200 to 1/2 times or greater, and about 2 to 3 times or less, than the normal dosage. Furthermore, in the event that two or more types of drugs are employed in combination, the dosage of each drug can be suitably adjusted when one drug has an impact on the metabolism of another drug, however the dosage that is generally employed is that when each drug is administered alone.

The following working examples, reference examples, experimental examples, and formulation examples are illustrated below to describe the present invention in further detail. However, these are merely examples, and do not limit the present invention in any way.

The following gene manipulation method is carried out in accordance with methods described in the textbook (Maniatis et al., Molecular Cloning, Cold Spring Harbor Laboratory, 1989) or the method described in the protocols that accompany the reagents.

In the reference examples and working examples below, silica gel 60 (Merck Corp., 70 to 230 or 230 to 400 mesh) was used as packing for column chromatography unless otherwise noted. ¹H NMR spectra were measured using tetramethylsilane as an internal reference with a Gemini 200 spectrometer (Varian, 200 MHz).

### Reference Example 1-1

### t-Butyl 4-(2-ethoxy-2-oxoethylidene)-1-piperidine carboxylate

To a solution of ethyl diethylphosphoryl acetate (28.3g) in tetrahydrofuran (200 ml) was added 60% sodium hydride (4.82g) under ice cooling and the mixture was stirred for 30 minutes, and then a solution of N-butoyxcarbonyl-4-piperidone (20g) in tetrahydrofuran (200 ml) was added dropwise thereto. The mixture was stirred for 22 hours at room temperature. After the completion of the reaction, water (200 ml) was added and extraction was carried out with ethyl acetate. After the extract was washed with saturated aqueous sodium chloride solution and dried over anhydrous magnesium sulfate, the concentrated residue obtained was then purified using silica gel column chromatography and eluted with hexane/ethyl acetate (6/1) to obtain the title compound (27.3 g, 100%) as a colorless powder.
¹H NMR (CDCl₃) δ 1.28 (3H, t, J=7.4Hz), 1.47 (9H, s), 2.24-2.33 (2H, m), 2.90-2.98 (2H, m), 3.43-3.55 (4H, m), 4.16 (2H, q, J=7.4Hz), 5.70-5.73 (1H, m).

### Reference Example 1-2

### [1-(Methylsulfonyl)-4-piperidylidene]ethyl acetate

The compound (10g) obtained in Reference Example 1-1 was dissolved in methanol (100 ml), and 4N hydrochloric acid-ethyl acetate (20 ml) and trifluoroacetic acid (2.5 ml) were added and the mixture was stirred for 3 hours. The solvent was evaporated, and the residue obtained was washed with ethyl acetate to obtain a colorless powder (6.64 g).

The colorless powder (6.64 g) obtained were added to tetrahydrofuran (100 ml) and triethylamine (9.9 ml), mesyl chloride (3 ml) was added dropwise with ice cooling, and the mixture was stirred at room temperature for 1 hour. After completion of the reaction, water (100 ml) was added and the mixture was extracted with ethyl acetate. After the extract was washed with saturated aqueous sodium chloride solution and dried over anhydrous magnesium sulfate, the concentrated residue obtained was then purified using silica gel column chromatography and eluted with hexane/ethyl acetate (1/1) to obtain the title compound (6.18g, 68%) as a colorless powder.
¹H NMR (CDCl₃) δ 1.29 (3H, t, J=7.0Hz), 2.39-2.48 (2H, m), 2.80 (3H, s), 2.99-3.14 (2H, m), 3.27-3.40 (4H, m), 4.17 (2H, q, J=7.0Hz), 5.37-5.77 (1H, m).

### Reference Example 1-3

### [1-(Methylsulfonyl)-4-piperidinyl]ethyl acetate

The compound obtained in Reference Example 1-2 and 10% palladium-carbon (0.3 g) were added to ethanol (50 ml) and the mixture was stirred under hydrogen for 5 hours. After the reaction was completed, insoluble materials were filtered off with celite, the filtrate was concentrated and the residue obtained was purified using silica gel column chromatography, and the title compound (1.62g, 100%) as a colorless oily material was obtained from the fraction eluted with hexane/ethyl acetate (1/1).
¹H NMR (CDCl₃) δ 1.26 (3H, t, J=7.0Hz), 1.33-1.48 (2H, m), 1.78-2.02 (3H, m), 2.28 (2H, d, J=6.6Hz), 2.68 (2H, dt, J=2.4, 12.0Hz), 2.77 (3H, s), 3.74-3.85 (2H, m), 4.15 (2H, q, J=7.0Hz).

### Reference Example 1-4

### [1-(Methylsulfonyl)-4-piperidinyl] acetate

The compound (1.61g) obtained in Reference Example 1-3 was dissolved in methanol (30 ml), and 8N sodium hydroxide solution (30 ml) and water (3 ml) were added and the mixture was stirred for 8 hours. After the reaction was completed, 6N hydrochloric acid (8 ml) was added and the organic solvent was evaporated. The aqueous layer was extracted with ethyl acetate, dried over anhydrous magnesium sulfate, and concentrated to obtain the title compound (1.29 g, 90%) as a colorless powder.
¹H NMR (CDCl₃) δ 1.29-1.54 (2H, m), 1.80-2.03 (3H, m), 2.35 (2H, d, J=.6.6Hz), 2.69 (2H, dt, J=2.2, 12.0Hz), 2,78 (3H, s), 3.75-3.88 (2H, m).

### Reference Example 1-5

### [1-(Methylsulfonyl)-4-piperidinyl]acetyl chloride

The compound (1.29g) obtained in Reference Example 1-4 was dissolved in dichloromethane (30 ml), and N,N-dimethyl formamide (0.045 ml) and oxalyl chloride (0.76 ml) were added and the mixture was stirred for 5 hours and concentrated to obtain the title compound (1.4 g, 100%) as a brown powder.

### Reference Example 2-1

### 4-[(1-Acetyl-4-piperidinyl)methyl]benzenesulphonyl chloride

A solution of 1-acetyl-4-benzylpiperidine (60.00 g) in dichloromethane (100 ml) was added dropwise over 1 hour to chlorosulfonic acid (92 ml) at 0°C with stirring, and the mixture was then stirred for 30 minutes at 0°C and for 1.5 hours at room temperature. The reaction mixture was poured into ice water (1 L), and extracted with dichloromethane (500 ml, 250 ml). The organic layer was washed with 5% sodium carbonate solution (500 ml x 2) and saturated saline solution (250 ml). The organic layer was dried over anhydrous magnesium sulfate, filtered and concentrated under reduced pressure. The residue was subjected to column chromatography (silica gel 250 g, ethyl acetate), and the target fraction was concentrated under reduced pressure to obtain the title compound (54.22 g) as a white solid.
¹H NMR (CDCl₃) δ 1.05-1.35 (2H, m), 1.6-1.95 (3H, m), 2.09 (3H, s), 2.35-2.65 (1H, m), 2.68 (2H, d, J=6.6Hz), 2.85-3.15 (1H, m), 3.7-3.9. (1H, m), 4.5-4.75 (1H, m), 7.39 (2H, d, J=8.4Hz), 7.97 (2H, d, J=8.4Hz).

### Reference Example 2-2

### 1-Acetyl-4-[4-(methylsulfonyl)benzyl]piperidine

4-[(1-Acetyl-4-piperidinyl)methyl]benzenesulphonyl chloride (11.46 g) was gradually added to a solution (40 ml) of sodium sulfite (4.57 g) and sodium bicarbonate (6.10 g) in water (40 ml) at 75°C with stirring, and the mixture was stirred for 1 hour at 75°C. Chloroacetic acid (5.14 g) and 50% sodium hydroxide solution in water (4.4 ml) were added, and the mixture was heated under reflux and stirred for 20 hours. To this was added 1N hydrochloric acid (20 ml) at 0°C, and then the mixture was extracted with ethyl acetate (60 ml, 30 ml). The organic layer was washed with saturated saline solution (10 ml x 2), and dried over anhydrous magnesium sulfate. The mixture was filtered, and concentrated under reduced pressure. The residue was subjected to column chromatography (silica gel 150 g, ethyl acetate/methanol=1/0 → 9/1), and the target fraction was concentrated under reduced pressure to obtain the title compound (8.76 g) as a colorless oily material.
¹H NMR (CDCl₃) δ 1.05-1.35 (2H, m), 1.55-1.95 (3H, m), 2.08 (3H, s), 2.4-2.6 (1H, m), 2.66 (2H, d, J=7.4Hz), 2.9-3.1 (1H, m), 3.06 (3H, s), 3.7-3.9 (1H, m), 4.55-4.7 (1H, m), 7.34 (2H, d, J=8.4Hz), 7.87 (2H, d, J=8.4Hz).

### Reference Example 2-3

### 4-[4-(Methylsulfonyl)benzyl]piperidine hydrochloride

A mixture of 1-acetyl-4-[4-(methylsulfonyl)benzyl]piperidine (8.76 g) and concentrated hydrochloric acid (100 ml) was heated under reflux, and stirred for 4 hours. The reaction mixture was concentrated under reduced pressure, 2-propanol (100 ml) was added, and the mixture was concentrated under reduced pressure. 2-Propanol (50 ml) was added to the residue, and the mixture was heated under reflux, and stirred for 30 minutes. This was cooled to room temperature, the precipitate was filtered off, the precipitate was washed with 2-propanol, and dried under reduced pressure to obtain the title compound (7.51 g) as a white solid.
¹H NMR (CD₃OD) δ 1.3-1.6 (2H, m), 1.75-2.1 (3H, m), 2.75 (2H, d, J=7.0Hz), 2.8-3.05 (2H, m), 3.10 (3H, s), 3.25-3.45 (2H, m), 7.49 (2H, d, J=8.1Hz), 7.89 (2H, d, J=8.1Hz).

### Reference Example 2-4

### 4-[4-(Methylsulfonyl)benzyl]piperidine

4-[4-(Methylsulfonyl)benzyl]piperidine hydrochloride (1 g) was dissolved in water (10 ml), 1N sodium hydroxide solution (5 ml) was added at 0°C, the mixture was stirred for 5 minutes, and then the aqueous layer was extracted with dichloromethane (10 ml x 3). The organic layer was dried over potassium carbonate, and then filtered and concentrated under reduced pressure. Diisoproylether (10 ml) was added to the residue, and the precipitate was filtered off. The precipitate was washed with diisopropylether, and then was dried at reduced pressure to obtain the title compound (712 mg) as a white solid.
¹H NMR (CDCl₃) δ 1.07-1.27 (2H, m), 1.50-1.73 (3H, m), 2.48-2.61 (2H, m), 2.62 (2H, d, J=6.6Hz), 3.03-3.08 (2H, m), 3.05 (3H, s), 7.34 (2H, d, J=8.4Hz), 7.85 (2H, d, J=8.4Hz).

### Reference Example 2-5

### N-(3-{4-[4-(4-Methylsulfonyl)benzyl]-1-piperidinyl}propyl)aniline dihydrochloride

A solution of 90% acrolein (3.7 ml) in tetrahydrofuran (10 ml) was added dropwise to a solution of 4-[4-(methylsulfonyl)benzyl]piperidine (12.6 g) and DBU (0.074 ml) in tetrahydrofuran (90 ml) with stirring at -28°C, and the mixture was stirred for 1 hour from -20°C to -10°C. 3,4-Dichloroaniline (8.07 g) and triacetoxy sodium borohydride (21.1 g) were sequentially added at -10°C, and then this mixture was raised to room temperature while stirring for 7 hours. Diethyl ether (150 ml) and 1N sodium hydroxide solution (240 ml) were added, and the mixture was stirred for 30 minutes. The separated aqueous layer was extracted twice with a mixture of diethyl ether and tetrahydrofuran (100 ml + 50 ml). The combined organic layer was dried over anhydrous magnesium sulfate, and then concentrated under reduced pressure. The residue was purified with silica gel column chromatography and a light brown oily material (15 g, 66%) was obtained from the fraction eluted with methanol/ethyl acetate (1/4). This compound was dissolved in 2-propanol (100 ml), a 4N hydrogen chloride ethyl acetate solution (100 ml) was added with stirring, and the precipitate was filtered off. The precipitate was washed with ethyl acetate, and dried at reduced pressure to obtain the title compound (16.3 g, 94%) as a light brown powder.
¹H NMR(CD₃OD) δ 1.59 - 2.35 (7H, m), 2.75 (2H, d, J = 6.4 Hz), 2.86 - 3.05 (2H, m), 3.13 (3H, s), 3.22 (2H, t, J = 7.4 Hz), 3.48 (2H, t, J = 8.0 Hz), 3.59 - 3.68 (2H, m), 6. 63 - 6.75 (3H, m), 7.10 - 7.25 (2H, m), 7.50 (2H, d, J = 8.2 Hz), 7.90 (2H, d, J = 8.2 Hz).

### Reference Example 3-1

### 1-Acetyl-4-[4-(isopropylsulfanyl)benzyl]piperidine

The compound obtained in Reference Example 2-1 (16.9 g) and powdered zinc (36.7 g) were added to a mixture of concentrated sulfuric acid (36 ml) and water (200 ml) under ice cooling, and the reaction mixture was stirred for 5 hours at 60°C. After cooling to room temperature, the filtrate was extracted with dichloromethane (200 ml x 2), the organic layer was dried over anhydrous magnesium sulfate and then concentrated under reduced pressure, and 1-acetyl-4-(4-mercaptobenzyl)piperidine was obtained as a colorless oily material.

The aforementioned compound was dissolved in N,N-dimethylformamide (300 ml), and 2-propyl iodide (7.3 ml) and potassium carbonate (8.86 g) were added and the mixture was stirred for 20 hours. After the solvent was evaporated, ethyl acetate (200 ml) and water (200 ml) were added to the residue obtained and the mixture was stirred for 10 minutes. The separated organic layer was washed with saturated saline solution, and then the concentrated. The residue obtained was purified with silica gel column chromatography, and the title compound (10.2 g, 66%) as a colorless oily material was obtained from the fraction eluted with ethyl acetate.
¹H NMR (CDCl₃) δ1.02-1.30 (2H, m), 1.29 (6H, d, J=6.6Hz), 1.60-1.82 (3H, m), 2.07 (3H, s), 2.40-2.58 (3H, m), 2.90-3.05 (1H, m), 3.25-3.42 (1H, m), 3.70-3.85 (1H, m), 4.55-4.65 (1H, m), 7.06 (2H, d, J=8.0Hz), 7.33 (2H, d, J=8.0Hz).

### Reference Example 3-2

### 1-Acetyl-4-[4-(isopropylsulfonyl)benzyl]piperidine

M-Chloroperbenzoic acid was added under ice cooling to a solution of the compound (1.06 g) obtained in Reference Example 3-1 in dichloromethane (30 ml), and the mixture was stirred at room temperature for 3 hours. The reaction mixture was diluted with dichloromethane (30 ml), the organic layer was washed twice with 5% sodium thiosulfate solution and a saturated sodium bicarbonate solution, and then washed with a saturated sodium bicarbonate solution and a saturated saline solution. After drying over anhydrous magnesium sulfate, the residue was concentrated under reduced pressure and subjected to silica gel column chromatography (ethyl acetate/methanol=10/1). The target fraction was concentrated under reduced pressure to obtain the title compound (1.12 g, 95%) as a colorless oily material.
¹H NMR (CDCl₃) δ1.03-1.40 (2H, m), 1.29 (6H, d, J=6.8Hz), 1.53-2.00 (3H, m), 2.07 (3H, s), 2.40-2.70 (3H, m), 2.82-3.05 (1H, m), 3.10-3.25 (1H, m), 3.70-3.85 (1H, m), 4.55-4.70 (1H, m), 7.32 (2H, d, J=8.2Hz), 7.80 (2H, d, J=8.2Hz).

### Reference Example 3-3

### 4-[9-(Isopropylsulfonyl)benzyl]piperidine

10N Hydrochloric acid (100 ml) was added to the compound (11.4 g) obtained in Reference Example 3-2, and heated under reflux for 6 hours. After the reaction was. completed, an 8N sodium hydroxide solution (200 ml) was added dropwise under ice cooling, and the mixture was extracted with dicyclomethane (200 ml). The reaction mixture was dried over anhydrous magnesium sulfate and concentration under reduced pressure to obtain the title compound (9.56 g, 96%) as a colorless powder.
¹H NMR (CDCl₃) δ1.07-1.25 (2H, m), 1.30 (6H, d, J=7.0Hz), 1.55-1.78 (3H, m), 2.55 (2H, ddd, J=2.6, 12.0, 12.0Hz), 2.62 (2H, d, J=6.8Hz), 3.00-3.30 (3H, m), 7.33 (2H, d, J=8.4Hz), 7.78 (2H,'d, J=8.4Hz).

### Reference Example 3-4

### N-(3-{4-[4-(9-Isopropylsulfonyl)benzyl]-1-piperidinyl}propyl)aniline dihydrochloride

The compound obtained in Reference Example 3-3 was employed, and the title compound was synthesized with the same method as that of Reference Example 2-5. Yield 36%
¹H NMR(CDCl₃; free) δ 1.18-1.95 (9H, m), 1.30 (6H, d, J=7.0Hz), 2.44 (2H, t, J=6.6Hz), 2.66 (2H, d, J=6.2Hz), 2.85-3.00 (2H, m), 3.05-3.27 (3H, m), 6.39 (1H, dd, J=2.6, 8.4Hz), 6.62 (1H, d, J=2.6Hz), 7.16 (1H, d, J=8.4Hz), 7.34 (2H, d, J = 8.4Hz), 7.80 (2H, d, J = 8.4Hz).

### Reference Example 4-1

### 4-Hydroxy-1-(methylsulfonyl)-4-piperidinecarbonitrile

A solution of 1-(methylsulfonyl)-4-piperidinone (synthesized by the method disclosed in U.S. Patent No. 6,051,582)(1 g, 5.64 mmol) and potassium cyanide (551 mg, 8.5 mmol) in acetic acid (6 ml) was stirred at room temperature for 18 hours. The reaction mixture was concentrated under reduced pressure, water (20 ml) was added to the residue obtained, and the mixture was extracted with ethyl acetate (20 ml x 2). The organic layer was washed with aqueous 10% sodium bicarbonate (20 ml x 2) and saturated saline solution (20 ml), and after drying over anhydrous sodium sulfate, the solvent was evaporated under reduced pressure. The residue obtained was purified by flash column chromatography (silica gel 25 g, ethyl acetate/hexane=1/5 → 1/1) to obtain the title compound (528.1 mg, 46%) as colorless powder crystals.
¹H NMR (CDCl₃) δ1.97-2.10 (2H, m), 2.18-2.28. (2H, m), 2.74 (1H, s), 2.83 (3H, s), 3.29-3.38 (2H, m), 3.49-3.63 (2H, m)

### Reference Example 4-2

### 4-Hydroxy-1-(methylsulfonyl)-4-piperidinecarboxylic acid

Concentrated hydrochloric acid (2 ml) was added to the compound (528 mg, 2.59 mmol) obtained in Reference Example 4-1, and the mixture was stirred at room temperature for 12 hours. The reaction mixture was concentrated under reduced pressure, the residue obtained was subjected to azeotropy with toluene, and the resulting solid was dried under reduced pressure to obtain the title compound (580 g, 100%) as colorless powder crystals.
¹H NMR (CD₃OD) δ1.64-1.95 (2H, m), 2.02-2.17 (2H, m), 2.85 (3H, s), 3.05-3.19 (2H, m), 3.53-3.65 (2H, m)

### Reference Example 5

### N-(3,4-Dichlorophenyl)-N-(3-{4-[4-(methylsulfonyl)benzyl]-1-piperidinyl}propyl)-2-[1-(methylsulfonyl)-4-piperidinyl]acetamide

The compound (0.6 g) obtained in Reference Example 1-5 was divided into 3 portions and added at 5 minute intervals to a mixture of the compound obtained in Reference Example 2-5 (0.88 g) and triethylamine (0.93 ml) in acetomitrile (15 ml) under ice cooling, and the reaction mixture was stirred for 16 hours at 50°C. After the reaction was completed, the reaction mixture was diluted with ethyl acetate at room temperature, and the diluent was washed with water, saturated sodium bicarbonate solution, and saturated saline solution. After drying over anhydrous magnesium sulfate, the concentrated residue obtained was subjected to silica gel column chromatography (Chromatorex NH), the oily material obtained from the fraction eluted with ethyl acetate was further purified with silica gel column chromatography, and a colorless amorphous material (0.699 g, 64%) was obtained from the fraction eluted with ethyl acetate/methanol/triethylamine (90/30/12).
¹H NMR (CDCl₃) δ1.10-1.40 (2H, m), 1.52-2.02 (14H, m), 2.82 (2H, t, J=7.2Hz), 2.55-2.71 (4H, m), 2.75 (3H, s), 2.75-2.88 (2H, m), 3.05 (3H, s), 3.62-3.81 (4H, m), 6.99 (1H, dd, J=2,2, 8.4Hz), 7.26 (1H, d, J=2.2Hz), 7.32 (2H, d, J=8.4Hz), 7.51 (1H, d, J=8.4Hz), 7.84 (2H, d, J=8.4Hz).

### Reference Example 6

### N-(3,4-Dichlorophenyl)-N-(3-{4-[4-(isopropylsulfonyl)benzyl]-1-piperidinyl}propyl)-2-[1-(methylsulfonyl)-4-piperidinyl] acetamide

The compound (0.29 g) obtained in Reference Example 1-5 was divided into 2 portions and added to a solution of the compound obtained in Reference Example 3-4 (0.292 g) and triethylamine (0.085 ml) in dichloromethane (5 ml) under ice cooling, and the mixture was stirred for 1.5 hours at room temperature. The compound (0.145 g) obtained in Reference Example 1-5 and triethylamine (0.085 ml) were added again, and the mixture was stirred for 1 hour at room temperature. Saturated sodium bicarbonate solution (10 ml) was added to the reaction mixture, and the organic solvent was evaporated. After the aqueous layer was extracted with ethyl acetate (50 ml), and the extract was washed with saturated sodium bicarbonate (30 ml x 2) and saturated saline solution (30 ml), dried over anhydrous magnesium sulfate. The concentrated residue obtained was subjected to silica gel column chromatography (Chromatorex NH), the oily material obtained from the fraction eluted with ethyl acetate was further purified with silica gel column chromatography, and a colorless amorphous material (0.231 g, 56%) was obtained from the fraction eluted with ethyl acetate.
¹H NMR (CDCl₃) δ1.05-1.40 (2H, m), 1.30 (6H, d, J=6.8Hz), 1.45-2.10 (14H, m), 2.29 (2H, t, J=7.3Hz), 2.55-2.73 (4H, m), 2.75 (3H, s), 2.75-2.89 (2H, m), 3.10-3.28 (1H, m), 3.62-3.85 (4H, m), 7.00 (1H, dd, J=2.6, 8.4Hz), 7.27 (1H, d, J=2.6Hz), 7.32 (2H, d, J=8.4Hz), 7.52 (1H, d, J=8.4Hz), 7.78 (2H, d, J=8.4Hz).

### Reference Example 7

### 3-(1-Acetyl-4-piperidinyl-N-(3,4-dichlorophenyl)-N-(3-{4-[4-(methylsulfonyl)benzyl]-1-piperidinyl}propyl)-propanamide

3-(1-Acetyl-4-piperidinyl)proprionic acid (0.597 g), N,N-dimethylformamide (0.044 ml) and oxalyl chloride (0.39 ml) were mixed and stirred for 1.5 hours. The reaction mixture was concentrated to obtain a yellow oily material. A solution of the aforementioned oily material in dichloromethane (15 ml) was added to a solution of the compound (0.792 g) obtained in Reference Example 2-5 and triethylamine (0.84 ml) in dichloromethane (5 ml) under ice cooling, and the mixture was stirred for 15 hours at room temperature. After the solvent was evaporated, ethyl acetate and water were added to the residue obtained, and the separated organic layer was washed with saturated saline solution, and the organic layer was dried over anhydrous magnesium sulfate. The concentrated residue obtained was subjected to silica gel column chromatography (Chromatorex NH), and a colorless oily material (54 mg, 6%) was obtained from the fraction eluted with ethyl acetate.
¹H NMR (CDCl₃) δ0.88-1.91 (15H, m), 1.95-2.12 (2H, m), 2.06 (3H, s), 2.20-2.55 (4H, m), 2.62 (2H, d, J=6.6Hz), 2.77-3.05 (3H, m), 3.05 (3H, s), 3.62-3.83 (3H, m), 4.48-4.61 (1H, m), 7.02 (1H, dd, J=2.4, 8.6Hz), 7.30 (1H, d, J=2.4Hz), 7.33 (2H, d, J=8.3Hz), 7.51 (1H, d, J=8.6Hz), 7.85 (2H, d, J=8.6Hz).

### Reference Example 8

### N-(3,4-Dichlorophenyl)-4-hydroxy-1-(methylsulfonyl)-N-(3-{4-(4-(methylsulfonyl)benzyl]-1-piperidinyl)propyl)-4-piperidinecarboxyamide

Oxalyl chloride (0.325 ml, 3.74 mmol) was added dropwise to a solution of the compound obtained in Reference Example 4-2 (555 mg, 2.49 mmol) and N,N-dimethylformamide (0.0193 ml, 0.249 mmol) in methylene chloride (10 ml), and the mixture was stirred for 1 hour at room temperature, and then concentrated under reduced pressure. A solution of the residue obtained in methylene chloride (5 ml) was added dropwise at 0°C to a solution of the compound obtained in Reference Example 2-5 (439 mg, 0.83 mmol) and triethylamine (1.39 ml, 9.96 mmol) in methylene chloride (10 ml), and the mixture was stirred for two hours at the same temperature and was stirred for 2 hours at room temperature. After the reaction mixture was washed with water (20 ml) and saturated saline solution (20 ml), and dried over anhydrous sodium sulfate, and the solvent was evaporated under reduced pressure. The residue obtained was purified by column chromatography [Chromatorex NH-DM-1020 (Fuji Sylysia Chemical); 30 g, ethyl acetate/hexane=1/1→3/1] to obtain the title compound (34.4 mg, 6.3 %) as colorless powder crystals.
¹H NMR (CDCλ₃) δ1.22-2.20 (13H, m), 2.33-2.40 (2H, m), 2.64 (2H, d, J = 6.0Hz), 2.78 (3H, s), 2.87-2.93 (2H, m), 3.06 (3H, s), 3.10-3.20 (2H, m), 3.56-3.61 (2H, m), 4.00-4.17 (2H, m), 7.06 (1H, dd, J = 8.0Hz, 2.6Hz), 7.32 (1H, d, J = 2.6Hz), 7.35 (2H, d, J = 8.0Hz), 7.49 (1H, d, J = 8.0Hz), 7.86 (2H, d, J = 8.0Hz).

### Experimental Example (Effects of a compound with respect to a mouse skin graft rejection model)

Abdominal skin (10 mm square) of a donor mouse B6-CH-2^{bm12} was grafted onto a recipient mouse (B6/129), and 10 mg/kg of an experimental compound (N,N-dimethyl-N-(4-[[[2-(4-methylphenyl)-6,7-dihydro-5H-benzocyclohepten-8-yl]carbonyl]amino]benzyl]tetrahydro-2H-pyran-4-ammonium chloride) was administered subdermally thereto once a day from the day of grafting. When the presence or absence of graft rejection was observed visually over time, the number of days of skin graft life for the control group and the group that was administered the compound were respectively 13.0±2.8 days (n=6) and 19.6±4.4 days (n=7), and thus a significant (P<0.05 vs. control, log-rank test) graft rejection suppression effect was observed with the administration of the compound.

| Example 1 (capsule) | |
|---|---|
| (1) 1-Acetyl-N-(3-{4-[4-(aminocarbonyl)benzyl]-1-piperidinyl}propyl)-N-(3-chloro-4-methylphenyl)-4-piperidine carboxamide | 40 mg |
| (2) Lactose | 70 mg |
| (3) Fine crystalline cellulose | 9 mg |
| (4) Magnesium stearate | 2 mg |
| One capsule | 120 mg |
| (1), (2) and (3) and 1/2 of (4) are mixed together, and then granulated. To the granules is added the reminder of (4), and the whole is filled into a gelatin capsule. | |

| Example 2 (tablet) | |
|---|---|
| (1) N-(3,4-Dichlorophenyl)-N-(3-{4-[4-(methylsulfonyl)benzyl]-1-piperidinyl}propyl)-2-[1-(methylsulfonyl)-4-piperidinyl]acetamide | 40 mg |
| (2) Lactose | 58 mg |
| (3) Corn starch | 18 mg |
| (4) Fine crystalline cellulose | 3.5 mg |
| (5) Magnesium stearate | 0.5 mg |
| One tablet | 120 mg |
| (1), (2), (3), 2/3 of (4) and 1/2 of (5) are mixed together, and then granulated. To the granules are added the reminders of (4) and (5), followed by subjecting the mixture to compression molding to prepare a tablet. | |

| Example 3 (capsules) | |
|---|---|
| (1) N-[3-(4-benzyl-1-piperidinyl)propyl]-N-phenyl-N'-phenylurea hydrochloride | 40 mg |
| (2) Lactose | 70 mg |
| (3) Fine crystalline cellulose | 9 mg |
| (4) Magnesium stearate | 1 mg |
| One capsule | 120 mg |
| (1), (2) and (3) and 1/2 of (4) are mixed together, and then granulated. To the granules is added the reminder of (4), and the whole is filled into a gelatin capsule. | |

| Example 4 (tablet) | |
|---|---|
| (1) N-[3-(4-benzyl-1-piperidinyl)propyl]-N'-(4-chlorophenyl)-N-(4-methylphenyl)urea | |
| hydrochloride | 40 mg |
| (2) Lactose | 58 mg |
| (3) Corn starch | 18 mg |
| (4) Fine crystalline cellulose | 3.5 mg |
| (5) Magnesium stearate | 0.5 mg |
| One tablet | 120 mg |
| (1), (2), (3), 2/3 of (4) and 1/2 of (5) are mixed together, and then granulated. To the granules are added the reminders of (4) and (5), followed by subjecting the mixture to compression molding to prepare a tablet. | |

### Industrial applicability

Compounds represented by the formulas (I), (II), (III), (IV) and (eI) employed in the present invention or salts thereof have a CCR antagonist effect, and particularly a CCR 5 antagonist effect, a CXCR4 antagonist effect, a CXCR3 antagonist effect, a CCR2 antagonist effect, and a CCR3 antagonist effect, and thus can be effectively used as an agent for the prevention and treatment of graft-versus-host disease and/or rejection reactions, and as an agent for the prevention and treatment of chronic rheumatoid arthritis, autoimmune diseases, allergic disorders, ischemic brain cell injury, myocardial infarction, chronic nephritis, and arteriosclerosis.

## Claims

1. An agent for the prevention or treatment of graft-versus host disease and/or rejection reactions during organ or bone marrow transplantation which comprises a compound having a CCR antagonistic effect represented by the formula: 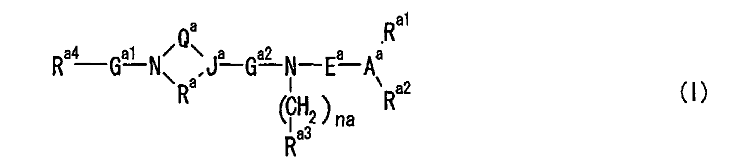 wherein R^{a1} is a hydrogen atom, a hydrocarbon group which may be substituted, a non-aromatic heterocyclic group which may be substituted, R^{a2} is a hydrocarbon group which may be substituted, a non-aromatic heterocyclic group which may be substituted, or R^{a1} and R^{a2} may combine with each other together with A^{a} to form a heterocyclic group which may be substituted, A^{a} is N or N⁺-R^{a5} · Y^{a-} (R^{a5} is a hydrocarbon group, Y^{a-} is a counter anion), R^{a3} is a cyclic hydrocarbon group which may be substituted or a heterocyclic group which may be substituted, na is 0 or 1, R^{a4} is a hydrogen atom, a hydrocarbon group which may be substituted, a heterocyclic group which may be substituted, an alkoxy group which may be substituted, an aryloxy group which may be substituted, or an amino group which may be substituted, E^{a} is a divalent aliphatic hydrocarbon group which may be substituted by a group other than an oxo group, G^{a1} is a bond, CO or SO₂, G^{a2} is CO, SO₂, NHCO, CONH or OCO, J^{a} is methine or a nitrogen atom, and each of Q^{a} and R^{a} is a bond or a divalent C₁₋₃ aliphatic hydrocarbon which may be substituted, with the proviso that J^{a} is methine when G^{a2} is OCO, one of Q^{a} and R^{a} is not a bond when the other is a bond, and each of Q^{a} and R^{a} is not substituted by an oxo group when G^{a1} is a bond,
the formula: 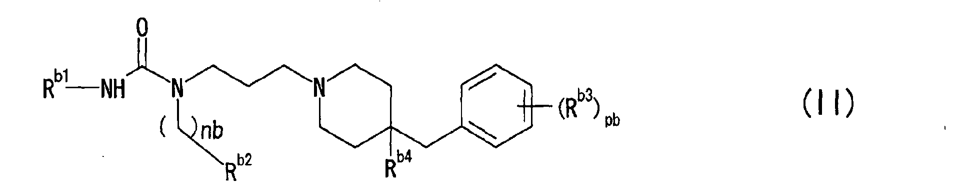 wherein R^{b1} is a hydrocarbon group which may be substituted; R^{b2} is a cyclic hydrocarbon group which may be substituted or a heterocyclic group which may be substituted; R^{b3} is a halogen atom, a carbamoyl group which may be substituted, a sulfamoyl group which may be substituted, an acyl group derived from a sulfonic acid, a C₁₋₄ alkyl group which may be substituted, a C₁₋₄ alkoxy group which may be substituted, an amino group which may be substituted, a nitro group or a cyano group; R^{b4} is a hydrogen atom or a hydroxy group; nb is an integer of 0 or 1; pb is an integer of 0 or 1 to 4,
the formula: 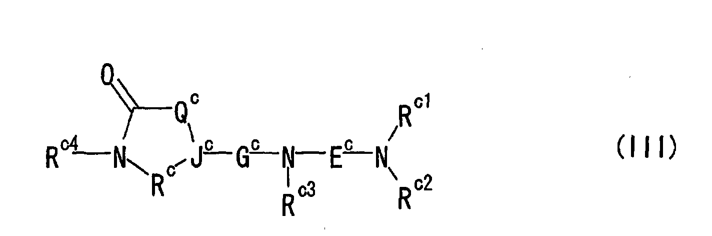 wherein R^{c1} is a hydrocarbon group, R^{c2} is a hydrocarbon group having 2 or more carbon atoms, or R^{c1} and R^{c2} may be bound together with the adjacent nitrogen atom to form a ring which may have a substituent or substituents, R^{c3} is a hydrocarbon group which may have a substituent or substituents or a heterocyclic group which may have a substituent or substituents, R^{c4} is a hydrogen atom, a hydrocarbon group which may have a substituent or substituents or a heterocyclic group which may have a substituent or substituents, E^{c} is a divalent aliphatic hydrocarbon group which may have a substituent or substituents other than an oxo group, G^{c} is CO or SO₂, J^{c} is a nitrogen atom or a methine group which may have a substituent or substituents, and Q^{c} and R^{c} are each a bond or a divalent aliphatic C₁₋₃ hydrocarbon group which may have a substituent or substituents,
the formula: 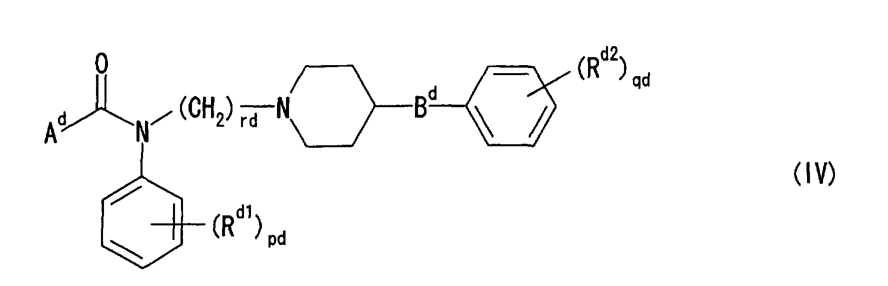 wherein A^{d} is a group represented by the formula: 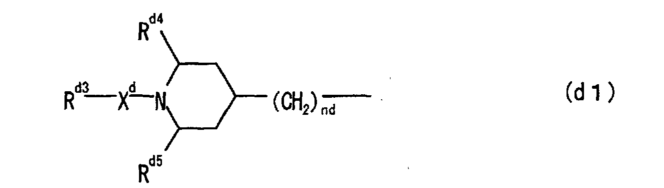 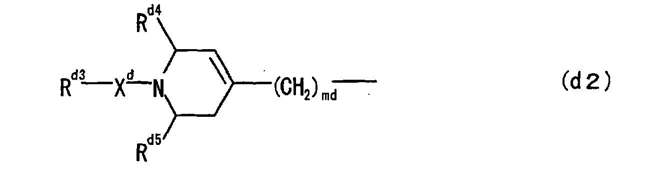 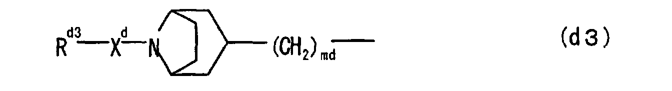 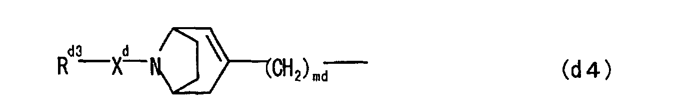 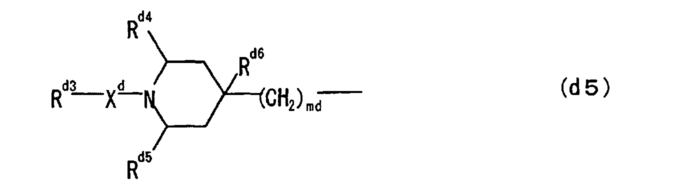 or 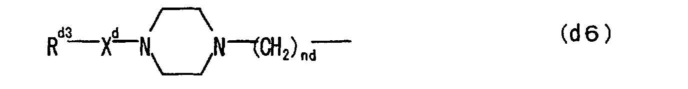 wherein, R^{d3} is (1) a hydrocarbon group which may be substituted, (2) a C₁₋₄ alkoxy group which may be substituted or (3) an amino group which may be substituted; X^{d} is a bond, -SO₂- or -CO-; nd is an integer of 1 to 3; md is 0 or an integer of 1 to 3; R^{d4} and R^{d5} are the same or different and each of which is a hydrogen atom or a C₁₋₆ alkyl group; R^{d6} is a hydroxyl group, a C₁₋₆ alkyl group or a C₂₋₆ alkenyl group; rd is an integer of 2 to 4; B^{d} is a bond, -CH₂-, -SO₂-, -SO-, -S-, -O-, -CO-, -NR^{da}-SO₂- or -NR^{da}-CO- (wherein, R^{da} is a hydrogen atom, a C₁₋₆ alkyl group, a C₂₋₆ alkenyl group or a C₃₋₈ cycloalkyl group); each of pd and qd is 0 or an integer of 1 to 4; R^{d1} is a halogen atom, a C₁₋₆ alkyl group, a C₂₋₄ alkenyl group, a C₁₋₄ alkanoyl group, a C₁₋₄ alkoxy group, a cyano group, a trifloromethyl group, a nitro group, a hydroxyl group, an amino group or an amidino group; R^{d2} is 1) a halogen, 2) a C₁₋₆ alkyl which may be substituted by a halogen or a C₁₋₄ alkoxy, 3) a C₁₋₄ alkoxy which may be substituted by a halogen or a C₁₋₄ alkoxy, 4) nitro, 5) cyano, 6) hydroxyl, 7) a C₁₋₄ alkanoylamino, 8) SO₂NR^{db}R^{dc}, 9) SO₂R^{dd}, 10) CONR^{db}R^{dc}, 11) NR ^{db}R^{dc} or 12) NR^{da}-SO₂R^{dd} (wherein, R^{da} has the meaning given above, and R^{db} and R^{dc} may be the same or different, and are (1) a hydrogen atom, (2) a C₁₋₆ alkyl group which may be substituted by a halogen or a C₁₋₄ alkoxy, or (3) a C₃₋₈ cycloalkyl group which may be substituted by a halogen or a C₁₋₄ alkoxy, or R^{db} and R^{dc} may bond with a nitrogen atom to form a cyclic amino group and R^{dd} is a C₁₋₆ alkyl group or a C₃₋₈ cycloalkyl group), each R^{d1} may be the same or different from each other when pd is two or more, and each R^{d2}, may be the same or different from each other when qd is two or more, or
the formula: 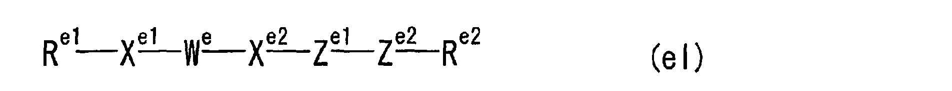 wherein R^{e1} represents a 5 to 6-membered cyclic ring group which may be substituted, X ^{e1} represents a bond or a bivalent group, in which the number of atoms constituting the straight-chain portion is 1 to 4, W^{e} represents a bivalent group represented by formula: 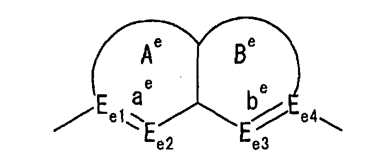 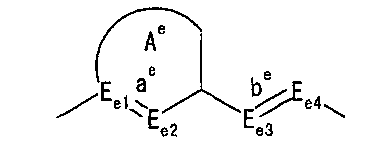 or 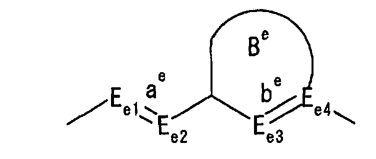 wherein each of ring A^{e} and ring B^{e} represents a 5- to 7-membered cyclic group which may be substituted, each of Ee₁ and Ee₄ is a carbon atom which may be substituted or a nitrogen atom which may be substituted, each of Ee₂ and Ee₃ is a carbon atom which may be substituted, a nitrogen atom which may be substituted, or a sulfur atom which may be oxidized or an oxygen atom, each of a^{e} and b^{e} is a single bond or a double bond), X^{e2} is a bivalent group in which the number of atoms constituting the straight-chain portion is 1 to 4, Z^{e1} is a bond or a bivalent cyclic ring group, Z^{e2} is a bond or a bivalent cyclic ring group in which the number of atoms constituting the straight-chain portion is 1 to 4, and R^{e2} is (1) an amino group which may be substituted, and the nitrogen atom may be converted into a quaternary ammonium or an N-oxide, (2) a nitrogen-containing heterocyclic ring group which may be substituted, may contain sulfur atom or an oxygen atom as a ring-constituting atom, and the nitrogen atom may be converted into a quaternary ammonium or a N-oxide, (3) a group which is bonded via the sulfur atom, (4) a group represented by formula: 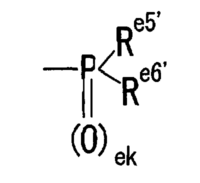 wherein ek is 0 or 1, the phosphorus atom may form a phosphonium salt when ek is 0, and each of R^{e5'} and R^{e6'} is a hydrocarbon atom which may be substituted, a hydroxyl group which may be substituted, or an amino group which may be substituted, and R^{e5'} and R^{e6'} may bond to each other to form a cyclic ring group together with the adjacent phosphorus atom, (5) an amidino group which may be substituted or (6) a guanidino group which may be substituted, or a salt thereof.

2. An agent for the prevention or treatment of chronic rheumatoid arthritis, autoimmune diseases, allergic disorders, ischemic brain cell damage, myocardial infarction, chronic nephritis, and arteriosclerosis which comprises a compound having a CCR antagonistic effect represented by the formula: 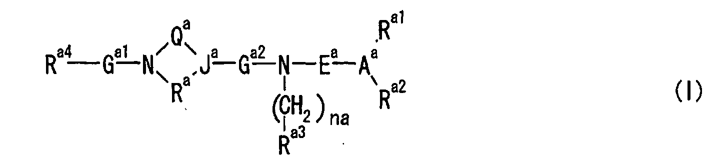 wherein R^{a1} is a hydrogen atom, a hydrocarbon group which may be substituted, a non-aromatic heterocyclic group which may be substituted, R^{a2} is a hydrocarbon group which may be substituted, a non-aromatic heterocyclic group which may be substituted, or R^{a1} and R^{a2} may combine with each other together with A^{a} to form a heterocyclic group which may be substituted, A^{a} is N or N⁺-R^{a5} · Y^{a-} (R^{a5} is a hydrocarbon group, Y^{a-} is a counter anion), R^{a3} is a cyclic hydrocarbon group which may be substituted or a heterocyclic group which may be substituted, na is 0 or 1, R^{a4} is a hydrogen atom, a hydrocarbon group which may be substituted, a heterocyclic group which may be substituted, an alkoxy group which may be substituted, an aryloxy group which may be substituted, or an amino group which may be substituted, E^{a} is a divalent aliphatic hydrocarbon group which may be substituted by a group other than an oxo group, G^{a1} is a bond, CO or SO₂, G^{a2} is CO, SO₂, NHCO, CONH or OCO, J^{a} is methine or a nitrogen atom, and each of Q^{a} and R^{a} is a bond or a divalent C₁₋₃ aliphatic hydrocarbon which may be substituted, with the proviso that J^{a} is methine when G^{a2} is OCO, one of Q^{a} and R^{a} is not a bond when the other is a bond, and each of Q^{a} and R^{a} is not substituted by an oxo group when G^{a1} is a bond,
the formula: 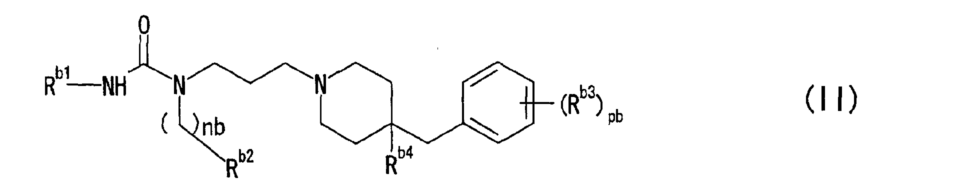 wherein R^{b1} is a hydrocarbon group which may be substituted; R^{b2} is a cyclic hydrocarbon group which may be substituted or a heterocyclic group which may be substituted; R^{b3} is a halogen atom, a carbamoyl group which may be substituted, a sulfamoyl group which may be substituted, an acyl group derived from a sulfonic acid, a C₁₋₄ alkyl group which may be substituted, a C₁₋₄ alkoxy group which may be substituted, an amino group which may be substituted, a nitro group or a cyano group; R^{b4} is a hydrogen atom or a hydroxy group; nb is an integer of 0 or 1; pb is an integer of 0 or 1 to 4,
the formula: 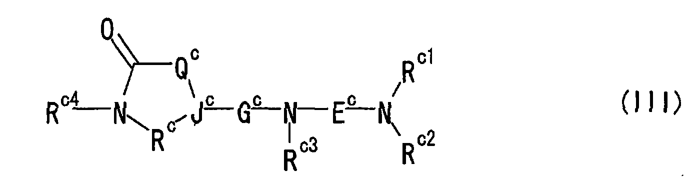 wherein R^{c1} is a hydrocarbon group, R^{c2} is a hydrocarbon group having 2 or more carbon atoms, or R^{c1} and R^{c2} may be bound together with the adjacent nitrogen atom to form a ring which may have a substituent or substituents, R^{c3} is a hydrocarbon group which may have a substituent or substituents or a heterocyclic group which may have a substituent or substituents, R^{c4} is a hydrogen atom, a hydrocarbon group which may have a substituent or substituents or a heterocyclic group which may have a substituent or substituents, E^{c} is a divalent aliphatic hydrocarbon group which may have a substituent or substituents other than an oxo group, G^{c} is CO or SO₂, J^{c} is a nitrogen atom or a methine group which may have a substituent or substituents, and Q^{c} and R^{c} are each a bond or a divalent aliphatic C₁₋₃ hydrocarbon group which may have a substituent or substituents,
the formula: 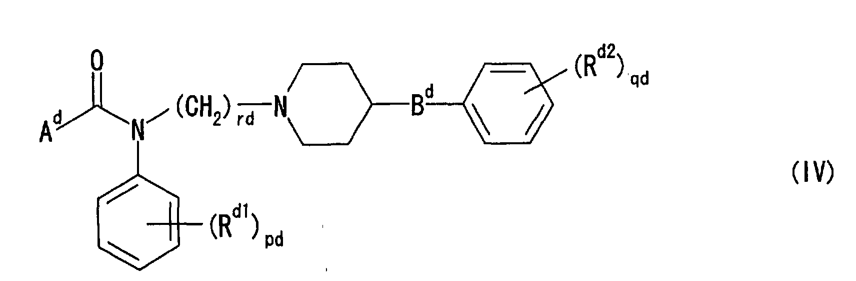 wherein A^{d} is a group represented by the formula: 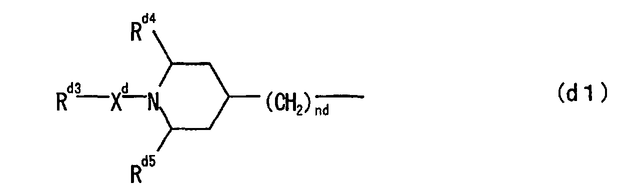 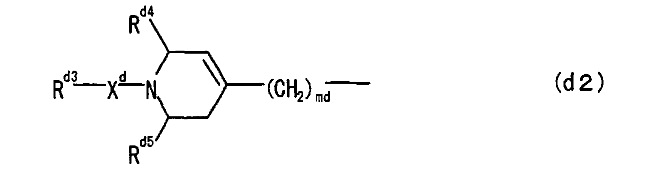 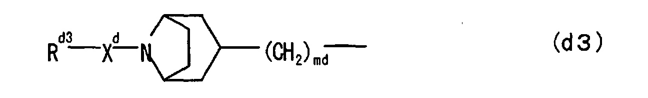 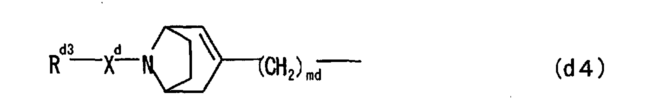 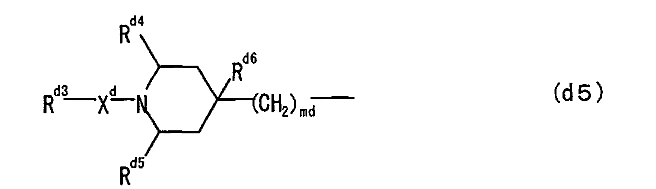 or 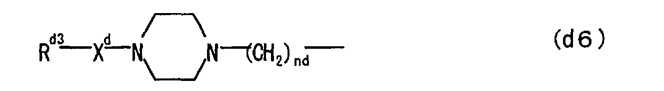 wherein, R^{d3} is (1) a hydrocarbon group which may be substituted, (2) a C₁₋₄ alkoxy group which may be substituted or (3) an amino group which may be substituted; X^{d} is a bond, -SO₂- or -CO-; nd is an integer of 1 to 3; md is 0 or an integer of 1 to 3; R^{d4} and R^{d5} are the same or different and each of which is a hydrogen atom or a C₁₋₆ alkyl group; R^{d6} is a hydroxyl group, a C₁₋₆ alkyl group or a C₂₋₆ alkenyl group; rd is an integer of 2 to 4; B^{d} is a bond, -CH₂-, -SO₂-, -SO-, -S-, -O-, -CO-, -NR^{da}-SO₂- or -NR^{da}-CO- (wherein, R^{da} is a hydrogen atom, a C₁₋₆ alkyl group, a C₂₋₆ alkenyl group or a C₃₋₈ cycloalkyl group); each of pd and qd is 0 or an integer of 1 to 4; R^{d1} is a halogen atom, a C₁₋₆ alkyl group, a C₂₋₄ alkenyl group, a C₁₋₄ alkanoyl group, a C₁₋₄ alkoxy group, a cyano group, a trifloromethyl group, a nitro group, a hydroxyl group, an amino group or an amidino group; R^{d2} is 1) a halogen, 2) a C₁₋₆ alkyl which may be substituted by a halogen or a C₁₋₄ alkoxy, 3) a C₁₋₄ alkoxy which may be substituted by a halogen or a C₁₋₄ alkoxy, 4) nitro, 5) cyano, 6) hydroxyl, 7) a C₁₋₄ alkanoylamino, 8) SO₂NR^{db}R^{dc}, 9) SO₂^{dd}, 10) CONR^{db}R^{dc}, 11) NR ^{db}R^{dc} or 12) NR^{da}-SO₂R^{dd} (wherein, R^{da} has the meaning given above, and R^{db} and R^{dc} may be the same or different, and are (1) a hydrogen atom, (2) a C₁₋₆ alkyl group which may be substituted by a halogen or a C₁₋₄ alkoxy, or (3) a C₃₋₈ cycloalkyl group which may be substituted by a halogen or a C₁₋₄ alkoxy, or R^{db} and R^{dc} may bond with a nitrogen atom to form a cyclic amino group and R^{dd} is a C₁₋₆ alkyl group or a C₃₋₈ cycloalkyl group), each R^{d1} may be the same or different from each other when pd is two or more, and each R^{d2}, may be the same or different from each other when qd is two or more, or
the formula: 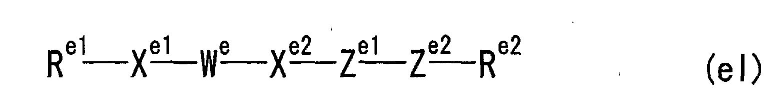 wherein R^{e1} represents a 5 to 6-membered cyclic ring group which may be substituted, X ^{e1} represents a bond or a bivalent group, in which the number of atoms constituting the straight-chain portion is 1 to 4, W^{e} represents a bivalent group represented by formula: 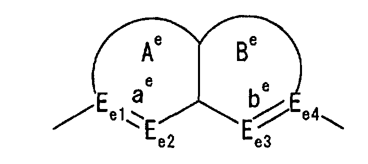 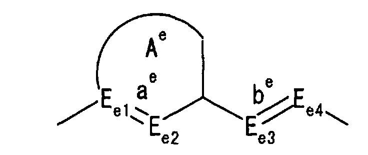 or 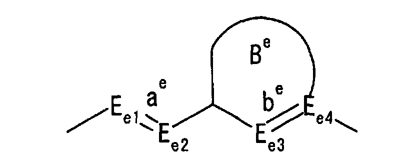 wherein each of ring A^{e} and ring B^{e} represents a 5- to 7-membered cyclic group which may be substituted, each of Ee₁ and Ee₄ is a carbon atom which may be substituted or a nitrogen atom which may be substituted, each of Ee₂ and Ee₃ is a carbon atom which may be substituted, a nitrogen atom which may be substituted, or a sulfur atom which may be oxidized or an oxygen atom, each of a^{e} and b^{e} is a single bond or a double bond), X^{e2} is a bivalent group in which the number of atoms constituting the straight-chain portion is 1 to 4, Z^{e1} is a bond or a bivalent cyclic ring group, Z^{e2} is a bond or a bivalent cyclic ring group in which the number of atoms constituting the straight-chain portion is 1 to 4, and R^{e2} is (1) an amino group which may be substituted, and the nitrogen atom may be converted into a quaternary ammonium or an N-oxide, (2) a nitrogen-containing heterocyclic ring group which may be substituted, may contain sulfur atom or an oxygen atom as a ring-constituting atom, and the nitrogen atom may be converted into a quaternary ammonium or a N-oxide, (3) a group which is bonded via the sulfur atom, (4) a group represented by formula: 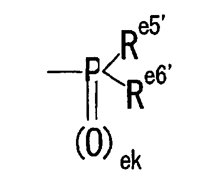 wherein ek is 0 or 1, the phosphorus atom may form a phosphonium salt when ek is 0, and each of R^{e5'} and R^{e6'} is a hydrocarbon atom which may be substituted, a hydroxyl group which may be substituted, or an amino group which may be substituted, and R^{e5'} and R^{e6'} may bond to each other to form a cyclic ring group together with the adjacent phosphorus atom, (5) an amidino group which may be substituted or (6) a guanidino group which may be substituted, or a salt thereof.

3. The agent for the prevention or treatment according to claim 1 or 2, wherein the compound having a CCR antagonistic effect or a salt thereof is N-(3,4-dichlorophenyl)-1-(methylsulfonyl)-N-{3-[4-({4-[(methylsulfonyl)-amino]phenyl}sulfonyl)-1-piperidinyl]propyl}-4-piperidinecarboxamide, N-(3-chlorophenyl)-1-(methylsulfonyl)-N-(3-{4-[4-(methylsulfonyl)benzyl]-1-piperidinyl}propyl)-4-piperidinecarboxamide, N-(3-{4-[4-(aminocarbonyl)benzyl]-1-piperidinyl}propyl)-N-(3,4-dichlorophenyl)-1-(methylsulfonyl)-4-piperidinecarboxamide, 1-acetyl-N-(3-{4-[4-(aminocarbonyl)benzyl]-1-piperidinyl}propyl)-N-(3-chloro-4-methylphenyl)-4-piperidinecarboxamide, N-(3,4-dichlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-1-(methylsulfonyl)-N-(3-{4-[4-(methylsulfonyl)benzyl]-1-piperidinyl}propyl)-4-piperidinecarboxamide, N-(3-{4-[4-(aminocarbonyl)benzyl]-1-piperidinyl}propyl)-N-(3-chloro-4-methylphenyl)-1-(methylsulfonyl)-4-piperidinecarboxamide, N-[3-(4-benzyl-1-piperidinyl)propyl]-N'-(4-chlorophenyl)-N-phenylurea, N'-(4-chlorophenyl)-N-{3-[4-(4-fluorobenzyl)-1-piperidinyl]propyl}-N-phenylurea, N'-(4-chlorophenyl)-N-(3-{4-[4-(4-morpholinylsulfonyl)benzyl]-1-piperidinyl}propyl)-N-phenylurea, N'-(4-chlorophenyl)-N-(3-{4-[4-(4-methylsulfonyl)benzyl]-1-piperidinyl}propyl)-N-phenylurea, 4-{[1-(3-{[(9-chloroanilino)carbonyl]anilino}propyl)-4-piperidinyl]methyl}benzamide, N-[3-(4-benzyl-1-piperidinyl)propyl]-N-(3,4-dichlorophenyl)-1-methyl-5-oxo-3-pyrrolidinecarboxamide, 1-benzyl-N-[3-(4-benzyl-1-piperidinyl)propyl]-5-oxo-N-phenyl-3-pyrrolidinecarboxamide, N-[3-(4-benzyl-1-piperidinyl)propyl]-1-(2-chlorobenzyl)-5-oxo-N-phenyl-3-pyrrolidinecarboxamide, N-(3,4-dichlorophenyl)-N-{3-[4-(4-fluorobenzyl)-1-piperidinyl]propyl}-1-methyl-5-oxo-3-pyrrolidinecarboxamide, N-[3-(4-benzyl-1-piperidinyl)propyl]-5-oxo-N-phenyl-1-(2,2,2-trifluoroethyl)-3-pyrrolidinecarboxamide, N-(3,4-dichlorophenyl)-N-(3-{4-[4-(methylsulfonyl)benzyl]-1-piperidinyl}propyl)-2-[1-(methylsulfonyl)-4-piperidinyl]acetamide, N-(3,4-dichlorophenyl)-N-(3-{4-[4-(isopropylsulfonyl)benzyl]-1-piperidinyl}propyl)-2-[1-(methylsulfonyl)-4-piperidinyl]acetamide, 3-(1-acetyl-4-piperidinyl)-N-(3,4-dichlorophenyl)-N-(3-{4-[4-(methylsulfonyl)benzyl]-1-piperidinyl}propyl)propanamide, or N-(3,4-dichlorophenyl)-4-hydroxy-1-(methylsulfonyl)-N-(3-{4-[4-(methylsulfonyl)benzyl]-1-piperidinyl}propyl)-4-piperidinecarboxamide or a salt thereof.

4. The prevention or treatment agent according to claim 1 or 2, wherein the compound having a CCR antagonistic effect or a salt thereof is N-methyl-N-[4-[[[2-(4-methylphenyl)-6,7-dihydro-5H-benzocyclohepten-8-yl]carbonyl]amino]benzyl]piperidinium iodide, N-methyl-N-[4-[[[7-(4-methylphenyl)-2,3-dihydro-1-benzoxepin-4-yl]carbonyl]amino]benzyl]piperidinium iodide, N-[4-[N-methyl-N-(tetrahydropyran-4-yl)aminomethyl]phenyl]-7-(4-methylphenyl)-2,3-dihydro-1-benzoxepin-4-carboxamide, N-[4-[N-methyl-N-(tetrahydropyran-4-yl)aminomethyl]phenyl]-7-(4-morpholinophenyl)-2,3-dihydro-1-benzoxepin-4-carboxamide, 7-(4-ethoxyphenyl)-N-[4-[N-methyl-N-(tetrahydropyran-4-yl)aminomethyl]phenyl]-2,3-dihydro-1-benzoxepin-4-carboxamide, N,N-dimethyl-N-[4-[[[2-(4-methylphenyl)-6,7-dihydro-5H-benzocyclohepten-8-yl]carbonyl]amino]benzyl]-N-(tetrahydropyran-4-yl) ammonium iodide, N-methyl-N-[4-[[[7-(4-methylphenyl)-3,4-dihydronaphthalen-2-yl]carbonyl]amino]benzyl]piperidinium iodide, N,N-dimethyl-N-(4-(((2-(4-methylphenyl)-6,7-dihydro-5H-benzocyclohepten-8-yl)carbonyl)amino)benzyl)-N-(4-tetrahydropyranyl)ammonium chloride, N,N-dimethyl-N-(((7-(4-methylphenyl)-2,3-dihydro-1-benzoxepin-4-yl)carbonyl)amino)benzyl)-N-(4-oxocyclohexyl)ammonium chloride, N-(4-(((7-(4-ethoxyphenyl)-2,3-dihydro-1-benzoxepin-4-yl)carbonyl)amino)benzyl)-N,N-dimethyl-N-(4-tetrahydropyranyl)ammonium chloride, N-[4-[N-methyl-N-(tetrahydropyran-4-yl)aminomethyl]phenyl]-7-(4-propoxyphenyl)-1,1-dioxo-2,3-dihydro-1-benzothiepin-4-carboxamide, 7-(4-butoxyphenyl)-N-[4-[N-methyl-N-(tetrahydropyran-4-yl)aminomethyl]phenyl]-1,1-dioxo-2,3-dihydro-1-benzothiepin-4-carboxamide, 7-[4-[N-methyl-N-(2-propoxyethyl)amino]phenyl]-N-[4-[[N-methyl-N-(tetrahydropyran-4-yl)amino]methyl]phenyl]-1,1-dioxo-2,3-dihydro-1-benzothiepin-4-carboxamide, 7-[4-(2-ethoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, N-[4-[[N-methyl-N-(tetrahydropyran-4-yl)amino]methyl]phenyl]-7-[4-(2-propoxyethoxy)phenyl]-1,1-dioxo-2,3-dihydro-1-benzothiepin-4-carboxamide, 7-[4-(2-butoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, 7-[4-(2-ethoxyethoxy)-3,5-dimethylphenyl]-N-[4-[[N-methyl-N-(tetrahydro-2H-pyran-4-yl)amino]methyl]phenyl]-1,1-dioxo-2,3-dihydro-1-benzothiepin-4-carboxamide, 7-[2-chloro-4-(2-propoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, 7-(3-methyl-4-propoxyphenyl)-N-[9-[[N-methyl-N-(tetrahydropyran-9-yl)amino]methyl]phenyl]-1,1-dioxo-2,3-dihydro-1-benzothiepin-9-carboxamide, 7-(3,4-dipropoxyphenyl)-N-(4-((N-methyl-N-(tetrahydro-2H-pyran-4-yl)amino)methyl)phenyl)-1,1-dioxo-2,3-dihydro-1-benzothiepin-4-carboxamide, 7-[4-(2-ethoxyethoxy)phenyl]-1-ethyl-N-[4-[[N-methyl-N-(tetrahydropyran-4-yl)amino]methyl]phenyl]-2,3-dihydro-1-benzoazepin-4-carboxamide, 1-ethyl-7-[4-(2-propoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-ethyl-N-[4-[[N-methyl-N-(tetrahydropyran-9-yl)amino]methyl]phenyl]-2,3-dihydro-1-benzoazepin-4-carboxamide, 7-[4-(2-ethoxyethoxy)phenyl]-1-formyl-N-[4-[[N-methyl-N-(tetrahydropyran-4-yl)amino]methyl]phenyl]-2,3-dihydro-1-benzoazepin-4-carboxamide, 1-formyl-7-[4-(2-propoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-formyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-5-yl)amino]methyl]phenyl]-1-propyl-2,3-dihydro-1-benzoazepin-4-carboxamide, N-[4-[[N-methyl-N-(tetrahydropyran-5-yl)amino]methyl]phenyl]-7-[4-(2-propoxyethoxy)phenyl]-1-propyl-2,3-dihydro-1-benzoazepin-4-carboxamide, 1-benzyl-7-[4-(2-butoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-cyclopropylmethyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-phenyl-2,3-dihydro-1-benzoazepin-4-carboxamide, 7-[4-(2-butoxyethoxy)phenyl]-1-(3,4-methylenedioxy)phenyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(2-methyloxazol-5-yl)-N-[4-[[N-methyl-N-(tetrahydropyran-4-yl)amino]methyl]phenyl]-2,3-dihydro-1-benzoazepin-4-carboxamide, 1-allyl-7-[4-(2-butoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(3-thienyl)methyl-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(thiazol-2-yl)methyl-2,3-dihydro-1-benzoazepin-4-carboxamide, 7-[4-(2-butoxyethoxy)phenyl]-1-(1-methylpyrazol-4-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(3-methylisothiazol-5-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(1-ethylpyrazol-4-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-isobutyl-N-[4-[[N-methyl-N-(tetrahydropyran-5-yl)amino]methyl]phenyl]-2,3-dihydro-1-benzoazepin-4-carboxamide, 1-isobutyl-N-[4-[[N-methyl-N-(tetrahydropyran-5-yl)amino]methyl]phenyl]- 7-[4-(2-propoxyethoxy)phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-2-(thiazol-5-yl)methyl-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(1-methyltetrazol-5-yl)methyl-2,3-dihydro-1-benzoazepin-4-carboxamide, or 7-[4-(2-butoxyethoxy)phenyl]-N-[4-[[N-methyl-N-(tetrahydropyran-4-yl)amino]methyl]phenyl]-1-(2-methyltetrazol-5-yl)methyl-2,3-dihydro-1-benzoazepin-4-carboxamide or a salt thereof.

5. A method of preventing or treating graft-versus host disease and/or rejection reactions during organ or bone marrow transplantation, which comprises the step of administering an effective amount of a compound having a CCR antagonist effect to a mammal.

6. A method of preventing or treating chronic rheumatoid arthritis, autoimmune diseases, allergic disorders, ischemic brain cell damage, myocardial infarction, chronic nephritis, and arteriosclerosis, which comprises the step of administering an effective amount of a compound having a CCR antagonist effect to a mammal.

7. Use of a compound having a CCR antagonist effect for manufacturing an agent for the prevention or treatment of graft-versus host disease and/or rejection reactions during organ or bone marrow transplantation.

8. Use of a compound having a CCR antagonist effect for manufacturing an agent for the prevention or treatment of chronic rheumatoid arthritis, autoimmune diseases, allergic disorders, ischemic brain cell damage, myocardial infarction, chronic nephritis, and arteriosclerosis.
